# EUROPEAN PATENT APPLICATION

(11) **EP 1 593 679 A1**
(43) Date of publication of application: **09.11.2005**
(21) Application number: 04291187.5
(22) Date of filing: 07.05.2004
(51) Int. Cl.: C07D 309/06, C07D 405/12, C07D 309/08, C07D 295/12, C07D 405/06, C07D 405/14, C07D 295/14, C07D 417/04, C07D 417/06, A61K 31/55, A61P 25/06

(54) **3- Or 4-monosubstituted phenol derivatives useful as H3 ligands**

(71) Applicant: Warner-Lambert Company LLC, Morris Plains, New Jersey 07950 (US)
(72) Inventor: Bernardelli, Patrick, 92260 Fontenay aux Roses (FR); Denis, Alexis, 75011 Paris (FR); Lorthiois, Edwige c/o Pfizer Limited, CT13 9NJ Sandwich, Kent (GB); Jacobelli, Henry, 91550 Paray Vieille Poste (FR); Vergne, Fabrice, 91190 Gif Sur Yvette (FR); Serradeil, Dephine, 92230 Cachan (FR); Rousseau, Fiona, Sandwich Kent CT13 9NJ (GB); Cronin, Andrew, Sandwich Kent CT13 9NJ (GB); Kemp, Mark, Sandwich Kent CT13 9NJ (GB)
(74) Representative: Dekker, Henrike Cornelie Christine

(57) **Abstract**

The invention relates to 3- or 4-monosubstituted phenol derivatives and to processes for the preparation of, intermediates used in the preparation of, compositions containing and the uses of, such derivatives. Said 3- or 4-monosubstituted phenol derivatives are H₃ ligands and are useful in numerous diseases, disorders and conditions, in particular inflammatory, allergic and respiratory diseases, disorders and conditions.

## Description

The present invention relates to 3- or 4-monosubstituted phenol derivatives of general formula : 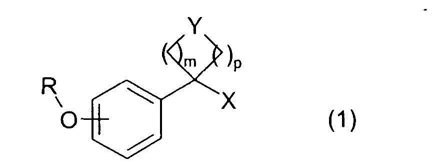 in which R, X, Y, m and p have the meanings indicated below,
and to processes for the preparation of, intermediates used in the preparation of, compositions containing and the uses of, such derivatives.

Histamine H₃ receptors are found inter alia on presynaptic terminals of peripheral nerves, where they modulate autonomic neurotransmission and modulate a variety of end organ responses under control of the autonomic nervous system. They are also heteroreceptors, modulating the release of numerous other neurotransmitters such as dopamine, glutamate, noradrenaline, serotonin, GABA, acetylcholine, some peptides and co-transmitters.

Recently numerous histamine H₃ receptor ligands have been developed. An overview of the current advance in H₃ ligand research and patenting is given in *Expert Opin. Ther. Patents* (2003) **13**(6). Examples of Histamine H₃ receptor ligands can be found in WO02/76925, WO00/06254, WO02/12190, WO02/12214 and WO02/06223.

H₃ receptor ligands are believed to be suitable for the treatment of various diseases including both disorders of the central nervous system and inflammatory disorders. Examples of diseases where treatment with H₃ ligands is believed to be useful are inflammatory bowel disease, Crohn's disease, colitis ulcerosa, sleep disorders, migraine, dyskinesia, stress-induced anxiety, psychotic disorders, epilepsy, Cognition deficiency diseases such as Alzheimer's disease or mild coginitive impairment, depression, mood disorders, schizophrenia, anxiety disorders, attention-deficit hyperactivity disorder (ADHD), psychotic disorders, obesity, dizziness, epilepsy, motion sickness, vertigo, respiratory diseases such as adult respiratory distress syndrome, acute respiratory distress syndrome, bronchitis, chronic bronchitis, chronic obstructive pulmonary disease, cystic fibrosis, asthma, emphysema, rhinitis, chronic sinusitis, allergy, allergy-induced airway responses, allergic rhinitis, viral rhinitis, non-allergic rhinitis, perennial and seasonal rhinitis, nasal congestion and allergic congestion.

Although H₃ ligands are known there is still a need to provide new H₃ ligands that are good drug candidates. In particular, preferred compounds should bind potently to the histamine H₃ receptor whilst showing little affinity for other receptors. They should be well absorbed from the gastrointestinal tract, be metabolically stable and possess favourable pharmacokinetic properties. They should be non-toxic and demonstrate few side-effects.

In this context, the present invention concerns new substituted phenol derivatives of general formula (1) : 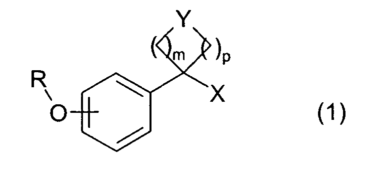 and pharmaceutically acceptable salts thereof wherein :
the substituent of formula -O-R is in the meta or para position of the phenyl group;
X is selected from -CN, -CH₂OH, -CH₂-O-(C₁-C₄)alkyl, -C(O)OH, -C(O)O(C₁-C₄)alkyl, -CH₂-NR¹R², -C(O)NR³R⁴ and het¹, het¹ being optionally substituted by one or two substituents independently selected from halo, cyano, (C₁-C₄)alkyl and (C₁-C₄)alkoxy;
R¹ is hydrogen or (C₁-C₄)alkyl;
R² is selected from the group consisting of :
   hydrogen,
   (C₁-C₄)alkyl optionally substituted by one or two substituents independently selected from (C₃-C₆)cycloalkyl, hydroxy, -S-(C₁-C₄)alkyl, -O-(C₁-C₄)alkyl, -SO₂-(C₁-C₄)alkyl, halo and phenyl, said phenyl being optionally substituted by one or two substituents independently selected from halo, cyano, (C₁-C₄)alkyl and (C₁-C₄)alkoxy,
   (C₃-C₆)cycloalkyl,
   het², optionally substituted by one or two substituents independently selected from halo, cyano, (C₁-C₄)alkyl and (C₁-C₄)alkoxy,
   -SO₂-R⁵ wherein R⁵ is selected from the group consisting of (C₁-C₄)alkyl, amino, (C₁-C₄)alkylamino, [(C₁-C₄)alkyl]₂amino, phenyl and -(C₁-C₄)alkyl-phenyl, said phenyl being optionally substituted by one or two substituents independently selected from halo, cyano, (C₁-C₄)alkyl and (C₁-C₄)alkoxy, and
   -C(O)-R⁶ wherein R⁶ is selected from the group consisting of (C₁-C₄)alkyl, amino, (C₁-C₄)alkylamino, [(C₁-C₄)alkyl]₂amino, phenyl and -(C₁-C₄)alkyl-phenyl, said phenyl being optionaly substituted by one or two substituents independently selected from halo, cyano, (C₁-C₄)alkyl and (C₁-C₄)alkoxy;
or R¹ and R² form together with the N atom to which they are attached a 4-, 5- or 6-membered saturated heterocycle wherein one C atom may be replaced by N, O, S, SO or SO₂ and wherein said saturated heterocycle is optionally substituted by one or two groups independently selected from hydroxy, halo, =O, (C₁-C₄)alkyl, (C₁-C₄)alkoxy, hydroxy(C₁-C₄)alkyl, (C₁-C₄)alkoxy(C₁-C₄)alkyl, -C(O)NH₂, C(O)O(C₁-C₄)alkyl and pyrrolidinone;
R³ and R⁴ are each independently selected from hydrogen and (C₁-C₄)alkyl or R³ and R⁴ form together with the N atom to which they are attached a 4-, 5- or 6-membered saturated heterocycle wherein one C atom may be replaced by N or O;
Y is selected from CH₂, CH(OH), O and C=O;
m and p are both integers which are independently 1, 2 or 3, with the condition that m+p is equal to or less than 4 so that the ring formed by : 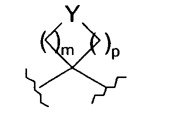 is a 4-, 5- or 6-membered ring;
and R is either a group of formula : 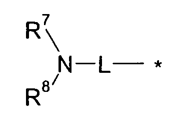 wherein * represents the attachment point to the O atom, L is a (C₂-C₆)alkylene and R⁷ and R⁸ are each independently selected from hydrogen, (C₁-C₆)alkyl, hydroxy(C₁-C₆)alkyl or R⁷ and R⁸ form together with the N atom to which they are attached a 4-, 5-, 6- or 7-membered saturated heterocycle wherein one C atom is optionally replaced by N, O, S, SO or SO₂ and wherein said saturated heterocycle is optionally substituted by one or two groups independently selected from (C₁-C₄)alkyl, (C₁-C₄)alkoxy, (C₁-C₄)alkoxy(C₁-C₄)alkyl, hydroxy(C₁-C₄)alkyl, hydroxy, C(O)O(C₁-C₄)alkyl, -C(O)-(C₁-C₄)alkyl-NH₂, -C(O)NH₂, halo, amino, (C₁-C₄)alkylamino and [(C₁-C₄)alkyl]₂amino,
   or a group of formula: 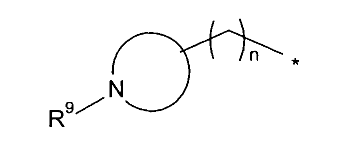 wherein * represents the attachment point to the O atom, the N-containing ring is a 4- to 7-membered saturated heterocycle, n is an integer equal to 0, 1 or 2, and R⁹ represents a substituent selected from hydrogen, (C₁-C₄)alkyl, hydroxy(C₁-C₆ alkyl) and (C₃-C₆)cycloalkyl;
het¹ is selected from monocyclic or bicyclic heteroaromatic groups having 5 to 10 ring members, which contain 1, 2, 3 or 4 heteroatom(s) selected from nitrogen, oxygen and sulphur and het² is selected from monocyclic or bicyclic heteroaromatic groups having 5 to 10 ring members, which contain 1, 2, 3 or 4 heteroatom(s) selected from nitrogen, oxygen and sulphur.

It has been found that these compounds are H3 ligands and are thus particularly useful for the treatment of H3-related diseases such as neurologic disorders, or inflammatory, respiratory and allergic diseases, disorders and conditions.

In the present description the following definitions are used, unless otherwise specified.
"halo" denotes a halogen atom selected from the group consisting of fluoro, chloro, bromo and iodo.
"(C₁-Cₓ)alkyl" denotes a saturated, straight-chain or branched hydrocarbon group having from 1 to x carbon atoms and includes for example (when x= 4) methyl, ethyl, propyl, i-propyl, n-butyl, i-butyl, sec-butyl and t-butyl and further (when x=6) pentyl, I-pentyl, n-pentyl and hexyl. This also applies if the alkyl group carries substituents or is a substituent for another group, e.g. in -S-(C₁-C₄)alkyl, -O-(C₁-C₄)alkyl, -SO₂-(C₁-C₄)alkyl.
"(C₁-C₄)alkoxy" denotes straight-chain and branched alkoxy groups and includes for example methoxy, ethoxy, n-propoxy, i-propoxy, n-butoxy, i-butoxy, sec-butoxy and t-butoxy.
"(C₂-C₆)alkylene" denotes a divalent radical derived from straight-chain or branched alkane containing from 2 to 6 carbon atoms. Examples of (C₂-C₆)alkylene radicals are methylene, ethylene (1,2-ethylene or 1,1-ethylene), trimethylene (1,3-propylene), tetramethylene (1,4-butylene), pentamethylene and hexamethylene.
"(C₃-C₆)cycloalkyl" denotes a saturated monocyclic carbocyclic group having 3 to 6 carbon atoms and includes for example cyclopropyl, cyclobutyl, cyclopentyl and cyclohexyl.
"saturated heterocycle" denotes a saturated monocyclic group having 4 to 7 ring members, which contains 1 nitrogen atom and optionally a further heteroatom selected from nitrogen, oxygen and sulphur. Examples of saturated heterocycles are azetidinyl, pyrrolidinyl, piperidinyl, morpholinyl, piperazinyl, and azepanyl.
het¹ and het² are monocyclic or bicyclic heteroaromatic groups having 5 to 10 ring members, which contain 1, 2, 3 or 4 heteroatom(s) selected from nitrogen, oxygen and sulphur. In particular the heteroaromatic group contains either (a) 1 to 4 nitrogen atoms, (b) one oxygen atom or one sulfur atom or (c) 1 oxygen atom or 1 sulfur atom and 1 or 2 nitrogen atoms. The heteroaryl group is preferably C-linked, which means that the group is linked to the adjacent atom by a ring carbon atom. The heteroaryl group can be unsubstituted, monosubstituted or disubstituted, as indicated in the definition of X and R² hereabove for general formula (1) according to the present invention. Examples of heteroaryl groups include, but are not limited to thiophenyl, furanyl, pyrrolyl, pyrazolyl, imidazolyl, oxazolyl, isoxazolyl, thiazolyl, isothiazolyl, triazolyl, oxadiazolyl, thiadiazolyl, tetrazolyl, pyranyl, pyridinyl, pyrazinyl, pyrimidinyl, pyridazinyl, triazinyl, thiadiazinyl, isobenzofuranyl, benzofuranyl, chromenyl, indolizinyl, isoindolyl, indolyl, indazolyl, purinyl, quinolinyl, isoquinolyl, cinnolinyl, phthalazinyl, naphthyridinyl, quinazolinyl, quinoxalinyl, benzoxazolyl, benzothiazolyl, benzimidazolyl, benzofuranyl and benzothiophenyl. Preferred definitions for het¹ and het² will follow hereafter.

In the general formula (1) according to the present invention, when a radical is mono- or poly-substituted, said substituent(s) can be located at any desired position(s), unless otherwise stated. Also, when a radical is polysubstituted, said substituents can be identical or different, unless otherwise stated.

According to a preferred aspect of the invention X is selected from -CN, -CH₂OH, -C(O)OH, -CH₂-NR¹R², -C(O)NR³R⁴ and het¹, more preferably from -CN, -CH₂-NR¹R², -CONR³R⁴ and het¹, het¹ being optionally substituted by one or two substituents independently selected from halo, cyano, (C₁-C₄)alkyl and (C₁-C₄)alkoxy, wherein R¹, R², R³, R⁴ and het¹ are as previously defined.

het¹ is preferably selected from 5 or 6 membered monocyclic heteroaromatic groups containing 1 to 2 nitrogen atoms or 1 nitrogen atom and 1 oxygen atom or 1 nitrogen atom and 1 sulphur atom, optionally substituted by one or two substituents independently selected from halo, cyano, (C₁-C₄)alkyl and (C₁-C₄)alkoxy, preferably (C₁-C₄)alkyl.

R¹ is preferably methyl or ethyl.

R² is preferably selected from the group consisting of
hydrogen,
(C₁-C₄)alkyl optionally substituted by one or two substituents independently selected from -S-(C₁-C₄)alkyl, -O-(C₁-C₄)alkyl, -SO₂-(C₁-C₄)alkyl, and phenyl, said phenyl being optionally substituted by one or two substituents independently selected from halo, cyano, (C₁-C₄)alkyl and (C₁-C₄)alkoxy,
(C₃-C₆)cycloalkyl,
het² wherein het² is defined as in claim 1,
-SO₂-R⁵ wherein R⁵ is selected from the group consisting of (C₁-C₄)alkyl, [(C₁-C₄)alkyl]₂amino, phenyl, and -(C₁-C₄)alkyl-phenyl, wherein said phenyl is optionally substituted by 1 substituent independently selected from halo and cyano and
-C(O)-R⁶ wherein R⁶ is selected from the group consisting of (C₁-C₄)alkyl, ((C₁-C₄)alkyl]₂amino, amino, and -(C₁-C₄)alkyl-phenyl, said phenyl being optionally substituted by one or two substituents independently selected from halo, cyano, (C₁-C₄)alkyl and (C₁-C₄)alkoxy.

More preferably R² is selected from the group consisting of
(C₁-C₃)alkyl optionally substituted by -O-(C₁-C₃)alkyl, preferably methoxy, (C₃-C₅)cycloalkyl,
het², wherein het² is selected from the group consisting of 5 or 6 membered monocyclic heteroaromatic ring systems containing 1 to 2 nitrogen atoms or 1 nitrogen atom and 1 oxygen atom or 1 nitrogen atom and 1 sulphur atom, optionally substituted by one or two substituents independently selected from halo, cyano, (C₁-C₄)alkyl and (C₁-C₄)alkoxy, preferably (C₁-C₄)alkyl,
SO₂-R⁵ wherein R⁵ is preferably (C₁-C₄)alkyl and
C(O)-R⁶ wherein R⁶ is preferably (C₁-C₄)alkyl.

het² is preferably selected from the group consisting of 5 or 6 membered monocyclic heteroaromatic ring systems containing 1 or 2 nitrogen atoms.

According to a further preferred aspect of the invention R¹ and R² form together with the N atom to which they are attached a 5- or 6-membered saturated heterocycle wherein a C atom may be replaced by N, O, S, SO or SO₂ and wherein said saturated heterocycle is optionally substituted by one or two groups independently selected from hydroxy, halo, =0 (carbonyl), (C₁-C₄)alkyl, (C₁-C₄)alkoxy, hydroxy(C₁-C₄)alkyl, (C₁-C₄)alkoxy(C₁-C₄)alkyl, -CONH₂, C(O)O(C₁-C₄)alkyl and pyrrolidinone, more preferably R¹ and R² form together with the N atom to which they are attached a 5- or 6-membered saturated heterocycle selected from 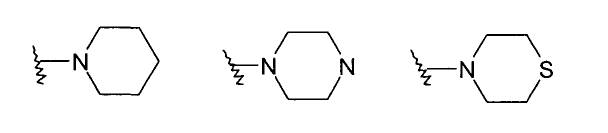 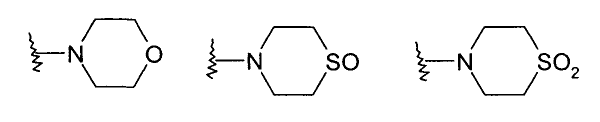 or 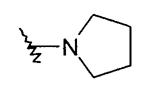 optionally substituted by one or two groups independently selected from hydroxy, fluoro, hydroxymethyl, methoxymethyl, -CONH₂, and pyrrolidinone.

Preferably R³ and R⁴ are each independently selected from hydrogen and (C₁-C₄)alkyl or R³ and R⁴ form together with the N atom to which they are attached a 5 or 6 membered saturated heterocycle, more preferably a 5 membered saturated heterocyle.

Preferably, Y is selected from CH₂, CH(OH), O and C=O, more preferably Y is O.

Preferably m is equal to 1 or 2 and p is equal to 2, more preferably m and p are both 2.

In one preferred aspect of the invention R is a group of formula : 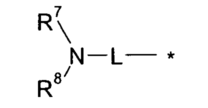 wherein * represents the attachment point to the O atom, L is a (C₂-C₅)alkylene and R⁷ and R⁸ are each independently selected from hydrogen, (C₁-C₆)alkyl, hydroxy(C₁-C₆)alkyl or R⁷ and R⁸ form together with the N atom to which they are attached a 5-, 6- or 7-membered saturated heterocycle wherein one C atom is optionally replaced by N, O, S, SO or SO₂ and wherein said saturated heterocycle is optionally substituted by one or two groups independently selected from (C₁-C₄)alkyl, (C₁-C₄)alkoxy, (C₁-C₄)alkoxy(C₁-C₄)alkyl, hydroxy(C₁-C₄)alkyl, hydroxy, C(O)O(C₁-C₄)alkyl, -C(O)-(C₁-C₄)alkyl-NH₂, -C(O)NH₂ and halo. Examples of the 5-, 6- or 7-membered saturated heterocycle are :

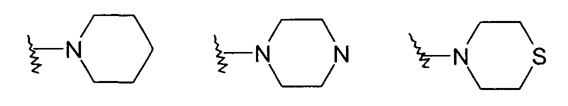

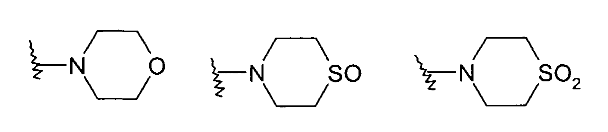

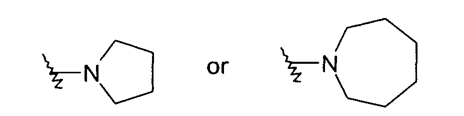

More preferably R⁷ and R⁸ form together with the N atom to which they are attached a 5 or 6 membered saturated heterocycle, optionally substituted by one or two (C₁-C₄)alkyl, preferably methyl.

According to another preferred aspect of the invention R is a group of formula: 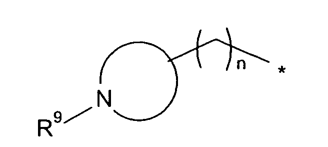 wherein * represents the attachment point to the O atom, the N-containing ring is a 6-membered saturated heterocycle, n is an integer equal to 0, and R⁹ represents a substituent selected from hydrogen, (C₁-C₄)alkyl and (C₃-C₆)cycloalkyl.

When one of the groups in the compound of formula (1) is substituted by halo, generally fluoro or chloro, in particular fluoro is preferred.

Particularly preferred compounds of the invention include those in which each variable in formula (1) is selected from the suitable and/or preferred groups for each variable. Even more preferable compounds of the invention include those where each variable in formula (1) is selected from the more preferred or most preferred groups for each variable.

According to a particular aspect of the invention the following compounds are excluded from the invention: compounds of formula (1), wherein Y is O, R is a group of formula : 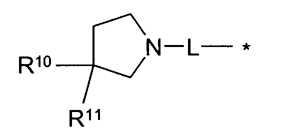 wherein R¹⁰ is hydrogen or (C₁-C₄)alkyl, R¹¹ is (C₁-C₄)alkoxy, hydroxy or N((C₁-C₄)alkyl)₂ and X is -CN, -CH₂OH, -CH₂-O-(C₁-C₄)alkyl, -C(O)OH, -C(O)O(C₁-C₄)alkyl, -CONR³R⁴ or CH₂NR¹R² wherein R¹ is hydrogen or a (C₁-C₄)alkyl group and R² is either hydrogen, (C₁-C₄)alkyl (optionally substituted by phenyl) or - C(O)(C₁-C₄)alkyl.

Particularly preferred examples of the phenol derivatives of formula (1) are as described in the Examples section hereafter, i.e. :
Dimethyl-{4-[3-(3-pyrrolidin-1-ylpropoxy)phenyl]tetrahydro-pyran-4-ylmethyl}amine
(4-{4-[3-(2,5-Dimethylpyrrolidin-1-yl)propoxy]phenyl}tetra-hydro-pyran-4-ylmethyl)dimethylamine
Dimethyl-(4-{4-[3-(2-methyl-pyrrolidin-1-yl)-propoxy]-phenyl}-tetrahydro-pyran-4-ylmethyl)-amine
(4-{4-[3-(2,6-Dimethylpiperidin-1-yl)propoxy]phenyl}tetra-hydro-pyran-4-ylmethyl)dimethyl-amine
Dimethyl-{4-[4-(3-thiomorpholin-4-ylpropoxy)phenyl]tetra-hydro-pyran-4-ylmethyl}amine
Dimethyl-(4-{4-[3-(1-oxothiomorpholin-4-yl)propoxy]phenyl}-tetrahydropyran-4-ylmethyl)amine
(4-{4-[3-(1,1-Dioxo-thiomorpholin-4-yl)propoxy]phenyl}tetra-hydro-pyran-4-ylmethyl)dimethyl-amine
1-{3-[4-(4-Dimethylaminomethyl-tetrahydro-pyran-4-yl)-phenoxy]-propyl}-piperidin-4-ol
(4-{4-[3-(4-Methoxy-piperidin-1-yl)-propoxy]-phenyl}-tetrahydro-pyran-4-ylmethyl)-dimethyl-amine
2-(1-{3-[4-(4-Dimethylaminomethyltetrahydropyran-4-yl)phenoxy]propyl}piperidin-4-yl)ethanol
[4-(4-{3-[4-(2-Methoxy-ethyl)-piperidin-1-yl]-propoxy}-phenyl)-tetrahydro-pyran-4-ylmethyl]-dimethyl-amine
1-{3-[4-(4-Dimethylaminomethyl-tetrahydro-pyran-4-yl)-phenoxy]-propyl}-piperidine-4-carboxylic acid amide
{3-[4-(4-Dimethylaminomethyltetrahydropyran-4-yl)phenoxy]propyl}piperazine-1-carboxylic acid ethyl ester
{3-[4-(4-Dimethylaminomethyltetrahydropyran-4-yl)phenoxy]-propyl}diethyl-amine
2-({3-[4-(4-Dimethylaminomethyltetrahydropyran-4-yl)phenoxy]propyl}ethylamino) ethanol
{3-[4-(4-Dimethylaminomethyltetrahydropyran-4-yl)phenoxy]-propyl} isopropylmethylamine
Dimethyl-{4-[4-(2-pyrrolidin-1-ylethoxy)phenyl]tetrahydro-pyran-4-ylmethyl}amine
Dimethyl-{4-[4-(2-methyl-3-pyrrolidin-1-yl-propoxy)-phenyl]-tetrahydro-pyran-4-ylmethyl}-amine
{4-[4-(1-Isopropylpiperidin-4-yloxy)phenyl]tetrahydropyran-4-ylmethyl}dimethylamine
{4-[4-(1-Cyclopentylpiperidin-4-yloxy)phenyl]tetrahydropyran-4-ylmethyl}dimethylamine
{3-[4-(4-Dimethylaminomethyl-tetrahydro-pyran-4-yl)-phenoxy]-propyl}-isopropylamine
Dimethyl-{4-[4-(4-pyrrolidin-1-ylbutoxy)phenyl]tetrahydro-pyran-4-ylmethyl}amine
{4-[4-(2,2-Dimethyl-3-pyrrolidin-1-yl-propoxy)-phenyl]-tetrahydro-pyran-4-ylmethyl}-dimethyl-amine
Dimethyl-{4-[4-(1-methyl-3-pyrrolidin-1-ylpropoxy)phenyl]-tetrahydropyran-4-ylmethyl}amine
Dimethyl-{4-[4-(3-pyrrolidin-1-yl-butoxy)-phenyl]-tetrahydro-pyran-4-ylmethyl}-amine
Dimethyl-(4-{4-[2-(1-methylpyrrolidin-2-yl)ethoxy]phenyl}-tetrahydropyran-4-ylmethyl)amine
4-[4-(3-Pyrrolidin-1-yl-propoxy)-phenyl]-tetrahydro-pyran-4-carbonitrile
{4-[4-(3-Pyrrolidin-1-ylpropoxy)phenyl]tetrahydropyran-4-yl} methylamine
Dimethyl-{4-[4-(3-pyrrolidin-1-ylpropoxy)phenyl]-tetrahydropyran-4-ylmethyl}amine
4-[4-(3-Piperid i n-1-yl-propoxy)-phenyl]-tetra hyd ro-pyran-4-carbonitri le
{4-[4-(3-Piperidin-1-ylpropoxy)phenyl]tetrahydropyran-4-yl} methylamine
Dimethyl-{4-[4-(3-piperidin-1-ylpropoxy)phenyl]tetra-hydropyran-4-ylmethyl}amine
1-[4-(3-pyrrolidin-1-ylpropoxy)phenyl]cyclohexanecarbonitrile
N-({1-[4-(3-pyrrolidin-1-ylpropoxy)phenyl]cyclohexyl}methyl)-N,N-dimethylamine
(1-{4-[4-(3-Pyrrolidin-1-ylpropoxy)phenyl]tetrahydropyran-4-ylmethyl}piperidin-4-yl)-methanol
1-{4-[4-(3-Pyrrolidin-1-ylpropoxy)phenyl]tetrahydropyran-4-ylmethyl}piperidin-4-ol
1-{4-[4-(3-Pyrrolidin-1-yl-propoxy)-phenyl]-tetrahydro-pyran-4-ylmethyl}-piperidine-4-carboxylic acid amide
1-Methyl-4-{4-[4-(3-pyrrolidin-1-yl-propoxy)-phenyl]-tetrahydro-pyran-4-ylmethyl}-piperazine
1-{4-[4-(3-Pyrrolidin-1-ylpropoxy)phenyl]tetrahydropyran-4-ylmethyl}piperazine
1-{4-[4-(3-Pyrrolidin-1-ylpropoxy)phenyl]tetrahydropyran-4-ylmethyl}pyrrolidin-3-ol
(R)-2-Methoxymethyl-1-{4-[4-(3-pyrrolidin-1-yl-propoxy)-phenyl]-tetrahydropyran-4-ylmethyl}-pyrrolidine
(S)-2-Methoxymethyl-1-{4-[4-(3-pyrrolidin-1-ylpropoxy)-phenyl]tetrahydropyran-4-ylmethyl}pyrrolidine
1-(1-{4-[4-(3-Pyrrolidin-1-ylpropoxy)phenyl]tetrahydropyran-4-ylmethyl}piperidin-4-yl)pyrrolidin-2-one
4-{4-[4-(3-Pyrrolidin-1-yl-propoxy)-phenyl]-tetrahydro-pyran-4-ylmethyl}-thiomorpholine
4-{4-[4-(3-Pyrrolidin-1-ylpropoxy)phenyl]tetrahydropyran-4-ylmethyl}thiomorpholine-1-oxide
4-{4-[4-(3-Pyrrolidin-1-yl-propoxy)-phenyl]-tetrahydro-pyran-4-ylmethyl}-thiomorpholine 1,1-dioxide
4-{4-[4-(3-Pyrrolidin-1-yl-propoxy)phenyl]tetrahydropyran-4-ylmethyl}piperazine-1-carboxylic acid ethyl ester
4-{4-[4-(3-Pyrrolidin-1-ylpropoxy)phenyl]tetrahydropyran-4-ylmethyl}piperazine-1-carboxylic acid amide
1-{4-[4-(3-Pyrrolidin-1-ylpropoxy)phenyl]tetrahydropyran-4-ylmethyl}piperidine
4,4-difluoro-1-{4-[4-(3-Pyrrolidin-1-ylpropoxy)phenyl]-tetrahydropyran-4-ylmethyl}piperidine
methyl-{4-[4-(3-pyrrolidin-1-ylpropoxy)phenyl]-tetrahydropyran-4-ylmethyl}amine
methyl-(3-methylsulfanyl-propyl)-{4-[4-(3-pyrrolidin-1-ylpropoxy)phenyl]tetrahydropyran-4-ylmethyl}amine
(3-methanesulfonyl-propyl)-methyl-{4-[4-(3-pyrrolidin-1-ylpropoxy)phenyl]tetrahydropyran-4-ylmethyl}amine
isopropyl-{4-[4-(3-pyrrolidin-1-ylpropoxy)phenyl]-tetrahydropyran-4-ylmethyl}amine
isopropyl-methyl-{4-[4-(3-pyrrolidin-1-ylpropoxy)phenyl]tetrahydropyran-4-ylmethyl}amine
cyclopentyl-{4-[4-(3-pyrrolidin-1-ylpropoxy)phenyl]tetrahydropyran-4-ylmethyl}amine
1-{4-[4-(3-Pyrrolidin-1-ylpropoxy)phenyl]tetrahydropyran-4-ylmethyl}pyrrolidine
4-{4-[4-(3-Pyrrolidin-1-ylpropoxy)phenyl]tetrahydropyran-4-ylmethyl}-morpholine
methyl-phenethyl-{4-[4-(3-pyrrolidin-1-ylpropoxy)phenyl]tetrahydropyran-4-ylmethyl}amine
methyl-benzyl-{4-[4-(3-pyrrolidin-1-ylpropoxy)phenyl]tetrahydropyran-4-ylmethyl}amine
(2-methoxyethyl)-methyl-{4-[4-(3-pyrrolidin-1-ylpropoxy)phenyl]tetrahydropyran-4-ylmethyl}amine
methyl-pyrymidin-2-yl-{4-[4-(3-pyrrolidin-1-ylpropoxy)-phenyl]tetrahydropyran-4-ylmethyl}amine
N-{4-[4-(3-Pyrrolidin-1-yl-propoxy)-phenyl]-tetrahydro-pyran-4-ylmethyl}-methanesulfonamide
C-Phenyl-N-{4-[4-(3-pyrrolidin-1-yl-propoxy)-phenyl]-tetrahydro-pyran-4-ylmethyl}-methanesulfonamide
3-Cyano-N-{4-[4-(3-pyrrolidin-1-yl-propoxy)-phenyl]-tetrahydro-pyran-4-ylmethyl}-benzenesulfonamide
2-Fluoro-N-{4-[4-(3-pyrrolidin-1-yl-propoxy)-phenyl]-tetrahydro-pyran-4-ylmethyl}-benzenesulfonamide
N-methyl-N-{4-[4-(3-Pyrrolidin-1-yl-propoxy)-phenyl]-tetrahydro-pyran-4-ylmethyl}-methanesulfonamide
*N*'-{[4-(4-(3-Pyrrolidin-1-yl-propoxy )-phenyl)tetrahydro-2*H*-pyran-4-yl]methyl}-*N*,*N*-dimethylsulfamide
N-{4-[4-(3-Pyrrolidin-1-yl-propoxy)-phenyl]-tetrahydro-pyran-4-ylmethyl}-acetamide
N-methyl-N-{4-[4-(3-Pyrrolidin-1-yl-propoxy)-phenyl]-tetrahydro-pyran-4-ylmethyl}-acetamide
2-Phenyl-N-{4-[4-(3-pyrrolidin-1-yl-propoxy)-phenyl]-tetrahydro-pyran-4-ylmethyl}-acetamide
1,1-Dimethyl-3-{4-[4-(3-pyrrolidin-1-yl-propoxy)-phenyl]-tetrahydro-pyran-4-ylmethyl}-urea
1,1,3-Trimethyl-3-{4-[4-(3-pyrrolidin-1-yl-propoxy)-phenyl]-tetrahydro-pyran-4-ylmethyl}-urea
{4-[4-(3-Pyrrolidin-1-yl-propoxy)-phenyl]-tetrahydro-pyran-4-ylmethyl}-urea
4-dimethylaminomethyl-4-[4-(3-pyrrolidin-1-yl-propoxy)-phenyl]-cyclohexanol
4-[3-(3-Pyrrolidin-1-ylpropoxy)phenyl]tetrahydropyran-4-carbonitrile
4-{4-[3-(2,5-Dimethylpyrrolidin-1-yl)propoxy]phenyl}-tetrahydro-pyran-4-carbonitrile
4-{4-[3-(2-Methylpyrrolidin-1-yl)-propoxy]-phenyl}tetrahydropyran-4-carbonitrile
4-[4-(3-Thiomorpholin-4-ylpropoxy)phenyl]tetrahydropyran-4-carbonitrile
4-{4-[3-(1-Oxo-thiomorpholin-4-yl)-propoxy]-phenyl}-tetrahydro-pyran-4-carbonitrile
4-{4-[3-(1,1-Dioxo-1λ6-thiomorpholin-4-yl)propoxy]phenyl}tetrahydro-pyran-4-carbonitrile
4-{4-[3-(4-Hydroxypiperidin-1-yl)propoxy]phenyl}tetrahydro-pyran-4-carbonitrile
4-(4-{3-[4-(2-Hydroxyethyl)-piperidin-1-yl]propoxy}phenyl)-tetrahydro-pyran-4-carbonitrile
1-{3-[4-(4-Cyano-tetrahydro-pyran-4-yl)-phenoxy]-propyl}-piperidine-4-carboxylic acid amide
4-{3-[4-(4-Cyanotetrahydropyran-4-yl)-phenoxy]propyl}-piperazine-1-carboxylic acid ethyl ester
4-{3-[4-(4-Cyano-tetrahydro-pyran-4-yl)-phenoxy]-propyl}-piperazine-1-carboxylic acid tert-butyl ester
4-[4-(3-Piperazin-1-ylpropoxy)phenyl]tetrahydropyran-4-carbonitrile
4-(4-{3-[4-(2-Aminopropionyl)piperazin-1-yl]propoxy}phenyl)-tetrahydropyran-4-carbonitrile
4-(4-{3-[(2-Hydroxyethyl)methylamino]propoxy}-phenyl)tetrahydropyran-4-carbonitrile
4-[4-(2-Pyrrolidin-1-ylethoxy)phenyl]tetrahydropyran-4-carbonitrile
4-[4-(2-Methyl-3-pyrrolidin-1-ylpropoxy)phenyl]-tetrahydropyran-4-carbonitrile
4-[4-(3-Pyrrolidin-1-ylbutoxy)phenyl]tetrahydropyran-4-carbonitrile
4-{4-[2-(1-Methylpyrrolidin-2-yl)ethoxy]phenyl}tetra-hydropyran-4-carbonitrile
4-[4-(1-Isopropylpiperidin-4-yloxy)phenyl]tetrahydropyran-4-carbonitrile
4-[4-(1-Cyclopentylpiperidin-4-yloxy)phenyl]tetrahydropyran-4-carbonitrile
4-[4-(3-morpholino-4-ylpropoxy)phenyl]tetrahydropyran-4-carbonitrile
4-{4-[3-(4-methyl-piperazin-1-yl)-propoxy]phenyl}-tetrahydropyran-4-carbonitrile
4-{4-[3-(2-methyl-piperidin-1-yl)-propoxy]phenyl}-tetrahydropyran-4-carbonitrile
4-{4-[3-(3-methyl-piperidin-1-yl)-propoxy]phenyl}-tetrahydropyran-4-carbonitrile
4-{4-[3-(4-methyl-piperidin-1-yl)-propoxy]phenyl}-tetrahydropyran-4-carbonitrile
4-[4-(3-azepan-1-ylpropoxy)phenyl]tetrahydropyran-4-carbonitrile
4-{4-[3-(4,4-difluoro-piperidin-1-yl)-propoxy]phenyl}-tetrahydropyran-4-carbonitrile
4-[4-(5-Pyrrolidin-1-ylpentyloxy)phenyl]tetrahydropyran-4-carbonitrile
1-[4-(3-piperidin-1-yl-propoxy)-phenyl]-cyclohexanecarbonitrile
4-[4-(2,2-Dimethyl-3-pyrrolidin-1-ylpropoxy)phenyl]tetrahydropyran-4-carbonitrile
4-[4-(3-Pyrrolidin-1-ylpropoxy)phenyl]tetrahydro-2H-pyran-4-carboxylic acid amide
4-[4-(3-Pyrrolidin-1-ylpropoxy)phenyl]tetrahydro-2H-pyran-4-carboxylic acid
{4-[4-(3-Pyrrolidin-1-ylpropoxy)phenyl]tetrahydro-2H-pyran-4-yl}methanol
1-[4-(3-pyrrolidin-1-ylpropoxy)phenyl]cyclo-pentanecarbonitrile
4-oxo-1-[4-(3-pyrrolidin-1-ylpropoxy)-phenyl]-cyclohexane-carbonitrile
4-hydroxy-1-[4-(3-pyrrolidin-1-ylpropoxy)-phenyl]-cyclohexanecarbonitrile
1-(4-{3-[(2R)-2-(methoxymethyl)pyrrolidin-1-yl]propoxy}phenyl)cyclohexanecarbonitrile
1-(4-{3-[(3*S*)-3-methoxypyrrolidin-1-yl]propoxy}phenyl)-cyclohexanecarbonitrile
1-(4-{3-[(3*R*)-3-methoxypyrrolidin-1-yl]propoxy}phenyl)-cyclohexanecarbonitrile
4-(4-{3-[(2*R*,5*S*)-2,5-dimethylpyrrolidin-1-yl]propoxy}-phenyl)tetrahydro-2*H*-pyran-4-carbonitrile
1-{4-[3-(2,2-dimethylpyrrolidin-1-yl)propoxy]phenyl}-cyclohexanecarbonitrile
1-[1-(4-{3-[(2*R*)-2-(methoxymethyl)pyrrolidin-1-yl]propoxy}phenyl)cyclohexyl]methanamine
{[1-(4-{3-[(3*S*)-3-methoxypyrrolidin-1-yl]propoxy}-phenyl)cyclohexyl]methyl}amine
*N*-methyl-1-{1-[4-(3-pyrrolidin-1-ylpropoxy)phenyl]-cyclohexyl}methanamine
[(1-{4-[3-(2,2-dimethylpyrrolidin-1-yl)propoxy]phenyl}-cyclohexyl)methyl]amine
*N*-ethyl-*N*-({4-[4-(3-pyrrolidin-1-ylpropoxy)phenyl]tetrahydro-2*H*-pyran-4-yl}methyl)ethanamine
*N*-ethyl-*N*-({4-[4-(3-pyrrolidin-1-ylpropoxy)phenyl]tetrahydro-2*H*-pyran-4-yl}methyl)acetamide
*N*-methyl-*N*-({4-[4-(3-pyrrolidin-1-ylpropoxy)phenyl]-tetrahydro-2*H*-pyran-4-yl}methyl)ethanesulfonamide
*N*-methyl-*N*-({4-[4-(3-pyrrolidin-1-ylpropoxy)phenyl]-tetrahydro-2*H*-pyran-4-yl}methyl)propane-1-sulfonamide
*N*-ethyl-*N*-({4-[4-(3-pyrrolidin-1-ylpropoxy)phenyl]tetrahydro-2*H*-pyran-4-yl}methyl)methanesulfonamide
*N*-ethyl-*N*-({4-[4-(3-pyrrolidin-1-ylpropoxy)phenyl]tetrahydro-2*H*-pyran-4-yl}methyl)ethanesulfonamide
*N*-({4-[4-(3-pyrrolidin-1-ylpropoxy)phenyl]tetrahydro-2*H*-pyran-4-yl}methyl)ethanamine
*N*-methyl-*N*-({4-[4-(3-pyrrolidin-1-ylpropoxy)phenyl]-tetrahydro-2*H*-pyran-4-yl}methyl)ethanamine
4-(4-{3-[(2*R*)-2-methylpyrrolidin-1-yl]propoxy}phenyl)-tetrahydro-2*H*-pyran-4-carboxylic acid
4-{4-[(1-cyclobutyl-4-piperidinyl)oxy]phenyl}tetrahydro-2*H*-pyran-4-carbonitrile
4-{4-[(1-isopropylpiperidin-4-yl)oxy]phenyl}tetrahydro-2*H*-pyran-4-carboxamide
4-{4-[(1-isopropylpiperidin-4-yl)oxy]phenyl}tetrahydro-2*H*-pyran-4-carboxylic acid
4-{4-[(1-isopropylpiperidin-4-yl)oxy]phenyl]-*N*,*N*-dimethyltetrahydro-2*H*-pyran-4-carboxamide
4-{4-[(1-isopropylpiperidin-4-yl)oxy]phenyl]-*N*,*N*-diethyltetrahydro-2*H*-pyran-4-carboxamide
4-{4-[(1-isopropylpiperidin-4-yl)oxy]phenyl)tetrahydro-2*H*-pyran-4-yl]carbonyl}pyrrolidine
4-methyl-2-[4-(4-(3-pyrrolidin-1-ylpropoxy)phenyl)tetrahydro-2H-pyran-4-yl]-1,3-thiazole
2-[4-(4-(3-pyrrolidin-1-ylpropoxy)phenyl)tetrahydro-2*H-*pyran-4-yl]-1,3-thiazole
*N*-[(4-{4-[(1-isopropylpiperidin-4-yl)oxy]phenyl}tetrahydro-2*H*-pyran-4-yl)methyl]-*N*-methylamine
*N*-{[4-(4-[(1-isopropylpiperidin-4-yl)oxy]phenyl)tetrahydro-2*H*-pyran-4-yl]methyl}-N-methylacetamide
*N*-{[4-(4-[(1-isopropylpiperidin-4-yl)oxy]phenyl)tetrahydro-2*H*-pyran-4-yl]methyl}-*N*-methylpropanamide
*N*-{[4-(4-[(1-isopropylpiperidin-4-yl)oxy]phenyl)tetrahydro-2*H*-pyran-4-yl]methyl}-acetamide
*N*-[(4-{4-[(1-isopropylpiperidin-4-yl)oxy]phenyl}tetrahydro-2*H*-pyran-4-yl)methyl]-*N*-ethylamine
*N*-{[4-(4-[(1-isopropylpiperidin-4-yl)oxy]phenyl)tetrahydro-2*H*-pyran-4-yl]methyl}-*N*-ethylacetamide
1-{[4-(4-[(1-isopropylpiperidin-4-yl)oxy]phenyl)tetrahydro-2*H*-pyran-4-yl]methyl}pyrrolidin-2-one
*N*-{[4-(4-[(1-isopropylpiperidin-4-yl)oxy]phenyl)tetrahydro-2*H*-pyran-4-yl]methyl}pyrimidin-2-amine
*N*-{[4-(4-(3-Pyrrolidin-1-ylpropoxy)phenyl)tetrahydro-2*H*-pyran-4-yl]methyl}pyrimidin-2-amine
2-{[4-(4-[(1-isopropylpiperidin-4-yl)oxy]phenyl)tetrahydro-2*H*-pyran-4-yl]methyl}isothiazolidine 1,1-dioxide.

Particularly preferred examples are:
{4-[4-(1-Isopropylpiperidin-4-yloxy)phenyl]tetrahydropyran-4-ylmethyl}dimethylamine
Dimethyl-{4-[4-(4-pyrrolidin-1-ylbutoxy)phenyl]tetrahydro-pyran-4-ylmethyl}amine
{4-[4-(3-Pyrrolidin-1-ylpropoxy)phenyl]tetrahydropyran-4-yl} methylamine
Dimethyl-{4-[4-(3-pyrrolidin-1-ylpropoxy)phenyl]-tetrahydropyran-4-ylmethyl}amine
{4-[4-(3-Piperidin-1-ylpropoxy)phenyl]tetrahydropyran-4-yl} methylamine
Dimethyl-{4-[4-(3-piperidin-1-ylpropoxy)phenyl]tetra-hydropyran-4-ylmethyl}amine
1-Methyl-4-{4-[4-(3-pyrrolidin-1-yl-propoxy)-phenyl]-tetrahydro-pyran-4-ylmethyl}-piperazine
(R)-2-Methoxymethyl-1-{4-[4-(3-pyrrolidin-1-yl-propoxy)-phenyl]-tetrahydro-pyran-4-ylmethyl}-pyrrolidine
(S)-2-Methoxymethyl-1-{4-[4-(3-pyrrolidin-1-ylpropoxy)-phenyl]tetrahydropyran-4-ylmethyl}pyrrolidine
1-{4-[4-(3-Pyrrolidin-1-ylpropoxy)phenyl]tetrahydropyran-4-ylmethyl}piperidine
methyl-{4-[4-(3-pyrrolidin-1-ylpropoxy)phenyl]-tetrahydropyran-4-ylmethyl}amine
isopropyl-methyl-{4-[4-(3-pyrrolidin-1-ylpropoxy)phenyl]-tetrahydropyran-4-ylmethyl}amine
1-{4-[4-(3-Pyrrolidin-1-ylpropoxy)phenyl]tetrahydropyran-4-ylmethyl}pyrrolidine
4-{4-[4-(3-Pyrrolidin-1-ylpropoxy)phenyl]tetrahydropyran-4-ylmethyl}-morpholine
(2-methoxyethyl)-methyl-{4-[4-(3-pyrrolidin-1-ylpropoxy)-phenyl]tetrahydropyran-4-ylmethyl}amine
4-[4-(1-Isopropylpiperidin-4-yloxy)phenyl]tetrahydropyran-4-carbonitrile
4-[4-(3-Pyrrolidin-1-ylpropoxy)phenyl]tetrahydro-2H-pyran-4-carboxylic acid amide
*N*-methyl-1-{1-[4-(3-pyrrolidin-1-ylpropoxy)phenyl]-cyclohexyl}methanamine
*N*-ethyl-*N*-({4-[4-(3-pyrrolidin-1-ylpropoxy)phenyl]tetrahydro-2*H*-pyran-4-yl}methyl)ethanamine
*N*-({4-[4-(3-pyrrolidin-1-ylpropoxy)phenyl]tetrahydro-2*H*-pyran-4-yl}methyl)ethanamine
*N*-methyl-*N*-({4-[4-(3-pyrrolidin-1-ylpropoxy)phenyl]-tetrahydro-2*H*-pyran-4-yl}methyl)ethanamine
4-{4-[(1-cyclobutyl-4-piperidinyl)oxy]phenyl}tetrahydro-2*H*-pyran-4-carbonitrile
4-{4-[(1-isopropylpiperidin-4-yl)oxy]phenyl}tetrahydro-2*H*-pyran-4-carboxamide
4-{4-[(1-isopropylpiperidin-4-yl)oxy]phenyl]-*N,N*-dimethyltetrahydro-2*H*-pyran-4-carboxamide
4-{4-[(1-isopropylpiperidin-4-yl)oxy]phenyl]-*N,N*-diethyltetra-hydro-2*H*-pyran-4-carboxamide
4-{4-[(1-isopropylpiperidin-4-yl)oxy]phenyl)tetrahydro-2*H*-pyran-4-yl]carbonyl}pyrrolidine
4-methyl-2-[4-(4-(3-pyrrolidin-1-ylpropoxy)phenyl)tetrahydro-2*H*-pyran-4-yl]-1,3-thiazole
2-[4-(4-(3-pyrrolidin-1-ylpropoxy)phenyl)tetrahydro-2*H*-pyran-4-yl]-1,3-thiazole
*N*-[(4-{4-[(1-isopropylpiperidin-4-yl)oxy]phenyl}tetrahydro-2*H*-pyran-4-yl)methyl]-*N*-methylamine
*N*-[(4-{4-[(1-isopropylpiperidin-4-yl)oxy]phenyl}tetrahydro-2*H*-pyran-4-yl)methyl]-*N*-ethylamine
*N*-{[4-(4-[(1-isopropylpiperidin-4-yl)oxy]phenyl)tetrahydro-2*H*-pyran-4-yl]methyl}pyrimidin-2-amine and
*N*-{[4-(4-(3-Pyrrolidin-1-ylpropoxy)phenyl)tetrahydro-2*H*-pyran-4-yl]methyl}pyrimidin-2-amine.

Pharmaceutically acceptable salts of the compounds of formula (1) include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula (1) may be prepared by one or more of three methods:
(i) by reacting the compound of formula (1) with the desired acid or base;
(ii) by removing an acid- or base-labile protecting group from a suitable precursor of the compound of formula (1) or by ring-opening a suitable cyclic precursor, for example, a lactone or lactam, using the desired acid or base; or
(iii) by converting one salt of the compound of formula (1) to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in both unsolvated and solvated forms. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non-ionised. For a review of such complexes, see J Pharm Sci, 64 (8), 1269-1288 by Haleblian (August 1975).

Hereinafter all references to compounds of formula (1) include references to salts, solvates and complexes thereof and to solvates and complexes of salts thereof.

The compounds of the invention include compounds of formula (1) as hereinbefore defined, including all polymorphs and crystal habits thereof, prodrugs and isomers thereof (including optical, geometric and tautomeric isomers) as hereinafter defined and isotopically-labeled compounds of formula (1).

As indicated, so-called 'pro-drugs' of the compounds of formula (1) are also within the scope of the invention. Thus certain derivatives of compounds of formula (1) which may have little or no pharmacological activity themselves can, when administered into or onto the body, be converted into compounds of formula (1) having the desired activity, for example, by hydrolytic cleavage. Such derivatives are referred to as 'prodrugs'. Further information on the use of prodrugs may be found in 'Pro-drugs as Novel Delivery Systems, Vol. 14, ACS Symposium Series (T. Higuchi and W. Stella) and 'Bioreversible Carriers in Drug Design', Pergamon Press, 1987 (ed. E. B Roche, American Pharmaceutical Association).

Prodrugs in accordance with the invention can, for example, be produced by replacing appropriate functionalities present in the compounds of formula (1) with certain moieties known to those skilled in the art as 'pro-moieties' as described, for example, in "Design of Prodrugs" by H. Bundgaard (Elsevier, 1985).

Some examples of prodrugs in accordance with the invention include:
(i) where the compound of formula (1) contains a carboxylic acid functionality (-C(O)OH), an ester thereof, for example, a compound wherein the hydrogen of the carboxylic acid functionality of the compound of formula (1) is replaced by (C₁-C₈)alkyl;
(ii) where the compound of formula (1) contains an alcohol functionality (-OH), an ether thereof, for example, a compound wherein the hydrogen of the alcohol functionality of the compound of formula (1) is replaced by (C₁-C₆)alkanoyloxymethyl; and
(iii) where the compound of formula (1) contains a primary or secondary amino functionality (-NH₂ or -NHR where R ≠ H), an amide thereof, for example, a compound wherein, as the case may be, one or both hydrogens of the amino functionality of the compound of formula (1) is/are replaced by (C₁-C₁₀)alkanoyl.

Further examples of replacement groups in accordance with the foregoing examples and examples of other prodrug types may be found in the aforementioned references.

Moreover, certain compounds of formula (1) may themselves act as prodrugs of other compounds of formula (1).

Also included within the scope of the invention are metabolites of compounds of formula (1), that is, compounds formed *in vivo* upon administration of the drug. Some examples of metabolites in accordance with the invention include :
(i) where the compound of formula (1) contains a methyl group, an hydroxymethyl derivative thereof (-CH₃ → -CH₂OH):
(ii) where the compound of formula (1) contains an alkoxy group, an hydroxy derivative thereof (-OR → -OH);
(iii) where the compound of formula (1) contains a tertiary amino group, a secondary amino derivative thereof (-NR^{a}R^{b} → -NHR^{a} or -NHR^{b});
(iv) where the compound of formula (1) contains a secondary amino group, a primary derivative thereof (-NHR^{a} → -NH₂);
(v) where the compound of formula (1) contains a phenyl moiety, a phenol derivative thereof (-Ph → -PhOH); and
(vi) where the compound of formula (1) contains an amide group, a carboxylic acid derivative thereof (-CONR^{c}R^{d} → COOH).

Compounds of formula (1) containing one or more asymmetric carbon atoms can exist as two or more stereoisomers. Where structural isomers are interconvertible *via* a low energy barrier, tautomeric isomerism ('tautomerism') can occur. This can take the form of proton tautomerism in compounds of formula (1) containing, for example, an imino, keto, or oxime group, or so-called valence tautomerism in compounds which contain an aromatic moiety. It follows that a single compound may exhibit more than one type of isomerism.

Included within the scope of the present invention are all stereoisomers, geometric isomers and tautomeric forms of the compounds of formula (1), including compounds exhibiting more than one type of isomerism, and mixtures of one or more thereof. Also included are acid addition or base salts wherein the counterion is optically active, for example, *d*-lactate or *l*-lysine, or racemic, for example, *dl*-tartrate or *dl*-arginine.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (1) contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1 % diethylamine. Concentration of the eluate affords the enriched mixture.

Stereoisomeric conglomerates may be separated by conventional techniques known to those skilled in the art - see, for example, "Stereochemistry of Organic Compounds" by E. L. Eliel (Wiley, New York, 1994).

The present invention includes all pharmaceutically acceptable isotopically-labelled compounds of formula (1) wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of formula (1), for example, those incorporating a radioactive isotope, are useful in drug and/or substrate tissue distribution studies. The radioactive isotopes tritium, *i*.*e*. ³H, and carbon-14, *i*.*e*. ¹⁴C, are particularly useful for this purpose in view of their ease of incorporation and ready means of detection.

Substitution with heavier isotopes such as deuterium, *i*.*e*. ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of formula (1) can generally be prepared by conventional techniques known to those skilled in the art or by processes analogous to those described in the accompanying Examples and Preparations using an appropriate isotopically-labeled reagents in place of the non-labeled reagent previously employed.

Pharmaceutically acceptable solvates in accordance with the invention include those wherein the solvent of crystallization may be isotopically substituted, e.g. D₂O, d₆-acetone, d₆-DMSO.

The phenol derivatives of formula (1) can be prepared using conventional procedures such as by the following illustrative methods in which R, X, Y, m, p, R¹, R², R³, R⁴, R⁵, R⁶, R⁷, R⁸ and R⁹ are as previously defined unless otherwise stated.

The compounds of formula (1), wherein X is -CH₂NR¹R², may be prepared from compounds of formula (2) : 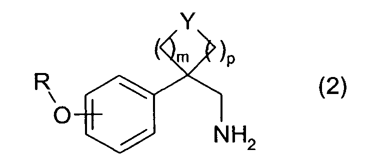 using standard techniques for functional group interconversion known to those skilled in the art, for example as described in *Comprehensive Organic Transformations,* R.C. Larock, 1^{st} Edition, 1989 VCH Publishers Inc.
These techniques include:
1) Reductive alkylation with aldehydes or ketones with a reducing agent (e.g. formic acid or NaHB(OAc)₃) in a suitable solvent (e.g. dichloromethane), optionally with the addition of acetic acid, at between room temperature (about 20°C) and reflux;
2) Alkylations with alkyl halides and a base (e.g. potassium carbonate) in a suitable solvent (e.g. acetonitrile) at between room temperature (about 20°C) and reflux;
3) Sulphonylation with a sulphonyl chloride and a base (e.g. triethylamine) in a suitable solvent (e.g. dichloromethane) at between room temperature and reflux;
4) Heteroarylation with a haloheteroaromatic compound and a base (e.g. potassium carbonate) in a suitable solvent (e.g. acetonitrile) at between room temperature (about 20°C) and reflux;
5) Urea formation with a dialkylcarbamoyl chloride or a cyanate salt, optionally in the presence of a base (e.g. triethylamine) or an acid (e.g. acetic acid) in a suitable solvent (e.g. dichloromethane or water) at between room temperature and reflux;
6) Sulphonyl urea formation with a dialkylsulphamoyl chloride and a base (e.g. triethylamine) in a suitable solvent (e.g. dichloromethane) at between room temperature and reflux;
7) Acylation with an activated acid (e.g. an acyl chloride) and a base (e.g. triethylamine) in a suitable solvent (e.g. dichloromethane) at between room temperature and reflux.
These transformations can be performed sequentially to prepare compounds in which R¹ and R² are as here above defined.

The compounds of formula (2) may be prepared by reduction of the corresponding cyano derivatives of formula (3) : 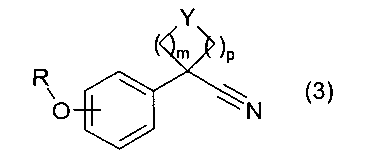 with a suitable reducing agent (e.g. LiAlH₄ or hydrogen in the presence of a catalyst such as PtO₂) in a suitable solvent (e.g. Et₂O/dichloromethane or isopropanol) at between room temperature and reflux.

The compounds of formula (3) above correspond actually to the compounds of formula (1) wherein X is a CN group.

The compounds of formula (3) may be prepared by alkylation of the corresponding hydroxy derivatives of formula (4) : 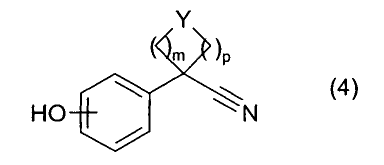 with a derivative of formula R-R^{LG} wherein R^{LG} is a leaving group such as halo, mesylate or tosylate, in the presence of a base (e.g. potassium carbonate) in a suitable solvent (e.g. dimethylformamide) at between room temperature and reflux.

When R is a group of formula: 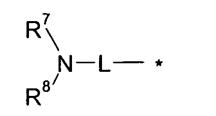 as previously defined, then the derivative of formula R-R^{LG} may be prepared by alkylation of the corresponding amino derivative of formula NHR⁷R⁸ with a derivative of formula R^{LG}-L-R^{LG} and a base (e.g. NaOH) in a suitable solvent (e.g. acetone/H₂O) at between room temperature and reflux. The amino derivative of formula NHR⁷R⁸ is either commercial or made using procedures known to the skilled person.

When R is a group of formula : 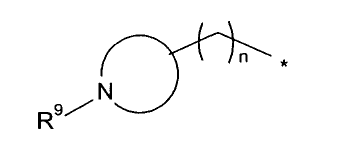 as previously defined, then the derivative of formula R-R^{LG} is either commercial or may be prepared using literature procedures well-known to the skilled person.

The compounds of formula (4) may be prepared by deprotection of the corresponding derivatives of formula (5) : 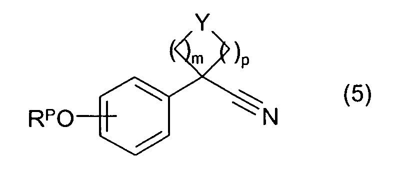 wherein R^{P} is a protecting group (e.g. methyl, deprotected with BBr₃ in dichloromethane at between 0°C and room temperature).

The compounds of formula (5) are either commercial or may be prepared by double alkylation of the compounds of formula (6) : 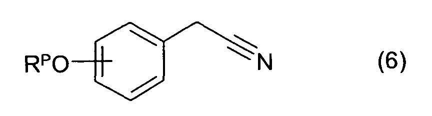 wherein R^{P} is as previously defined,
with the compounds of formula (7) : 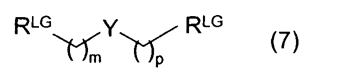 wherein R^{LG} is as previously defined, and a base (e.g. NaH) in a suitable solvent (e.g. dimethylformamide) at between room temperature and reflux.

The compounds of formula (6) and (7) are each either commercially available or made using literature procedures well-known to the skilled person.
Alternatively, the compounds of formula (3) may be prepared by alkylation of the compounds of formula (4) with the alcohol derivative of formula ROH, which is either commercial or made using literature procedures well-known to the skilled person, using Mitsunobu reagents such as PPh₃ and DIAD in a suitable solvent (e.g. THF) at between 0°C and reflux.

Persons skilled in the art will appreciate that, in order to obtain compounds of formula (1) in an alternative or more convenient manner, the individual process steps mentioned in this section may be performed in a different order. For example compounds of formula (1) wherein X is -CH₂NR¹R², may be prepared by alkylation of the compounds of formula (8) : 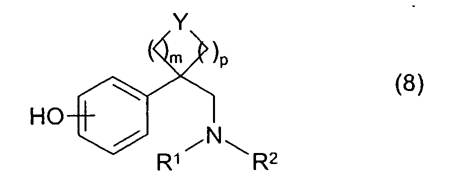 with a derivative of formula R-R^{LG} or ROH as previously defined, using conditions analogous to those decribed for the preparation of compounds of formula (3).

The compounds of formula (8) may be prepared by deprotection of the compounds of formula (9) : 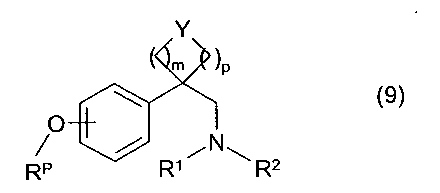 wherein R^{P} is as previously defined,
using conditions appropriate for the protecting group and the nature of R¹ and R² (e.g. using NaSMe in dimethylformamide at 130°C wherein R^{P} is methyl and R¹ and R² are (C₁-C₄)alkyl).

The compounds of formula (9) may be prepared from the corresponding amino derivatives of formula (10) : 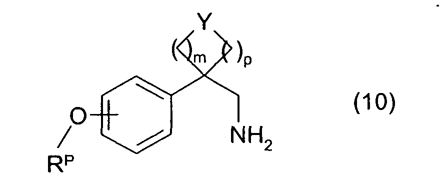 using analogous conditions to those described for the preparation of compounds of formula (1) from compounds of formula (2).

The compounds of formula (10) may be prepared from compounds of formula (5) using analogous reduction conditions to those described for the preparation of compounds of formula (2) from compounds of formula (3).

A further example of performing the individual process steps described in this section in a different order to obtain compounds of formula (1) in an alternative or more convenient manner, is the preparation of compounds of formula (1) by double alkylation of the compounds of formula (11) : 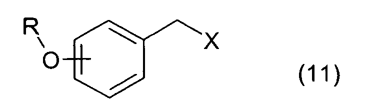 with the compounds of formula (7) as previously defined, using analogous conditions described for the preparation of compounds of formula (5) from compounds of formula (6).

The compounds of formula (11) may be prepared from the corresponding hydroxy derivatives of formula (12) : 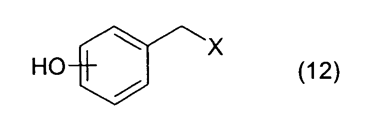 using analogous conditions described for the preparation of compounds of formula (3) from compounds of formula (4).

The compounds of formula (12) are either commercial or made using literature procedures well-known to the skilled person.

An example of performing the individual process steps described in this section in a different order to obtain compounds of formula (1), wherein R is a group of formula : 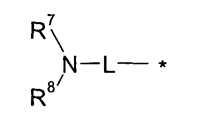 is the preparation of these compounds by reaction of the compounds of formula (13) : 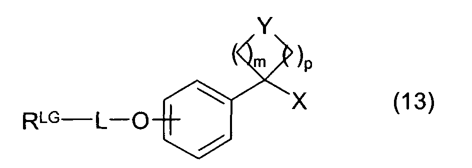 wherein R^{LG} is as previously defined, with the amino derivatives of formula NHR⁷R⁸ using analogous conditions to those previously described for the preparation of R-R^{LG}.

Alternatively, the same transformation can be achieved by heating the compounds of formula (13) with the amino derivatives of formula NHR⁷R⁸ and a base (e.g. diisopropylethylamine) in a suitable solvent (e.g. N-methylpyrrolidinone) at 150-200°C for 5-10 min using a microwave oven.

Alternatively, the compounds of formula (1) wherein X is -CH₂NR¹R² and R¹ and R² are hydrogen or an optionally substituted (C₁-C₄)alkyl or together with the N atom to which they are attached form an optionally substituted 4-, 5- or 6-membered saturated heterocycle wherein a C atom may be replaced by N, O, S, SO or SO₂, may be prepared by reductive amination of the compounds compounds of formula (14) : 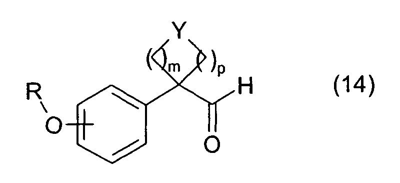 with the amino derivative of formula HNR¹R² and a reducing agent (e.g. NaHB(OAc)₃), optionally in the presence of a Lewis acid (e.g. Ti(OⁱPr)₄) in a suitable solvent (e.g. EtOH) at between 0°C and reflux.

The compounds of formula (14) may be prepared by reduction of the compounds of formula (3) with a reducing agent (e.g. diisobutylaluminium hydride) in a suitable solvent (e.g. toluene) at between -78°C and room temperature (about 20°C).

Alternatively, the compounds of formula (14) may be prepared by oxidation of the compounds of formula (1) wherein X is -CH₂OH, using an oxidant (e.g. pyridinium chlorochromate) in a suitable solvent (e.g. dichloromethane) at between 0°C and reflux.

The compounds of formula (1) wherein X is -CH₂OH, may be prepared by reduction of the compounds of formula (1) wherein X is -C(O)O(C₁-C₄)alkyl, with a reducing agent (e.g. LiAlH₄) in a suitable solvent (e.g. tetrahydrofuran) at between -78°C and reflux.

The compounds of formula (1) wherein X is -C(O)O(C₁-C₄)alkyl, may be prepared by esterification of the compounds of formula (1) wherein X is COOH, using the standard procedures well-known to the skilled person. For example, the esterification can be achieved by using a reagent capable of activating a carboxylic acid (e.g. thionyl chloride) and HO(C₁-C₄)alkyl, optionally in the presence of an additional solvent (e.g. dichloromethane) at between 0°C and reflux.

The compounds of formula (1) wherein X is C(O)OH, may be prepared by hydrolysis of the compounds of formula (3) as previously defined with a mineral acid (e.g. concentrated aqueous HCl), optionally in the presence of a suitable cosolvent (e.g. dioxane), at between 0°C and reflux.

The compounds of formula (1) wherein m and p are both equal to 2 and Y is CH(OH), may be prepared by reduction of the compounds of formula (1) wherein m and p are both equal to 2 and Y is C=O, with a reducing agent (e.g. LiAlH₄) in a suitable solvent (e.g. Et₂O) at between 0°C and reflux.

The compounds of formula (1) wherein m and p are both equal to 2 and Y is C=O, may be prepared by treatment of the compounds of formula (15) : 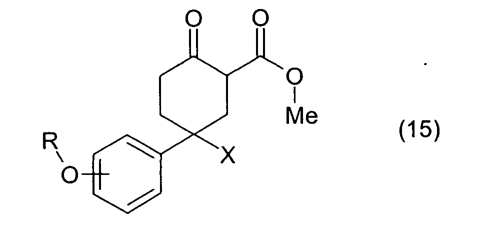 with sodium chloride in a suitable solvent (e.g. dimethylsulphoxide/water) at between 100°C and reflux.

The compounds of formula (15) may be prepared by treatment of the compounds of formula (16) : 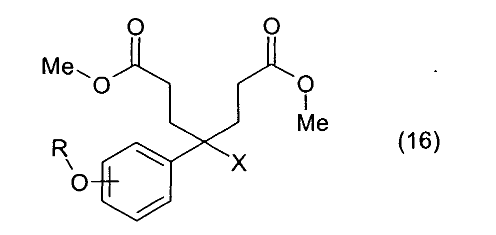 with a base (e.g. NaH) in a suitable solvent (e.g. 1,2-dimethoxyethane) at between room temperature and reflux.

The compounds of formula (16) may be prepared by alkylation of the compounds of formula (11) as previously defined with methyl acrylate and a base (e.g. benzyltrimethylammonium hydroxide) in a suitable solvent (e.g. methanol/acetonitrile) at between room temperature and reflux.
The compounds of formula (1) wherein X is -CONH₂, may be prepared by treatment of the compounds of formula (3) as previously defined with polyphosphoric acid at between room temperature and 100°C.
The compounds of formula (1) wherein X is -CONR³R⁴, may be prepared by reaction of the compounds of formula (1) wherein X is COOH, sequentially with a reagent capable of activating a carboxylic acid (e.g. thionyl chloride) and then with the amino derivative of formula HNR³R⁴, in a suitable solvent (e.g. dichloromethane) at between 0°C and reflux.

The compounds of formula (1) wherein X is -CH₂-O-(C₁-C₄)alkyl may be prepared by alkylation of the compounds of formula (1) wherein X is -CH₂-OH, with a derivative of formula R^{LG}(C1-C4)alkyl, wherein R^{LG} is as previously defined, and a base (e.g. NaH) in a suitable solvent (e.g. dimethylformamide) at between room temperature and reflux.

Compounds of formula (1 ), where X is het¹, may be prepared from compounds of formula (1), where X is COOH, or alternatively from compounds of formula (3) using methods known to those skilled in the art, such as those described in general heterocyclic texts such as *Heterocyclic Chemistry,* J.A. Joule and K. Mills, 4^{th} Edition, Blackwell publishing, 2000.

For example, compounds of formula (1) where X is 2-thiazolyl, may be prepared by treating compounds of formula (17) : 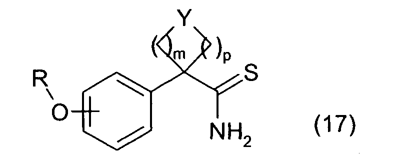 with bromoacetaldehyde dimethyl acetal and HCl in a suitable solvent (e.g. EtOH) at reflux.

Compounds of formula (17) may be prepared by reacting compounds of formula (3) with diethyldithiophosphate and water at 60°C.

The compounds of formula (1) and their precursors which contain a sulphide group can be oxidised to the corresponding sulphoxides or sulphones using standard techniques well-known to those skilled in the art, for example as described in *Comprehensive Organic Transformations,* R.C. Larock, 1^{st} Edition, 1989 VCH Publishers Inc.

It will be appreciated by those skilled in the art that, in the course of carrying out the processes described above, the functional groups of intermediate compounds may need to be protected by protecting groups.

These functional groups include hydroxyl, amino and carboxylic acid. Suitable protecting groups for hydroxyl include trialkylsilyl and diarylalkylsilyl (e.g. *tert*-butyldimethylsilyl, tert-butyldiphenylsilyl or trimethylsilyl), alkyl (e.g. methyl or methoxyethyl) and tetrahydropyranyl. Suitable protecting groups for amino include *tert*-butyloxycarbonyl, 9-fluorenylmethoxycarbonyl or benzyloxycarbonyl. Suitable [protecting groups for carboxylic acid include (C₁-C₄)alkyl or benzyl esters.

The protection and deprotection of functional groups may take place before or after any of the reaction steps described hereinbefore.

The introduction and removal of protecting groups is fully described in *Protective Groups in Organic Chemistry,* edited by J.W.F. McOmie, Plenum Press (1973), *Protective Groups in Organic Synthesis*, 2^{nd} edition, T.W. Greene & P.G.M. Wutz, Wiley-Interscience Publication, 1991) and *Protecting Groups,* P.J. Kocienski, Thieme, 1994.

Also, the compounds of formula (1) as well as intermediates for the preparation thereof, can be purified according to various well-known methods such as recrystallisation and chromatography.

The compounds of formula (1), their pharmaceutically acceptable salts and/or derived forms, are valuable pharmaceutically active compounds, which are suitable for the therapy and prophylaxis of numerous disorders in which the histamine H₃ receptor is involved or in which agonism or antagonism of this receptor may induce benefit.

Compounds of the invention intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

They may be administered alone or in combination with one or more other compounds of the invention or in combination with one or more other drugs (or as any combination thereof). Generally, they will be administered as a formulation in association with one or more pharmaceutically acceptable excipients. The term 'excipient' is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in Remington's Pharmaceutical Sciences, 19th Edition (Mack Publishing Company, 1995).

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, solid solution, liposome, films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human or veterinary use are typically pliable water-soluble or water-swellable thin film dosage forms which may be rapidly dissolving or mucoadhesive and typically comprise a compound of formula (1), a film-forming polymer, a binder, a solvent, a humectant, a plasticiser, a stabiliser or emulsifier, a viscosity-modifying agent and a solvent. Some components of the formulation may perform more than one function.

The compound of formula (1) may be water-soluble or insoluble. A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes. Alternatively, the compound of formula (1) may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, cosolvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line, 25(2), 1-14, by Verma *et al* (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (1) used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and poly(*dl*-lactic-coglycolic)acid (PGLA) microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Other means of topical administration include delivery by electroporation, iontophoresis, phonophoresis, sonophoresis and microneedle or needle-free (*e.g.* Powderject™, Bioject™, etc.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as I-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula (1), propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff' containing from 1 µg to 4000 µg of the compound of formula (1). The overall daily dose will typically be in the range 1 µg to 20 mg which may be administered in a single dose or, more usually, as divided doses throughout the day.
The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i*.*e*. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula (1) in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the compounds of the invention is typically in the 0.001 mg to 2000 mg depending, of course, on the mode of administration. For example, oral administration may require a total daily dose of from 1 mg to 2000 mg, while an intravenous dose may only require from 0.01 mg to 100 mg. The total daily dose may be administered in single or divided doses and may, at the physician's discretion, fall outside of the typical range given herein.

These dosages are based on an average human subject having a weight of about 60kg to 70kg. The physician will readily be able to determine doses for subjects whose weight falls outside this range, such as infants and the elderly.

For the avoidance of doubt, references herein to "treatment" include references to curative, palliative and prophylactic treatment.

According to another embodiment of the present invention, the compounds of the formula (1), or pharmaceutically acceptable salts, derived forms or compositions thereof, can also be used as a combination with one or more additional therapeutic agents to be co-administered to a patient to obtain some particularly desired therapeutic end result. The second and more additional therapeutic agents may also be a compound of the formula (1), or a pharmaceutically acceptable salt, derived forms or compositions thereof, or one or more histamine H₃ receptor ligands known in the art. More typically, the second and more therapeutic agents will be selected from a different class of therapeutic agents.

As used herein, the terms "co-administration", "co-administered" and "in combination with", referring to the compounds of formula (1) and one or more other therapeutic agents, is intended to mean, and does refer to and include the following:
- simultaneous administration of such combination of compound(s) of formula (1) and therapeutic agent(s) to a patient in need of treatment, when such components are formulated together into a single dosage form which releases said components at substantially the same time to said patient,
- substantially simultaneous administration of such combination of compound(s) of formula (1) and therapeutic agent(s) to a patient in need of treatment, when such components are formulated apart from each other into separate dosage forms which are taken at substantially the same time by said patient, whereupon said components are released at substantially the same time to said patient,
- sequential administration of such combination compound(s) of formula (1) and therapeutic agent(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of compound(s) of formula (1) and therapeutic agent(s) to a patient in need of treatment, when such components are formulated together into a single dosage form which releases said components in a controlled manner whereupon they are concurrently, consecutively, and/or overlapingly administered at the same and/or different times by said patient,
where each part may be administered by either the same or different route.

Suitable examples of other therapeutic agents which may be used in combination with the compound(s) of formula (1), or pharmaceutically acceptable salts, derived forms or compositions thereof, include, but are by no means limited to :
- Histamine H₁ receptor antagonists, for instance loratidine, desloratidine, fexofenadine and cetirizine
- Histamine H₄ receptor antagonists
- Histamine H₂ receptor antagonists
- Leukotriene antagonists, including antagonists of LTB₄, LTC₄, LTD₄, and LTE₄, in particular Montelukast
- Phosphodiesterase PDE4 inhibitors
- neurotransmitter re-uptake inhibitors, for instance fluoxetine, sertraline, paroxetine, ziprasidone
- 5-Lipoxygenase (5-LO) inhibitors or 5-lipoxygenase activating protein (FLAP) antagonists,
- α₁- and α₂-adrenoceptor agonist vasoconstrictor sympathomimetic agents for decongestant use,
- Muscarinic M3 receptor antagonists or anticholinergic agents,
- β₂-adrenoceptor agonists,
- Theophylline,
- Sodium cromoglycate,
- COX-1 inhibitors (NSAIDs) and COX-2 selective inhibitors,
- Oral or inhaled Glucocorticosteroids,
- Monoclonal antibodies active against endogenous inflammatory entities,
- Anti-tumor necrosis factor (anti-TNF-α) agents,
- Adhesion molecule inhibitors including VLA-4 antagonists,
- Kinin-B₁ - and B₂-receptor antagonists,
- Immunosuppressive agents,
- Inhibitors of matrix metalloproteases (MMPs),
- Tachykinin NK₁, NK₂ and NK₃ receptor antagonists,
- Elastase inhibitors,
- Adenosine A2a receptor agonists,
- Inhibitors of urokinase,
- Compounds that act on dopamine receptors, e.g. D2 agonists,
- Modulators of the NFκβ pathway, e.g. IKK inhibitors,
- Agents that can be classed as mucolytics or anti-tussive,
- antibiotics,
- p38 MAP kinase inhibitors, and
- Phosphodiesterase PDE5 inhibitors

According to the present invention, combination of the compounds of formula (1) with :
- Histamine H₁ receptor antagonists, for instance loratidine, desloratidine, fexofenadine and cetirizine
- Histamine H₄ receptor antagonists
- Histamine H₂ receptor antagonists
- Leukotriene antagonists, including antagonists of LTB₄, LTC₄, LTD₄, and LTE₄, in particular Montelukast
- Phosphodiesterase PDE4 inhibitors
- neurotransmitter re-uptake inhibitors, for instance fluoxetine, sertraline, paroxetine, ziprasidone
are preferred.

The compounds of formula (1) have the ability to interact with the H₃ receptor and thereby have a wide range of therapeutic applications, as described further below, because of the essential role which the H₃ receptor plays in the physiology of all mammals. According to this invention H₃ ligands are meant to include H₃ receptor antagonists, agonists and inverse agonists. For the preferred indications to be treated according to the invention, H₃ antagonists are believed to be most suitable.

Therefore, a further aspect of the present invention relates to the compounds of formula (1), or pharmaceutically acceptable salts, derived forms or compositions thereof, for use in the treatment of diseases, disorders, and conditions in which the H₃ receptor is involved. More specifically, the present invention also concerns the compounds of formula (1), or pharmaceutically acceptable salts, derived forms or compositions thereof, for use in the treatment of diseases, disorders, and conditions selected from the group consisting of :
- diseases of the central nervous system: sleep disorders, migraine, dyskinesia, stress-induced anxiety, psychotic disorders, epilepsy, Cognition deficiency diseases such as Alzheimer's disease or mild cognitive impairment, depression, mood disorders, schizophrenia, anxiety disorders, attention-deficit hyperactivity disorder (ADHD), psychotic disorders, obesity, dizziness, vertigo, epilepsy, motion sickness
- inflammatory diseases
- respiratory diseases (adult respiratory distress syndrome, acute respiratory distress syndrome, bronchitis, chronic bronchitis, chronic obstructive pulmonary disease, cystic fibrosis, asthma, emphysema, rhinitis, chronic sinusitis), allergy, allergy-induced airway responses, allergic rhinitis, viral rhinitis, non-allergic rhinitis, perennial and seasonal rhinitis, nasal congestion, allergic congestion
- female and male sexual dysfunction,
- cardiac dysfunctions such as myocardial ischaemia and arrythmia
- diseases of the gastrointestinal tract such as inflammatory bowel disease, Crohn's disease and colitis ulcerosa
- cancer
- hypotension
- pain and
- overactive bladder conditions

The compounds of formula (1) of the invention are particularly suitable for the treatment of allergy, allergy-induced airway responses, allergic rhinitis, viral rhinitis, non-allergic rhinitis, perennial and seasonal rhinitis, nasal congestion, allergic congestion.

A still further aspect of the present invention also relates to the use of the compounds of formula (1), or pharmaceutically acceptable salts, derived forms or compositions thereof, for the manufacture of a drug being a H₃ ligand. In particular, the present inventions concerns the use of the compounds of formula (1), or pharmaceutically acceptable salts, derived forms or compositions thereof, for the manufacture of a drug for the treatment of H3-mediated diseases and/or conditions, in particular the diseases and/or conditions listed above.

As a consequence, the present invention provides a particularly interesting method to treat a mammal, including a human being, with an effective amount of a compound of formula (1), or a pharmaceutically acceptable salt, derived form or composition thereof. More precisely, the present invention provides a particularly interesting method for the treatment of a H3-mediated diseases and/or conditions in a mammal, including a human being, in particular the diseases and/or conditions listed above, comprising administering to said mammal an effective amount of a compound of formula (1), its pharmaceutically acceptable salts and/or derived forms.

The following examples illustrate the preparation of the phenol derivatives of the formula (1):

### EXAMPLES

In the examples the following abbreviations were used:
DIAD diisopropyl azodicarboxylate
THF tetrahydrofuran
DMSO dimethyl sulphoxide
DCM dichloromethane
TBME tert-butylmethylether
TFA trifluoro acetic acid
PTFA peroxytrifluoroacetic acid
STAB: sodium triacetoxy borohydride

### Intermediate 1: 4-(4-methoxyphenyl)-tetrahydro-2H-pyran-4-carbonitrile

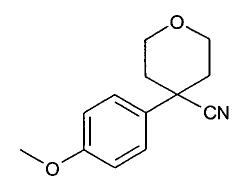

4-Methoxyphenylacetonitrile (10 g, 67.9 mmol) in DMF (50 ml) was added slowly to a suspension of NaH (5.04g, 150mmol) in DMF (50ml) at 0°C under N₂. The reaction was allowed to warm up to room temperature and stirred for 30 min. The reaction was cooled to 0°C and bis(2-chloroethyl)ether (1 0.7g, 74.7mmol) in DMF (100ml) was added dropwise over 80 min. The reaction was allowed to warm up to room temperature and stirred for 1 hour. The reaction was quenched with water (200ml) and extracted with ethyl acetate (3 x 300ml). The combined organic extracts were washed with water (2 x 200ml), brine (200ml), dried over MgSO₄, filtered, washed with ethyl acetate and concentrated *in vacuo* to give the title compound (17.2g, 100%th).
¹H NMR (400MHz, DMSO-*d*₆) δ7.45 (d, 2H), 7.00 (d, 2H), 4.03-3.96 (m, 2H), 3.77 (s, 3H), 3.65 (td, 2H), 2.12-1.94 (m, 4H).

### Intermediate 2: 4-(4-Hydroxy-phenyl)-tetrahydro-pyran-4-carbonitrile

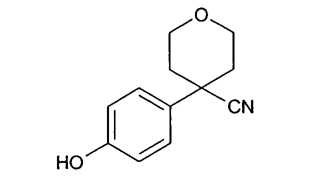

BBr₃ (1M in DCM, 262ml) was added to a solution of 4-(4-methoxyphenyl)-tetrahydro-2H-pyran-4-carbonitrile (14.7g, 67.9mmol) in DCM (294ml) at 0°C under N₂ keeping the temperature below 5°C. The reaction was allowed to warm to room temperature and stirred for 48hours. The mixture was cooled to -10-0°C with dry ice acetone and quenched with sat bicarbonate (294ml). The mixture was allowed to warm to room temperature and stirred for 1 hour. The mixture was separated and the aqueous extracted with DCM (3 x 250ml). The combined organic extracts were dried over MgSO₄, filtered, washed with DCM and concentrated *in vacuo* to give the title compound (10.8g, 78%th).
¹H NMR (400MHz, DMSO-*d*₆) δ9.62 (br s, 1H), 7.34 (d, 2H), 6.83 (d, 2H), 4.06-3.94 (m, 2H), 3.65 (td, 2H), 2.12-1.92 (m, 4H).

### Intermediate 3: 4-(4-Dimethylaminomethyl-tetrahydro-pyran-4-yl)-phenol

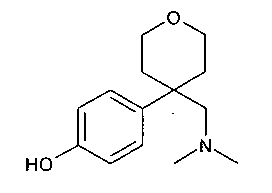

### Step 1:

To a stirred suspension of LiAlH₄ (79g, 2.08mol, 5eq) in THF (900ml) at 0 to 5°C under an atmosphere of nitrogen was added 4-(4-methoxyphenyl)- tetrahydro-2H-pyran-4-carbonitrile (90g, 0.414mol) in THF (900ml) over a 25 minutes maintaining the temperature at 5 to 10°C. The reaction mixture was allowed to warm to ambient temperature and stirred until complete. Sodium hydroxide (2N, 850ml) was added dropwise, the resulting solids filtered and washed with THF (2x800ml), the organics concentrated *in vacuo* at 40°C. The residue was dissolved in EtOAc (300ml) and dried over MgSO₄, filtered and concentrated *in vacuo* at 40°C to provide {[4-(4-methoxyphenyl)- tetrahydro-2H-pyran-4-yl]methyl}amine as a light yellow oil (92g, quantitative).
¹H NMR (400MHz, CDCl₃) δ7.21 (d, 2H), 6.92 (d, 2H), 3.82 (s, 3H), 3.85-3.74 (m, 2H), 3.59-3.49 (m, 2H), 2.76 (s, 2H), 2.17-2.07 (m, 2H), 1.81 (ddd, 2H).

### Step 2:

To a solution of {[4-(4-methoxyphenyl)- tetrahydro-2H-pyran-4-yl]methyl}amine (60g, 0.271mol) in H₂O (444mL) and AcOH (213ml) was charged formaldehyde (37%ww solution in H₂O, 408 mL) and the mixture cooled to 0 to 5 °C. Sodium triacetoxyborohydride (345g, 1.626mol, 6eq) was added portion wise while maintaining the temperature below 12°C. The mixture was stirred at ambient temperature until complete. Sodium hydroxide (2M, 1000ml) was added slowly and the mixture extracted with DCM (4x250ml). The organic extracts were dried over MgSO₄, filtered and concentrated *in vacuo* at 30°C to provide {[4-(4-methoxyphenyl)- tetrahydro-2H-pyran-4-yl]methyl}dimethylamine as a yellow oil (53.5g, 80%th).
¹H NMR (400MHz, CDCl₃) δ7.23 (d, 2H), 6.88 (d, 2H), 3.81 (s, 3H), 3.75 (dt, 2H), 3.55 (td, 2H), 2.40 (s, 2H), 2.16-2.05 (m, 2H), 1.97 (s, 6H), 1.89 (ddd, 2H).

### Step 3:

To a suspension of NaSMe (49.2g, 0.702mol, 5eq) in DMF (140ml) was added {[4-(4methoxyphenyl)- tetrahydro-2H-pyran-4-yl]methyl}dimethylamine (35g, 0.140mol) and the resulting mixture was heated to 130°C. Once complete, allowed to cool to ambient temperature and saturated aqueous NH₄Cl (525ml) was added. The resultant was extracted with EtOAc (3x500ml), dried over MgSO₄, filtered and concentrated in vacuo at 35°C to provide 4-(4-Dimethylaminomethyl-tetrahydro-pyran-4-yl)-phenol (intermediate 3) as a yellow solid (35.7g, 108%th) containing residual DMF and EtOAc.
¹H NMR (400MHz, CDCl₃) δ7.14 (d, 2H), 6.72 (d, 2H), 3.77 (dt, 2H), 3.55 (td, 2H), 2.45 (s, 2H), 2.15-2.07 (m, 2H), 2.01 (s, 6H), 1.86 (ddd, 2H).

### Intermediate 4: 3-{4-[(dimethylamino)methyl]tetrahydro-pyran-4-yl}phenol

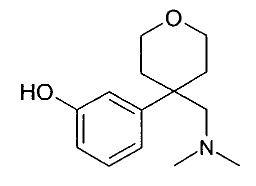

### Step 1:

3-Methoxyphenylacetonitrile (10g, 67.9mmol) in DMF (50ml) was added slowly to a suspension of NaH (5.04g, 150mmol) in DMF (50ml) at 0°C under N₂. The reaction was allowed to warm to room temperature and stirred for 30mns. The reaction was cooled to 0°C and bis(2-chloroethyl)ether (10.7g, 74.7mmol) in DMF (100ml) was added dropwise over 80 min. The reaction was allowed to warm to room temperature and stirred for 1 hour. The reaction was quenched with water (500ml) and extracted with ethyl acetate (3 x 100ml). The combined organic extracts were washed with brine (3 x 50ml), dried over MgSO₄, filtered, washed with ethyl acetate and concentrated *in vacuo*. The crude mixture was purified by column chromatography, eluting with heptane increasing the polarity to heptane:ethyl acetate (90 :10), to give 4-(3-methoxyphenyl)tetrahydro-pyran-4-carbonitrile (4.1 g, 28%th).
¹H NMR (400MHz, CDCl₃) δ7.34 (t, 1 H), 7.07 (ddd, 1 H), 7.03 (t, 1 H), 6.88 (ddd, 1 H), 4.11-4.04 (m, 2H), 3.90 (td, 2H), 3.83 (s, 3H), 2.18-2.00 (m, 4H).

### Step 2:

To a stirred suspension of LiAlH₄ (3.7 g, 96.7 mmol) in diethyl ether (100 mL) cooled at 0°C was added dropwise a solution of 4-(3-methoxyphenyl)-tetrahydropyran-4-carbonitrile (4.2 g, 19.3 mmol) in diethyl ether (100 mL). After being stirred at room temperature for 30 min, the reaction mixture was refluxed for another 15 min. The mixture was cooled to 10°C and a solution of water, sodium hydroxide (15% w/v, 0.48 mL) and water again were successively added dropwise. After being stirred for 15 min at room temperature, the mixture was filtrated over diatomaceous earth and concentrated. The residue was purified with flash chromatography (CH₂Cl₂/MeOH: 95/5) to provide [4-(3-methoxyphenyl)tetrahydro-pyran-4-yl]methylamine (4 g, 94%).
¹H NMR (CDCl₃) δ0.95 (br s, 2H), 1.80 (m, 2H), 2.15 (m, 2H), 2.80 (s, 2H), 3.55 (m, 2H), 3.75 (m, 2H), 3.85 (s, 3H), 6.80 (d, 1H), 6.85 (s, 1H), 6.90 (d, 1 H), 7.30 (t, 1H).

### Step 3:

A mixture of [4-(3-methoxyphenyl)tetrahydro-pyran-4-yl]methylamine (0.51 g, 2.3 mmol), acetic acid (0.535 mL, 9.2 mmol), sodium triacetoxyborohydride (0.974 g, 4.6 mmol), 37% solution of formaldehyde in H₂O (0.55 mL) and CH₂Cl₂ (40 mL) was stirred 72 h at ambient temperature. The organic phase was washed twice with water. Concentrated NaOH was added to the aqueous phase (pH 12) and the mixture was extracted twice with dichloromethane. The organic extracts were dried over Na₂SO₄ , filtered and concentrated *in vacuo* to provide N-{[4-(3-methoxyphenyl)tetrahydro-pyran-4-yl]methyl}-N,N-dimethylamine as an oil (0.35 g, 61 %).
¹H NMR (400MHz, CDCl₃) δ7.28-7.20 (m,1H); 6.95-6.90 (m,1H); 6.88 (s,1H); 6.70 (d,1H); 3.8 (s,3H); 3.8-3.70 (m,2H); 3.60-3.55 (m,2H); 2.40 (s,2H); 2.15-2.08 (m,2H); 2.0 (s,2H); 2.0-1.88 (m,2H).

### Step 4:

To a suspension of NaSMe (0.42 g, 6 mmol) in DMF (1.2 mL) was added N-{[4-(3-methoxyphenyl)tetrahydro-pyran-4-yl]methyl}-N,N-dimethylamine (0.3 g, 1.2 mmol) and the resulting mixture was heated to 65°C for 7 h, allowed to cool to ambient temperature and quenched with saturated aqueous NH₄Cl (6 mL). The resultant was extracted with DCM, dried over Na₂SO₄, filtered and concentrated *in vacuo* to provide 0.25 g of crude product. A mixture of the curde and HBr (3 mL) was heated to reflux for 1 h. After cooling, water was added to quench the reaction and the mixture was basified with NaHCO₃ and extracted with DCM. The organic extracts were dried over Na₂SO₄, filtered and concentrated. The residue was purified by flash chromatography (CH₂Cl₂ / MeOH (10% NH₄OH)) to provide 3-{4-[(dimethylamino)methyl]tetrahydro-pyran-4-yl}phenol (0.100 g, 35.5%).
¹H NMR (400MHz, CDCl₃) δ7.25-7.20 (m,1H); 6.90 (d,1H); 6.8 (s,1H); 6.70 (d,1H); 5.0 (bs,1H); 3.80-3.70 (m,2H); 3.60-3.40 (m,2H); 2.50 (s,2H); 2.15-2.08 ( m,2H); 2.0 (s,6H); 1.9-1.80 (m,2H).

### General procedure A for the synthesis of chlorides (R-R^{LG} wherein R^{LG} is chloride):

Amine (1eq.) was charged to a reaction flask followed by acetone (3vol or 20vol), 5M NaOH solution (1.2eq.) and 1-bromo-3-chloropropane (1.5 eq. or 3eq.). The reaction was stirred overnight at room temperature. The phases were separated and the acetone layer concentrated *in vacuo.* The aqueous was acidified with 2M HCl solution to pH 1 (~10vol). The concentrate was diluted with TBME (30vol) and washed with water (15vol). The aqueous was extracted with TBME (2 x 15ml). The combined TBME was washed with 2M HCl solution (15ml) and combined with the first acidic phase. The combined acidic layers were washed with TBME (15vol) and basified to pH 14 with 4M NaOH solution (~40ml). The aqueous was extracted with TBME (3 x 30ml). The combined organic layers were dried over MgSO₄, filtered, washed with TBME (5vol) and concentrated *in vacuo* to give the required chloride.

### General procedure B:

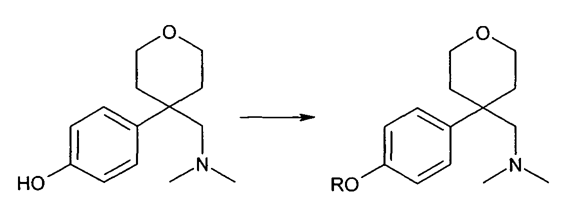

4-(4-Dimethylaminomethyl-tetrahydro-pyran-4-yl)-phenol (500mg, 2.13mmol), chloride (378mg, 2,13mmol), DMF (10ml), water (17ml) and K₂CO₃ (1.18g, 8.52mmol) was heated to 130°C. Cooled to ambient temperature, water (40ml) added, extracted with EtOAc (3x25ml) and the combined organic extracts washed with water (2x25ml). The organics were dried over MgSO₄, filtered and concentrated *in vacuo* at 35°C. Purification of the crude material by chromatography on silica, eluant (4% MeOH, 1% NH₄OH, in DCM) provided the title compounds.

### Intermediate 5: 1-(3-Chloro-propyl)-pyrrolidine

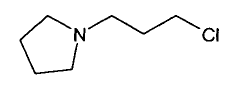

Pyrrolidine (10g, 0.14mol), acetone (28ml), 5M NaOH solution (21ml) and 1-bromo-3-chloropropane (24.4g, 0.15mol) were stirred together under N₂ for 8hours. The organic layer was separated and concentrated *in vacuo*. The crude product was purified by vacuum distillation (b.p. 90°C/30mbar) to give the title compound (11.7g, 57%th) as a colourless oil.
¹H NMR (400MHz, CDCl₃) δ3.67 (t, 2H), 2.49 (t, 2H), 2.46-2.37 (m, 4H), 1.88 (p, 2H), 1.72-1.64 (m, 4H).

### Example 1: Dimethyl-{4-[3-(3-pyrrolidin-1-ylpropoxy)phenyl]tetrahydropyran-4-ylmethyl}amine

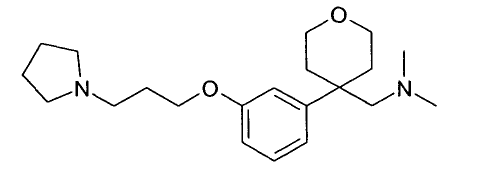

Alkylation of 3-[4-(dimethylamino)methyltetrahydro-2H-pyran-4-yl]phenol (0.1 g, 0.425 mmol) with 1-(3-chloropropyl)pyrrolidine (0.125 g, 0.85 mmol) according to general procedure B gave the desired compound after purification by flash chromatography (0.030 g, 20%).
¹H NMR (400MHz, CDCl₃) δ7.27-7.18 (m,1H), 6.92-6.84(m,2H),6.75 (dd,1H), 4.05 (t,2H), 3.8-3.7 (m, 2H), 3.55 (t, 2H), 2.65 (t, 2H), 2.6 - 2.5 (m, 4H), 2.4(s,2H), 2.15-1.85 (m, 6H), 2 (s,6H), 1.85-1.75 (m, 4H).

### Intermediate 6: 1-(3-Chloro-propyl)-2(R),5(R)-trans-dimethyl-pyrrolidine

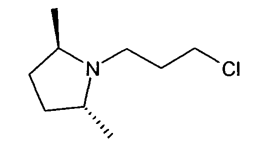

2(R),5(R)-*trans*-Dimethyl-pyrrolidine (0.75g, 7.56mmol), acetone (15ml, 20vol), 5M NaOH solution (1.8ml, 1.2eq.) and 1-bromo-3-chloropropane (3.57g, 22.7mmol, 3eq.) were reacted together according to general procedure A to give the title compound (0.5g, 38%th) as a yellow oil.
¹H NMR (400MHz, CDCl₃) δ3.61 (td, 2H), 3.07-2.97 (m, 2H), 2.72 (dt, 1H), 2.52 (dt, 1 H), 2.05-1.90 (m, 4H), 1.43-1.30 (m, 2H), 0.96 (d, 6H).

### Example 2: (4-{4-[3-(2,5-Dimethylpyrrolidin-1-yl)propoxy]phenyl}tetrahydro-pyran-4-ylmethyl)dimethylamine

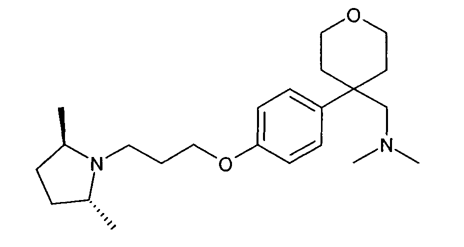

4-(4-Dimethylaminomethyl-tetrahydro-pyran-4-yl)-phenol (330mg, 1.43mmol), 1-(3-chloro-propyl)-2,5-*trans*-dimethyl-pyrrolidine (221 mg, 1.26mmol), DMF (7.6ml) and K₂CO₃ (790mg, 5.72mmol) were reacted together according to general procedure B. The isolated material was subjected to chromatography on silica, eluant 95 :4 :1 (DCM, MeOH, NH₃) to give the title compound as a yellow oil (180mg, 34%th).
¹H NMR (400MHz, CDCl₃) δ7.20 (d, 2H), 6.87 (d, 2H), 4.08-3.95 (m, 2H), 3.79-3.70 (m, 2H), 3.59-3.49 (m, 2H), 3.11-3.00 (m, 2H), 2.77 (m, 1 H), 2.54 (m, 1 H), 2.40 (s, 2H), 2.14-1.81 (m, 8H), 1.96 (s, 6H), 1.44-1.31 (m, 2H), 0.97 (d, 6H).

### Intermediate 7: 1-(3-Chloro-propyl)-2-methyl-pyrrolidine

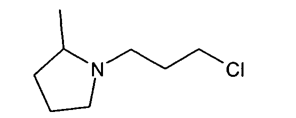

2-Methylpyrrolidine (0.9g, 10.6mmol), acetone (18ml, 20vol), 5M NaOH solution (2.50ml) and 1-bromo-3-chloropropane (5g, 31.8mmol, 3eq.) were reacted together according to general procedure A to give the title compound (1 g, 59%th) as a pale yellow oil.
¹H NMR (400MHz, CDCl₃) δ3.61 (t, 2H), 3.12 (td, 1H), 2.94 (dt, 1H), 2.28 (m, 1 H), 2.19-2.02 (m, 2H), 2.00-1.85 (m, 3H), 1.83-1.61 (m, 2H), 1.40 (m, 1H), 1.08 (d, 3H).

### Example 3: Dimethyl-(4-{4-[3-(2-methyl-pyrrolidin-1-yl)-propoxy]-phenyl}-tetrahydro-pyran-4-ylmethyl)-amine

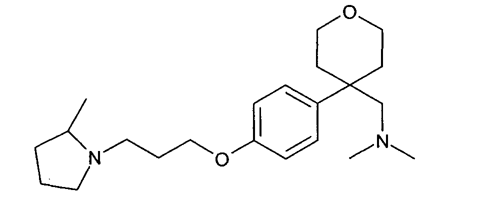

4-(4-Dimethylaminomethyl-tetrahydro-pyran-4-yl)-phenol (500mg, 2.13mmol), 1-(3-chloro-propyl)-2-methyl-pyrrolidine (344mg, 2,13mmol), DMF (10ml) and K₂CO₃ (1.18g, 8.52mmol) were reacted together according to general procedure B. The isolated material was subjected to chromatography on silica, eluant 95:4:1 (DCM, MeOH, NH₃) to give the title compound as a yellow oil (120mg, 16%th).
¹H NMR (400MHz, CDCl₃) δ7.20 (d, 2H), 6.87 (d, 2H), 4.08-3.96 (m, 2H), 3.75 (dt, 2H), 3.55 (td, 2H), 3.18 (td, 1 H), 2.98 (m, 1H), 2.40 (s, 2H), 2.29 (m, 1H), 2.20 (m, 1H), 2.16-1.62 (m, 10H), 1.96 (s, 6H), 1.42 (m, 1 H), 1.09 (d, 3H).

### Intermediate 8: 1-(3-Chloro-propyl)-2,6-cis-dimethyl-piperidine

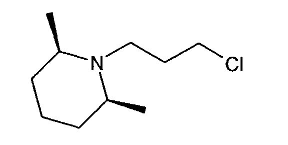

### Step 1:

2,6-*cis*-Dimethyl-piperidine (1.0g, 8.83mmol), K₂CO₃ (1.52g, 1.25eq.) and 3-bromopropanol (6.14g, 44.2mmol, 4vol) were reacted together at 100°C for 2hours. The reaction was allowed to cool to room temperature, diluted with DCM (20ml) and quenched with 2M HCl solution (20ml). The aqueous was extracted with DCM (2 x 20ml) and basified to pH 14 with 2M NaOH solution (~15ml). The aqueous was extracted with DCM (3 x 20ml). The combined DCM layers were dried over MgSO₄, filtered washed with DCM and concentrated *in vacuo* to give 1-(propan-1'-ol)-2,6-*cis*-dimethyl-piperidine (1.22g, 81%th) as a pale yellow oil.
¹H NMR (400MHz, CDCl₃) δ4.91 (br s, 1H), 3.77 (t, 2H), 2.82 (t, 2H), 2.43-2.33 (m, 2H), 1.73-1.50 (m, 5H), 1.38-1.23 (m, 3H), 1.19 (d, 6H).

### Step 2:

1-(Propan-1'-ol)-2,6-*cis*-dimethyl-piperidine (1.22g, 7.12mmol) was dissolved in DCM (24ml) and cooled to 0-5°C under N₂. Thionyl chloride (1.04ml, 14.25mmol) was added dropwise. The reaction was allowed to warm to room temperature and stirred for 30mins. The reaction was quenched with 2M HCl solution (25ml), The aqueous was extracted with DCM (25ml) and basified to pH 14 with 5M NaOH solution (~25ml). The aqueous was extracted with TBME (3 x 25ml). The combined organic layers were dried over MgSO₄, filtered washed with TBME and concentrated *in vacuo* to give the title compound (1.26g, 93%th) as a yellow oil.
¹H NMR (400MHz, CDCl₃) δ3.51 (t, 2H), 2.93-2.85 (m, 2H), 2.47-2.36 (m, 2H), 1.93-1.83 (m, 2H), 1.66 (m, 1H), 1.60-1.52 (m, 2H), 1.33-1.23 (m, 3H), 1.12 (d, 6H).

### Example 4: (4-{4-[3-(2,6-Dimethylpiperidin-1-yl)propoxy]phenyl}tetra-hydropyran-4-ylmethyl)dimethyl-amine

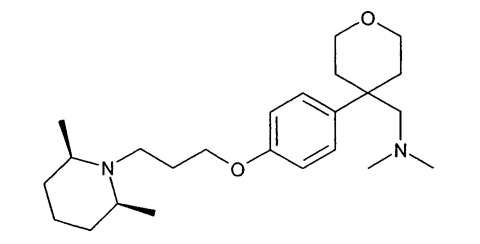

4-(4-Dimethylaminomethyl-tetrahydro-pyran-4-yl)-phenol (500mg, 2.13mmol), 1-(3-chloro-propyl)-2,6-*cis*-dimethyl-piperidine (410mg, 2,13mmol), DMF (10ml) and K₂CO₃ (1.18g, 8.52mmol) were reacted together according to general procedure B. The isolated material was subjected to chromatography on silica, eluant 95 :4 :1 (DCM, MeOH, NH₃) to give the title compound as a yellow oil (280mg, 33.9%th).
¹H NMR (400MHz, CDCl₃) δ7.20 (d, 2H), 6.85 (d, 2H), 3.93 (t, 2H), 3.75 (dt, 2H), 3.55 (td, 2H), 2.95 (t, 2H), 2.51-2.36 (m, 2H), 2.40 (s, 2H), 2.14-2.04 (m, 2H), 1.96 (s, 6H), 1.93-1.82 (m, 4H), 1.74-1.51 (m, 3H), 1.41-1.21 (m, 3H), 1.13 (d, 6H).

### Intermediate 9: 4-(3-Chloro-propyl)-thiomorpholine

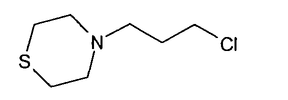

Thiomorpholine (5g, 49mmol), acetone (15ml, 3vol), 5M NaOH solution (11.8ml) and 1-bromo-3-chloropropane (11.6g, 73.5mmol, 1.5eq.) were reacted together according to general procedure A to give the title compound (8.5g, 96%th) as a pale yellow oil.
¹H NMR (400MHz, CDCl₃) δ3.59 (t, 2H), 2.74-2.64 (m, 8H), 2.50 (t, 2H), 1.92 (p, 2H).

### Example 5: Dimethyl-{4-[4-(3-thiomorpholin-4-ylpropoxy)phenyl]tetra-hydropyran-4-ylmethyl}amine

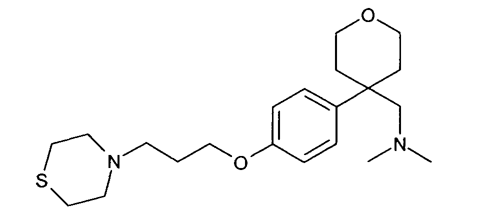

4-(4-Dimethylaminomethyl-tetrahydro-pyran-4-yl)-phenol (1000mg, 4.25mmol), 4-(3-chloro-propyl)-thiomorpholine (764mg, 2,13mmol), DMF (20ml), and K₂CO₃ (2,34g, 17mmol) were reacted together according to general procedure B. The isolated material was subjected to chromatography on silica, eluant DCM :MeOH :NH₃ (95 :5 :1) to give the title compound (320mg, 20%th).
¹H NMR (400MHz, CDCl₃) δ7.20 (d, 2H), 6.86 (d, 2H), 3.99 (t, 2H), 3.75 (dt, 2H), 3.55 (td, 2H), 2.79-2.64 (m, 8H), 2.55 (t, 2H), 2.40 (s, 2H), 2.16-2.04 (m, 2H), 2.01-1.82 (m, 4H), 1.97 (s, 6H).

### Example 6: Dimethyl-(4-{4-[3-(1-oxothiomorpholin-4-yl)propoxylphenyl}-tetrahydropyran-4-ylmethyl)amine

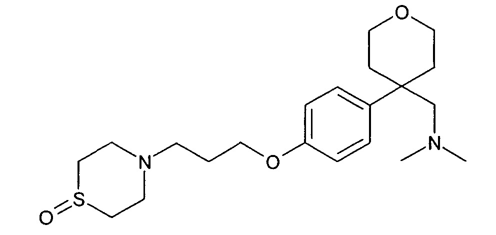

Dimethyl-{4-[4-(3-thiomorpholin-4-ylpropoxy)phenyl]tetrahydro-pyran-4-ylmethyl}amine (300mg, 0.792mmol) and TFA (0.99ml) were cooled to 0 to 5 °C and PTFA (4M, 0.77ml)) [4M solution prepared by the addition of 27.5% H₂O₂ (0.94ml) to TFA (1.56ml)] was added and the reaction stirred for six hours at 0 to 5°C. The mixture was diluted with DCM (6ml), basified with NaOH (2M, 8ml) and extracted with DCM (2x20ml). The DCM extracts were dried over MgSO₄, filtered and concentrated *in vacuo* at 35°C. Purification by chromatography on silica, eluant DCM:MeOH:NH₃ (96:4:1) provided the title compound (200mg, 61%th) as a white solid.
¹H NMR (400MHz, CDCl₃) δ7.21 (d, 2H), 6.86 (d, 2H), 4.01 (t, 2H), 3.75 (dt, 2H), 3.55 (td, 2H), 3.15-3.02 (m, 2H), 2.93-2.79 (m, 4H), 2.72 (dt, 2H), 2.64 (t, 2H), 2.40 (s, 2H), 2.16-1.82 (m, 6H), 1.97 (s, 6H).

### Example 7: (4-{4-[3-(1,1-Dioxo-thiomorpholin-4-yl)propoxy]phenyl}tetrahydro-pyran-4-ylmethyl)dimethyl-amine

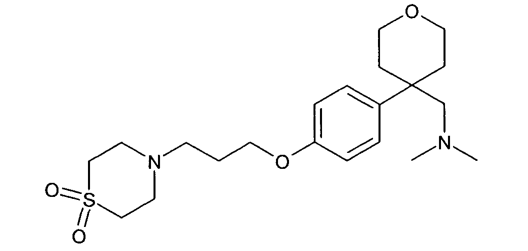

Dimethyl-{4-[4-(3-thiomorpholin-4-ylpropoxy)phenyl]tetrahydro-pyran-4-ylmethyl}amine (300mg, 0.792mmol) and TFA (0.99ml) were cooled to 0 to 5°C and PTFA (4M, 0.77ml) was added and the reaction allowed to warm to ambient temperature overnight. The mixture was diluted with DCM (6ml), basified with NaOH (2M, 8ml) and extracted with DCM .(2x20ml). The DCM extracts were dried over MgSO₄, filtered and concentrated *in vacuo* at 35°C. Purification by chromatography on silica, eluant DCM:MeOH:NH₃ (96:4:1) provided the title compound (151 mg, 46%th) as a white solid.
¹H NMR (400MHz, CDCl₃) δ7.22 (d, 2H), 6.85 (d, 2H), 4.01 (t, 2H), 3.75 (dt, 2H), 3.55 (td, 2H), 3.10-2.97 (m, 8H), 2.72 (t, 2H), 2.40 (s, 2H), 2.14-1.82 (m, 6H), 1.97 (s, 6H).

### Intermediate 10: 1-(3-Chloro-propyl)-piperidin-4-ol

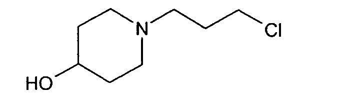

4-Hydroxypiperidine (3g, 30mmol), acetone (60ml, 20vol), 5M NaOH solution (7.2ml) and 1-bromo-3-chloropropane (14.2g, 90mmol, 3eq.) were reacted together according to general procedure A to give the title compound (1.5g, 28%th) as a pale yellow oil.
¹H NMR (400MHz, CDCl₃) δ3.69 (m, 1H), 3.59 (t, 2H), 2.79-2.71 (m, 2H), 2.47 (t, 2H), 2.19-2.09 (m, 2H), 1.98-1.85 (m, 4H), 1.63-1.49 (m, 3H).

### Example 8: 1-{3-[4-(4-Dimethylaminomethyl-tetrahydro-pyran-4-yl)-phenoxy]-propyl}-piperidin-4-ol

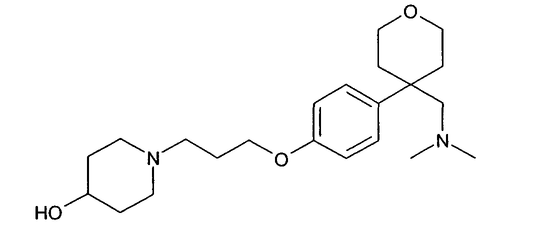

4-(4-Dimethylaminomethyl-tetrahydro-pyran-4-yl)-phenol (500mg, 2.13mmol), 1-(3-chloro-propyl)-piperidin-4-ol (378mg, 2,13mmol), DMF (10ml) and K₂CO₃ (1.18g, 8.52mmol) were reacted together according to general procedure B. The isolated material was dissolved in EtOAc (30ml), washed with NaOH (2M, 2x20ml) and water (25ml), dried over MgSO₄ filtered and concentrated in vacuo at 35°C to give the title compound (411 mg, 51%th).
¹H NMR (400MHz, CDCl₃) δ7.20 (d, 2H), 6.86 (d, 2H), 4.00 (t, 2H), 3.80-3.65 (m, 3H), 3.55 (td, 2H), 2.85-2.73 (m, 2H), 2.52 (t, 2H), 2.40 (s, 2H), 2.22-1.82 (m, 10H), 1.97 (s, 6H), 1.66-1.54 (m, 2H), 1.52 (br s, 1 H).

### Intermediate 11: 1-(3-Chloro-propyl)-4-methoxy-piperidine

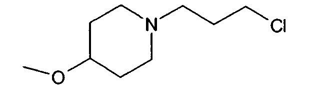

4-Methoxy-piperidine (1.5g, 13mmol), acetone (30ml, 20vol), 5M NaOH solution (3.13ml) and 1-bromo-3-chloropropane (6.14g, 39mmol, 3eq.) were reacted together according to general procedure A to give the title compound (1.5g, 60%th) as a pale yellow oil.
¹H NMR (400MHz, CDCl₃) δ3.58 (t, 2H), 3.33 (s, 3H), 3.20 (m, 1 H), 2.81-2.65 (m, 2H), 2.45 (t, 2H), 2.13 (t, 2H), 2.00-1.82 (m, 4H), 1.67-1.49 (m, 2H).

### Example 9: (4-{4-[3-(4-Methoxy-piperidin-1-yl)-propoxy]-phenyl}-tetrahydropyran-4-ylmethyl)-dimethyl-amine

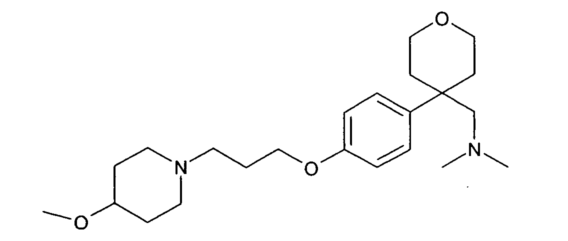

4-(4-Dimethylaminomethyl-tetrahydro-pyran-4-yl)-phenol (500mg, 2.13mmol), 1-(3-chloro-propyl)-4-methoxy-piperidine (410mg, 2,13mmol), DMF (10ml) and K₂CO₃ (1.18g, 8.52mmol) were reacted together according to general procedure B. The isolated material was subjected to chromatography on silica, eluant 95 :4 :1 (DCM: MeOH: NH₃) to give the title compound as a yellow oil (415mg, 50%th).
¹H NMR (400MHz, CDCl₃) δ7.20 (d, 2H), 6.86 (d, 2H), 4.00 (t, 2H), 3.75 (dt, 2H), 3.55 (td, 2H), 3.34 (s, 3H), 3.22 (m, 1 H), 2.83-2.71 (m, 2H), 2.51 (t, 2H), 2.40 (s, 2H), 2.23-1.81 (m, 10H), 1.97 (s, 6H), 1.67-1.53 (m, 2H).

### Intermediate 12: 2-[1-(3-Chloro-propyl)-piperidin-4-yl]-ethanol

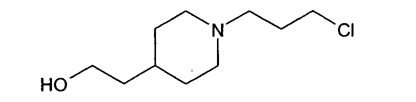

4-Piperidine ethanol (0.9g, 7.5mmol), acetone (18ml, 20vol), 5M NaOH solution (1.8ml) and 1-bromo-3-chloropropane (3.54g, 22.5mmol, 3eq.) were reacted together according to general procedure A to give the title compound (0.6g, 37%th) as an orange oil.
¹H NMR (400MHz, CDCl₃) δ3.75-3.65 (m, 2H), 3.58 (t, 2H), 2.88 (d, 2H), 2.44 (t, 2H), 2.00-1.87 (m, 4H), 1.69 (d, 2H), 1.56-1.36 (m, 3H), 1.33-1.16 (m, 3H).

### Example 10: 2-(1-{3-[4-(4-Dimethylaminomethyltetrahydropyran-4-yl)phenoxy]propyl}piperidin-4-yl)ethanol

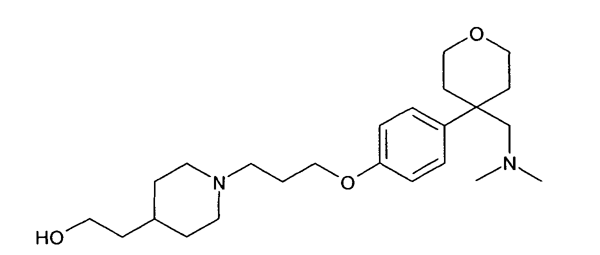

4-(4-Dimethylaminomethyl-tetrahydro-pyran-4-yl)-phenol (288mg, 1.22mmol), 2-[1-(3-chloro-propyl)-piperidin-4-yl]-ethanol (252mg, 1.22mmol), DMF (8ml) and K₂CO₃ (674mg, 4.88mmol) were reacted together according to general procedure B. Purification by chromatography on silica, eluant DCM :MeOH : NH₃ (96 :4 :1) provided the title compound (180mg, 36%th) as an off white solid.
¹H NMR (400MHz, CDCl₃) δ7.20 (d, 2H), 6.86 (d, 2H), 3.99 (t, 2H), 3.75 (dt, 2H), 3.70 (t, 2H), 3.55 (td, 2H), 2.98-2.88 (m, 2H), 2.49 (t, 2H), 2.40 (s, 2H), 2.14-1.82 (m, 6H), 1.97 (s, 6H), 1.75-1.15 (m, 10H).

### Intermediate 13: 1-(3-Chloro-propyl)-4-(2-methoxy-ethyl)-piperidine

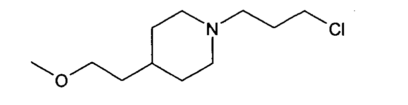

### Step 1:

*N*-Boc-4-(2-hydroxy-ethyl)-piperidine (5g, 21.8mmol) in THF (25ml) was added to a suspension of NaH (1.47g, 43.7mmol) in THF (75ml) at 0°C under N₂. The reaction was stirred for 1hour at 0°C and Mel (2.72ml, 43.7mmol) was added slowly. The reaction was allowed to warm to room temperature and stirred overnight. The reaction was quenched with water (100ml) and extracted with DCM (3 x 100ml). The combined DCM layers were dried over MgSO₄, filtered, washed with DCM and concentrated *in vacuo.* The crude reaction (7.5g) was treated with 3M HCl solution in EtOH (150ml) for 12hours. The reaction was concentrated *in vacuo* and azeo-dried with toluene (2 x 150ml) to give 4-(2-methoxy-ethyl)-piperidine hydrochloride (3.5g, 89%th) as a pale yellow solid.
¹H NMR (400MHz, DMSO *d*_{*6*}) δ9.25 (br s, 1 H), 9.04 (br s, 1H), 3.48 (s, 3H), 3.34 (t, J = 7Hz, 2H), 3.25-3.08 (m, 2H), 2.86-2.69 (m, 2H), 1.81-1.69 (m, 2H), 1.59 (m, 1H), 1.47-1.26 (m, 4H).

### Step 2:

4-(2-Methoxy-ethyl)-piperidine hydrochloride (1.5g, 8.4mmol), acetone (30ml, 20vol), 5M NaOH solution (6.03ml, 3.6eq.) and 1-bromo-3-chloropropane (3.96g, 25.1mmol, 3eq.) were reacted together according to general procedure A to give the title compound (1.6g, 88%th) as a pale yellow oil.
¹H NMR (400MHz, CDCl₃) δ3.58 (t, 2H), 3.41 (t, 2H), 3.32 (s, 3H), 2.92-2.83 (m, 2H), 2.44 (t, 2H), 2.00-1.88 (m, 4H), 1.72-1.63 (m, 2H), 1.55-1.47 (m, 2H), 1.40 (m, 1 H), 1.31-1.18 (m, 2H).

### Example 11: [4-(4-{3-[4-(2-Methoxy-ethyl)-piperidin-1-yl]-propoxy}-phenyl)-tetrahydro-pyran-4-ylmethyl]-dimethyl-amine

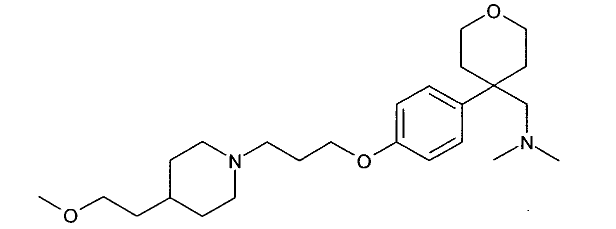

4-(4-Dimethylaminomethyl-tetrahydro-pyran-4-yl)-phenol (500mg, 2.13mmol), 1-(3-chloro-propyl)-4-(2-methoxy-ethyl)-piperidine (466mg, 2,13mmol), DMF (10ml) and K₂CO₃ (1.18g, 8.52mmol) were reacted together according to general procedure B. Purification by chromatography on silica, eluant DCM :MeOH, NH₃ (96 :4 :1) provided the title compound (409mg, 46%th) as a yellow oil.
¹H NMR (400MHz, CDCl₃) δ7.20 (d, 2H), 6.86 (d, 2H), 3.99 (t, 2H), 3.75 (dt, 2H), 3.55 (td, 2H), 3.41 (t, 2H), 3.33 (s, 3H), 2.97-2.86 (m, 2H), 2.49 (t, 2H), 2.40 (s, 2H), 2.15-1.78 (m, 8H), 1.97 (s, 6H), 1.74-1.17 (m, 7H).

### Intermediate 14: 1-(3-Chloro-propyl)-piperidine-4-carboxylic acid amide

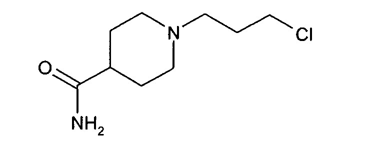

Piperidine-4-carboxylic acid amide (0.5g, 3.90mmol), acetone (1.5ml, 3vol), 5M NaOH solution (1.20ml, 1.2eq.) and 1-bromo-3-chloropropane (0.50ml, 5.04mmol, 1eq.) were reacted together according to general procedure A to give the title compound (0.16g, 20%th) as a pale yellow oil.
¹H NMR (400MHz, CDCl₃) δ5.46 (br s, 1 H), 5.34 (br s, 1 H), 3.59 (t, 2H), 2.98-2.90 (m, 2H), 2.47 (t, 2H), 2.15 (m, 1 H), 2.06-1.85 (m, 6H), 1.75 (ddd, 2H).

### Example 12: 1-{3-[4-(4-Dimethylaminomethyl-tetrahydro-pyran-4-yl)-phenoxy]-propyl}-piperidine-4-carboxylic acid amide

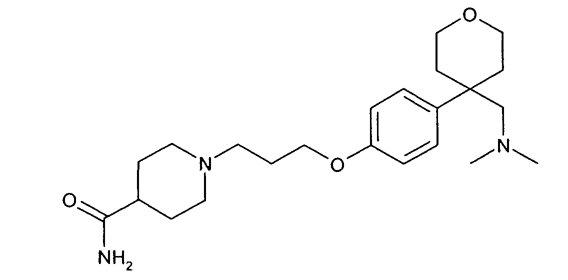

4-(4-Dimethylaminomethyl-tetrahydro-pyran-4-yl)-phenol (500mg, 2.13mmol), 1-(3-chloro-propyl)-piperidine-4-carboxylic acid amide (436mg, 2,13mmol), DMF (10ml) and K₂CO₃ (1.18g, 8.52mmol) were reacted together according to general procedure B. The isolated material was subjected to chromatography on silica, eluant 95 :4 :1 (DCM : MeOH : NH₃) to give the title compound as a white solid (175mg, 20%th).
¹H NMR (400MHz, CDCl₃) δ7.21 (d, 2H), 6.86 (d, 2H), 5.44 (br s, 1H), 5.25 (br s, 1H), 4.00 (t, 2H), 3.75 (dt, 2H), 3.55 (td, 2H), 3.03-2.93 (m, 2H), 2.51 (t, 2H), 2.40 (s, 2H), 2.22-1.66 (m, 13H), 1.97 (s, 6H).

### Intermediate 15: 4-(3-Chloro-propyl)-piperazine-1-carboxylic acid ethyl ester

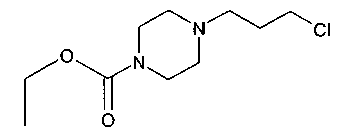

Piperazine-1-carboxylic acid ethyl ester (3.0g, 18.9mmol), K₂CO₃ (2.8g, 1.1eq.), DMF (30ml, 10vol) and 1-bromo-3-chloropropane (1.8ml, 18.9mmol, 1eq.) were reacted together at room temperature overnight. The reaction was quenched with water (100ml) and extracted with DCM (3 x 50ml). The combined DCM extracts were dried over MgSO₄, filtered washed with DCM and concentrated *in vacuo* to give the title compound (3.1 g, 70%th) as a pale yellow oil.
¹H NMR (400MHz, CDCl₃) δ4.06 (q, 2H), 3.53 (t, 2H), 3.40 (t, 4H), 2.42 (t, 2H), 2.32 (t, 4H), 1.87 (p, 2H), 1.18 (t, 3H).

### Example 13: {3-[4-(4-Dimethylaminomethyltetrahydropyran-4-yl)phenoxy]-propyl}piperazine-1-carboxylic acid ethyl ester

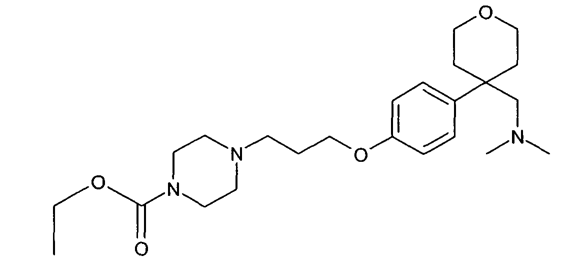

4-(4-Dimethylaminomethyl-tetrahydro-pyran-4-yl)-phenol (500mg, 2.13mmol), 4-(3-chloro-propyl)-piperazine-1-carboxylic acid ethyl ester (499mg, 2,13mmol), DMF (10ml) and K₂CO₃ (1.18g, 8.52mmol) were reacted together according to general procedure B. Purification by chromatography on silica, eluant (10%MeOH in DCM) the title compound (276mg, 26%th).
¹H NMR (400MHz, CDCl₃) δ 7.21 (d, 2H), 6.86 (d, 2H), 4.14 (q, 2H), 4.01 (t, 2H), 3.75 (dt, 2H), 3.60-3.42 (m, 6H), 2.54 (t, 2H), 2.47-2.36 (m, 4H), 2.40 (s, 2H), 2.16-1.82 (m, 6H), 1.97 (s, 6H), 1.26 (t, 3H).

### Intermediate 16 : (3-Chloro-propyl)-diethyl-amine

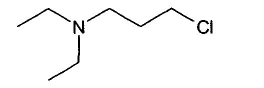

Diethyl-amine (7.27ml, 70mmol), acetone (15ml, 3vol), 5M NaOH solution (16.8ml, 1.2eq.) and 1-bromo-3-chloropropane (16.5g, 105mmol, 1.5eq.) were reacted together according to general procedure A to give the title compound (6.2g, 59%th) as a colourless oil.
¹H NMR (400MHz, CDCl₃) δ3.60 (t, 2H), 2.56 (t, 2H), 2.51 (q, 4H), 1.90 (p, 2H), 1.02 (t, 6H).

### Example 14: {3-[4-(4-Dimethylaminomethyltetrahydropyran-4-yl)phenoxy]-propyl}diethyl-amine

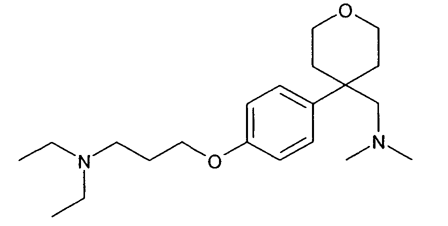

4-(4-Dimethylaminomethyl-tetrahydro-pyran-4-yl)-phenol (500mg, 2.13mmol), (3-chloro-propyl)-diethyl-amine (319mg, 2,13mmol), DMF (10ml) and K₂CO₃ (1.18g, 8.52mmol) were reacted together according to general procedure B. Purification by chromatography on silica, eluant (3%EtOH: 1%NH₄OH in DCM) gave the title compound as a pale yellow clear oil (160mg, 22%th).
¹H NMR (400MHz, CDCl₃) δ7.20 (d, 2H), 6.86 (d, 2H), 4.00 (t, 2H), 3.75 (dt, 2H), 3.55 (td, 2H), 2.61 (t, 2H), 2.54 (q, 4H), 2.40 (s, 2H), 2.15-1.82 (m, 6H), 1.97 (s, 6H), 1.02 (t, 6H).

### Intermediate 17: 2-[(3-Chloro-propyl)-ethyl-amino]-ethanol

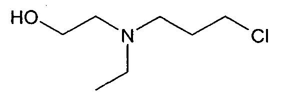

2-Ethylamino ethanol (6.2g, 70mmol), acetone (18ml, 3vol), 5M NaOH solution (16.8ml, 1.2eq.) and 1-bromo-3-chloropropane (16.5g, 105mmol, 1.5eq.) were reacted together according to general procedure A to give the title compound (4.5g, 39%th) as a colourless oil.
¹H NMR (400MHz, CDCl₃) δ3.59 (t, 2H), 3.56 (t, 2H), 2.81 (br s, 1 H), 2.63 (t, 2H), 2.59 (t, 2H), 2.56 (q, 2H), 1.92 (p, 2H), 1.04 (t, 3H).

### Example 15: 2-({3-[4-(4-Dimethylaminomethyltetrahydropyran-4-yl)-phenoxy]propyl}ethylamino)ethanol

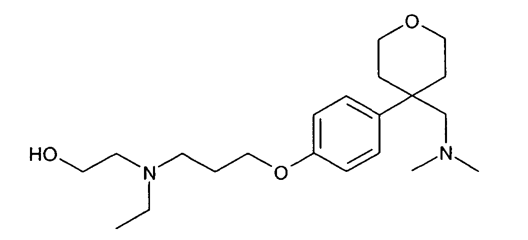

4-(4-Dimethylaminomethyl-tetrahydro-pyran-4-yl)-phenol (500mg, 2.13mmol), 2-[(3-chloro-propyl)-ethyl-amino]-ethanol (353mg, 2,13mmol), DMF (10ml) and K₂CO₃ (1.18g, 8.52mmol) were reacted together according to general procedure B. The isolated material was subjected to chromatography on silica, eluant 95 :4 :1 (DCM: MeOH: NH₃) to give the title compound as a white solid (150mg, 19%th).
¹H NMR (400MHz, CDCl₃) δ7.21 (d, 2H), 6.86 (d, 2H), 4.00 (t, 2H), 3.75 (dt, 2H), 3.60-3.49 (m, 4H), 2.68 (t, 2H), 2.62 (t, 2H), 2.59 (q, 2H), 2.40 (s, 2H), 2.14-1.82 (m, 6H), 1.97 (s, 6H), 1.62 (br s, 1 H), 1.03 (t, 3H).

### Intermediate 18: (3-Chloro-propyl)-isopropyl-methyl-amine

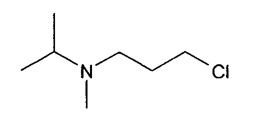

Isopropylmethylamine (4g, 56mmol), acetone (12ml, 3vol), 5M NaOH solution (13.44ml, 1.2eq.) and 1-bromo-3-chloropropane (13.22g, 84mmol, 1.5eq.) were reacted together according to general procedure A to give the title compound (4.8g, 66%th) as a colourless oil.
¹H NMR (400MHz, CDCl₃) δ3.60 (t, 2H), 2.81 (m, 1H), 2.50 (t, 2H), 2.19 (s, 3H), 1.90 (m, 2H), 1.00 (d, 6H).

### Example 16: {3-[4-(4-Dimethylaminomethyltetrahydropyran-4-yl)phenoxy]-propyl} isopropylmethylamine

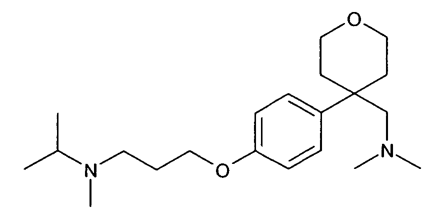

4-(4-Dimethylaminomethyl-tetrahydro-pyran-4-yl)-phenol (500mg, 2.13mmol), (3-chloro-propyl)-isopropyl-methyl-amine (318mg, 2,13mmol), DMF (10ml) and K₂CO₃ (1.18g, 8.52mmol) were reacted together according to general procedure B. Purification by chromatography on silica, eluant DCM :MeOH :NH₃ (96 :3 :1) provided the title compound (200mg, 43%th) as a white solid.
¹H NMR (400MHz, CDCl₃) δ7.20 (d, 2H), 6.87 (d, 2H), 4.00 (t, 2H), 3.75 (dt, 2H), 3.55 (td, 2H), 2.83 (sep, 1 H), 2.55 (t, 2H), 2.40 (s, 2H), 2.22 (s, 3H), 2.14-2.05 (m, 2H), 1.96 (s, 6H), 2.00-1.82 (m, 4H), 1.00 (d, 6H).

### Intermediate 19: 1-(2-chloro-ethyl)-pyrrolidine

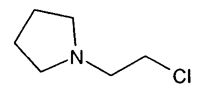

Pyrrolidine (10.4g, 0.15mol), acetone (200ml), 5M NaOH solution (35ml) and 1-bromo-2-chloroethane (62.9g, 0.44mol) were reacted together according to general procedure A. The crude mixture was purified by column chromatography eluting with ethyl acetate to give the title compound (2.0g, 10%th) as a pale yellow oil.
¹H NMR (400MHz, CDCl₃) δ3.59 (t, 2H), 2.83 (t, 2H), 2.61-2.54 (m, 4H), 1.84-1.76 (m, 4H).

### Example 17: Dimethyl-{4-[4-(2-pyrrolidin-1-ylethoxy)phenyl]tetrahydropyran-4-ylmethyl}amine

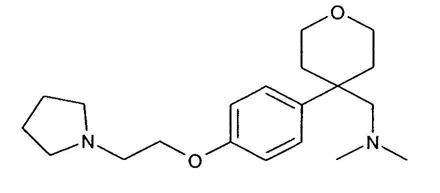

4-(4-Dimethylaminomethyl-tetrahydro-pyran-4-yl)-phenol (389mg, 1.65mmol), 1-(2-chloro-ethyl)-pyrrolidine (210mg, 1.57mmol), DMF (4.5ml) and K₂CO₃ (885g, 6.40mmol) was heated to 130°C for 30 minutes. The mixture was cooled to ambient temperature; water (9ml) was added and the mixture was extracted with EtOAc (3 x 4.5ml). The organic phase was washed with brine (10ml), NaOH solution (2M, 10ml) and water (10ml). The organic layer was dried over MgSO₄, filtered and concentrated *in vacuo* at 35°C and the residue subjected to chromatography on silica eluting with DCM:MeOH:NH₄OH (98:1:1) to give the title compound as a white solid (150mg, 27%).
¹H NMR (400MHz, CDCl₃) δ7.21 (d, 2H), 6.89 (d, 2H), 4.10 (t, 2H), 3.75 (dt, 2H), 3.55 (td, 2H), 2.89 (t, 2H), 2.67-2.58 (m, 4H), 2.40 (s, 2H), 2.13-2.05 (m, 2H), 1.97 (s, 6H), 1.88 (ddd, 2H), 1.84-1.77 (m, 4H).

### Intermediate 20: 1-(3-Chloro-2-methyl-propyl)-pyrrolidine

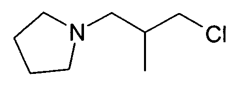

Pyrrolidine (3.0g, 42mmol), acetone (60ml), 5M NaOH solution (10ml) and 1-bromo-3-chloro-2-methyl-propane (21.7g, 0.13mol) were reacted together according to general procedure A to give the title compound (3.9g, 58%th) as a pale yellow oil.
¹H NMR (400MHz, CDCl₃) δ3.65 (dd, 1 H), 3.49 (dd, 1 H), 2.57-2.38 (m, 5H), 2.24 (dd, 1H), 2.02 (m, 1 H), 1.81-1.70 (m, 4H), 1.03 (d, 3H).

### Example 18: Dimethyl-{4-[4-(2-methyl-3-pyrrolidin-1-yl-propoxy)-phenyl]-tetrahydro-pyran-4-ylmethyl}-amine

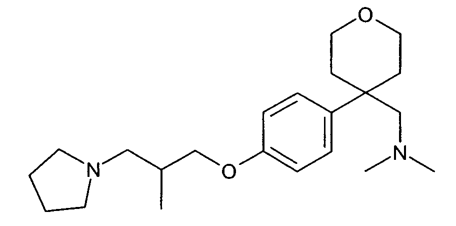

4-(4-Dimethylaminomethyl-tetrahydro-pyran-4-yl)-phenol (0.91g, 3.87mmol), 1-(3-chloro-2-methyl-propyl)-pyrrolidine (500mg, 3.10mmol), DMF (5ml) and K₂CO₃ (2.14g, 15.50mmol) were reacted together according to general procedure B. The organic phase was washed with 2M NaOH (3 x 20ml), water (2 x 20ml), dried over MgSO₄, fitered and concentrated *in vacuo* at 35°C. The crude material was subjected to chromatography on silica eluting with DCM :MeOH :NH₄OH (97 :2 :1) to provide the title compound (464mg, 42%th) as a pale yellow oil.
¹H NMR (400MHz, CDCl₃) δ7.20 (d, 2H), 6.88 (d, 2H), 4.00 (dd, 1H), 3.80-3.70 (m, 3H), 3.55 (td, 2H), 2.59-2.43 (m, 5H), 2.40 (s, 2H), 2.33 (dd, 1 H), 2.21-2.04 (m, 3H), 1.97 (s, 6H), 1.88 (ddd, 2H), 1.82-1.70 (m, 4H), 1.08 (d, 3H).

### Intermediate 21: Methanesulfonic acid 1-isopropyl-piperidin-4-yl ester

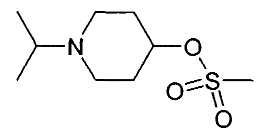

### Step 1:

4-Hydroxypiperidine (2.13g, 21.1mmol), K₂CO₃ (5.83g, 2eq.), 2-bromopropane (11.2g, 91 mmol, 4.3eq.) and MeOH (21.3ml) were refluxed together overnight. The reaction was allowed to cool to room temperature and quenched with 2M HCl solution (40ml) and extracted with TBME (40ml). The aqueous phase was basified to pH 14 with 2M NaOH solution and extracted with DCM (9 x 50ml). The combined organic extracts were dried over MgSO₄, filtered, washed with DCM and concentrated *in vacuo* to give 1-isopropyl-4-hydroxypiperidine (2.41g, 80%th) as a pale yellow oil.
¹H NMR (400MHz, CDCl₃) δ3.67 (m, 1H), 2.85-2.66 (m, 3H), 2.33-2.20 (m, 2H), 1.99-1.38 (m, 5H), 1.04 (d, 6H).

### Step 2:

1-Isopropyl-4-hydroxypiperidine (1g, 6.98mmol), DCM (10ml) and triethylamine (1.08ml, 7.68mmol) were cooled to 0-5°C and mesyl chloride (0.54ml, 6.98mmol) was added dropwise under N₂. The reaction was allowed to warm to room temperature and stirred for 1 hour. The reaction was quenched with sat. NaHCO₃ (20ml) and the aqueous phase was extracted with DCM (2 x 5ml). The combined organic extracts were dried over MgSO₄, filtered, washed with DCM and concentrated *in vacuo* to give the title compound (1.55g, 100%th) as a pale yellow oil.
¹H NMR (400MHz, CDCl₃) δ4.72 (m, 1H), 3.02 (s, 3H), 2.82-2.68 (m, 3H), 2.48-2.30 (m, 2H), 2.14-1.82 (m, 4H), 1.03 (d, 6H).

### Example 19: {4-[4-(1-Isopropylpiperidin-4-yloxy)phenyl]tetrahydropyran-4-ylmethyl}dimethyl-amine

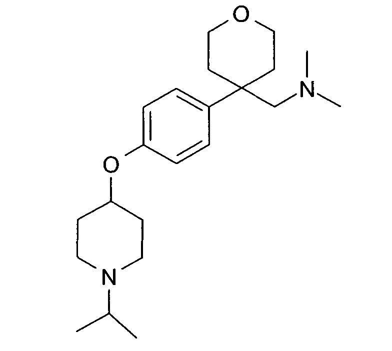

4-(4-Dimethylaminomethyl-tetrahydro-pyran-4-yl)-phenol (532mg, 2.26mmol) in DMF (2ml) was added to a solution of NaH (100mg, 2.5mmol) in DMF (2ml) at room temperature under N₂. The reaction was stirred for 1 hr and a solution of methanesulfonic acid 1-isopropyl-piperidin-4-yl ester (400mg, 1.81 mmol) in DMF (1.3ml) was slowly added. The reaction was heated to 75°C and stirred for 6hrs. The reaction was allowed to cool to room temperature and diluted with TBME (20ml), water (10ml) and 5M NaOH solution (10ml). The organic layer was washed with 2.5M NaOH (2 x 20ml), brine (2 x 20ml), dried over MgSO₄, filtered and concentrated *in vacuo.* The crude reaction was purified by column chromatography, eluting with DCM :MeOH : NH₄OH (98 :1 :1) to give the title compound as a white crystalline solid (113mg, 17%th).
¹H NMR (400MHz, CDCl₃) δ7.20 (d, 2H), 6.87 (d, 2H), 4.27 (m, 1H), 3.75 (dt, 2H), 3.55 (td, 2H), 2.85-2.68 (m, 3H), 2.45-2.32 (m, 2H), 2.40 (s, 2H), 2.14-1.94 (m, 4H), 1.96 (s, 6H), 1.93-1.75 (m, 4H), 1.06 (d, 6H).

### Intermediate 22: Methanesulfonic acid 1-cyclopentyl-piperidin-4-yl ester

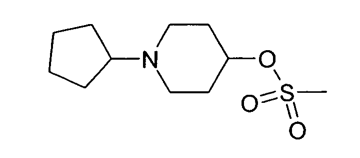

### Step 1:

4-Hydroxypiperidine (2.08g, 21.2mmol), K₂CO₃ (5.86g, 2eq.), cylopentylbromide (11.51 g, 77.6mmol, 3.7eq.) and MeOH (20.8ml) were refluxed together overnight. The reaction was allowed to cool to room temperature and quenched with 2M HCl solution (40ml) and extracted with TBME (40ml). The aqueous phase was basified to pH 14 with a 2M NaOH solution and extracted with DCM (4 x 50ml). The combined organic extracts were dried over MgSO₄, filtered, washed with DCM and concentrated *in vacuo* to give 1-cyclopentyl-4-hydroxypiperidine (2.55g, 71 %th) as a pale yellow oil.
¹H NMR (400MHz, CDCl₃) δ3.69 (m, 1 H), 2.94-2.76 (m, 2H), 2.48 (m, 1 H), 2.23-2.07 (m, 2H), 1.96-1.32 (m, 13H).

### Step 2:

1-Cyclopentyl-4-hydroxypiperidine (1 g, 5.91 mmol), DCM (10ml) and triethylamine (0.91 ml, 6.5mmol) were cooled to 0-5°C and mesyl chloride (0.46ml, 5.91 mmol) was added dropwise under N₂. The reaction was allowed to warm to room temperature and stirred for 1 hour. The reaction was quenched with sat. NaHCO₃ (20ml) and the aqueous phase was extracted with DCM (2 x 5ml). The combined organic extracts were dried over MgSO₄, filtered, washed with DCM and concentrated *in vacuo* to give the title compound (1.38g, 95%th) as a pale yellow oil.
¹H NMR (400MHz, CDCl₃) δ4.74 (m, 1H), 3.02 (s, 3H), 2.88-2.71 (m, 2H), 2.51 (m, 1H), 2.42-2.22 (m, 2H), 2.12-1.29 (m, 12H).

### Example 20: {4-[4-(1-Cyclopentylpiperidin-4-yloxy)phenyl]tetrahydropyran-4-ylmethyl}dimethylamine

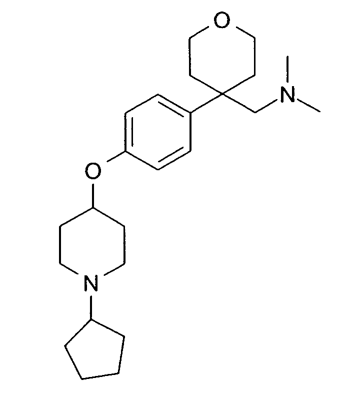

4-(4-Dimethylaminomethyl-tetrahydro-pyran-4-yl)-phenol (487mg, 2.07mmol) in DMF (2ml) was added to a solution of NaH (100mg, 2.5mmol) in DMF (2ml) at room temperature under N₂. The reaction was stirred for 1 hr and a solution of methanesulfonic acid 1-cyclopentyl-piperidin-4-yl ester (409mg, 1.65mmol) in DMF (1.5ml) was slowly added. The reaction was heated to 75°C and stirred for 6hrs. The reaction was allowed to cool to room temperature and diluted with TBME (20ml), water (10ml) and 5M NaOH solution (10ml). The organic layer was washed with 2.5M NaOH (2 x 20ml), brine (2 x 20ml), dried over MgSO₄, filtered and concentrated *in vacuo*. The crude reaction was purified by column chromatography, eluting with DCM :MeOH : NH₄OH (98 :1 :1) to give the title compound as a white solid (78mg, 12%th).
¹H NMR (400MHz, CDCl₃) δ7.20 (d, 2H), 6.87 (d, 2H), 4.28 (m, 1H), 3.75 (dt, 2H), 3.55 (td, 2H), 2.89-2.76 (m, 2H), 2.52 (p, 1 H), 2.40 (s, 2H), 2.37-2.25 (m, 2H), 2.14-1.94 (m, 4H), 1.96 (s, 6H), 1.93-1.77 (m, 6H), 1.76-1.34 (m, 6H).

### Example 21: {3-[4-(4-Dimethylaminomethyl-tetrahydro-pyran-4-yl)-phenoxy]-propyl}-isopropyl-amine

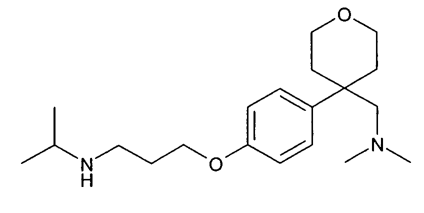

4-(4-Dimethylaminomethyl-tetrahydro-pyran-4-yl)-phenol (730mg, 3.10mmol), 3-chloro-1-bromopropane (980mg, 6.20mmol), DMF (10ml) and K₂CO₃ (1.72g, 12.40mmol) were reacted together according to general procedure B. Purification by chromatography on silica, eluant DCM :MeOH (20 : 1) provided a mixture of bromo and chloro phenoxy ether (250mg) which was used in the next stage. The mixture (250mg) was refluxed with isopropyl amine (34°C) (10ml, 40vol) for 7 days. The reaction was concentrated *in vacuo* and partitioned between 2M NaOH (10ml) and DCM (10ml). The aqueous phase was extracted with DCM (3 x 10mL). The combined DCM layers were dried over MgSO₄, filtered, washed with DCM and concentrated *in vacuo.* Purification by chromatography on silica, eluant DCM :MeOH :NH₃ (95 :5 :0) increasing gradually to DCM :MeOH :NH₃ (90 :9 :1) gave the title compound as a pale yellow solid (180mg, 17%th over the 2 steps).
¹H NMR (400MHz, CDCl₃) δ7.20 (d, 2H), 6.88 (d, 2H), 4.03 (t, 2H), 3.75 (dt, 2H), 3.59-3.50 (m, 2H), 2.89-2.76 (m, 3H), 2.40 (s, 2H), 2.15-2.05 (m, 2H), 2.03-1.93 (m, 2H), 1.96 (s, 6H), 1.88 (ddd, 2H), 1.49 (br s, 1 H), 1.07 (d, 6H).

### Example 22: Dimethyl-{4-[4-(4-pyrrolidin-1-ylbutoxy)phenyl]tetrahydropyran-4-ylmethyl}amine

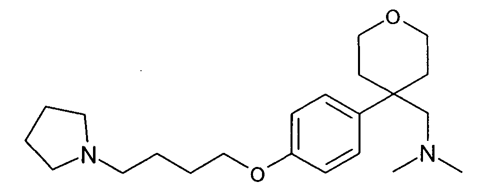

4-(4-Dimethylaminomethyl-tetrahydro-pyran-4-yl)-phenol (1.5g, 6.38mmol), 1-bromo-4-chlorobutane (1.5ml, 12.77mmol), DMF (20ml) and K₂CO₃ (3.5g, 25.53mmol) were reacted together according to general procedure B. Purification of a fraction of the crude (900mg) by chromatography on alumina, eluant ethyl acetate :heptanes (12 : 88) provided a mixture of bromo and chloro phenoxy ether (220mg) which was used in the next stage. The purified mixture (220mg, 0.68mmol) and pyrrolidine (0.17ml, 2.03mmol) were refluxed in EtOH (3ml, 15vol) overnight. The reaction was concentrated *in vacuo* and partitioned between 2M NaOH (10ml) and ethyl acetate (10ml). The aqueous was extracted with ethyl acetate (3 x 5mL). The combined ethyl acetate layers were washed with brine (3 x 5ml), dired over MgSO₄, filtered, washed with ethyl acetate and concentrated *in vacuo.* Purification by chromatography on silica, eluant 95 :3 :2 (DCM: MeOH: NH₃) followed by a second column eluting with DCM :MeOH :NH₃ (96.5 :3 :0.5), gave the title compound as a colourless oil (134mg, 55%th).
¹H NMR (400MHz, CDCl₃) δ7.21 (d, 2H), 6.86 (d, 2H), 3.97 (t, 2H), 3.75 (dt, 2H), 3.59-3.50 (m, 2H), 2.57-2.45 (m, 6H), 2.40 (s, 2H), 2.14-2.05 (m, 2H), 1.96 (s, 6H), 1.92-1.65 (m, 10H).

### General procedure C:

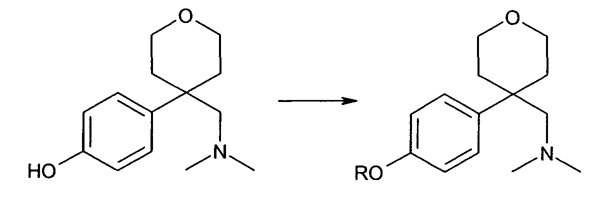

4-(4-Dimethylaminomethyl-tetrahydro-pyran-4-yl)-phenol (1eq.), alcohol (R-OH) (0.8eq.) and PPh₃ (1eq.) were mixed together in THF (10vol) and cooled to 0°C under N₂. DIAD (1eq.) in THF (10vol) was added slowly to the reaction and allowed to warm to room temperature overnight. The reaction was quenched with 2M HCl solution (10vol) and extracted with ethyl acetate (3 x 10vol). The aqueous phase was basified to pH 14 with NaOH (~30vol) and extracted with ethyl acetate (3 x 10vol). The organic phase was dried over MgSO₄, filtered and concentrated *in vacuo* at 35°C. Purification of the crude material by chromatography on silica, eluant (10%MeOH in DCM) provided the title compounds.

### Intermediate 23: 2,2-Dimethyl-3-pyrrolidin-1-yl-propan-1-ol

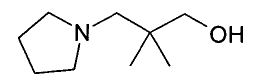

Pyrrolidine (1.82g, 25mmol), K₂CO₃ (3.0g, 1.04eq.), 3-bromo-2,2-dimethyl-propan-1-ol (8.45g, 50mmol, 2eq.) were heated together at 90°C overnight. The reaction was allowed to cool to room temperature and quenched with 2M HCl solution (50ml) and extracted with TBME (3 x 50ml). The aqueous phase was basified to pH 14 with 2M NaOH solution and extracted with DCM (3 x 50ml). The combined organic extracts were dried over MgSO₄, filtered, washed with DCM and concentrated *in vacuo* to give the title compound (1.03g, 26%th) as a pale yellow oil.
¹H NMR (400MHz, CDCl₃), δ3.51 (s, 2H), 2.65 (br s, 4H), 2.57 (s, 2H), 1.80 (m, 1 H), 1.76-1.71 (m, 4H), 0.94 (s, 6H).

### Example 23: {4-[4-(2,2-Dimethyl-3-pyrrolidin-1-yl-propoxy)-phenyl]-tetrahydropyran-4-ylmethyl}-dimethyl-amine

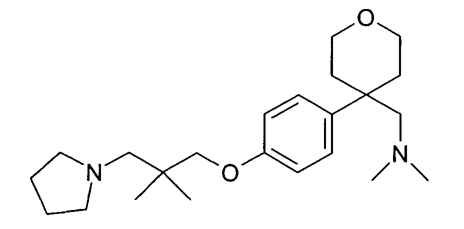

4-(4-Dimethylaminomethyl-tetrahydro-pyran-4-yl)-phenol (0.28g, 1.19mmol), 2,2-dimethyl-3-pyrrolidin-1-yl-propan-1-ol (0.15g, 0.96mmol), PPh₃ (0.31g, 1.19mmol), THF (3ml) and DIAD (0.24ml, 1.19mmol) were reacted together according to general procedure C. The crude material was subjected to chromatography on silica eluting with DCM :MeOH :NH₄OH (97 :2 :1) to provide the title compound (85mg, 24%th) as an off-white solid.
¹H NMR (400MHz, CDCl₃) δ7.23 (d, 2H), 6.91 (d, 2H), 3.84-3.69 (m, 4H), 3.69-3.54 (m, 2H), 2.67-2.56 (m, 4H), 2.50 (s, 2H), 2.44 (s, 2H), 2.18-2.08 (m, 2H), 2.01 (s, 6H), 1.92 (ddd, 2H), 1.79-1.67 (m, 4H), 1.04 (s, 6H).

### Intermediate 24: 4-Pyrrolidin-1-yl-butan-2-ol

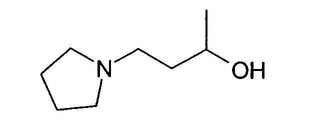

Pyrrolidine (2ml, 24mmol), THF (20ml) and methyl vinylketone (2.5ml, 31 mmol) were mixed together and stirred overnight. NaBH₄ (1.17g, 31mmol) was added to the reaction mixture and stirred for 3hours. The reaction was quenched with water (20ml) and extratcted with TBME (2 x 20ml). The combined organic extracts were dried over MgSO₄, filtered, washed with TBME and concentrated in *vacuo.* The crude product was purified by column chromatography, eluting with DCM DCM :MeOH :NH₃ (94 :5 :1), to give the title compound as a pale yellow oil (1.3g, 38%th).
¹H NMR (400MHz, CDCl₃) δ3.96 (m, 1 H), 2.90 (m, 1 H), 2.71-2.60 (m, 2H), 2.56 (dt, 1H), 2.51-2.40 (m, 2H), 1.82-1.66 (m, 5H), 1.65-1.48 (m, 2H), 1.16 (d, 3H).

### Example 24: Dimethyl-{4-[4-(1-methyl-3-pyrrolidin-1-ylpropoxy)phenyl]-tetrahydropyran-4-ylmethyl}amine

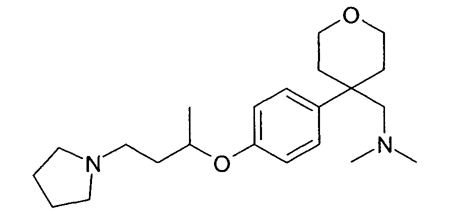

4-(4-Dimethylaminomethyl-tetrahydro-pyran-4-yl)-phenol (1.03g, 4.40mmol), 4-pyrrolidin-1-yl-butan-2-ol (0.5g, 3.50mmol), PPh₃ (1.15g, 4.40mmol), THF (10ml) and DIAD (0.86ml, 4.40mmol) were reacted together according to general procedure C. The crude product was purified by preparative HPLC, eluting with acetonitrile/water/0.1%TFA as a gradient, to give the title compound as a yellow oil (125mg, 10%th).
¹H NMR (400MHz, CDCl₃) δ 7.19 (d, 2H), 6.86 (d, 2H), 4.43 (m, 1H), 3.75 (dt, 2H), 3.60-3.51 (m, 2H), 2.67-2.46 (m, 6H), 2.40 (s, 2H), 2.14-2.04 (m, 2H), 1.97 (s, 6H), 1.93-1.73 (m, 8H), 1.31 (d, 3H).

### Intermediate 25: 3-Pyrrolidin-1-yl-butan-1-ol

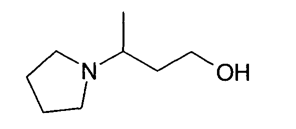

Pyrrolidine (28.15g, 0.4mol), toluene (200ml), catalytic p-TsOH (200mg) and ethyl acetoacetate (20g, 0.15mol) were refluxed together in a Dean-Stark apparatus under N₂ for 3hours. The reaction was cooled to room temperature and concentrated *in vacuo*. NaBH₄ (3.1 g, 82mmol) was dissolved in MeOH (50ml) and cooled to 0-5°C. A portion of the previous crude reaction (5g, 27mmol) in MeOH (25ml) was added to the reaction mixture and stirred for 72hours. The reaction was quenched with an aqueous solution of NaOH (1%w/w, 50ml) and concentrated *in vacuo.* The aqueous phase was extracted with TBME (3 x 50ml). The combined organic extracts were dried over MgSO₄, filtered, washed with TBME and concentrated *in vacuo* to give a mixture of the title compound, 3-pyrrolidin-1-yl-butanoic acid ethyl ester and residual TBME (3.5g). The mixture (3.5g) in THF (20ml) was added to a solution of LiALH₄ (1 M in THF, 33.5ml, 33.5mmol) at 0-5°C under N₂. The reaction was allowed to warm to room temperature and stirred for 3hours. The reaction was quenched with an aqueous solution of NaOH (1%w/w, 50ml) at 0-5°C, filtered and washed with THF (3 x 20ml). The combined organic layers were dried over MgSO₄, filtered, washed with THF and concentrated *in vacuo* to give the title compound as a yellow oil (2g, 73%th).
¹H NMR (400MHz, CDCl₃) δ5.98 (br s, 1 H), 3.90 (ddd, 1 H), 3.77 (ddd, 1 H), 2.90 (m, 1 H), 2.70-2.55 (m, 4H), 1.80-1.56 (m, 6H), 1.09 (d, 3H).

### Example 25: Dimethyl-{4-[4-(3-pyrrolidin-1-yl-butoxy)-phenyl]-tetrahydropyran-4-ylmethyl}-amine

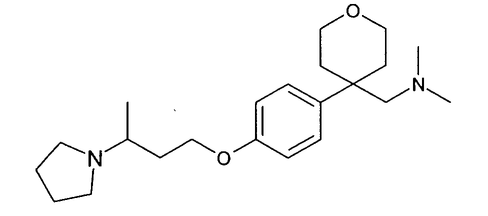

4-(4-Dimethylaminomethyl-tetrahydro-pyran-4-yl)-phenol (925mg, 3.93mmol), 3-pyrrolidin-1-yl-butan-1-ol (450mg, 3.14mmol), PPh₃ (1.03g, 3.93mmol), THF (9ml) and DIAD (0.77ml, 3.93mmol) were reacted together according to general procedure C. The crude material was subjected to chromatography on silica eluting with DCM :MeOH (99.5 :0.5) to provide the title compound (268mg, 24%th) as a colourless oil.
¹H NMR (400MHz, CDCl₃) δ7.21 (d, 2H), 6.88 (d, 2H), 4.11-3.97 (m, 2H), 3.75 (dt, 2H), 3.55 (td, 2H), 2.66-2.52 (m, 5H), 2.40 (s, 2H), 2.19-2.05 (m, 3H), 1.96 (s, 6H), 1.93-1.70 (m, 7H), 1.16 (d, 3H).

### Example 26: Dimethyl-(4-{4-[2-(1-methylpyrrolidin-2-yl)ethoxylphenyl}-tetrahydropyran-4-ylmethyl)amine

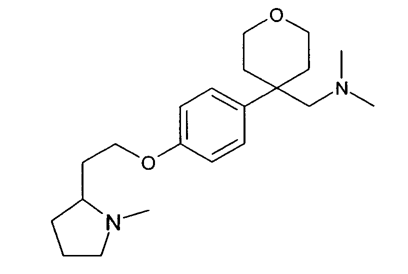

4-(4-Dimethylaminomethyl-tetrahydro-pyran-4-yl)-phenol (405mg, 1.72mmol), 2-(1-methyl-pyrrolidin-2-yl)-ethanol (178mg, 1.38mmol), PPh₃ (451 mg, 1.72mmol), THF (8ml) and DIAD (348mg, 1.72mmol) were reacted together according to general procedure C. The crude product was subjected to chromatography on alumina eluting with ethyl acetate :heptane (50 :50) to provide the title compound (130mg, 27%th) as a colourless oil.
¹H NMR (400MHz, CDCl₃) δ7.21 (d, 2H), 6.87 (d, 2H), 4.09-3.95 (m, 2H), 3.75 (dt, 2H), 3.55 (td, 2H), 3.09 (m, 1 H), 2.40 (s, 2H), 2.35 (s, 3H), 2.30-1.99 (m, 6H), 1.96 (s, 6H), 1.93-1.63 (m, 6H).

### Example 27: 4-[4-(3-Pyrrolidin-1-yl-propoxy)-phenyl]-tetrahydro-pyran-4-carbonitrile

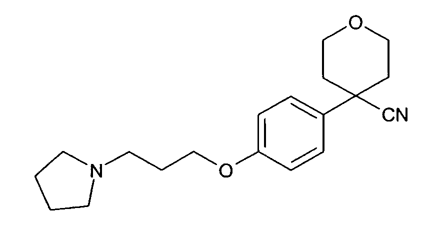

4-(4-Hydroxy-phenyl)-tetrahydro-pyran-4-carbonitrile (2g, 9.86mmol), 1-(3-chloropropyl)-pyrrolidine (2.33g, 15.78mmol), DMF (40ml) and K₂CO₃ (5.45g, 39.44mmol) were reacted together according to general procedure B. The crude reaction product was diluted with 2M HCl solution (200ml) and washed with ethyl acetate (3 x 50ml). The aqueous phase was basified to pH 14 with 2M NaOH (200ml) and extracted with ethyl acetate (4 x 50ml). The combined organic extracts were dried over MgSO₄, filtered, washed with ethyl acetate and concentrated *in vacuo* to give a white solid. The solid was slurried in TBME (15ml) and heptanes (15ml), filtered, washed with heptanes (8ml) and dried on the filter for 2 hours to give the title compound as a white solid (1.6g, 53%th).
¹H NMR (400MHz, DMSO-*d*₆) δ7.43 (d, 2H), 6.99 (d, 2H), 4.08-3.94 (m, 4H), 3.64 (dt, 2H), 2.57-2.47 (m, 2H), 2.46-2.36 (m, 4H), 2.12-1.94 (m, 4H), 1.87 (p, 2H), 1.73-1.62 (m, 4H).

Alternatively, 4-[4-(3-Pyrrolidin-1-yl-propoxy)-phenyl]-tetrahydro-pyran-4-carbonitrile can be prepared by the following steps:

### Step 1:

4-Hydroxybenzylcyanide (20 g, 0.15 mol) was dissolved in DMF (500 mL) and then K₂CO₃ (41.4 g, 0.3 mol) and Nal (7.8 g, 0.052 mol) were added followed by dropwise addition of 3-(1-pyrrolidine)-propylchloride (26.5 g, 0.18 mol) at 60 °C. Heating was continued at 90 °C for 3 h and then the mixture was heated overnight at 60 °C. 3-(1-Pyrrolidine)-propylchloride (10 g, 0.067 mol) was again added as the reaction was not complete and heating was continued for a further 6 h. The reaction mixture was concentrated and taken up in EtOAc. The organic layer was washed with water, finally with brine. Crude [4-(3-pyrrolidin-1-ylpropoxy)phenyl]acetonitrile was purified by DCM/MeOH 9.5/0.5 over a silica gel column. Yield: 25.5g (70 %).
¹ H-NMR (400 MHz, CDCl₃) δ1.77 (m, 4H); 1.98 (m, 2H); 2.50 (m, 4H); 2.59 (t, 2H); 3.66 (s, 2H); 4.00 (t, 2H); 6.87(d, 2H); 7.19(d, 2H).

### Step 2:

NaH (14.6 g, 0.61 mol) was suspended in DMF (100 mL). [4-(3-Pyrrolidin-1-ylpropoxy)phenyl]acetonitrile (25.5 g, 0.10 mol) was dissolved in DMF (100 mL) and added dropwise to the ice-cooled suspension of NaH. The reaction mixture was stirred at room temperature for 30 min followed by dropwise addition of 2-bromoethylether (36.2 g, 0.16 mol) in DMF (200 mL) in ice-cold condition. The reaction mixture was poured into ice-cold water. The solid was filtered, washed with water, dried and the resulting solid was washed with 5 % EtOAc in hexane to give the title compound (22 g, 67 %).

### Example 28: {4-[4-(3-Pyrrolidin-1-ylpropoxy)phenyl]tetrahydropyran-4-yl} methylamine dihydrochloride

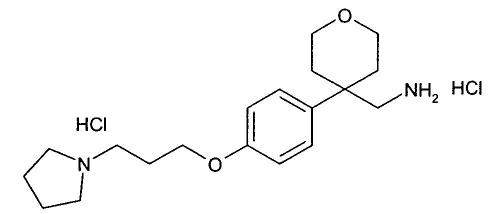

To a stirred suspension of LiAlH₄ (1.81g, 47.7mmol, 5eq) in Et₂O/DCM (1:1, 30mL) at 0 to 5°C under an nitrogen atmosphere was added 4-[4-(3-pyrrolidin-1-yl-propoxy)-phenyl]-tetrahydro-pyran-4-carbonitrile (3g, 9.55 mmol) in Et₂O/DCM (1:1, 30mL) over 15 minutes maintaining the temperature at 5 to 10°C. The reaction mixture was allowed to warm up to ambient temperature and stirred until complete. Sodium hydroxide (2N, 15mL) was added dropwise and the resulting solids were filtered, washed with Et₂O/DCM (1:1, 6 x 50mL) and concentrated *in vacuo* at 40°C. A portion of the crude product from the above reaction (0.5 g, 1.57 mmol) was dissolved in dioxane (5mL) and 4M HCl in dioxane (1.18mL) added under N₂. The reaction was stirred for 3 hours and filtered under a blanket of N₂ (hygroscopic), washed with TBME and dried in the oven at 40°C to give the title compound as a white solid (0.5g, 81%th).
¹H NMR (400MHz, DMSO-*d*₆) δ11.24 (br s, 1 H), 8.00 (br s, 3H), 7.33 (d, 2H), 6.98 (d, 2H), 4.08 (t, 2H), 3.76-3.63 (m, 2H), 3.60-3.46 (m, 2H), 3.45-3.18 (m, 4H), 3.07-2.87 (m, 4H), 2.25-1.77 (m, 10H).

### Example 29: Dimethyl-{4-[4-(3-pyrrolidin-1-ylpropoxy)phenyl]-tetrahydropyran-4-ylmethyl}amine

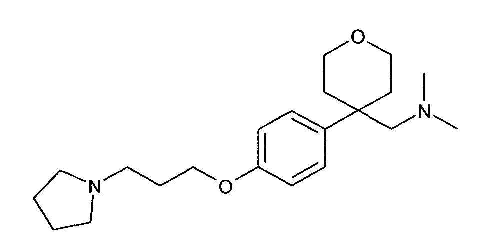

To a mixture of {4-[4-(3-Pyrrolidin-1-ylpropoxy)phenyl]tetrahydropyran-4-yl} methylamine (2.5g, 7.86 mmol), acetic acid (6ml), and water (12.5ml) was added formaldehyde (11.5ml) and stirred for 15 minutes. STAB (10g, 4.72mmol) was added portionwise and stirred for one hour. Sodium hydroxide (2M, 100ml) was added slowly to attain a pH of 9. The resulting mixture was extracted with DCM (3x100ml) and the combined organic phases were dried over MgSO₄, filtered and concentrated *in vacuo* at 35°C. The crude mixture was purified by column chromatography on silica eluting with DCM:MeOH:NH₄OH (95:4:1) to give the title compound as a white solid (1.09g, 40%th).
¹H NMR (400MHz, CDCl₃) δ7.21 (d, 2H), 6.87 (d, 2H), 4.02 (t, 2H), 3.75 (dt, 2H), 3.55 (td, 2H), 2.65 (t, 2H), 2.56 (br s, 4H), 2.40 (s, 2H), 2.15-1.74 (m, 10H), 1.97 (s, 6H).

### Example 30: 4-[4-(3-Piperidin-1-yl-propoxy)-phenyl]-tetrahydro-pyran-4-carbonitrile

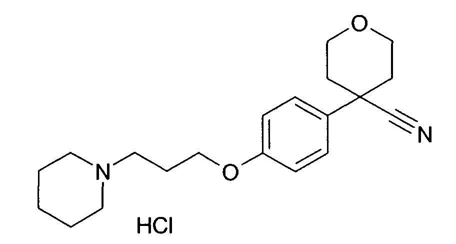

A mixture of 4-(4-hydroxyphenyl)tetrahydro-2H-pyran-4-carbonitrile ( 1.0 g, 5 mmol), 1-(3-chloropropyl)piperidine ( 1.2 g, 7.5 mmol) obtained following the procedure described in the *Patent DD 60557,* cesium carbonate ( 2.4g, 7.5 mmol), KI (1.2 g, 7.5 mmol) in DMF (50 mL) was heated to 70°C overnight. After DMF removal under reduced pressure, the residue was quenched with water and extracted with DCM. The organic phases were dried over Na₂SO₄, filtered and concentrated. The residue was purified by flash chromatography (50 g silica gel, CH₂Cl₂ / MeOH ) to provide the title compound (1.45 g, 88%).
The hydrochloride salt as analytical sample was prepared with ether/HCl 2M in dichloromethane and crystallization in ether.
¹H NMR (500MHz, CDCl₃) δ12.14 (bs, 1H), 7.32 (d, 2H), 6.83(d,2H), 4.07 -3.97 (m, 4H), 3.85 -3.78 (m, 2H), 3.57-3.48 (m, 2H), 3.14-3.07 (m, 2H), 2.67 - 2.56 (m, 2H), 2.45 - 2.36 (m, 2H), 2.32 - 2.19 (m, 2H), 2.06 - 1.92 (m, 4H), 1.90-1.75 (m, 4H), 1.43-1.32 (m, 1H).

### Example 31: {4-[4-(3-Piperidin-1-ylpropoxy)phenyl]tetrahydropyran-4-yl} methylamine dihydrochloride

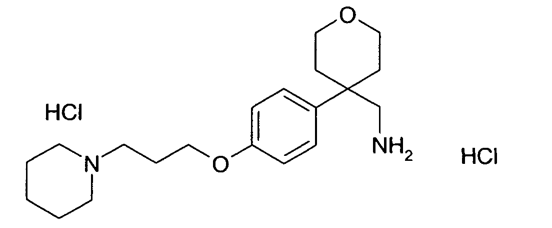

A mixture of 4-[4-(3-piperidin-1-yl-propoxy)-phenyl]-tetrahydro-pyran-4-carbonitrile (1.45 g, 4.4 mmol), isopropanol (12 mL), HCl conc. (350µL), and PtO₂ (115 mg) was stirred and hydrogenated to 50°C overnight at atmospheric pressure. The mixture was filtrated over diatomaceous earth and concentrated. The residue was dissolved in DCM /MeOH mixture and washed with NaOH (1M). The organic layers were dried over Na₂SO₄ , filtered and concentrated, purified by flash chromatography (10 g silica gel, CH₂Cl₂/ MeOH (10% NH₄OH)) to provide the title compound as free base (0.650 g, 44.5%).
¹H NMR (400MHz, DMSO-D6) δ10.66 (bs, 1H), 7.74 (bs, 3H ), 7.32 (d, 2H), 6.97 (d, 2H), 4.07 (t, 2H), 3.73-3.65 (m, 2H), 3.47-3.28 (m, 4H), 3.18-3.10 (m, 2H), 2.98-2.81 (m, 4H), 2.25-2.16 (m, 2H), 2.14-2.06 (m, 2H), 1.90-1.67 (m, 7H),1.44-1.31 (m, 1 H).

### Example 32: Dimethyl-{4-[4-(3-piperidin-1-ylpropoxy)phenyl]tetrahydropyran-4-ylmethyl}amine dihydrochloride

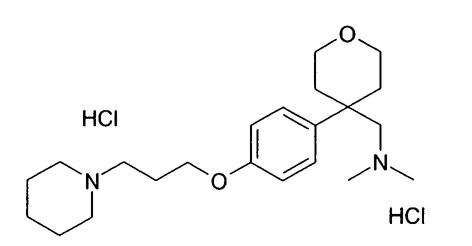

To (0.50 g, 1.5 mmol) of {4-[4-(3-Piperidin-1-ylpropoxy)phenyl]tetrahydropyran-4-yl} methylamine dihydrochloride was added 1.5 mL of formaldehyde (37% w/w solution in H₂O). 1.9 mL of formic acid was added and the mixture was heated to 100°C for 1.5 h. After cooling, H₂O was added and the mixture extracted with ether. NaOH was added to the aqueous phase and the mixture extracted 3 times with ether. The organic extracts were washed with brine, dried over Na₂SO₄ filtered and concentrated *in vacuo*. The residue was purified by flash chromatography (25 g silica gel, CH₂Cl₂/ MeOH (10% NH₄OH ).
¹H NMR (400MHz, DMSO-D6) δ10.63 (bs, 1H), 9.73 (bs, 1H), 7.43 (d, 2H), 6.99(d,2H), 4.11-4.03 (t, 2H), 3.76-3.67 (m, 2H), 3.47-3.29 (m, 7H), 3.19-3.11 (m, 2H), 2.92-2.81 (m, 2H), 2.41-2.35 (m, 5H), 2.32-2.16 (m, 4H), 1.95-1.66 (m, 7H), 1.45-1.32 (m, 1H).

### Intermediate 26: 1-(4-hydroxyphenyl)cyclohexanecarbonitrile

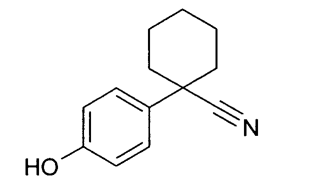

To a solution of 1-(4-methoxyphenyl)cyclohexanecarbonitrile (2 g, 9.29 mmol) in DCM (40 mL) at 0°C under N₂ was added dropwise BBr₃ (2.63 mL, 27.87 mmol). The reaction was allowed to warm to room temperature and stirred for 48hours. The mixture was stirred for 3 hours at 0°C and quenched with water (30 mL). The mixture was separated and the organic layer was washed with a saturated aqueous solution of NaHCO₃ then water. The combined organic extracts were dried over Na₂SO₄, filtered, concentrated *in vacuo* and purified by colomn chromatography on silica gel eluting with DCM:acetone (97:3) to give the title compound (1.71 g, 91%).

### Example 33: 1-[4-(3-pyrrolidin-1-ylpropoxy)phenyl]cyclohexanecarbonitrile

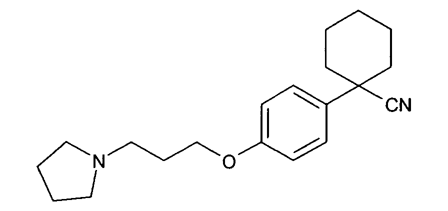

1-(4-hydroxyphenyl)cyclohexanecarbonitrile (1.70 g, 8.45 mmol), 1-(3-chloropropyl)-pyrrolidine (1.87 g, 12.67 mmol), acetone (50 mL), sodium iodide (0.444 g, 2.96 mmol) and K₂CO₃ (5.45g, 39.44 mmol) were heated to 50°C overnight. The solvent was evaporated and the crude product was taken into DCM, washed with 0.5N NaOH , extracted with 0.5N HCl solution and washed with DCM. The aqueous layer was basified with 0.5N NaOH and extracted with DCM. The combined organic extracts were dried over Na₂SO₄, filtered, washed with DCM and concentrated *in vacuo* to give a yellowish solid. The solid was slurried in ether, filtered, washed with ether to give the title compound as a white solid (1.34 g, 51%).
¹H NMR (400MHz, CDCl₃) δ7.30 (d, 2H), 6.83 (d, 2H), 3.95 (t, 2H), 2.54 (t, 2H), 2.44 (m, 4H), 2.06 (m, 2H), 1.92 (m, 2H), 1.84-1.58 (m, 12H).

### Example 34: N-({1-[4-(3-pyrrolidin-1-ylpropoxy)phenyl]cyclohexyl}methyl)-N,N-dimethylamine

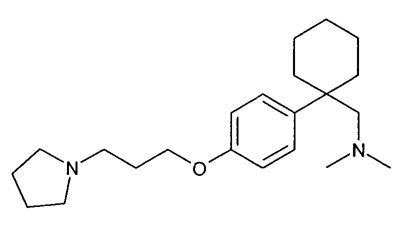

### Step 1:

To a stirred suspension of LiAlH₄ (0.607 g, 16 mmol) in diethyl ether (15 mL) cooled at 0°C was added dropwise a solution of 1-[4-(3-pyrrolidin-1-ylpropoxy)phenyl]cyclohexanecarbonitrile (1 g, 3.2 mmol) in diethyl ether (10 mL). After being stirred at room temperature for 30 min, the reaction mixture was refluxed for another 30 min. The mixture was cooled to 0°C and water (1.38 mL), a 5N aqueous solution of sodium hydroxide (1.38 mL) and water (5.52 mL) again were successively added dropwise. After being stirred for 15 min at room temperature, the mixture was filtered over diatomaceous earth and concentrated. The crude {1-[4-(3-pyrrolidin-1-ylpropoxy)phenyl]cyclohexyl}methylamine (0.961 g, 95%) was used in the next step without further purification.

### Step 2:

To {1-[4-(3-pyrrolidin-1-ylpropoxy)phenyl]cyclohexyl}methylamine (0.96 g, 3 mmol) was added formic acid (5 mL) followed by formaldehyde (37% w/w solution in H₂O, 0.915 mL, 33 mmol). The mixture was refluxed for 3 h and allowed to stir overnight at room temperature. It was then diluted with DCM, basified to pH 12 with an aqueous solution of NaOH and the mixture was extracted with DCM. The organic extracts were dried over Na₂SO₄, filtered, concentrated *in vacuo* and purified by column chromatography on silica gel eluting with a gradient of DCM:MeOH:NH₄OH (from 99:0:1 to 90:10:1) to provide the title compound (0.447 g, 43%) as an oil.
¹H NMR (400MHz, CDCl₃) δ7.18 (d, 2H), 6.78 (d, 2H), 3.94 (t, 2H), 2.55 (t, 2H), 2.45 (m, 4H), 2.22 (m, 2H), 2.06-1.86 (m, 10H), 1.72 (m, 4H), 1.58-1.19 (m, 8H).

### Intermediate 27: 4-[4-(3-pyrrolidin-1-ylpropoxy)phenyl]tetrahydro-2H-pyran-4-carbaldehyde

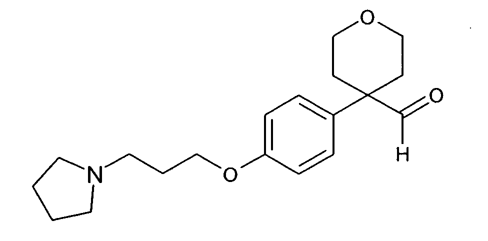

### Step 1:

A solution of 4-[4-(3-pyrrolidin-1-ylpropoxy)phenyl]tetrahydro-2*H*-pyran-4-carbonitrile (20g, 0.0636mol) in concentrated hydrochloric acid (200ml, 10vol) was heated and stirred at reflux for 16h after which time LC/LCMS analysis showed the reaction had stalled at 85-90% conversion. The solution was evaporated to dryness *in vacuo,* azeo-dried with methanol (200ml, 20vol) and toluene (2x 200ml, 2x 20vol) to yield 4-[4-(3-pyrrolidin-1-ylpropoxy)phenyl]tetrahydro-2*H*-pyran-4-carboxylic acid as a beige solid which was used directly (no NMR data recorded).

### Step 2:

A suspension of 4-[4-(3-pyrrolidin-1-ylpropoxy)phenyl]tetrahydro-2*H*-pyran-4-carboxylic acid (23.5g, 0.0636mol) in methanol (235ml, 10vol) was stirred and cooled to 0 to -5°C under nitrogen. Thionyl chloride (9.3ml, 0.1272mol, 0.40vol) was charged dropwise over 20minutes, maintaining the temperature at -5 to +5°C. The resulting slurry was then heated and stirred at reflux under nitrogen for 16h after which time LC analysis showed 1.4% by area of acid remaining. The solution was evaporated to dryness *in vacuo* at 40°C to give a brown sludge. The sludge was dissolved in ethyl acetate (118ml, 5vol) and water (235ml, 10vol) and the layers separated. The aqueous layer was washed with ethyl acetate (118ml, 5vol) and basified to pH 11 with saturated aqueous potassium carbonate (118ml, 5vol). The product was extracted into dichloromethane (3x 118ml, 3x 5vol) and the combined extracts dried over magnesium sulfate (23g) and concentrated *in vacuo* at 40°C to yield methyl 4-[4-(3-pyrrolidin-1-ylpropoxy)phenyl]tetrahydro-2H-pyran-4-carboxylate (21.7g, 98%th from the nitrile) as a cream solid.
¹H NMR (400MHz, DMSO-*d*₆) δ7.15 (d, 2H), 6.76 (d, 2H), 3.90 (t, 2H), 3.70 (dt, 2H), 3.50 (s, 3H), 3.30 (td, 2H), 2.40-2.20 (m, 6H), 1.80-1.70 (m, 4H), 1.6-1.5 (m, 4H).

### Step 3:

Anhydrous tetrahydrofuran (141ml, 10vol) was charged rapidly onto stirred and cooled (0-5°C) lithium aluminium hydride (6.2g, 0.1634mol, 0.44wt) under nitrogen. The resulting slurry was cooled to 0-5°C and then a solution of methyl 4-[4-(3-pyrrolidin-1-ylpropoxy)phenyl]tetrahydro-2H-pyran-4-carboxylate (14.1g, 0.04058mol) in tetrahydrofuran (99ml, 7vol) was added dropwise over 30 minutes maintaining 0-5°C. On complete addition the reaction was allowed to warm to room temperature over 30 minutes and stirred at 18-25°C for 30 minutes, LC analysis showed the reaction to be complete. The mixture was cooled to 0 to 5°C, a 1:1 mixture of tetrahydrofuran and water (18.6ml, 1.32vol) was added very slowly. The mixture was further quenched with 20% w/v sodium hydroxide solution (6.2ml, 0.44vol) and water (18.6ml, 1.32vol) and the resulting white suspension stirred vigorously for 30 minutes at 18-25°C. The salts were removed by filtration and rinsed with tetrahydrofuran (3x 150ml). The combined filtrates were evaporated to dryness at 40°C to yield {4-[4-(3-pyrrolidin-1-ylpropoxy)phenyl]tetrahydro-2*H*-pyran-4-yl}methanol (12.1 g, 94%th) as a white solid.
¹H NMR (400MHz, CDCl₃) δ7.25 (d, 2H), 6.90 (d, 2H), 4.05 (t, , 2H), 3.80 (dt, 2H), 3.55 (td, 2H), 2.60 (t, 2H), 2.62-2.41 (m, 4H), 2.10 (dt, 2H), 2.00 (p, 2H), 2.00-1.85 (m, 2H), 1.95-1.72 (m, 4H)

### Step 4:

Pyridinium chlorochromate (20.3g, 0.09415mol), 1.69wt) was charged to a stirred suspension of celite (24g, 2wt) and {4-[4-(3-pyrrolidin-1-ylpropoxy)phenyl]tetrahydro-2*H*-pyran-4-yl}methanol (12.03g, 0.03766mol) in dichloromethane (120ml, 10vol) with stirring. The mixture was stirred at 18-25°C for 3.5 hours after which time LC analysis showed the reaction to be 70% complete. Magnesium sulphate (12g, 1wt) was added and the resulting slurry filtered through a pad of aluminium oxide (activated, basic, Brockmann I, standard grade, ~150 mesh) (481 g, 40wt) eluting with 1% methanol/dichloromethane. Clean fractions were combined and evaporated to dryness at 40°C. The resulting brown solid was stirred in tertbutylmethylether (120ml, 10vol). Undissolved residue was removed by filtration and rinsed with *tert*butylmethylether (3x120ml, 3x 10vol). The combined filtrates were evaporated at 40°C to yield 4-[4-(3-Pyrrolidin-1-ylpropoxy)phenyl]tetrahydro-2*H*-pyran-4-carbaldehyde (6.0g, 50.2%th) as a beige waxy solid.
¹H NMR (400MHz, CDCl₃) δ9.35 (s, 1H), 7.20 (d, 2H), 6.95 (d, 2H), 4.05 (t, 2H), 3.90 (dt, 2H), 3.60 (td, 2H), 2.60 (t, 2H) 2.50 (m, 4H), 2.35 (br d, 2H), 2.10-1.90 (m, 4H), 1.92-1.72 (m, 4H).

Alternatively, 4-[4-(3-Pyrrolidin-1-ylpropoxy)phenyl]tetrahydro-2*H*-pyran-4-carbaldehyde can be prepared as follows:
To a solution of 4-[4-(3-pyrrolidin-1-ylpropoxy)phenyl]tetrahydro-2H-pyran-4-carbonitrile (250 mg, 0.79 mmol) in anhydrous toluene (2 mL) at -78°C was added dropwise a 1.5M solution of diisobutyl aluminium hydride in toluene (1.59 mL, 2.38 mmol, 3 equiv.). The reaction mixture was stirred for 2 h at -78°C. Ethyl acetate was then added followed by a saturated solution of sodium potassium tartrate and dichloromethane. After stirring at room temperature for 1 h, the organic layer was separated. The aqueous layer was extracted three times with dichloromethane. The combined organic extracts were dried over sodium sulfate, filtered and concentrated under reduced pressure to give a 8:2 mixture of the desired aldehyde and starting nitrile as an orange oil.

### General procedure D:

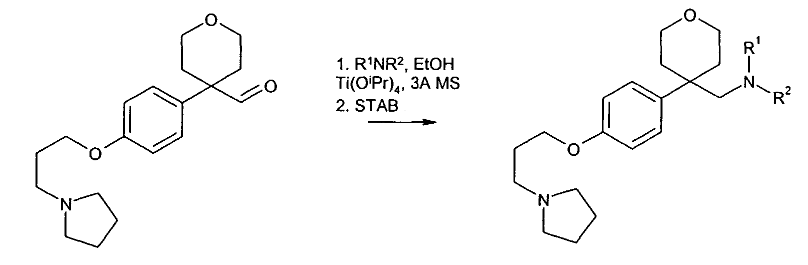

A solution of the 4-[4-(3-Pyrrolidin-1-ylpropoxy)phenyl]tetrahydro-2*H*-pyran-4-carbaldehyde (700mg, 2.205mmol, 1wt) and secondary amine (NR¹R²) (4.21 mmol) in absolute ethanol (28ml, 40vol) was stirred 18 to 25°C under nitrogen over activated 3Å molecular sieves (700mg, 1wt). Titanium (IV) isopropoxide (3.25ml, 11.025mmol, 4.62vol) was added dropwise over 2 minutes. The resulting solution was stirred at 18 to 25°C under nitrogen for 20 to 24h, over which time precipitation was observed. Sodium triacetoxyborohydride (3.97g, 0.0187mol, 5.67wt) was then added in one portion (Note: not exothermic, but gentle gas evolution was observed) and the reaction was stirred for 1 to 8h until LC analysis showed the reaction to be either complete or stalled at 1-10% aldehyde by area). The reaction mixture was decanted from the sieves and evaporated to dryness *in vacuo* at 40°C. *tert*-Butylmethyl ether (28ml, 40vol), 50% w/v Rochelle's solution (28ml, 40vol) and sat. aqueous potassium carbonate solution (28ml, 40vol) were added and the mixture stirred vigorously for 1 to 2h until two layers were visible (the organic layer was clear, the aqueous cloudy). The layers were separated and the aqueous layer re-extracted with *tert*-butylmethyl ether (28ml, 40vol). The extracts were combined, washed with sat. aqueous sodium chloride solution (28ml, 40vol), dried over magnesium sulfate (700mg, 1wt) and evaporated *in vacuo* at 40°C to yield a viscous oil. The oil was purified by column chromatography on silica (20g, 28.6wt) eluting with DCM(95):MeOH(4):NH₄OH(1) to remove unreacted aldehyde, alcohol by-product (typically 1-10% by area) and the excess secondary amine to yield the pure product as a viscous oil contaminated by dichloromethane after evaporation. The oil was dissolved in ethanol (5ml, 7.1vol) and evaporated to dryness *in vacuo* at 40°C, then in a vacuum oven at 40°C for 12 to 24h to yield the required tertiary amine product as a viscous oil or waxy solid.

### Example 35: (1-{4-[4-(3-Pyrrolidin-1-ylpropoxy)phenyl]tetrahydropyran-4-ylmethyl}piperidin-4-yl)-methanol

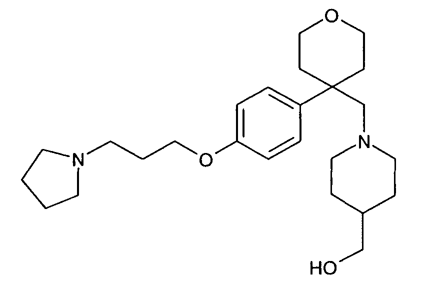

4-[4-(3-Pyrrolidin-1-ylpropoxy)phenyl]tetrahydro-2*H*-pyran-4-carbaldehyde (700mg, 2.205mmol, 1wt), 4-piperidinemethanol (485mg, 4.21 mmol), absolute ethanol (28ml, 40vol), activated 3Å molecular sieves (700mg), titanium (IV) isopropoxide (3.25ml, 11.03mmol) and STAB (3.97g, 18.7mmol) was reacted in accordance with the general procedure D. The isolated waxy solid was purified by column chromatography on silica eluting with DCM:MeOH:NH₄OH 95:4:1 to give the title compound as a colorless oil (820mg, 72%th).
¹H NMR (400MHz, CDCl₃) δ7.20 (d, 2H), 6.85 (d, 2H), 4.02 (t, 2H), 3.75 (dt, 2H), 3.59-3.46 (m, 2H), 3.42 (d, 2H), 2.63 (t, 2H), 2.53 (br s, 4H), 2.39-2.26 (m, 4H), 2.15-1.03 (m, 18H).

### Example 36: 1-{4-[4-(3-Pyrrolidin-1-ylpropoxy)phenyl]tetrahydropyran-4-ylmethyl}piperidin-4-ol

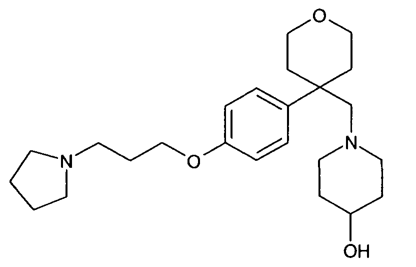

4-[4-(3-Pyrrolidin-1-ylpropoxy)phenyl]tetrahydro-2*H*-pyran-4-carbaldehyde (420mg, 1.30mmol, 1wt), 4-hydroxypiperidine (268mg, 2.60mmol), absolute ethanol (17ml, 40vol), activated 3Å molecular sieves (420mg), titanium (IV) isopropoxide (1.94ml, 4.62vol) and STAB (2.34g, 11.1mmol) was reacted in accordance with the general procedure D. The isolated waxy solid was purified by preparative HPLC, eluting with acetonitrile/water/0.1%TFA as a gradient, to give the title compound as a white solid (380mg, 73%th).
¹H NMR (400MHz, CDCl₃) δ7.21 (d, 2H), 6.86 (d, 2H), 4.02 (t, 2H), 3.75 (dt, 2H), 3.59-3.47 (m, 2H), 2.63 (t, 2H), 2.53 (br s, 4H), 2.35 (s, 2H), 2.33-2.25 (m, 2H), 2.13-1.96 (m, 6H), 1.93-1.83 (m, 2H), 1.82-1.76 (m, 4H), 1.72-1.59 (m, 3H), 1.47-1.29 (m, 3H).

### Example 37: 1-{4-[4-(3-Pyrrolidin-1-yl-propoxy)-phenyl]-tetrahydro-pyran-4-ylmethyl}-piperidine-4-carboxylic acid amide

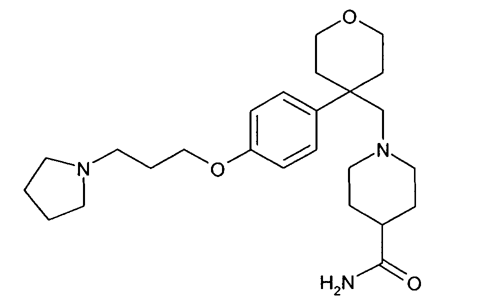

4-[4-(3-Pyrrolidin-1-ylpropoxy)phenyl]tetrahydro-2*H*-pyran-4-carbaldehyde (500mg, 1.58mmol), isonipecotamide (434mg, 3.03mmol), absolute ethanol (20ml), activated 3Å molecular sieves (500mg), titanium (IV) isopropoxide (2.31 ml, 7.88mmol) and STAB (2.84g, 13.6mmol) were reacted in accordance with the general procedure D. The isolated crude product was purified by column chromatography on silica eluting with DCM:MeOH:NH₄OH (97:2:1) to give the title compound as a colorless solid (400mg, 59%th).
¹H NMR (400MHz, CDCl₃) δ7.20 (d, 2H), 6.85 (d, 2H), 5.45 (br s, 2H), 4.01 (t, 2H), 3.79-3.69 (m, 2H), 3.57-3.46 (m, 2H), 2.63 (t, 2H), 2.53 (br s, 4H), 2.35 (s, 2H), 2.31 (br s, 1H), 2.14-1.72 (m, 14H), 1.67-1.51 (m, 4H).

### Example 38: 1-Methyl-4-{4-[4-(3-pyrrolidin-1-yl-propoxy)-phenyl]-tetrahydropyran-4-ylmethyl}-piperazine

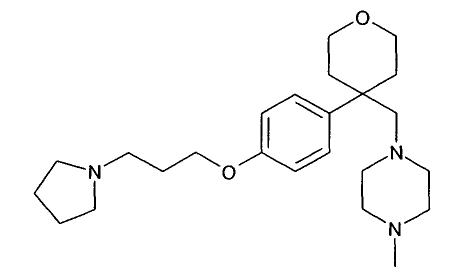

A solution of 4-[4-(3-Pyrrolidin-1-ylpropoxy)phenyl]tetrahydro-2*H*-pyran-4-carbaldehyde (500mg, 1.58mmol, 1wt), 1-methylpiperazine (300mg, 2.99mmol), absolute ethanol (20ml, 40vol), activated 3Å molecular sieves (500mg), titanium (IV) isopropoxide (2.32ml, 7.83mmol) and STAB (1.43g, 6.73mmol) were reacted in accordance with the general procedure D. The isolated oil was purified by column chromatography on silica (20g, 28.6wt) eluting with DCM(95):MeOH(4):NH₄OH(1) to give the title compound as a clear viscous oil (360mg, 56%th).
¹H NMR (400MHz, CDCl₃) δ7.21 (d, 2H), 6.86 (d, 2H), 4.02 (t, 2H), 3.75 (dt2H), 3.60-3.47 (m, 2H), 2.63 (t, 2H), 2.53 (br s, 4H), 2.38 (s, 2H), 2.34-2.14 (m, 11 H), 2.13-1.73 (m, 10H).

### Example 39: 1-{4-[4-(3-Pyrrolidin-1-ylpropoxy)phenyl]tetrahydropyran-4-ylmethyl}piperazine

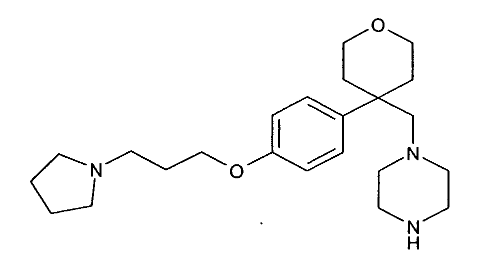

4-[4-(3-Pyrrolidin-1-ylpropoxy)phenyl]tetrahydro-2*H*-pyran-4-carbaldehyde (350mg, 1.103mmol), piperazine (372mg, 1.98mmol), absolute ethanol (40ml), activated 3Å molecular sieves (350mg), titanium (IV) isopropoxide (1.62ml, 5.48mmol) and STAB (2.0g, 9.74mmol) were reacted in accordance with the general procedure D. The isolated crude product was purified by column chromatography on silica eluting with DCM:MeOH:NH₄OH (98:1:1) to give a white solid (295mg, 69%th). The material was re-dissolved in methanol (3.2ml) and 1,4-dioxane (8ml), HCl in dioxane (4M, 1.95ml) was added. The mixture was stirred overnight and concentrated *in vacuo* at 40°C. To the concentrate, TBME (13ml) and NaOH (2M aqueous) were added to attain a pH of 12-14. The organic phase was separated and dried over MgSO₄ and concentrated *in vacuo* at 35°C. The crude product was subjected to chromatography on silica eluting with DCM:MeOH:NH₄OH (94:4:2) to give the title compound as a yellow oil (100mg, 23%th).
¹H NMR (400MHz, CDCl₃) δ7.21 (d, 2H), 6.85 (d, 2H), 4.02 (t, 2H), 3.80-3.70 (m, 2H), 3.58-3.46 (m, 2H), 2.73-2.66 (m, 4H), 2.63 (t, 2H), 2.53 (br s, 4H), 2.34 (s, 2H), 2.17-1.48 (m, 15H).

### Example 40: 1-{4-[4-(3-Pyrrolidin-1-ylpropoxy)phenyl]tetrahydropyran-4-ylmethyl}pyrrolidin-3-ol

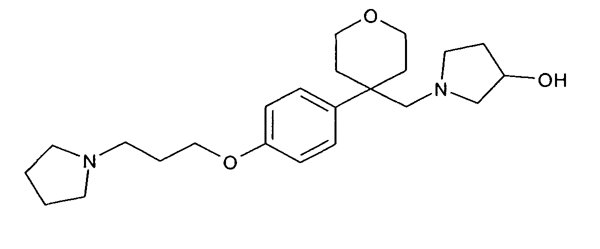

4-[4-(3-Pyrrolidin-1-ylpropoxy)phenyl]tetrahydro-2*H*-pyran-4-carbaldehyde (350mg, 1.103mmol), 3-pyrrolidinol (200mg, 2.02mmol), absolute ethanol (40ml), activated 3Å molecular sieves (350mg), titanium (IV) isopropoxide (1.62ml, 5.48mmol) and STAB (2.0g, 9.74mmol) were reacted in accordance with the general procedure D. The isolated crude product was purified by column chromatography on silica eluting with DCM:MeOH:NH₄OH (97:2:1) to give the title compound as a white solid (390mg, 91%th).
¹H NMR (400MHz, CDCl₃) δ7.20 (d, 2H), 6.87 (d, 2H), 4.10-4.01 (m, 3H), 3.81-3.68 (m, 3H), 3.55 (t, 2H), 2.67-2.48 (m, 9H), 2.28-1.61 (m, 15H).

### Example 41: (R)-2-Methoxymethyl-1-{4-[4-(3-pyrrolidin-1-yl-propoxy)-phenyl]-tetrahydro-pyran-4-ylmethyl}-pyrrolidine

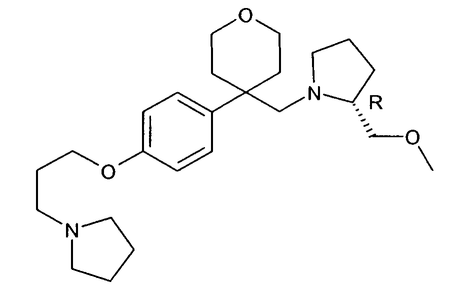

4-[4-(3-Pyrrolidin-1-ylpropoxy)phenyl]tetrahydro-2*H*-pyran-4-carbaldehyde (500mg, 1.575mmol), (R)-2-(methoxymethyl)pyrrolidine (347mg, 4.21 mmol), absolute ethanol (20ml), activated 3Å molecular sieves (500mg), titanium (IV) isopropoxide (2.33ml, 7.88mmol) and STAB (2.84g, 13.4mmol) were reacted in accordance with the general procedure D. The isolated waxy solid was purified by column chromatography on silica eluting with DCM:MeOH:NH₄OH 95:14:1 to give the title compound as a viscous pale yellow oil (390mg, 59%th).
¹H NMR (400MHz, CDCl₃) δ7.18 (d, 2H), 6.86 (d, 2H), 4.02 (t, 2H), 3.85-3.69 (m, 2H), 3.59 (td, 1H), 3.45 (td, 1 H), 3.27 (s, 3H), 3.10 (dd, 1H), 3.02 (dd, 1H), 2.94 (d, 1 H), 2.63 (t, 2H), 2.58-2.45 (m, 6H), 2.39 (m, 1H), 2.21 (m, 1H), 2.07-1.67 (m, 11 H), 1.57-1.33 (m, 3H).

### Example 42: (S)-2-Methoxymethyl-1-{4-[4-(3-pyrrolidin-1-ylpropoxy)-phenyl]tetrahydropyran-4-ylmethyl}pyrrolidine

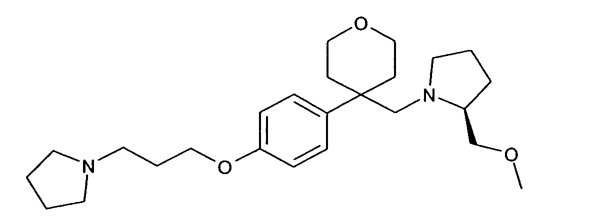

4-[4-(3-Pyrrolidin-1-ylpropoxy)phenyl]tetrahydro-2*H*-pyran-4-carbaldehyde (350mg, 1.103mmol), (s)-(+)-2-(methoxymethyl)pyrrolidine (230mg, 2.00mmol), absolute ethanol (40ml), activated 3Å molecular sieves (350mg), titanium (IV) isopropoxide (1.62ml, 5.48mmol) and STAB (2.0g, 9.74mmol) were reacted in accordance with the general procedure D. The isolated crude product was purified by column chromatography on silica eluting with DCM:MeOH:NH₄OH (98:1:1) to give the title compound as a white solid (330mg, 72%th).
¹H NMR (400MHz, CDCl₃) δ7.18 (d, 2H), 6.86 (d, 2H), 4.02 (t, 2H), 3.84-3.70 (m, 2H), 3.59 (td, 1 H), 3.45 (td, 1 H), 3.27 (s, 3H), 3.10 (dd, 1H), 3.02 (dd, 1 H), 2.94 (d, 1H), 2.63 (t, 2H), 2.59-2.45 (m, 5H), 2.39 (m,1H), 2.21 (m, 1H), 2.06-1.34 (m, 15H).

### Example 43: 1-(1-{4-[4-(3-Pyrrolidin-1-ylpropoxy)phenyl]tetrahydropyran-4-ylmethyl}piperidin-4-yl)pyrrolidin-2-one

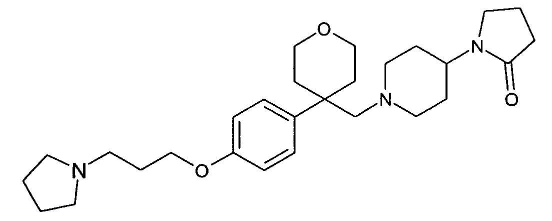

4-[4-(3-Pyrrolidin-1-ylpropoxy)phenyl]tetrahydro-2*H*-pyran-4-carbaldehyde (400mg, 1.260mmol), 4-(N-2-pyrrolidinone)piperidine hydrochloride (493mg, 4.407mmol), absolute ethanol (16ml), activated 3Å molecular sieves (400mg), titanium (IV) isopropoxide (1.86ml, 6.30mmol) and STAB (2.27g, 10.71mmol) were reacted in accordance with the general procedure D. The isolated waxy solid was purified by column chromatography on silica eluting with DCM(95):MeOH(4):NH₄OH(1) to give the title compound as a pale yellow oil (133mg, 22%th).
¹H NMR (400MHz, CDCl₃) δ7.20 (d, 2H), 6.86 (d, 2H), 4.02 (t, 2H), 3.83-3.71 (m, 3H), 3.52 (dt, 2H), 3.32 (t, 2H), 2.63 (t, 2H), 2.53 (br s, 4H), 2.41-2.30 (m, 6H), 2.18 (dt, 2H), 2.12-1.36 (m, 16H).

### Example 44: 4-{4-[4-(3-Pyrrolidin-1-yl-propoxy)-phenyl]-tetrahydro-pyran-4-ylmethyl}-thiomorpholine

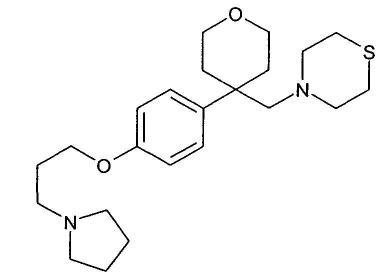

4-[4-(3-Pyrrolidin-1-ylpropoxy)phenyl]tetrahydro-2*H*-pyran-4-carbaldehyde (1.5g, 4.726mmol, 1wt), thiomorpholine (930mg, 9.027mmol), absolute ethanol (60ml), activated 3Å molecular sieves (1.5g), titanium (IV) isopropoxide (6.97ml, 23.630mmol) and STAB (8.51 g, 40.15mmol) were reacted in accordance with the general procedure D. The isolated waxy solid was purified by column chromatography on silica eluting with DCM(95):MeOH(4):NH₄OH(1) to give the title compound as a pale yellow oil (1.06g, 55%th).
¹H NMR (400MHz, CDCl₃) δ7.20 (d, 2H), 6.86 (d, 2H), 4.02 (t, 2H), 3.75 (dt, 2H), 3.57-3.45 (m, 2H), 2.63 (t, 2H), 2.59-2.39 (m, 12H), 2.36 (s, 2H), 2.14-1.95 (m, 4H), 1.92-1.73 (m, 6H).

### Example 45: 4-{4-[4-(3-Pyrrolidin-1-ylpropoxy)phenyl]tetrahydropyran-4-ylmethyl}thiomorpholine-1-oxide

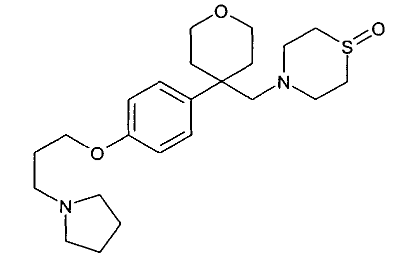

To a solution of 4-{4-[4-(3-Pyrrolidin-1-yl-propoxy)-phenyl]-tetrahydro-pyran-4-ylmethyl}-thiomorpholine (200mg, 0.494mmol) in TFA (0.67ml) at 5 to 10°C was added dropwise a solution of PTFA [4M solution prepared by the addition of 27.5% H₂O₂ (0.94ml) to TFA (1.56ml)] and stirred at 5 to 10°C for 1 hour. The reaction mixture was diluted with DCM (5ml) and NaOH (5M solution, 4ml) was added to attain pH14. The mixture was extracted with DCM ((2x5ml), the combined DCM extracts were washed with saturated aqueous brine (10ml), dried over MgSO₄, filtered and concentrated *in vacuo.* The isolated yellow oil was purified by column chromatography on silica eluting with DCM(95):MeOH(4):NH₄0H(1) to give the title compound as a yellow oil (165mg, 79%th).
¹H NMR (400MHz, CDCl₃) δ7.20 (d, 2H), 6.87 (d, 2H), 4.02 (t, 2H), 3.81-3.69 (m, 2H), 3.59-3.43 (m, 2H), 2.99-2.82 (m, 2H), 2.76-2.2.59 (m, 6H), 2.53 (br s, 4H), 2.46 (s, 2H), 2.31-1.73 (m, 12H).

### Example 46: 4-{4-[4-(3-Pyrrolidin-1-yl-propoxy)-phenyl]-tetrahydro-pyran-4-ylmethyl}-thiomorpholine 1,1-dioxide

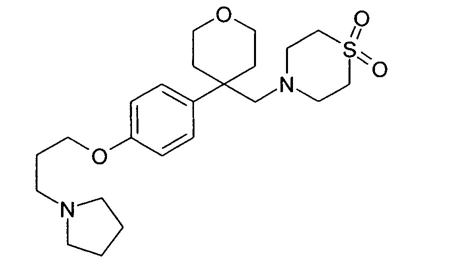

To a solution of 4-{4-[4-(3-Pyrrolidin-1-yl-propoxy)-phenyl]-tetrahydro-pyran-4-ylmethyl}-thiomorpholine (230mg, 0.56mmol) in TFA (0.77ml) at 0 to 5°C was added dropwise a solution of PTFA [4M solution prepared by the addition of 27.5% H₂O₂ (0.94ml) to TFA (1.56ml)]. The reaction was allowed to warm to room temperature and stirred overnight. The reaction mixture was cooled to 0 to 5°C, diluted with DCM (5ml) and quenched with NaOH (5M solution, 5ml) to attain pH14. The mixture was extracted with DCM (4x5ml), the combined DCM extracts were dried over MgSO₄, filtered and concentrated *in vacuo.* The isolated yellow oil was purified by column chromatography on silica eluting with DCM(95):MeOH(4):NH₄OH(1) to give the title compound as a colourless oil (96.6mg, 39%th).
¹H NMR (400MHz, CDCl₃) δ7.20 (d, 2H), 6.88 (d, 2H), 4.02 (t, 2H), 3.75 (dt, *J* = 11 and 4Hz, 2H), 3.57-3.45 (m, 2H), 2.89-2.79 (m, 4H), 2.73-2.65 (m, 4H), 2.63 (t, 2H), 2.55 (br s, 4H), 2.53 (s, 2H), 2.22-2.11 (m, 2H), 2.01 (m, 2H), 1.87-1.73 (m, 6H).

### Example 47: 4-{4-[4-(3-Pyrrolidin-1-yl-propoxy)phenyl]tetrahydropyran-4-ylmethyl}piperazine-1-carboxylic acid ethyl ester

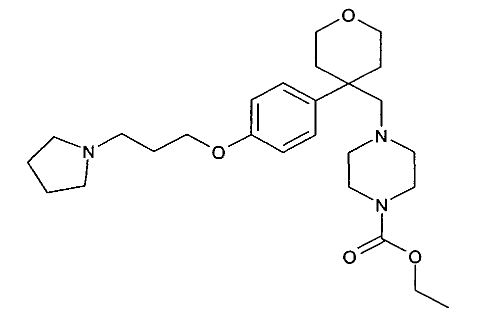

4-[4-(3-Pyrrolidin-1-ylpropoxy)phenyl]tetrahydro-2*H*-pyran-4-carbaldehyde (500mg, 1.58mmol), ethyl piperazine-1-carboxylate (471mg, 3.00mmol), absolute ethanol (20ml), activated 3Å molecular sieves (500mg), titanium (IV) isopropoxide (2.31 ml, 7.88mmol) and STAB (2.84g, 13.6mmol) were reacted in accordance with the general procedure D. The isolated crude product was purified by column chromatography on silica eluting with DCM:MeOH:NH₄OH (97:2:1) to give the title compound as a white solid containing residual 4-[4-(3-Pyrrolidin-1-ylpropoxy)phenyl]tetrahydro-2*H*-pyran-4-carbaldehyde (350mg). The material was dissolved in DCM (14ml) and PS-AMPS (loading 2.04mmol⁻¹g, 23mg) was added and shaken for 24 hours. The resin was filtered and the solution concentrated *in vacuo* at 30°C, the crude material was subjected to chromatography on silica eluting with DCM:MeOH:NH₄OH (97:2:1) to give the title compound as a white solid (130mg, 18%).
¹H NMR (400MHz, CDCl₃) δ7.20 (d, 2H), 6.86 (d, 2H), 4.08 (q, 2H), 4.02 (t, 2H), 3.79-3.70 (m, 2H), 3.57-3.47 (m, 2H), 3.27 (br s, 4H), 2.63 (t, 2H), 2.53 (br s, 4H), 2.38 (s, 2H), 2.21-1.73 (m, 14H), 1.21 (t, 3H).

### Intermediate 28: Piperazine-1-carboxylic acid amide hydrochloride

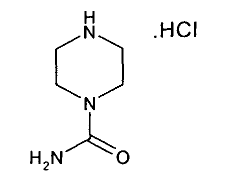

4-Piperazine-1-carboxylic acid *tert*-butyl ester (1.0g, 5.4mmol), acetic acid (3ml) and water (5ml) were mixed together. Potassium cyanate (2.25g, 27.7mmol) was added portionwise as a solution in water (5ml) and stirred for 4 hours, during which time a solid precipitated. The solid was filtered, re-dissolved in DCM (20ml), dried over MgSO₄, filtered, the filter cake washed with DCM (5vol) and concentrated *in vacuo* to give a white solid (0.38g). The white solid (0.38g) was dissolved in methanol (3.8ml) and 1,4-dioxane (0.7ml), 4M HCl in 1,4-dioxane (2.5ml, 10mmol) was added to the reaction and stirred overnight. The reaction was concentrated *in vacuo* to give the title compound (0.28g, 31% over 2 steps) as a white solid.
¹H NMR (400MHz, DMSO-*d*₆) δ9.18 (br s, 2H), 3.91 (br s, 2H), 3.45 (br s, 4H), 2.93 (br s, 4H).

### Example 48: 4-{4-[4-(3-Pyrrolidin-1-ylpropoxy)phenyl]tetrahydropyran-4-ylmethyl}piperazine-1-carboxylic acid amide

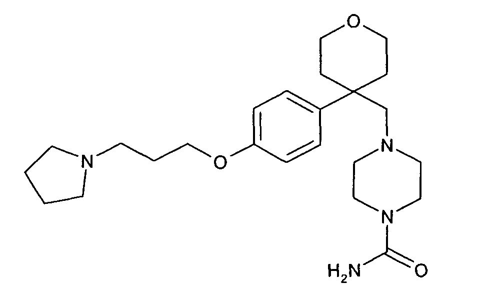

4-[4-(3-Pyrrolidin-1-ylpropoxy)phenyl]tetrahydro-2*H*-pyran-4-carbaldehyde (317mg, 1mmol), piperazine-1-carboxylic acid amide hydrochloride (273mg, 1.65mmol), absolute ethanol (12.7ml), activated 3Å molecular sieves (320mg), Et₃N (0.278ml), titanium (IV) isopropoxide (1.46ml, 5.20mmol) and STAB (1.8g, 8.5mmol) were reacted in accordance with the general procedure D. The isolated crude product was purified by column chromatography on silica eluting with DCM:MeOH:NH₄OH (98:1:1) to give the title compound as a off white solid (70mg, 16%).
¹H NMR (400MHz, CDCl₃) δ7.21 (d, 2H), 6.86 (d, 2H), 4.30 (br s, 2H), 4.02 (t, 2H), 3.80-3.71 (m, 2H), 3.58-3.48 (m, 2H), 3.23-3.15 (m, 4H), 2.63 (t, 2H), 2.53 (br s, 4H), 2.40 (s, 2H), 2.19-1.73 (m, 14H).

### Example 49: 1-{4-[4-(3-Pyrrolidin-1-ylpropoxy)phenyl]tetrahydropyran-4-ylmethyl}piperidine

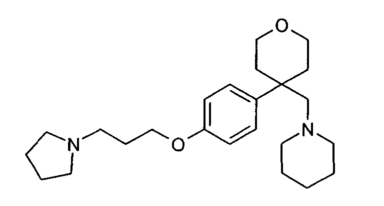

4-[4-(3-Pyrrolidin-1-ylpropoxy)phenyl]tetrahydro-2*H*-pyran-4-carbaldehyde (272 mg, 0.86 mmol), piperidine (0.161 mL, 1.63 mmol), absolute ethanol (11 mL), activated 4Å molecular sieves, titanium (IV) isopropoxide (1.205 mL, 4.28 mmol) and STAB (1.54 g, 7.28 mmol) were reacted in accordance with the general procedure D. The isolated waxy solid was purified by column chromatography on silica eluting with a gradient of DCM:MeOH:NH₄OH (from 99:0:1 to 90:9:1) to give the title compound (97.4 mg, 25%).
¹H NMR (400MHz, CDCl₃) δ7.21(d, 2H), 6.85 (d, 2H), 4.02 (t, 2H), 3.77 -3.72 (m, 2H), 3.54 -3.48 (t, 2H), 2.63 (t, 2H), 2.53 (m, 4H), 2.29 (s, 2H), 2.13-1.87 (m, 10H), 1.79 (quint, 4H), 1.41-1.36 (m, 4H), 1.30-1.26 (m, 2H).

### Example 50: 4,4-difluoro-1-{4-[4-(3-Pyrrolidin-1-ylpropoxy)phenyl]tetrahydropyran-4-ylmethyl}piperidine

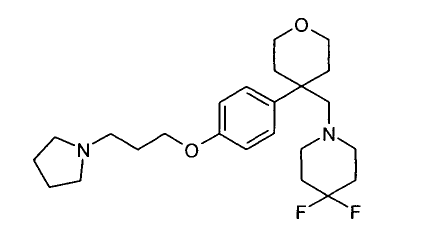

4-[4-(3-Pyrrolidin-1-ylpropoxy)phenyl]tetrahydro-2H-pyran-4-carbaldehyde (260 mg, 0.82 mmol), 4,4-difluoropiperidine hydrochloride (323 mg, 2.05 mmol), absolute ethanol (11 mL), activated 4Å molecular sieves, titanium (IV) isopropoxide (1.209 mL, 4.09 mmol) and STAB (1.475 g, 6.96 mmol) were reacted in accordance with the general procedure D. The isolated waxy solid was purified by reverse phase preparative HPLC on a Xterra column eluting with a gradient of MeCN:H₂O:Et₃N (from 29.9:70:0.1 to 94.9:5:0.1 in 13 min) to give the title compound (26 mg, 6%).
¹H NMR (400MHz, CDCl₃) δ7.21 (d, 2H), 6.88 (d, 2H), 4.02 (t, 2H), 3.77 -3.73 (m, 2H), 3.53 -3.47 (t, 2H), 2.64 (t, 2H), 2.54 (m, 4H), 2.39 (s, 2H), 2.30-2.24 (m, 6H), 2.13 (d, 2H), 2.02 (m, 2H), 1.90-1.71 (m, 6H).

### Example 51: methyl-{4-[4-(3-pyrrolidin-1-ylpropoxy)phenyl]-tetrahydropyran-4-ylmethyl}amine

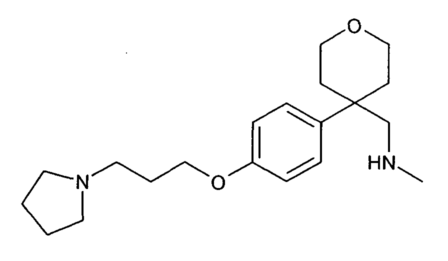

### Step 1:

To a stirred solution of {4-[4-(3-Pyrrolidin-1-ylpropoxy)phenyl]tetrahydropyran-4-yl} methylamine (10.83 g, 34.05 mmol) and triethylamine (10.31 g, 102.16 mmol) in dry dichloromethane at 0 ° C was added dropwise a solution of Di-*tert*-butyldicarbonate (9.65 g, 44.27 mmol) in dry dichloromethane. Stirring was continued for another 1 h at 0 °C and then overnight at r. t. Reaction mixture was then diluted with dichloromethane, washed with water and brine, dried over Na₂SO₄ and concentrated under reduced pressure to provide a crude product. The crude product was purified by column chromatography on silica gel (EtOAc-MeOH-Et₃N) to give methyl-{4-[4-(3-pyrrolidin-1-ylpropoxy)phenyl]tetrahydropyran-4-ylmethyl}carbamic acid *tert*-butyl ester (9.80 g, 69 % yield).
¹H NMR (400 MHz, CDCl₃) δ1.37 (s, 9H); 1.75-1.88 (m, 6H); 1.96-2.07 (m, 4H); 2.48-2.55 (m, 4H); 2.61 (t, 2H); 3.28 (d, 2H); 3.51-3.59 (m, 2H); 3.76-3.80 (m, 2H); 4.01 (t, 2H); 4.15 (brm, 1 H); 6.89 (d, 2H); 7.16 (d, 2H).

### Step 2:

To a stirred solution of LiAlH₄ (2.59 g, 68.18 mmol) in dry THF at 0 ° C was added dropwise a solution of methyl-{4-[4-(3-pyrrolidin-1-ylpropoxy)phenyl]tetrahydropyran-4-ylmethyl}carbamic acid *tert*-butyl ester (9.50 g, 22.72 mmol). Reaction mixture was then brought to r. t. and finally allowed to reflux for 3 h. Reaction mixture was quenched with aqueous NaOH solution and filtered over a short pad of celite, dried over anhy. Na₂SO₄ and concentrated under reduced pressure to provide the title compound (7.1 g, 94 % yield).
¹H NMR (400 MHz, CDCl₃) δ1.71-1.78 (m, 4H); 1.80-1.90 (m, 2H); 1.88-2.02 (m, 2H); 2.06-2.14 (m, 2H); 2.23 (s, 3H); 2.46-2.53 (m, 4H); 2.51-2.63 (m, 4H); 3.47-3.56 (m, 2H); 3.70-3.77 (m, 2H); 3.99 (t, 2H); 6.86 (d, 2H); 7.17 (d, 2H).

### Example 52: methyl-(3-methylsulfanyl-propyl)-{4-[4-(3-pyrrolidin-1-ylpropoxy)phenyl]tetrahydropyran-4-ylmethyl}amine

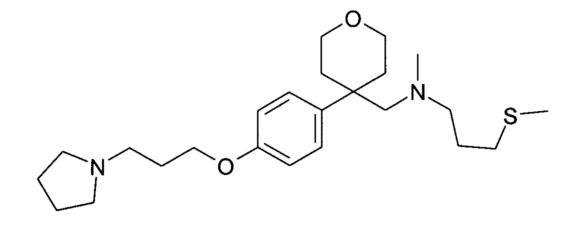

To a solution of methyl-{4-[4-(3-pyrrolidin-1-ylpropoxy)phenyl]tetrahydropyran-4-ylmethyl}amine (200 mg, 0.629 mmol) in dichloroethane (5 mL) under nitrogen atmosphere was added acetic acid (45 mg, 1.258 mmol), triethylamine (76 mg, 1.258 mmol) and 3-(methylthio)propanal (108 mg, 1.04 mmol). The mixture was stirred for 30 min and a solution of NaHB(OAc)₃ (236 mg, 1.15 mmol) in dichloroethane (5 mL) was added. After stirring at room temperature overnight, the reaction mixture was filtered over celite, concentrated *in vacuo* and purified by column chromatography on silica gel eluting with DCM:MeOH:NH₄OH (94.9:5:0.1) to give the title compound (29 mg, 7%).
¹H NMR (400MHz, CDCl₃) δ7.19 (d, 2H), 6.86 H), 4.02 (t, 2H), 3.78 - 3.73 (m, 2H), 3.54 - 3.49 (m, 2H), 2.67 (t, 2H), 2.59 (m, 4H), 2.42 (s, 2H), 2.35 (t, 2H), 2.22 (t, 2H), 2.11 - 2.00 (m, 7H), 1.91-1.86 (m, 5H), 1.85- 1.82 (m, 4H), 1.56 (q, 2H).

### Example 53: (3-methanesulfonyl-propyl)-methyl-{4-[4-(3-pyrrolidin-1-yl-propoxy)phenyl]tetrahydropyran-4-ylmethyl}amine

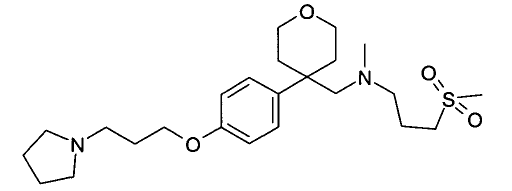

To a solution of methyl-(3-methylsulfanyl-propyl)-{4-[4-(3-pyrrolidin-1-ylpropoxy)phenyl]tetrahydropyran-4-ylmethyl}amine (170 mg, 0.404 mmol) in DCM (10 mL) under nitrogen atmosphere at 0°C was added TFA (0.240 mL, 3.2 mmol) followed by meta-chloroperbenzoic acid (70% pure, 199 mg, 0.808 mmol). The reaction mixture was stirred overnight allowing it to warm up to room temperature. It was quenched with an aqueous solution of Na₂S2O₃. The organic layer was separated and washed with an aqueous solution of NaHCO₃. The organic layer was then dried over sodium sulphate, filtered, concentrated and purified by column chromatography on silica gel eluting with DCM:MeOH:NH₄OH (89.9:10:0.1) to give the title compound.
¹H NMR (400MHz, CDCl₃) δ7.18 (d, 2H), 6.88 (d, 2H), 4.03 (t, 2H), 3.78 - 3.73 (m, 2H), 3.53 - 3.48 (m, 2H), 2.82 (s, 3H), 2.69-2.73 (m, 2H), 2.65 (m, 2H), 2.55 (m, 4H), 2.47 (s, 2H), 2.22 (t, 2H), 2.12 - 2.09 (m, 2H), 1.89 - 2.05 (m, 5H), 1.68-1.85 (m, 8H).

### Example 54: isopropyl-{4-[4-(3-pyrrolidin-1-ylpropoxy)phenyl]-tetrahydropyran-4-ylmethyl}amine

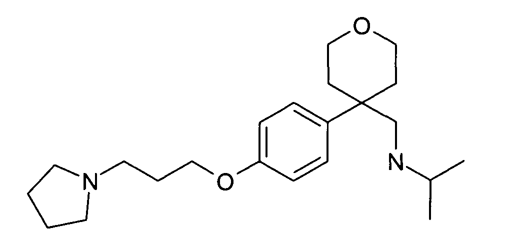

To a solution of {4-[4-(3-Pyrrolidin-1-ylpropoxy)phenyl]tetrahydropyran-4-yl} methylamine (400 mg, 1.256 mmol) in dichloroethane (10 mL) under nitrogen atmosphere was added acetic acid (180.5 mg, 2.76 mmol), triethylamine (306.4 mg, 2.76 mmol) and acetone (80.2 mg, 1.38 mmol). The mixture was stirred for 1 h at ambient temperature and NaHB(OAc)₃ (601 mg, 2.76 mmol) was added. After stirring overnight, the reaction mixture was filtered over celite, concentrated *in vacuo* and purified by column chromatography on silica gel eluting with DCM:MeOH:NH₄OH (89:10:1) to give the title compound as an oil (282 mg, 60%).
¹H NMR (400 MHz, CDCl₃) δ 7.13 (d, 2H), 6.82 (d, 2H), 3.96 (t, 2H), 3.68 (m, 2H), 3.49 (m, 2H), 2.61 (s, 2H), 2.56 (t, 2H), 2.46 (m, 5H), 2.04 (m, 2H), 1.94 (m, 2H), 1.84 (m, 2H), 1.72 (m, 4H), 1.19 (s, 1 H), 0.83 (d, 6H).

### Example 55: isopropyl-methyl-{4-[4-(3-pyrrolidin-1-ylpropoxy)phenyl]-tetrahydropyran-4-ylmethyl}amine

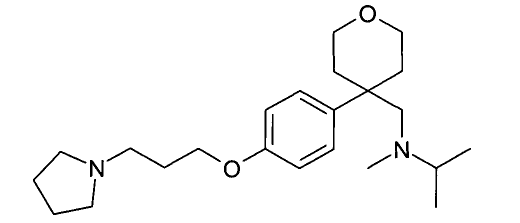

To isopropyl-{4-[4-(3-pyrrolidin-1-ylpropoxy)phenyl]tetrahydropyran-4-ylmethyl}amine (258 mg, 0.71 mmol) was added formaldehyde (37% w/w solution in H₂O, 1 mL) and formic acid (1.5 mL).The resulting mixture was heated to 60°C overnight. After cooling, H₂O and ethyl acetate were added. The aqueous layer was separated, basified with 30% NaOH and extracted 3 times with ethyl acetate. The combined organic extracts were dried over Na₂SO₄, filtered and concentrated *in vacuo* to give the title compound as an oil (170 mg, 65%).
¹H NMR (400MHz, CDCl₃) δ7.19 (d, 2H), 6.86 (d, 2H), 4.02 (t, 2H), 3.76 (m, 2H), 3.50 (m, 2H), 2.63 (t, 2H), 2.53 (m, 4H), 2.44 (m, 1H), 2.38 (s, 2H), 2.08 (m, 2H), 2.01 (m, 2H), 1.89 (m, 2H), 1.85 (s, 3H), 1.79 (m, 4H), 0.84 (d, 6H).

### Example 56: cyclopentyl-{4-[4-(3-pyrrolidin-1-ylpropoxy)phenyl]tetrahydropyran-4-ylmethyl}amine

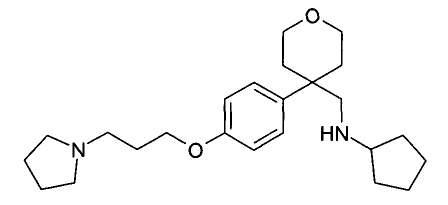

To a solution of {4-[4-(3-Pyrrolidin-1-ylpropoxy)phenyl]tetrahydropyran-4-yl} methylamine (254 mg, 0.798 mmol) in dichloroethane (5 mL) under nitrogen atmosphere were added acetic acid (37 mg, 1.6 mmol), triethylamine (97 mg, 1.6 mmol) and cyclopentanone (10 mg, 1.19 mmol). The mixture was stirred for 30 min at ambient temperature and NaHB(OAc)₃ (204 mg, 1.6 mmol) was added at 0°C. After stirring overnight, the reaction mixture was filtered over celite, concentrated *in vacuo* and purified by column chromatography on silica gel eluting with DCM:MeOH:NH₄OH (89:10:1) to give the title compound (226 mg, 58%).
¹H NMR (400MHz, CDCl₃) δ7.20 (d, 2H), 6.88 H), 4.03 (t, 2H), 3.78-3.73 (m, 2H), 3.59-3.53 (m, 2H), 2.84 (q, 1 H), 2.73 (t, 2H), 2.65-2.68 (m, 6H), 2.03-2.12 (m, 4H), 1.84-1.94 (m, 6H), 1.65-1.72 (m, 2H), 1.51-1.58 (m, 2H), 1.41-1.46 (m, 2H), 1.07-1.16 (m, 2H).

### Example 57: 1-{4-[4-(3-Pyrrolidin-1-ylpropoxy)phenyl]tetrahydropyran-4-ylmethyl}pyrrolidine

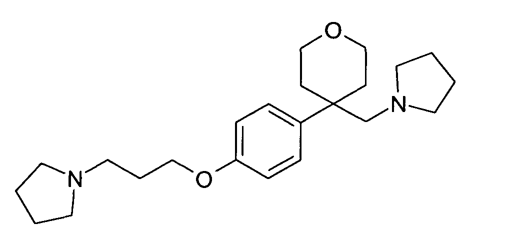

To a stirred solution of {4-[4-(3-Pyrrolidin-1-ylpropoxy)phenyl]tetrahydropyran-4-yl} methylamine (150 mg, 0.47 mmol) and 1-bromo-4-chloroethane (54.2 µL, 0.47 mmol) in anhydrous acetonitrile (5 mL) was added potassium carbonate (65 mg, 0.47 mmol). The mixture was heated to 60°C overnight then concentrated under reduced pressure. The crude product was purified by column chromatography on silica gel eluting with DCM:MeOH:NH₄OH (94:5:1) to give the title compound (40 mg, 23 % yield).

¹H NMR (400MHz, CDCl₃) δ7.20 (d, 2H), 6.86 (d, 2H), 4.02 (t, 2H), 3.73 -3.78 (m, 2H), 3.57-3.53 (m, 2H), 2.65 -2.61 (m, 4H), 2.53 (m, 4H), 2.20 (m, 4H), 2.10 - 1.88 (m, 6H), 1.56 (m, 4H).

### Example 58: 4-{4-[4-(3-Pyrrolidin-1-ylpropoxy)phenyl]tetrahydropyran-4-ylmethyl}-morpholine

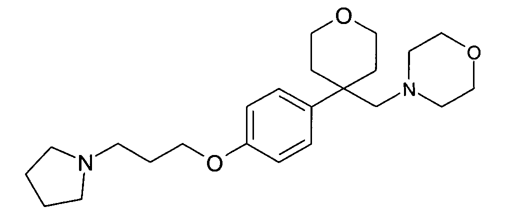

To a stirred solution of {4-[4-(3-Pyrrolidin-1-ylpropoxy)phenyl]tetrahydropyran-4-yl} methylamine (207 mg, 0.65 mmol) and bis(2-bromoethyl)ether (82 µL, 1.3 mmol) in anhydrous acetonitrile (10 mL) was added potassium carbonate (180 mg, 1.3 mmol). The mixture was heated to 60°C overnight then concentrated under reduced pressure. The crude product was purified by column chromatography on silica gel eluting with DCM:MeOH (97:3) with 10% NH₄OH to give the title compound (90 mg, 23 % yield).
¹H NMR (400MHZ, CDCL₃) δ7.22 (D, 2H), 6.83 (D, 2H), 4.04 (M, 2H), 3.73 (M, 2H), 3.51 (M, 6H), 3 (M, 6H), 2.38 (M, 2H), 2.25 (M, 2H), 2.13 - 2.03 (M, 10H), 1.88 (M, 2H).

### Example 59 : methyl-phenethyl-{4-[4-(3-pyrrolidin-1-ylpropoxy)phenyl]tetrahydropyran-4-ylmethyl}amine

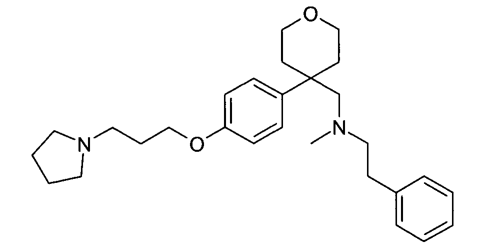

Methyl-{4-[4-(3-pyrrolidin-1-ylpropoxy)phenyl]tetrahydropyran-4-ylmethyl}amine (150 mg, 0.45 mmol) was treated with (2-bromoethyl)benzene (100 mg, 0.54 mmol) and potassium carbonate (187 mg, 1.35 mmol) in a sealed tube under microwave (55°C) for 80 min. The reaction mixture was taken in DCM (10 mL) and water (5 mL). The organic layer was separated and washed twice with water and brine. The combined orgnaic extracts were dried over Na₂SO₄, filtered, concentrated *in vacuo* and purified by column chromatography on silica gel eluting with DCM:MeOH (90:10) with 10% NH₄OH to give the title compound as a colorless oil (41.4 mg, 20%).
¹H NMR (400MHz,CDCl₃) δ7.23 (m, 7H), 6.87 (d, 2H), 4.00 (t, 2H), 3.66 (m, 2H), 3.51 (m, 2H), 3.29 (t, 2H), 3.20 (t, 2H), 2.62 (t, 2H), 2.51 (m, 6H), 2.10 (m, 2H), 1.99 (m, 2H), 1.90 (m, 2H), 1.86 (s, 3H), 1.78 (m, 4H).

### Example 60 : methyl-benzyl-{4-[4-(3-pyrrolidin-1-ylpropoxy)phenyl]tetrahydropyran-4-ylmethyl}amine

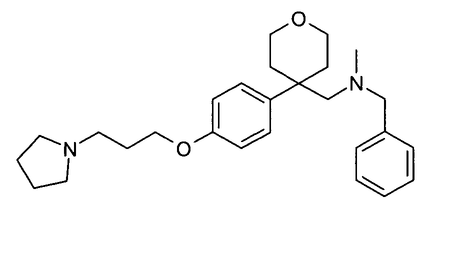

This example was prepared from methyl-{4-[4-(3-pyrrolidin-1-ylpropoxy)phenyl]tetrahydropyran-4-ylmethyl}amine similarly to the procedure used for example 56.
¹H NMR (400MHz, CDCl₃) δ7.28-7.19 (m, 7H), 6.87 (d, 2H), 4.00 (t, 2H), 3.69 - 3.65 (m, 2H), 3.53 -3.48 (t, 2H), 3.29 (s, 2H), 2.61 (t, 2H), 2.54-2.50 (m, 6H), 2.10-2.07 (m, 2H), 2.01-1.97 (m, 2H), 1.93-1.88 (m, 2H), 1.86 (s, 3H), 1.78-1.76 (m, 4H).

### Example 61 : (2-methoxyethyl)-methyl-{4-[4-(3-pyrrolidin-1-ylpropoxy)-phenyl]tetrahydropyran-4-ylmethyl}amine

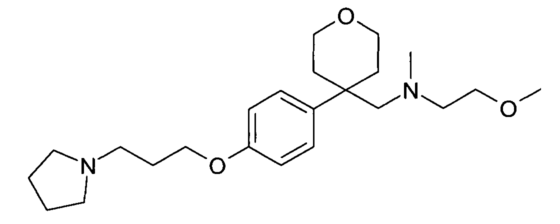

To a stirred solution of methyl-{4-[4-(3-pyrrolidin-1-ylpropoxy)phenyl]tetrahydropyran-4-ylmethyl}amine (150 mg, 0.45 mmol) and 2-bromoethylmethylether (43 µL, 0.45 mmol) in anhydrous acetonitrile (5 mL) was added potassium carbonate (62.4 mg, 0.45 mmol). The mixture was heated to 60°C overnight then to 80°C for a day and concentrated under reduced pressure. The crude product was purified by column chromatography on silica gel eluting with DCM:MeOH (95:5) with 10% NH₄OH to give the title compound as an oil (69 mg, 18% yield).
¹H NMR (400MHz, CDCl₃) δ7.20 (d, 2H), 6.86 (d, 2H), 4.02 (t, 2H), 3.78 - 3.48 (m, 4H), 3.29 (t, 2H), 3.27 (s, 3H), 2.67 (t, 2H), 2.59 (m, 4H), 2.48 (s, 2H), 2.39 (t, 2H), 2.11 - 2.00 (m, 4H), 1.98 (s, 3H), 1.92 - 1.85 (m, 2H), 1.84 - 1.80 (m, 4H).

### Example 62: methyl-pyrymidin-2-yl-{4-[4-(3-pyrrolidin-1-ylpropoxy)-phenyl]-tetrahydropyran-4-ylmethyl}amine

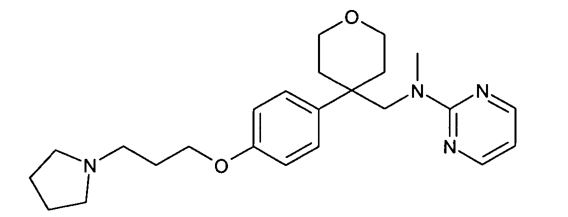

To a solution of methyl-{4-[4-(3-pyrrolidin-1-ylpropoxy)phenyl]tetrahydropyran-4-ylmethyl}amine (150 mg, 0.45 mmol) and 2-chloropyrymidine (52 mg, 0.45 mmol) in anhydrous acetonitrile (5 mL) was added potassium carbonate (62 mg, 0.45 mmol). The mixture was heated to 60°C overnight then refluxed for 2 days, filtered and concentrated under reduced pressure. The crude product was purified by column chromatography on silica gel eluting with DCM:MeOH (97:3) with 10% NH₄OH to give the title compound (40 mg, 22% yield).
¹H NMR (400MHz, CDCl₃) δ8.24 (m, 2H), 7.20 (d, 2H), 6.88 (d, 2H), 6.42 (m, 1 H), 4.02 (m, 2H), 3.80 (m, 4H), 3.52 (t, 2H), 2.59 (m, 9H), 2.01 (m, 8H), 1.79 (s, 3H).

### Example 63: N-{4-[4-(3-Pyrrolidin-1-yl-propoxy)-phenyl]-tetrahydro-pyran-4-ylmethyl}-methanesulfonamide

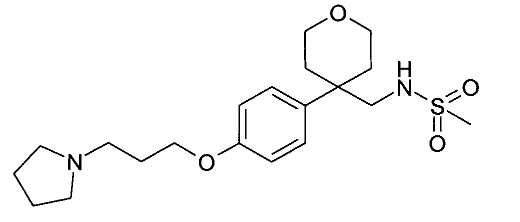

To a stirred solution of {4-[4-(3-pyrrolidin-1-ylpropoxy)phenyl]tetrahydropyran-4-yl} methylamine (200 mg, 0.628 mmol) and triethylamine (106 µL, 0.754 mmol) in anhydrous dichloromethane (8 mL) under nitrogen atmosphere was added dropwise a solution of mesyl chloride (58 µL, 0.754 mmol) in anhydrous dichloromethane (2 mL). After stirring overnight, the reaction mixture was quenched with a saturated aqueous solution of NaHCO₃. The organic layer was separated and washed with a saturated aqueous solution of NaHCO₃ and water then dried over magnesium sulphate, filtered and concentrated under reduced pressure to give the title compound (140 mg, 56 %).
¹H NMR (400MHz, CDCl₃) δ7.19 (d, 2H), 6.94 (d, 2H), 4.04 (t, 2H), 3.79 (m, 2H), 3.57 (m, 2H), 3.24 (d, 2H), 2.75 (s, 3H), 2.63 (t, 2H), 2.54 (m, 4H), 2.12 (m, 2H), 2.01 (m, 2H), 1.88 (m, 2H), 1.80 (m, 4H).

### Example 64: C-Phenyl-N-{4-[4-(3-pyrrolidin-1-yl-propoxy)-phenyl]-tetrahydropyran-4-ylmethyl}-methanesulfonamide

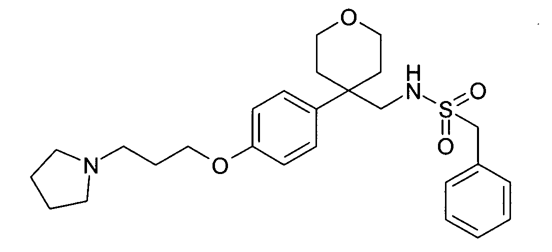

To a stirred solution of {4-[4-(3-pyrrolidin-1-ylpropoxy)phenyl]tetrahydropyran-4-yl} methylamine (200 mg, 0.628 mmol) and triethylamine (106 µL, 0.754 mmol) in anhydrous dichloromethane (8 mL) under nitrogen atmosphere was added dropwise a solution of α-toluenesulfonylchloride (143 mg, 0.754 mmol) in anhydrous dichloromethane (2 mL). After stirring overnight, the reaction mixture was quenched with a saturated aqueous solution of NaHCO₃. The organic layer was separated and washed with water, dried over magnesium sulphate, filtered and concentrated under reduced pressure to give the title compound (207 mg, 69 %).
¹H NMR (400MHZ, CDCL₃) δ7.27-7.22 (M, 3H), 7.16-7.14 (M, 2H), 7.07 (D, 2H), 6.83 (D, 2H), 4.05 (S, 2H), 3.95 (T, 2H), 3.68-3.63 (M, 2H), 3.55 (T, 1 H), 3.49-3.44 (M, 2H), 3.01(D,2H), 2.57 (T,2H), 2.47 (S,4H), 2.02-1.93 (M, 4H), 1.78-1.72 (M, 6H).

### Example 65: 3-Cyano-N-{4-[4-(3-pyrrolidin-1-yl-propoxy)-phenyl]-tetrahydropyran-4-ylmethyl}-benzenesulfonamide

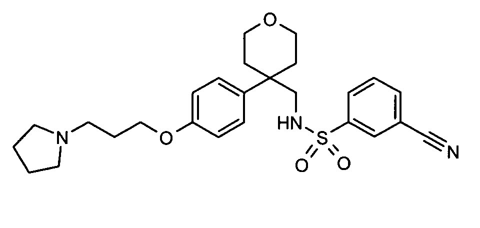

To a stirred solution of {4-[4-(3-pyrrolidin-1-ylpropoxy)phenyl]tetrahydropyran-4-yl} methylamine (100 mg, 0.314 mmol) and triethylamine (0.1 mL, 0.63 mmol) in anhydrous dichloromethane (3 mL) under nitrogen atmosphere at 0°C was added 3-cyanobenzenesulfonylchloride (100 mg, 0.47 mmol). The reaction mixture was stirred for 2 h allowing it to warm up to room temperature. It was then diluted with dichloromethane and quenched with water. The organic layer was separated and washed with water, dried over sodium sulfate, filtered and concentrated under reduced pressure. The crude product was purified by column chromatography on silica gel eluting with DCM:MeOH (95 :5) folowed by DCM:MeOH (90 :10) to give the title compound.
¹H NMR (400MHz, CDCl₃) δ7.97 (s, 1 H), 7.93-7.91 (d, 1 H), 7.83-7.81 (d, 1 H), 7.62-7.58 (m, 1H), 7.11-7.09 (d, 2H), 6.89-6.86 (d, 2H), 4.06-4.03 (m, 3H), 3.77-3.72 (m, 2H), 3.56-3.51 (m, 2H),3.08 (br s,2H), 2.70-2.67 (m, 2H), 3.08 (br s,4H), 2.08-2.01 (m, 4H), 1.85-1.82 (m, 6H).

### Example 66: 2-Fluoro-N-{4-[4-(3-pyrrolidin-1-yl-propoxy)-phenyl]-tetrahydropyran-4-ylmethyl}-benzenesulfonamide

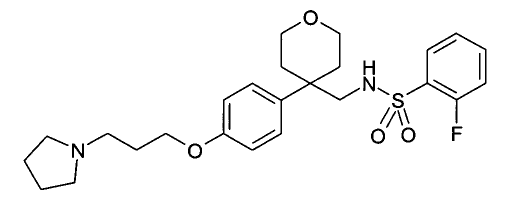

To a stirred solution of {4-[4-(3-pyrrolidin-1-ylpropoxy)phenyl]tetrahydropyran-4-yl} methylamine (100 mg, 0.314 mmol) and triethylamine (0.1 mL, 0.63 mmol) in anhydrous dichloromethane (3 mL) under nitrogen atmosphere at 0°C was added 2-fluorobenzenesulfonylchloride (91 mg, 0.47 mmol). The reaction mixture was stirred for 2 h allowing it to warm up to room temperature. It was then diluted with dichloromethane and quenched with a saturated aqueous solution of NaHCO₃. The organic layer was separated and washed with water, dried over sodium sulfate, filtered and concentrated under reduced pressure to give the title compound (120 mg, 80%).
¹H NMR (400MHz, CDCl₃) δ7.86-7.82 (t, 1 H), 7.58-7.52 (m, 1 H), 7.28-7.24 (m, 1 H), 7.13-7.12 (m, 3H), 6.90-6.88 (m, 2H), 4.19 (m, 1H), 4.05-4.02 (t, 2H), 3.73-3.70 (m, 2H), 3.56-3.51 (t, 2H), 3.10-3.08 (d, 2H), 2.66-2.62 (t, 2H), 2.54 (m, 4H), 2.06-1.99 (m, 4H), 1.85-1.8 (m, 6H).

### Example 67: N-methyl-N-{4-[4-(3-Pyrrolidin-1-yl-propoxy)-phenyl]-tetrahydropyran-4-ylmethyl}-methanesulfonamide

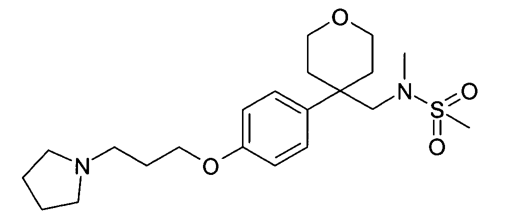

To a stirred solution of methyl-{4-[4-(3-pyrrolidin-1-ylpropoxy)phenyl]tetrahydropyran-4-ylmethyl}amine (200 mg, 0.60 mmol) and triethylamine (102 µL, 0.72 mmol) in anhydrous dichloromethane (8 mL) under nitrogen atmosphere was added dropwise a solution of mesyl chloride (56 µL, 0.72 mmol) in anhydrous dichloromethane (2 mL). After stirring overnight, the reaction mixture was quenched with a saturated aqueous solution of NaHCO₃. The organic layer was separated, washed with water then dried over magnesium sulfate, filtered and concentrated under reduced pressure to give the title compound (175 mg, 71 %).
¹H NMR (400MHz, CDCl₃) δ7.22 (d, 2H), 6.92-6.90 (d, 2H), 4.03 (t, 2H), 3.83-3.78 (m, 2H), 3.52 (t, 2H), 3.16 (s, 2H), 2.68 (s, 3H), 2.64 (t, 2H), 2.54 (m, 4H), 2.19-2.15 (m, 5H), 2.05-1.91 (m,4H), 1.81-1.78 (m, 4H).

### Example 68 : N'-{[4-(4-(3-Pyrrolidin-1-yl-propoxy)-phenyl)tetrahydro-2H-pyran-4-yl]methyl}-N,N-dimethylsulfamide

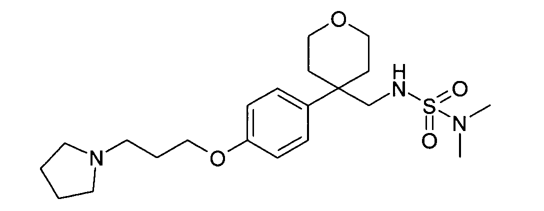

To a stirred solution of {4-[4-(3-pyrrolidin-1-ylpropoxy)phenyl]tetrahydropyran-4-yl} methylamine (200 mg, 0.628 mmol) and triethylamine (106 µL, 0.754 mmol) in anhydrous dichloromethane (8 mL) under nitrogen atmosphere was added dropwise a solution of dimethylsulfamoyl chloride (81 µL, 0.754 mmol) in anhydrous dichloromethane (2 mL). After stirring overnight, the reaction mixture was quenched with a saturated aqueous solution of NaHCO₃. The organic layer was separated and washed with water then dried over magnesium sulfate, filtered and concentrated under reduced pressure to give the title compound (207 mg, 77 %).
¹H NMR (400MHZ, CDCL₃) δ7.19 (D, 2H), 6.93-6.91 (D, 2H), 4.05 (T, 2H), 3.78-3.76 (M, 2H), 3.59-3.54 (M, 2H), 3.17-3.15 (D, 2H), 2.76-2.70 (M, 12H), 2.15-2.06 (M, 4H), 1.90-1.87 (M, 6H).

### Example 69: N-{4-[4-(3-Pyrrolidin-1-yl-propoxy)-phenyl]-tetrahydro-pyran-4-ylmethyl}-acetamide

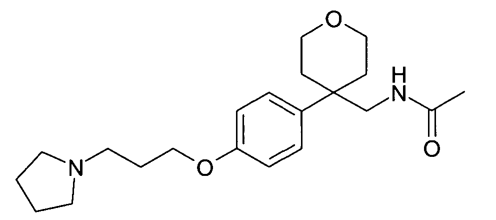

To a stirred solution of {4-[4-(3-pyrrolidin-1-ylpropoxy)phenyl]tetrahydropyran-4-yl} methylamine (200 mg, 0.628 mmol) and triethylamine (106 µL, 0.754 mmol) in anhydrous dichloromethane (8 mL) under nitrogen atmosphere was added dropwise a solution of acetyl chloride (54 µL, 0.754 mmol) in anhydrous dichloromethane (2 mL). After stirring overnight, the reaction mixture was quenched with a saturated aqueous solution of NaHCO₃. The organic layer was separated and washed with a saturated aqueous solution of NaHCO₃ and water then dried over magnesium sulfate, filtered and concentrated under reduced pressure to give the title compound (102 mg, 45 %).
¹H NMR (400MHz, CDCl₃) δ7.18 (d, 2H), 6.94 (d, 2H), 4.97 (s, 1 H), 4.05 (t, 2H), 3.80 (m, 2H), 3.59 (m, 2H), 3.46 (d, 2H), 2.66 (t, 2H), 2.57 (m, 4H), 2.03 (m, 4H), 1.80 (m,8H).

### Example 70: N-methyl-N-{4-[4-(3-Pyrrolidin-1-yl-propoxy)-phenyl]-tetrahydro-pyran-4-ylmethyl}-acetamide

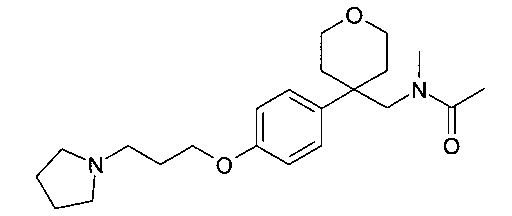

To a stirred solution of methyl-{4-[4-(3-pyrrolidin-1-ylpropoxy)phenyl]tetrahydropyran-4-ylmethyl}amine (200 mg, 0.60 mmol) and triethylamine (102 µL, 0.72 mmol) in anhydrous dichloromethane (8 mL) under nitrogen atmosphere was added dropwise a solution of acetyl chloride (52 µL, 0.72 mmol) in anhydrous dichloromethane (2 mL). After stirring overnight, the reaction mixture was quenched with a saturated aqueous solution of NaHCO₃. The organic layer was separated and washed with water then dried over magnesium sulfate, filtered, concentrated under reduced pressure and purified by column chromatography on silica gel eluting with DCM:MeOH (95 :5) folowed by DCM:MeOH (90 :10) to give the title compound (86 mg, 36 %).
¹H NMR (400MHz, CDCl₃) δShows rotomers 7.19-7.13 (2d, 2H), 6.91-6.88 (d, 2H), 4.05-4.02 (m, 2H), 3.85-3.80 (m, 2H), 3.56-3.45 (m, 4H), 2.67-2.65 (m, 2H), 2.60-2.56 (m, 4H), 2.32-1.96 (several multiplets, 12H).

### Example 71: 2-Phenyl-N-{4-[4-(3-pyrrolidin-1-yl-propoxy)-phenyl]-tetrahydropyran-4-ylmethyl}-acetamide

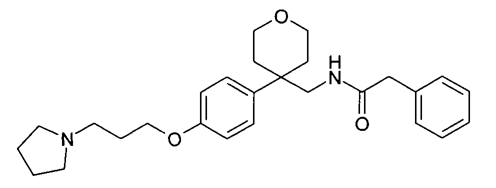

To a stirred solution of {4-[4-(3-pyrrolidin-1-ylpropoxy)phenyl]tetrahydropyran-4-yl} methylamine (200 mg, 0.63 mmol) and triethylamine (93 µL, 0.66 mmol) in anhydrous dichloromethane (8 mL) under nitrogen atmosphere at 0°C was added dropwise a solution of phenylacetyl chloride (93 µL, 0.66 mmol) in anhydrous dichloromethane (2 mL). After stirring for 3 h at 0°C, the reaction mixture was quenched with a saturated aqueous solution of NaHCO₃. The organic layer was separated and washed with water then dried over magnesium sulfate, filtered, concentrated under reduced pressure and purified by column chromatography on silica gel eluting with DCM:MeOH (98 : 2) to give the title compound (34 mg, 12 %).
¹H NMR (400MHz, CDCl₃) δ7.32-7.31 (m, 3H), 7.12-7.10 (m, 2H), 6.89-6.87 (d, 2H), 6.76-6.74 (d, 2H), 4.91 (t, 1 H), 4.02 (t, 2H), 3.79-3.74 (m, 2H), 3.53-3.51 (m, 2H), 3.47 (s, 2H), 3.37-3.35 (d, 2H), 2.62 (m, 6H), 2.08-2.06 (m, 2H), 1.93-1.85 (m, 6H), 1.76-1.74 (m, 2H).

### Example 72: 1,1-Dimethyl-3-{4-[4-(3-pyrrolidin-1-yl-propoxy)-phenyl]-tetrahydro-pyran-4-ylmethyl}-urea

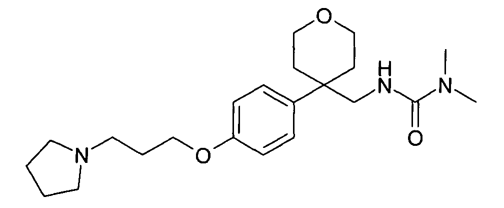

To a stirred solution of {4-[4-(3-pyrrolidin-1-ylpropoxy)phenyl]tetrahydropyran-4-yl} methylamine (200 mg, 0.63 mmol) and triethylamine (106 µL, 0.754 mmol) in anhydrous dichloromethane (9 mL) under nitrogen atmosphere was added dropwise a solution of dimethylcarbamoylchloride (69 µL, 0.754 mmol) in anhydrous dichloromethane (1 mL). After stirring for 3 h at 0°C, the reaction mixture was quenched with a saturated aqueous solution of NaHCO₃. The organic layer was separated and washed with water then dried over magnesium sulfate, filtered, concentrated under reduced pressure and purified by column chromatography on silica gel eluting with DCM:MeOH (95: 5) followed by DCM:MeOH (90 : 10) to give the title compound (80 mg, 32 %).
¹H NMR (400MHz, CDCl₃) δ7.13 (d, 2H), 6.86 (d, 2H), 3.98 (t, 2H), 3.84 (t, 1 H), 3.74 (m, 2H), 3.52 (m, 2H), 3.36 (d, 2H), 2.69 (s, 6H), 2.65 (m, 2H), 2.57 (m, 4H), 2.02-1.95 (m,4H), 1.84-1.78 (m, 6H).

### Example 73: 1,1,3-Trimethyl-3-{4-[4-(3-pyrrolidin-1-yl-propoxy)-phenyl]-tetrahydro-pyran-4-ylmethyl}-urea

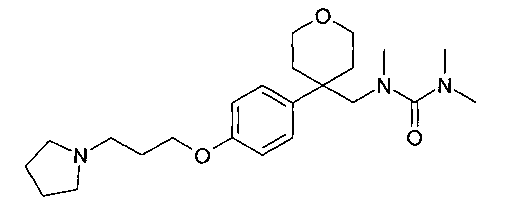

To a stirred solution of methyl-{4-[4-(3-pyrrolidin-1-ylpropoxy)phenyl]tetrahydropyran-4-ylmethyl}amine (200 mg, 0.60 mmol) and triethylamine (102 µL, 0.72 mmol) in anhydrous dichloromethane (8 mL) under nitrogen atmosphere was added dropwise a solution of dimethylcarbamoylchloride (67 µL, 0.72 mmol) in anhydrous dichloromethane (2 mL). After stirring overnight, the reaction mixture was quenched with a saturated aqueous solution of NaHCO₃. The organic layer was separated, washed with a saturated aqueous solution of NaHCO₃ and water then dried over magnesium sulfate, filtered, concentrated under reduced pressure and purified by column chromatography on silica gel eluting with DCM:MeOH (95 :5) followed by DCM:MeOH:NH₄OH (90:5:5) to give the title compound (84 mg, 39 %).
¹H NMR (400MHz, CDCl₃) δ7.19 (d, 2H), 6.90-6.88 (d, 2H), 4.03 (t, 2H), 3.81-3.78 (m, 2H), 3.56-3.52 (m, 4H), 2.70 (s, 6H), 2.66 (t, 2H), 2.29 (s, 3H), 2.05-2.01 (m, 4H), 1.86-1.81 (m, 6H).

### Example 74: {4-[4-(3-Pyrrolidin-1-yl-propoxy)-phenyl]-tetrahydro-pyran-4-ylmethyl}-urea

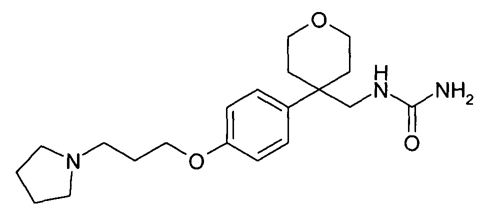

To a stirred solution of {4-[4-(3-pyrrolidin-1-ylpropoxy)phenyl]tetrahydropyran-4-yl} methylamine (200 mg, 0.63 mmol) and acetic acid (108 µL, 1.89 mmol) in water (12 mL) was added portionwise potassium cyanate (153 mg, 1.89 mmol). After stirring overnight, the reaction mixture was quenched with a 0.1 N aqueous solution of NaOH. The organic layer was separated and the aqueous layer extracted twice with dichloromethane. The combined organic extracts were dried over magnesium sulfate, filtered, concentrated under reduced pressure and purified by column chromatography on silica gel eluting with DCM:MeOH (95:5) followed by DCM:MeOH (90:10) and DCM:MeOH:NH₄OH (90:5:5) to give the title compound (25 mg, 11 %).
¹H NMR (400MHz, CDCl₃) δ7.18 (d, 2H), 6.92-6.90 (d, 2H), 4.24 (s, 2H), 4.17 (t, 1H), 4.03 (t, 2H), 3.84-3.79 (m, 2H), 3.61-3.55 (m, 2H), 3.36-3.35 (d, 2H), 2.63 (t, 2H), 2.53 (m, 4H), 2.07-1.97 (m, 4H), 1.88-1.79 (m, 6H).

### Intermediate 29: 4-aminomethyl-4-[4-(3-pyrrolidin-1-yl-propoxy)-phenyl]-cyclohexanol

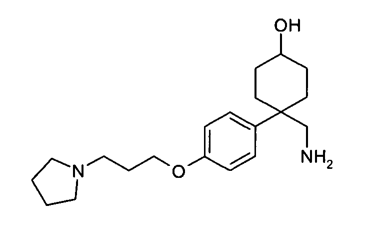

### Step 1:

A three neck reaction vessel was charged with [4-(3-pyrrolidin-1-ylpropoxy)phenyl]acetonitrile (7g, 28.6 mmol), benzyltrimethylammonium hydroxide (40% in methanol, 1.3 mL) in acetonitrile (185 mL). The solution was heated to reflux and methyl acrylate (25 mL, 286 mmol) was added dropwise. After refluxing for 5 hr, the mixture was concentrated to half, diethyl ether was added and the organics were washed with HCl 1N and brine, dried over Na₂SO₄, filtered and concentrated. The residue was purified by flash-chromatography to provide 4-cyano-4-[4-(3-pyrrolidin-1-ylpropoxy)-phenyl]-heptanedioic acid dimethyl ester (8.8 g, 74%).
¹H NMR (400MHz, CDCl₃) δ7.28 ( d,2H); 6.95 (d,2H); 4.08-4.0 ( m,2H ); 3.6 (s,6H); 2.68-2.60 (m,2H); 2.58-2.45 (m,6H); 2.40-2.20 (m,4H); 2.20-2.12 (m,2H); 2.08-2.0 (m,2H); 1.80-1.75 (m,4H)

### Step 2:

To a solution of 4-cyano-4-[4-(3-pyrrolidin-1-ylpropoxy)-phenyl]-heptanedioic acid dimethyl ester (8.8 g, 21.1 mmol) in 1,2-dimethoxyethane (176 mL) was charged portions of NaH ( 60% dispersion, 2.55 g, 63.4 mmol). The reaction mixture was heated to reflux. After 4.5 hr, the ¾ of solvent were evaporated and the mixture cooled to 20°C with an ice bath and quenched with water (100 mL), HCl 1N (100 mL). The aqueous layer was extracted with ether (100 mL). After basification with NaOH the aqueous phases were extracted with dichloromethane. The combined organics were washed with water, dried over Na₂SO₄, filtrered and concentrated to provide 5-cyano-2-oxo-5-[4-(3-pyrrolidin-1-ylpropoxy)-phenyl]-cyclohexanecarboxylic acid methyl ester (6.6 g , 81 %) as an oil.
¹H NMR (400MHz, CDCl₃) δ12.2 (bs, 1 H); 7.33 (d, 2H); 6.95 (d, 2H); 4.08-4.00 (m, 2H); 3.75 (s, 3H); 2.95 (d, 1 H); 2.80-2.70 (m, 1 H); 265-2.60 (m, 2H); 2.50-2.42 (m, 6H); 2.30-2.15 (m, 2H); 2.05-1.95 (m, 2H); 1.90-1.85 (m, 4H).

### Step 3:

To a solution of 5-cyano-2-oxo-5-[4-(3-pyrrolidin-1-ylpropoxy)-phenyl]-cyclohexanecarboxylic acid methyl ester (6.6 g, 17 mmol) and dimethylsulfoxide (128 mL) was added water (8.0 mL) and sodium chloride (6.4 g, 109 mmol). The reaction mixture was heated to 142-146°C. After 5 hr, the mixture was concentrated and the residue was dissolved in CH₂Cl₂ (120 mL), washed with water (100 mL), and brine, dried over Na₂SO₄, filtered and concentrated. The residue was purified by flash chromatography (70 g silica gel, eluant : gradient of CH₂Cl₂/MeOH 95/5 to 90/10) providing 4-oxo-1-[4-(3-pyrrolidin-1-ylpropoxy)-phenyl]-cyclohexanecarbonitrile as a crystallizing oil (2.2 g 45.5 %). After crystallization in ether, 0.2g of analytical sample were obtained.
¹H NMR (400MHz, CDCl₃) δ7.32 (d, 2H), 6.88(d,2H), 4. (t, 2H), 2.95 -2.8 (m, 2H), 2.65-2.38 (m, 10H), 2.3-2.15 (m, 2H), 2 - 1.9 (m, 2H),18 - 1.7 (m, 4H),.

### Step 4:

To a stirred suspension of LiAlH₄ (0.59 g, 15 mmol, 10 eq) in diethyl ether (8 mL) was added dropwise at ambient temperature a solution of 4-oxo-1-[4-(3-pyrrolidin-1-ylpropoxy)-phenyl]-cyclohexanecarbonitrile (0.5 g, 1.5 mmol) in diethyl ether (25 mL). The reaction mixture was heated to reflux for 2 h. The reaction mixture was cooled to 0°C and, successively, a solution of water, sodium hydroxide (15% w/v, 0.68 mL) and water again were carefully dropped. After being stirred for 15 min at room temperature, the mixture was filtered over diatomaceous earth and concentrated. The residue was purified by flash chromatography (10 g silica gel, CH₂Cl₂/ MeOH ( 10% NH₄OH) to provide the title compound (0.27 g, 54%) as an oil.
¹H NMR (400MHz, CDCl₃) δ7.25-7.18 (m,2H); 6.92-6.80 (m,2H); 4.08-3.98 (m,2H); 3.85-3.80 (m,1H); 2.72 (s,2H); 2.65-2.55 (m,2H); 2.55-2.45 (m,4H); 2.05-1.20 (m,17H).

### Example 75: 4-dimethylaminomethyl-4-[4-(3-pyrrolidin-1-yl-propoxy)-phenyl]-cyclohexanol

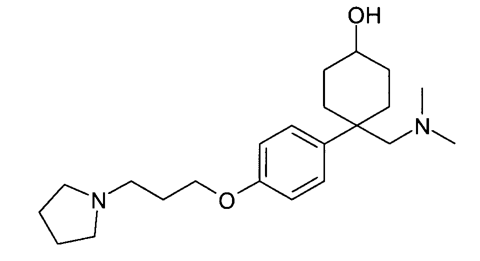

To 4-aminomethyl-4-[4-(3-pyrrolidin-1-yl-propoxy)-phenyl]-cyclohexanol (0.26 g, 0.78 mmol) was added 0.8 mL of formaldehyde (37% w/w solution in H₂O). 1 mL of formic acid was added and the mixture was heated to about 100°C for 20 min. After cooling, water was added and the mixture extracted with ether. NaOH was added at the aqueous phase and the mixture extracted 3 times with ether. The organic extracts were washed with brine, dried over Na₂SO₄, filtered and concentrated *in vacuo* to provide 0.23 g of oil. The residue was purified by flash chromatography (25 g silica gel, CH₂Cl₂/ MeOH (10% NH₄OH) to provide 0.18 g of product. After trituration with 10 mL hexanes (0.13 g, 46.4%) of title compound were obtained.
¹H NMR (400MHz, CDCl₃) δ7.25 (d, 2H), 6.85(d,2H), 4. (t, 2H), 3.8 (bs, 1 H), 2.62 (t, 2H), 2.56-2.49 (m, 4H), 2.39 (s, 2H), 2.08 - 1.92 (m, 3H), 1.95 (s,6H), 1.85 - 1.74 (m, 6H), 1.73-1.64 (m, 4H), 1.62-1.52 (m, 2H).

### General procedure E:

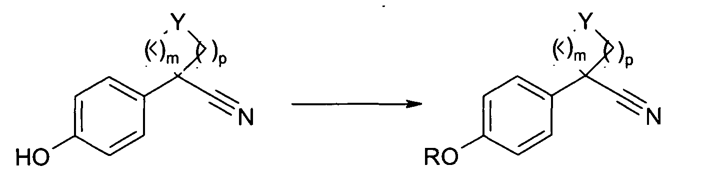

Starting phenol (3.69mmol), chloride (5.90mmol), DMF (15ml) and K₂CO₃ (14.83mmol) was heated to 130°C until the reaction is complete (1 to 2 hrs). Cooled to ambient temperature, water (30ml) was added, extracted with EtOAc (3x9ml) and the combined organic extracts were washed with NaOH (2M, 2x25ml). The organics were dried over MgSO₄, filtered and concentrated in *vacuo* at 35°C. Purification of the crude material by chromatography on silica, eluant (10%MeOH in DCM) provided the title compounds.

### Example 76: 4-[3-(3-Pyrrolidin-1-ylpropoxy)phenyl]tetrahydropyran-4-carbonitrile

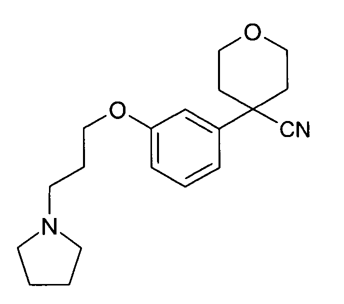

4-(3-Hydroxy-phenyl)-tetrahydro-2H-pyran-4-carbonitrile (1.03g, 5.10mmol), 1-(3-chloro-propyl)-pyrrolidine (600mg, 4.08mmol), DMF (6.8ml) and K₂CO₃ (2.82g, 20.4mmol) were reacted together according to general procedure E. The organic phase was washed with 2M NaOH (3 x 50ml), brine (3 x 50ml), dried over MgSO₄, fitered and concentrated *in vacuo* at 35°C. The crude mixture was slurried in TBME :heptane (1 :20, 10ml). The solid was filtered, washed with heptane (10ml) and dried on the filter for 2hours to give the title compound (0.4g, 31%th).
¹H NMR (400MHz, CDCl₃) δ7.32 (t, 1H), 7.07-6.99 (m, 2H), 6.87 (dd, 1H), 4.11-4.04 (m, 2H), 4.05 (t, 2H), 3.90 (td, 2H), 2.63 (t, 2H), 2.57-2.49 (m, 4H), 2.18-1.97 (m, 6H), 1.84-1.75 (m, 4H).

### Example 77: 4-{4-[3-(2,5-Dimethylpyrrolidin-1-yl)propoxy]phenyl}-tetrahydropyran-4-carbonitrile

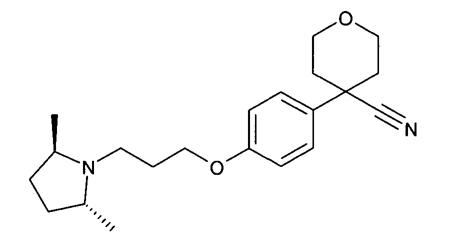

4-[4-(3-pyrrolidin-1-ylpropoxy)phenyl]tetrahydro-2*H*-pyran-4-carbonitrile (318mg, 1.56mmol), 1-(3-chloro-propyl)-2,5-*trans*-dimethyl-pyrrolidine (250mg, 1.42mmol), DMF (5ml) and K₂CO₃ (785mg, 5.70mmol) were reacted together according to general procedure E. The crude was dissolved in ethyl acetate (20ml), washed with brine (2 x 10ml), dried over MgSO₄, filtered and concentrated *in vacuo* to give the title compound (200mg, 56%th) as a light brown solid.
¹H NMR (400MHz, CDCl₃), δ7.37 (d, 2H), 6.93 (d, 2H), 4.13-3.97 (m, 4H), 3.89 (td, 2H), 3.14-2.98 (m, 2H), 2.76 (m, 1 H), 2.55 (m, 1 H), 2.16-1.88 (m, 8H), 1.46-1.30 (m, 2H), 0.97 (d, 6H).

### Example 78: 4-{4-[3-(2-Methylpyrrolidin-1-yl)-propoxy]-phenyl}tetrahydropyran-4-carbonitrile

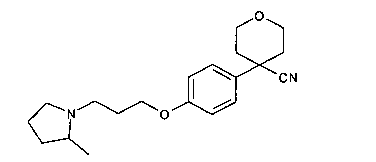

4-[4-(3-pyrrolidin-1-ylpropoxy)phenyl]tetrahydro-2*H*-pyran-4-carbonitrile (315mg, 1.55m mol), 1-(3-chloro-propyl)-2-methyl-pyrrolidine (400mg, 2.48mmol), DMF (6ml) and K₂CO₃ (833mg, 6.03mmol) were reacted together according to general procedure E. Purification by chromatography on silica, eluant DCM :MeOH, NH₃ (92 :6 :2) provided the title compound (450mg, 88%th) as an orange oil.
¹H NMR (400MHz, CDCl₃), δ7.37 (d, 2H), 6.93 (d, 2H), 4.12-3.99 (m, 4H), 3.89 (td, 2H), 2.35-1.62 (m, 14H), 1.42 (m, 1H), 1.09 (d, 3H).

### Example 79: 4-[4-(3-Thiomorpholin-4-ylpropoxy)phenyl]tetrahydropyran-4-carbonitrile

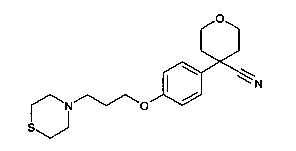

4-[4-(3-pyrrolidin-1-ylpropoxy)phenyl]tetrahydro-2*H*-pyran-4-carbonitrile(750mg, 3.69m mol), 4-(3-chloro-propyl)-thiomorpholine (1.06g, 5.91 mmol), DMF (15ml) and K₂CO₃ (2.05g, 14.83mmol) were reacted together according to general procedure E. Purification by chromatography on silica, eluant DCM :MeOH : NH₃ (96 :3 :1) provided the title compound (365mg, 29%th) as an off white solid.
¹H NMR (400MHz, CDCl₃), δ7.37 (d, 2H), 6.93 (d, 2H), 4.12-4.04 (m, 2H), 4.01 (t, 2H), 3.89 (td, 2H), 2.77-2.64 (m, 8H), 2.54 (t, 2H), 2.16-1.99 (m, 4H), 1.95 (p, 2H).

### Example 80: 4-{4-[3-(1-Oxo-thiomorpholin-4-yl)-propoxy]-phenyl}-tetrahydropyran-4-carbonitrile

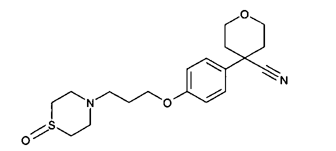

Sodium perborate tetrahydrate (221 mg, 1.43mmol) and glacial acetic acid (5ml) was heated to 50 to 60°C and 4-[4-(3-Thiomorpholin-4-ylpropoxy)phenyl]tetrahydropyran-4-carbonitrile (500mg, 1.43mmol) was added in one portion and the heating was maintained for 3 hours. The reaction mixture was cooled to ambient temperature and filtered. The filtrate was added to ice water (15ml) and extracted with EtOAc (3x5ml), which was discarded. The aqueous phase was basified to pH 8-9 with 2M sodium hydroxide and extracted with DCM (3x25ml), dried over MgSO₄, filtered and concentrated *in vacuo* at 35°C. The crude material was subjected to chromatography on silica eluting with DCM:MeOH:NH₄OH (97:2:1 to 92:6:2) to provided the title compound (300mg, 58%th) as a white solid.
¹H NMR (400MHz, CDCl₃), δ7.38 (d, 2H), 6.92 (d, 2H), 4.11-4.05 (m, 2H), 4.03 (t, 2H), 3.89 (td, 2H), 3.15-3.03 (m, 2H), 2.94-2.67 (m, 6H), 2.63 (t, 2H), 2.16-1.92 (m, 6H).

### Example 81: 4-{4-[3-(1,1-Dioxo-1λ6-thiomorpholin-4-yl)propoxy]phenyl}tetrahydro-pyran-4-carbonitrile

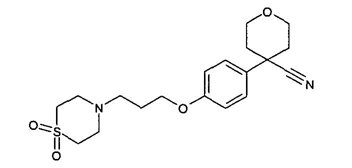

To a solution of 4-[4-(3-Thiomorpholin-4-ylpropoxy)phenyl]tetrahydropyran-4-carbonitrile (500mg, 1.44mmol) in TFA (1.65ml) at 0 to 5°C was added drop wise a solution of PTFA (4M in TFA, 0.72ml). The reaction was allowed to warm to room temperature and stirred for 3 hours. A further portion of PTFA (4M in TFA, 0.097ml) [4M solution prepared by the addition of 27.5% H₂O₂ (0.94ml) to TFA (1.56ml)] was added to the reaction and stirred overnight. The reaction mixture was cooled to 0 to 5°C, diluted with DCM (20ml) and quenched with NaOH (5M solution, 12ml) to attain pH10. The mixture was extracted with DCM (3 x 20ml), the combined DCM extracts were dried over MgSO₄, filtered and concentrated *in vacuo*. The isolated yellow oil was purified by column chromatography on basic alumina eluting with DCM(95):MeOH(4):NH₄OH(1) to give the title compound as an off-white solid (230mg, 42%th).
¹H NMR (400MHz, CDCl₃), δ7.39 (d, 2H), 6.92 (d, 2H), 4.12-4.05 (m, 2H), 4.03 (t, 2H), 3.89 (td, 2H), 3.11-2.96 (m, 8H), 2.71 (t, 2H), 2.15-1.99 (m, 4H), 1.96 (p, 2H).

### Example 82: 4-{4-[3-(4-Hydroxypiperidin-1-yl)propoxy]phenyl}tetrahydropyran-4-carbonitrile

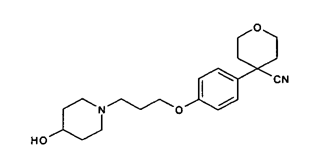

4-[4-(3-pyrrolidin-1-ylpropoxy)phenyl]tetrahydro-2*H*-pyran-4-carbonitrile (533mg, 2.62mmol), 1-(3-chloro-propyl)-piperidin-4-ol (750mg, 4.22mmol), DMF (10ml) and K₂CO₃ (1.44g, 10.42mmol) were reacted together according to general procedure E. Purification by chromatography on silica, eluant DCM : MeOH, NH₃ (92 :6 :2) provided the title compound (550mg, 61 %th) as an off white solid.
¹H NMR (400MHz, CDCl₃), δ7.38 (d, 2H), 6.93 (d, 2H), 4.13-4.04 (m, 2H), 4.02 (t, 2H), 3.89 (td, 2H), 3.70 (m, 1 H), 2.87-2.71 (m, 2H), 2.50 (t, 2H), 2.23-1.84 (m, 10H), 1.68-1.49 (m, 3H).

### Example 83: 4-(4-{3-[4-(2-Hydroxyethyl)-piperidin-1-yl]propoxy}phenyl)-tetrahydro-pyran-4-carbonitrile

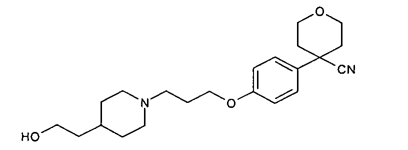

4-[4-(3-pyrrolidin-1-ylpropoxy)phenyl]tetrahydro-2*H*-pyran-4-carbonitrile (185mg, 0.91mmol), 2-[1-(3-chloro-propyl)-piperidin-4-yl]-ethanol (300mg, 1.46mmol), DMF (4ml) and K₂CO₃ (497mg, 3.60mmol) were reacted together according to general procedure E. Purification by chromatography on silica, eluant DCM : MeOH, NH₃ (92 :6 :2) provided the title compound (220mg, 65%th) as a pale pink solid.
¹H NMR (400MHz, CDCl₃), δ7.37 (d, 2H), 6.93 (d, 2H), 4.11-4.04 (m, 2H), 4.01 (t, 2H), 3.89 (td, 2H), 3.70 (t, 2H), 2.97-2.87 (m, 2H), 2.48 (t, 2H), 2.15-1.88 (m, 8H), 1.77-1.57 (m, 3H), 1.57-1.38 (m, 3H), 1.35-1.21 (m, 2H).

### Example 84: 1-{3-[4-(4-Cyano-tetrahydro-pyran-4-yl)-phenoxy]-propyl}-piperidine-4-carboxylic acid amide

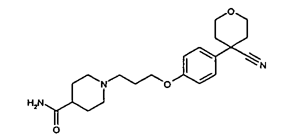

-[4-(3-pyrrolidin-1-ylpropoxy)phenyl]tetrahydro-2*H*-pyran-4-carbonitrile (200mg, 0.98mmol), 1-(3-chloro-propyl)-piperidine-4-carboxylic acid amide (200mg, 0.98mmol), DMF (4ml) and K₂CO₃ (550mg, 3.98mmol) were reacted together according to general procedure E. The reaction mixture was poured onto water (30ml), the resulting precipitate was filtered and redissolved in DCM (20ml). The DCM solution was washed with NaOH solution (2M, 2x5ml) and water (5ml), dried over MgSO₄, filtered and concentrated *in vacuo* at 35°C, displacing residual DCM with EtOH to provide the title compound (210mg, 58%th) as an off white solid.
¹H NMR (400MHz, CDCl₃), δ7.38 (d, 2H), 6.93 (d, 2H), 5.45 (br s, 1 H), 5.35 (br s, 1 H), 4.12-4.05 (m, 2H), 4.02 (t, 2H), 3.89 (td, 2H), 3.03-2.92 (m, 2H), 2.50 (t, 2H), 2.22-1.84 (m, 11H), 1.81-1.67 (m, 2H).

### Example 85: 4-{3-[4-(4-Cyanotetrahydropyran-4-yl)-phenoxy]propyl}-piperazine-1-carboxylic acid ethyl ester

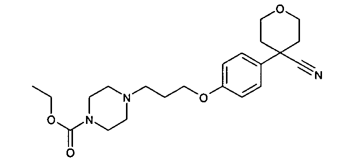

4-[4-(3-pyrrolidin-1-ylpropoxy)phenyl]tetrahydro-2*H*-pyran-4-carbonitrile (500mg, 2.46mmol), 4-(3-chloro-propyl)-piperazine-1-carboxylic acid ethyl ester (570mg, 2.43mmol), DMF (10ml) and K₂CO₃ (1.36g, 9.84mmol) were reacted together according to general procedure E. The organic phase was washed with 2M NaOH (3.x 20ml), water (2x 20ml), dried over MgSO₄, fitered and concentrated *in vacuo* at 35°C. The crude product was subjected to chromatography on silica eluting with DCM :MeOH :NH₄OH (97:2:1) to provide the title compound (280mg, 28%th) as an off white solid.
¹H NMR (400MHz, CDCl₃), δ7.38 (d, 2H), 6.93 (d, 2H), 4.14 (q, 2H), 4.11-4.05 (m, 2H), 4.03 (t, 2H), 3.89 (td, 2H), 3.55-3.42 (m, 4H), 2.53 (t, 2H), 2.47-2.36 (m, 4H), 2.15-1.92 (m, 6H), 1.26 (t, 3H).

### Intermediate 30: 4-(3-Chloro-propyl)-piperazine-1-carboxylic acid tert-butyl ester

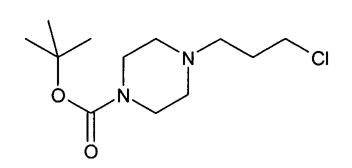

4-Piperazine-1-carboxylic acid *tert*-butyl ester (4.0g, 21.5mmol), acetone (80ml, 20vol), 5M NaOH solution (5.16ml, 1.2eq.) and 1-bromo-3-chloropropane (10.15g, 64.5mmol, 3eq.) were reacted together according to general procedure A to give the title compound (2.78g, 66%th) as a colourless oil.
¹H NMR (400MHz, CDCl₃) δ3.60 (t, 2H), 3.42 (t, 4H), 2.49 (t, 2H), 2.38 (t, 4H), 1.94 (p, 2H), 1.46 (s, 9H).

### Example 86: 4-{3-[4-(4-Cyano-tetrahydro-pyran-4-yl)-phenoxy]-propyl}-piperazine-1-carboxylic acid tert-butyl ester

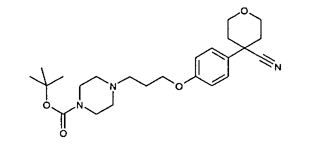

4-[4-(3-pyrrolidin-1-ylpropoxy)phenyl]tetrahydro-2*H*-pyran-4-carbonitrile (200mg, 0.99mmol), 4-(3-chloro-propyl)-piperazine-1-carboxylic acid *tert*-butyl ester (203mg, 0.76mmol), DMF (4ml) and K₂CO₃ (553g, 4.00mmol) was heated to 70°C and work up according to general procedure E. The organic phase was washed with 2M NaOH (3.x 20ml), water (2x 20ml), dried over MgSO₄, fitered and concentrate in vacuo at 35°C to provided the title compound (272mg, 64%th) as an off white solid.
¹H NMR (400MHz, CDCl₃), δ7.38 (d, 2H), 6.93 (d, 2H), 4.11-4.04 (m, 2H), 4.03 (t, 2H), 3.89 (td, 2H), 3.50-3.37 (m, 4H), 2.53 (t, 2H), 2.45-2.34 (m, 4H), 2.15-2.00 (m, 4H), 1.97 (p, 2H), 1.46 (s, 9H).

### Example 87: 4-[4-(3-Piperazin-1-ylpropoxy)phenyl]tetrahydropyran-4-carbonitrile

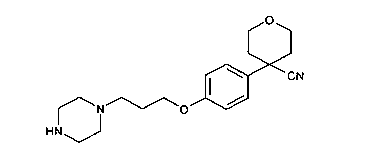

4-{3-[4-(4-Cyano-tetrahydro-pyran-4-yl)-phenoxy]-propyl}-piperazine-1-carboxylic acid *tert*-butyl ester (500mg, 1.16mmol), MeOH (5ml), 1,4-dioxane (1ml) and HCl in dioxane (4M, 1.8ml) was stirred at ambient temperature overnight. The reaction mixture was concentrated *in vacuo* at 40°C, and diluted with TBME (5ml). Aqueous NaOH (2M solution) was added to attain a pH of 14 and the mixture separated and extracted with TBME (2x5ml). The combined organic extracts were dried over MgSO₄, filtered and concentrated *in vacuo* to provide title compound as a yellow oil (275mg, 72%th).
¹H NMR (400MHz, CDCl₃), δ7.38 (d, 2H), 6.93 (d, 2H), 4.11-4.05 (m, 2H), 4.03 (t, 2H), 3.89 (td, 2H), 2.90 (t, 4H), 2.51 (t, 2H), 2.44 (br s, 4H), 2.15-2.00 (m, 4H), 1.97 (p, 2H), 1.74 (br s, 1H).

### Example 88: 4-(4-{3-[4-(2-Aminopropionyl)piperazin-1-yl]propoxy}phenyl)-tetrahydropyran-4-carbonitrile

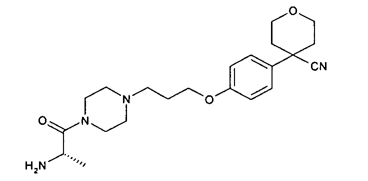

4-[4-(3-Piperazin-1-ylpropoxy)phenyl]tetrahydropyran-4-carbonitrile (265mg, 0.81mmol), DCM (10.6ml), HOBT (120mg, 0.89mmmol), FMoc (L)-aniline (277mg, 0.89mmol) and EDCl.HCl (171 mg, 0.89mmol) were stirred at ambient temperature overnight. Water (10ml) was added and stirred for one hour, the mixture was filtered and the organic phase separated and washed with water (10ml), the organic phase was dried over MgSO₄, filtered and concentrated *in vacuo* at 35°C. The residue was dissolved in DCM (9ml) and piperidine (86mg, 10mmol) was added, the mixture was stirred at ambient temperature for one hour. To the reaction mixture, water (5ml) was added and the organic phase separated, dried over MgSO₄, filtered and concentrated *in vacuo* at 35°C. The crude material was subjected to chromatography on silica eluting with 2% MeOH in DCM to provide title compound (136mg, 42%th).
¹H NMR (400MHz, CDCl₃), δ7.38 (d, 2H), 6.93 (d, 2H), 4.11-4.05 (m, 2H), 4.03 (t, 2H), 3.89 (td, 2H), 3.77 (q, 1 H), 3.65 (br s, 2H), 3.49 (br s, 2H), 2.55 (t, 2H), 2.45 (br s, 4H), 2.14-2.00 (m, 4H), 1.97 (p, 2H), 1.58 (br s, 2H), 1.25 (d, 3H).

### Intermediate 31: 2-[(3-Chloro-propyl)-methyl-amino]-ethanol

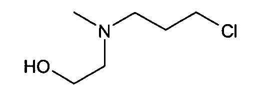

2-(Methylamino)-ethanol (1.0g, 13.3mmol), acetone (20ml, 20vol), 5M NaOH solution (3.19ml, 1.2eq.) and 1-bromo-3-chloropropane (6.28g, 39.9mmol, 3eq.) were reacted together according to general procedure A to give the title compound (1.14g, 55%th) as a colourless oil.
¹H NMR (400MHz, CDCl₃) δ3.60 (t, 2H), 3.57 (t, 2H), 2.82 (br s, 1 H), 2.57 (t, 2H), 2.54 (t, 2H), 2.26 (s, 3H), 1.95 (p, 2H).

### Example 89: 4-(4-{3-[(2-Hydroxyethyl)methylamino]propoxy}-phenyl)tetrahydropyran-4-carbonitrile

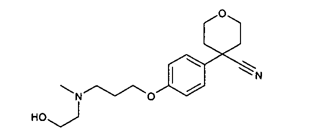

4-[4-(3-pyrrolidin-1-ylpropoxy)phenyl]tetrahydro-2*H*-pyran-4-carbonitrile (800mg, 3.94mmol), 2-[(3-chloro-propyl)-methyl-amino]-ethanol (595mg, 3.94mmol), DMF (8ml) and K₂CO₃ (2.18g, 15.76mmol) were reacted together according to general procedure E. The organic phase was washed with 2M NaOH (3.x 20ml), water (2x 20ml), dried over MgSO₄, fitered and concentrated *in vacuo* at 35°C to provided the title compound (550mg, 44%th) as a pale yellow oil.
¹H NMR (400MHz, CDCl₃), δ7.37 (d, 2H), 6.92 (d, 2H), 4.11-4.04 (m, 2H), 3.88 (dt, 2H), 3.60 (t, 2H), 2.61 (t, 2H), 2.56 (t, 2H), 2.37 (br s, 1 H), 2.29 (s, 3H), 2.15-1.91 (m, 6H)

### Example 90: 4-[4-(2-Pyrrolidin-1-ylethoxy)phenyl]tetrahydropyran-4-carbonitrile

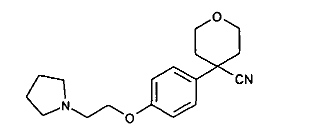

4-[4-(3-pyrrolidin-1-ylpropoxy)phenyl]tetrahydro-2*H*-pyran-4-carbonitrile (570mg, 2.82mmol), 1-(2-chloro-ethyl)-pyrrolidine (300mg, 2.25mmol), DMF (5.7ml) and K₂CO₃ (1.56g, 1.13mmol) were reacted together according to general procedure E. The organic phase was washed with 2M NaOH (3.x 20ml), water (2x 20ml), dried over MgSO₄, fitered and concentrated *in vacuo* at 35°C. The crude material was subjected to chromatography on silica eluting with 5% MeOH in DCM and gradient to 10% MeOH in DCM to provided the title compound (400mg, 47%th) as an off white solid.
¹H NMR (400MHz, CDCl₃), δ7.38 (d, 2H), 6.95 (d, 2H), 4.13 (t, 2H), 4.10-4.03 (m, 2H), 3.89 (td, 2H), 2.92 (t, 2H), 2.64 (br s, 4H), 2.15-1.99 (m, 4H), 1.88-1.76 (m, 4H).

### Example 91: 4-[4-(2-Methyl-3-pyrrolidin-1-ylpropoxy)phenyl]-tetrahydropyran-4-carbonitrile

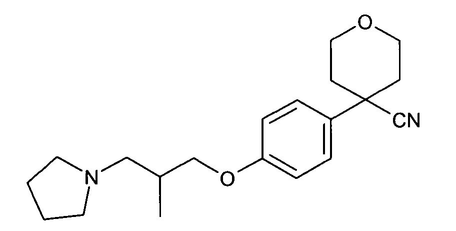

4-(4-Hydroxy-phenyl)-tetrahydro-pyran-4-carbonitrile (790mg, 3.87mmol), 1-(3-chloro-2-methyl-propyl)-pyrrolidine (500mg, 3.10mmol), DMF (5ml) and K₂CO₃ (2.14g, 15.50mmol) were reacted together according to general procedure E. The organic phase was washed with 2M NaOH (3 x 20ml), water (2 x 20ml), dried over MgSO₄, fitered and concentrated *in vacuo* at 35°C to provide the title compound (979mg, 96%th) as a yellow solid.
¹H NMR (400MHz, CDCl₃), δ7.37 (d, 2H), 6.95 (d, 2H), 4.11-4.03 (m, 2H), 4.02 (dd, 1 H), 3.89 (td, 2H), 3.77 (dd, 1 H), 2.60-2.42 (m, 5H), 2.32 (dd, 1 H), 2.22-1.99 (m, 5H), 1.82-1.72 (m, 4H), 1.07 (d, 3H).

### Intermediate 32: 1-(3-Chloro-1-methyl-propyl)-pyrrolidine hydrochloride

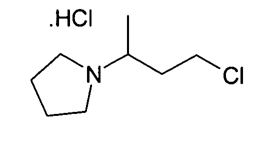

### Step 1:

Pyrrolidine (28.15g, 0.4mol), toluene (200ml), catalytic *p*-TsOH (200mg) and ethyl acetoacetate (20g, 0.15mol) were refluxed together with a Dean-Stark apparatus under N₂ for 3hours. The reaction was cooled to room temperature and concentrated *in vacuo.* NaBH₄ (3.1g, 82mmol) was dissolved in MeOH (50ml) and cooled to 0-5°C. A portion of the previous crude reaction (5g, 27mmol) in MeOH (25ml) was added to the reaction mixture and stirred for 72hours. The reaction was quenched with an aqueous solution of NaOH (1%w/w, 50ml) and concentrated *in vacuo.* The aqueous was extratcted with TBME (3 x 50ml). The combined organic extracts were dried over MgSO₄, filtered, washed with TBME and concentrated *in vacuo* to give a mixture of the title compound, 3-pyrrolidin-1-yl-butanoic acid ethyl ester and residual TBME (3.5g). The mixture (3.5g) in THF (20ml) was added to a solution of LiALH₄ (1 M in THF, 33.5ml, 33.5mmol) at 0-5°C under N₂. The reaction was allowed to warm to room temperature and stirred for 3hours. The reaction was quenched with an aqueous solution of NaOH (1%w/w, 50ml) at 0-5°C, filtered and washed with THF (3 x 20ml). The combined organics were dried over MgSO₄, filtered, washed with THF and concentrated *in vacuo* to give 3-pyrrolidin-1-yl-butan-1-ol as a yellow oil (2g, 73%th).
¹H NMR (400MHz, CDCl₃) δ5.98 (br s, 1 H), 3.90 (ddd, 1 H), 3.77 (ddd, 1H), 2.90 (m, 1 H), 2.70-2.55 (m, 4H), 1.80-1.56 (m, 6H), 1.09 (d, 3H).

### Step 2:

3-Pyrrolidin-1-yl-butan-1-ol (1.0g, 7mmol) was dissolved in DCM (20ml). The reaction was cooled to 0-5°C and thionyl chloride (1.65g, 14mmol) was added slowly. The reaction was allowed to warm to room temperature and stirred overnight. The reaction was concentrated *in vacuo* and azeo-dried with DCM (20ml) to give the title compound (1.4g, 100%th) as a brown oil.
¹H NMR (400MHz, CDCl₃) δ12.45 (br s, 1 H), 3.84-3.71 (m, 3H), 3.56 (m, 1H), 3.38 (m, 1 H), 2.92-2.79 (m, 2H), 2.45-1.99 (m, 6H), 1.51 (d, 3H).

### Example 92: 4-[4-(3-Pyrrolidin-1-ylbutoxy)phenyl]tetrahydropyran-4-carbonitrile

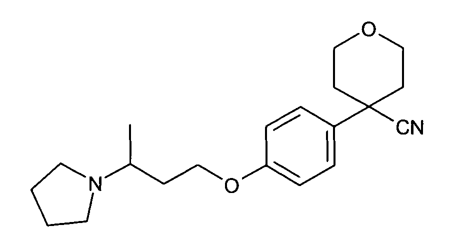

4-(4-Hydroxy-phenyl)-tetrahydro-pyran-4-carbonitrile (1.0g, 4.9mmol), 1-(3-chloro-1-methyl-propyl)-pyrrolidine (780mg, 3.90mmol), DMF (20ml) and K₂CO₃ (2.71 g, 19.60mmol) were reacted together according to general procedure E. The organic phase was washed with 2M NaOH (3 x 20ml), water (2 x 20ml), dried over MgSO₄, fitered and concentrated *in vacuo* at 35°C and purified by prep HPLC, eluting with acetonitrile/water/0.1%TFA as a gradient, to provide the title compound (120mg, 9%th) as a yellow oil.
¹H NMR (400MHz, CDCl₃), δ7.38 (d, 2H), 6.93 (d, 2H), 4.15-3.98 (m, 4H), 3.89 (td, 2H), 2.75-2.51 (m, 5H), 2.22-1.74 (m, 10H), 1.17 (d, 3H).

### Example 93: 4-{4-[2-(1-Methylpyrrolidin-2-yl)ethoxy]phenyl}tetrahydropyran-4-carbonitrile

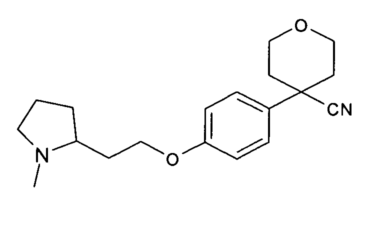

4-(4-Hydroxy-phenyl)-tetrahydro-pyran-4-carbonitrile (1.0g, 4.9mmol), 2-(2-chloro-ethyl)-1-methyl-pyrrolidine (722mg, 3.90mmol), DMF (20ml) and K₂CO₃ (2.71 g, 19.60mmol) were reacted together according to general procedure E. The organic phase was washed with 2M NaOH (3 x 20ml), water (2 x 20ml), dried over MgSO₄, fitered and concentrated *in vacuo* at 35°C and purified by prep TLC, eluting with DCM :MeOH :NH₄OH (95 :5 :3), to provide the title compound (180mg, 15%th) as a yellow oil.
¹H NMR (400MHz, CDCl₃), δ7.38 (d, 2H), 6.93 (d, 2H), 4.12-4.03 (m, 4H), 3.89 (td, 2H), 3.08 (m, 1 H), 2.35 (s, 3H), 2.29-1.94 (m, 8H), 1.85-1.65 (m, 2H), 1.62-1.50 (m, 2H).

### Example 94: 4-[4-(1-Isopropylpiperidin-4-yloxy)phenyl]tetrahydropyran-4-carbonitrile

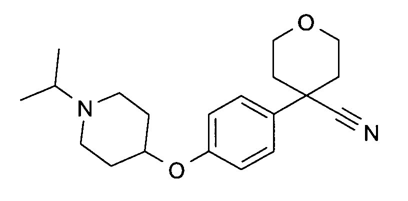

4-(4-Hydroxy-phenyl)-tetrahydro-pyran-4-carbonitrile (459mg, 2.26mmol) in DMF (2ml) was added to a solution of NaH (100mg, 2.5mmol) in DMF (2ml) at room temperature under N₂. The reaction was stirred for 1 hr and a solution of methanesulfonic acid 1-isopropyl-piperidin-4-yl ester (400mg, 1.81mmol) in DMF (1.3ml) was slowly added. The reaction was heated to 75°C and stirred for 6hrs. The reaction was allowed to cool to room temperature and diluted with TBME (20ml), water (10ml) and 5M NaOH solution (10ml). The organic layer was washed with 2.5M NaOH (2 x 20ml), brine (2 x 20ml), dried over MgSO₄, filtered and concentrated *in vacuo*. The crude reaction was purified by successive column chromatography, eluting with DCM :MeOH : NH₄OH (98 :1 :1) to give the title compound as a pale yellow solid (116mg, 19%th).
¹H NMR (400MHz, CDCl₃) δ7.37 (d, 2H), 6.93 (d, 2H), 4.30 (m, 1H), 4.12-4.02 (m, 2H), 3.89 (td, 2H), 2.84-2.68 (m, 3H), 2.45-2.34 (m, 2H), 2.15-1.96 (m, 6H), 1.88-1.75 (m, 2H), 1.06 (d, 6H).

### Example 95: 4-[4-(1-Cyclopentylpiperidin-4-yloxy)phenyl]tetrahydropyran-4-carbonitrile

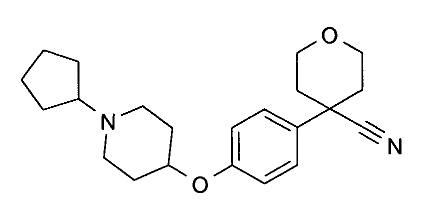

4-(4-Hydroxy-phenyl)-tetrahydro-pyran-4-carbonitrile (459mg, 2.26mmol) in DMF (2ml) was added to a solution of NaH (100mg, 2.5mmol) in DMF (2ml) at room temperature under N₂. The reaction was stirred for 1 hr and a solution of methanesulfonic acid 1-cyclopentyl-piperidin-4-yl ester (447mg, 1.81mmol) in DMF (1.3ml) was slowly added. The reaction was heated to 75°C and stirred for 6hrs. The reaction was allowed to cool to room temperature and diluted with TBME (20ml), water (10ml) and 5M NaOH solution (10ml). The organic layer was washed with 2.5M NaOH (2 x 20ml), brine (2 x 20ml), dried over MgSO₄, filtered and concentrated *in vacuo.* The crude reaction was purified by successive column chromatography, eluting with DCM :MeOH : NH₄OH (98 :1 :1) to give the title compound as a pale yellow solid (81 mg, 13%th).
¹H NMR (400MHz, CDCl₃) δ7.37 (d, 2H), 6.93 (d, 2H), 4.36 (m, 1H), 4.11-4.04 (m, 2H), 3.89 (td, 2H), 2.91-2.77 (m, 2H), 2.67-2.25 (m, 2H), 2.15-1.99 (m, 7H), 1.95-1.81 (m, 4H), 1.78-1.64 (m, 2H), 1.62-1.39 (m, 4H).

### Intermediates 33 and 34: 4-[4-(3-chloro-propoxy)-phenyl]-tetrahydropyran-4-carbonitrile and 4-[4-(3-bromo-propoxy)-phenyl]-tetrahydropyran-4-carbonitrile

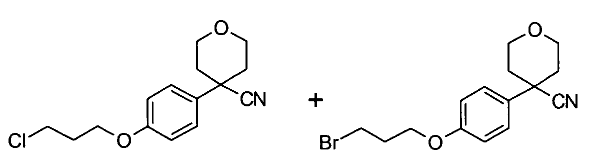

4-(4-hydroxy-phenyl)-tetrahydro-pyran-4-carbonitrile (3.4 g, 16.73 mmol) was taken in DMF (75 ml) and then Cs₂CO₃ (4.84 g, 25.095 mmol) was added followed by dropwise addition of 1-bomo-3-chloroproprane (2.45 ml) at 60 °C. The heating was continued at 60 °C overnight. First the reaction mixture was concentrated and quenched by adding water and extracted with ethyl acetate. The compound was purified by coloumn chromatography (0-14 %, ethyl acetate-hexane). Yield: 3.3 g (70 % of a 10 : 3 mixture of chloro and bromo compound).
¹H-NMR (400 MHz, CDCl₃): δ2.01-2.12 (m, 4H); 2.23 (p, 1.5H); 2.32 (p, 0.5H); 3.59 (t, 0.5H); 3.74 (t, 1.5H); 3.88 (dt, 2H); 4.09-4.08 (m, 2H); 4.12 (t, 2H); 7.34 (d, 2H); 6.93 (d, 2H).

### General procedure F:

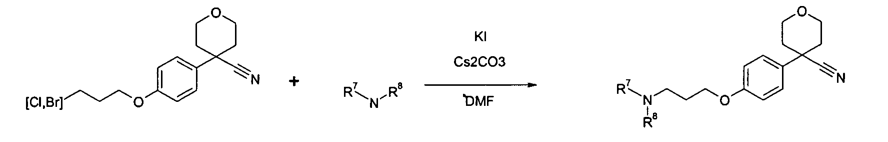

A mixture of halide (0.7 mmol), Cs₂CO₃ (0.350 g, 1.07 mmol), potassium iodide (0.180 g, 1.07 mmol) and amines (NR⁷R⁸) ( 1.07 mmol) in DMF (7 mL) was heated to 70°C overnight. The mixture was cooled to ambient temperature, water was added, and the resulting mixture was extracted with dichloromethane and the organic extracts were dried over Na₂SO₄, filtered and concentrated *in vacuo*. Purification of the crude material was carried out with flash chromatography (20 g silica gel, CH₂Cl₂/ MeOH gradient).

### Example 96: 4-[4-(3-morpholino-4-ylpropoxy)phenyl]tetrahydropyran-4-carbonitrile

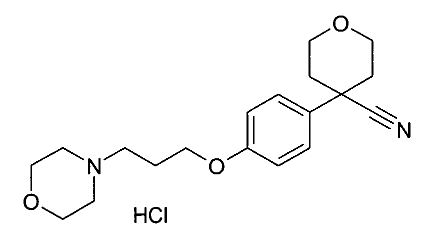

The compound was obtained according the general procedure F, using morpholine as amine. The hydrochloride salt as analytical sample was prepared with ether/HCl 2M in dichloromethane and crystallization in ether.
Yield = 34%
¹H NMR (400MHz, DMSO-D6) δ1.95-2.12 (m,4H); 2.16-2.25 (m,2H); 3.01-3.12 (m,2H); 3.19-3.27 (m,2H); 3.40-3.48 (m,2H); 3.60-3.69 (t,2H); 3.80-3.89 (m,2H); 3.91-4.03 (m,4H); 4.08-4.13 (m,2H); 7.20 (d,2H); 7.46 (d,2H); 11.33 (bs,1 H)

### Example 97: 4-{4-[3-(4-methyl-piperazin-1-yl)-propoxy]phenyl}tetrahydropyran-4-carbonitrile

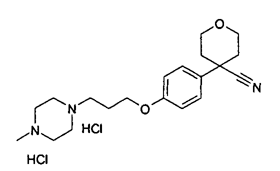

The compound was obtained according the general procedure F, using 1-methylpiperazine as amine. The hydrochloride salt as analytical sample was prepared with ether/HCl 1M in dichloromethane and triturated with pentane.
Yield = 57%
¹H NMR (400MHz, DMSO-D₆) δ11.85 (bs, 1H), 7.46 (d, 2H), 7.02 (d,2H), 4.13 - 4.07 (m, 2H), 4.03 -3.96 (m, 2H), 3.84-3.18 (m, 12H), 2.87-2.78 (m, 3H), 2.24 - 2.13 (m, 2H), 2.11 - 1.96 (m, 4H).

### Example 98: 4-{4-[3-(2-methyl-piperidin-1-yl)-propoxy]phenyl}-tetrahydropyran-4-carbonitrile

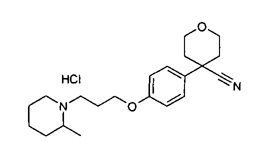

The compound was obtained according the general procedure F, using 2-methylpiperidine as amine. The hydrochloride salt as analytical sample was prepared with ether/HCl 1M in dichloromethane and trituration with pentane.
Yield = 28%
¹H NMR (400MHz, DMSO-D6) δ10.22 (bs, 1 H), 7.46 (d, 2H), 7.01 (d,2H), 4.13 - 4.06 (m, 2H), 4.03-3.96 (dd, 2H), 3.69-3.60 (t, 2H), 3.48-3.38 (m, 1 H), 3.25-3.06 (m, 3H), 3.01-2.90 (m, 1 H), 2.21-1.95 (m, 7H), 1.88-1.57 (m, 5H), 1.56-1.39 (m, 1 H), 1.32 (d, 2H), 1.24 (d, 1H).

### Example 99: 4-{4-[3-(3-methyl-piperidin-1-yl)-propoxy]phenyl}-tetrahydropyran-4-carbonitrile

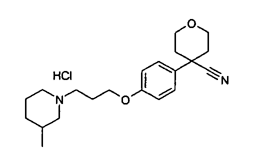

The compound was obtained according the general procedure F, using 3-methylpiperidine as amine. The hydrochloride salt as analytical sample was prepared with ether/HCl 1 M in dichloromethane and trituration with pentane.
Yield = 52%
¹H NMR (400MHz, DMSO-D6) δ10.32 (bs, 1H), 7.46 (d, 2H), 7.01(d,2H), 4.11 - 4.05 (t, 2H), 4.03-3.96 (dd, 2H), 3.69-3.60 (t, 2H), 3.48-3.35 (m, 2H), 3.19-3.11 (m, 2H), 2.82-2.72 (m, 1 H), 2.58-2.45 (m, 1H), 2.24-2.16 (m, 2H), 2.12-1.90 (m, 5H), 1.85-1.70 (m, 3H), 1.12-1.02 (m, 1H), 0.89 (d, 3H).

### Example 100: 4-{4-[3-(4-methyl-piperidin-1-yl)-propoxy]phenyl}-tetrahydropyran-4-carbonitrile

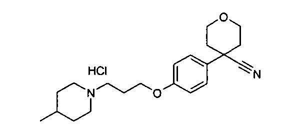

The compound was obtained according the general procedure F, using 4-methylpiperidine as amine. The hydrochloride salt as analytical sample was prepared with ether/HCl 1 M in dichloromethane and trituration with pentane.
Yield = 60%
¹H NMR: (400MHz, DMSO-D6) δ10.18 (bs, 1H), 7.46 (d, 2H), 7.01(d,2H), 4.11 - 4.05 (t, 2H), 4.03-3.96 (dd, 2H), 3.69-3.60 (t, 2H), 3.49-3.42 (m, 2H), 3.19-3.11 (m, 2H), 2.94-2.83 (m, 2H), 2.22-2.13 (m, 2H), 2.11-1.95 (m, 4H), 1.81-1.73 (m, 2H), 1.65-1.54 (m, 1 H), 1.53-1.41 (m, 2H), 0.91 (d, 3H).

### Example 101: 4-[4-(3-azepan-1-ylpropoxy)phenyl]tetrahydropyran-4-carbonitrile

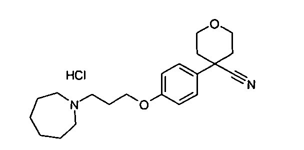

The compound was obtained according the general procedure F, using hexamethyleneimine as amine. The hydrochloride salt as analytical sample was prepared with ether/HCl 1M in dichloromethane and trituration with ether.
Yield = 9%
¹H NMR (400MHz, DMSO-D6) δ10.54 (bs, 1 H), 7.41 (d, 2H), 7.01 (d,2H), 4.11-4.05 (t, 2H), 4.03-3.96 (dd, 2H), 3.69-3.60 (dt, 2H), 3.41-3.30 (m, 2H), 3.24-3.17 (m, 2H), 3.16-3.06 (m, 2H), 2.24-2.16 (m, 2H), 2.12-1.95 (m, 4H), 1.92-1.76 (m, 4H), 1.72-1.52 (m, 4H).

### Example 102: 4-{4-[3-(4,4-difluoro-piperidin-1-yl)-propoxylphenyl}-tetrahydropyran-4-carbonitrile

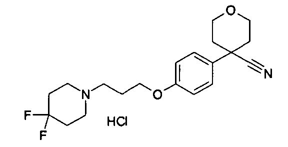

The compound was obtained according the general procedure F, using 4,4-difluoropiperidine hydrochloride as amine (add (C₂H₅)₃N to obtain the free base ). The hydrochloride salt as analytical sample was prepared with ether/HCl 1M in dichloromethane and trituration with ether.
Yield = 9%
¹H NMR: (400MHz, DMSO-D6) δ10.28 (bs, 1 H), 7.46 (d, 2H), 7.01 (d,2H), 4.12-4.07 (t, 2H), 4.03-3.96 (dd, 2H), 3.69-3.59 (dt, 4H), 3.33-3.25 (m, 2H), 3.21-3.11 (m, 2H), 2.39-2.27 (m, 2H), 2.27-2.17 (m, 2H), 2.12-1.95 (m, 4H).

### Example 103: 4-[4-(5-Pyrrolidin-1-ylpentyloxy)phenyl]tetrahydropyran-4-carbonitrile

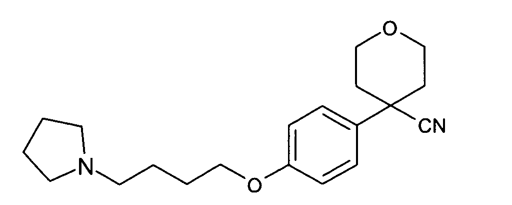

4-(4-Hydroxy-phenyl)-tetrahydro-pyran-4-carbonitrile (1.5g, 7.39mmol), 1-bromo-4-chlorobutane (1.7ml, 14.77mmol), DMF (20ml) and K₂CO₃ (4.1g, 29.56mmol) were reacted together according to general procedure E. Purification by chromatography on silica, eluant ethyl acetate :heptanes (33 : 66) provided a mixture of bromo and chloro phenoxy ether (1.3g, 60%th) which was used in the next stage. A portion of the mixture (0.5g, 1.70mmol) and pyrrolidine (0.42ml, 5.11mmol) were refluxed in EtOH (5ml, 10vol) overnight. The reaction was concentrated *in vacuo* and partitioned between 2M NaOH (10ml) and ethyl acetate (10ml). The aqueous was extracted with ethyl acetate (3 x 10mL). The combined ethyl acetate layers were washed with brine (3 x 10ml), dried over MgSO₄, filtered, washed with ethyl acetate and concentrated *in vacuo.* Purification by chromatography on silica, eluant DCM :MeOH :NH₃ (95 :4 :1) increasing gradually to DCM :MeOH :NH₃ (93 :6 :1), gave the title compound as a pale yellow solid (350mg, 63%th).
¹H NMR (400MHz, DMSO-*d*₆) δ7.38 (d, 2H), 6.92 (d, 2H), 4.11-4.03 (m, 2H), 3.99 (t, 2H), 3.89 (td, 2H), 2.58-2.43 (m, 6H), 2.15-1.99 (m, 4H), 1.89-1.64 (m, 8H).

### Intermediate 35: 1-[4-(3-bromo-propoxy)-phenyl]-cyclohexanecarbonitrile

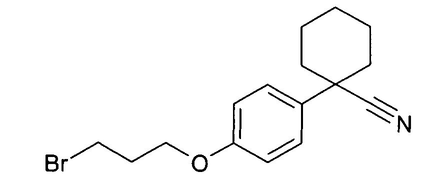

1-(4-hydroxyphenyl)cyclohexanecarbonitrile (2.0 g, 10 mmol) was taken in acetone (150 mL) and then Cs₂CO₃ (8.1 g, 25 mmol) was added followed by dropwise addition of 1,3 dibromopropane (5.1 mL, 50 mmol). The mixture was heated for 3 h at 70°C. After cooling and filtration, the mixture was concentrated and the residue was purified by flash chromatography (50 g silica gel, CH₂Cl₂) to provide the title compound (2.8 g, 87%). Note: presence of allyl derivative as impurity.
¹H NMR (400MHz, DMSO-D6) δ7.4 (d,2H); 6.92 (d,2H); 4.1-4.02 (m,2H); 3.6-3.5 (m,2H); 2.35-2.25 (m,2H); 2.18-2.05 (m,2H); 1.9-1.7 ( m,8H).

### General procedure G:

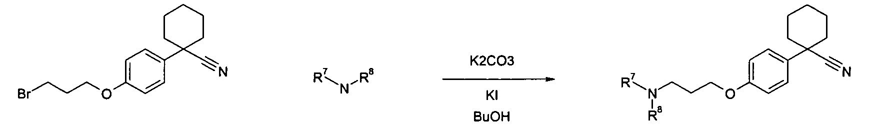

A mixture of 1-[4-(3-bromo-propoxy)-phenyl]-cyclohexanecarbonitrile (0.660 g, 2.0 mmol), amine (NR⁷R⁸) (0.260g, 3.0 mmol), sodium carbonate (0.320 g, 3 mmol), potassium iodide (20 mg) and 20 mL of butanol was heated for 4 h at 100°C. After cooling, water was added to quench the reaction and the mixture was extracted with DCM. The organics extracts were washed with a saturated aqueous solution of NaHCO₃, water and dried over Na₂SO₄, filtered and concentrated. The residue was purified by flash chromatography (CH₂Cl₂ and CH₂Cl₂ / MeOH (10% NH₄OH) to provide (0.440 g, 67% )

### Example 104: 1-[4-(3-piperidin-1-yl-propoxy)-phenyl]-cyclohexane-carbonitrile

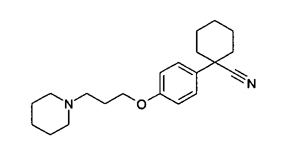

A mixture of 1-[4-(3-bromo-propoxy)-phenyl]-cyclohexanecarbonitrile (0.660 g, 2.0 mmol), piperidine (0.260g, 3.0 mmol), sodium carbonate (0.320 g, 3 mmol), potassium iodide (20 mg) and 20 mL of butanol was heated for 4 h at 100°C according to general procedure G. After cooling, water was added to quench the reaction and the mixture was extracted with DCM. The organics extracts were washed with a saturated aqueous solution of NaHCO₃, water and dried over Na₂SO₄, filtered and concentrated. The residue was purified by flash chromatography (CH₂Cl₂ and CH₂Cl₂ / MeOH (10% NH₄OH) to provide the title compound (0.440 g, 67%)
¹H NMR (400MHz, CDCl₃) δ7.37 (d, 2H), 6.90(d,2H), 4.00 (t, 2H), 2.49-2.36 (m, 6H), 2.17-2.09 (m, 2H), 1.97 (qt, 2H), 1.88-1.67 (m, 7H), 1.62-1.55 (m, 4H), 1.47-1.40 (m, 2H), 1.32-1.20 (m, 1 H).

### General procedure h:

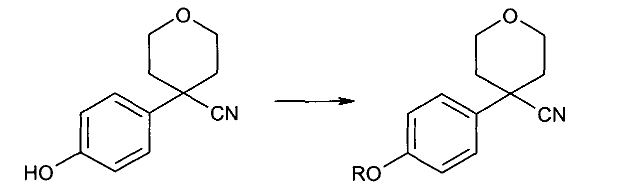

4-(4-Hydroxy-phenyl)-tetrahydro-pyran-4-carbonitrile (1 eq.), alcohol (R-OH) (0.8eq.) and PPh₃ (1eq.) were mixed together in THF (10vol) and cooled to 0°C under N₂. DIAD (1eq.) in THF (10vol) was added slowly to the reaction and allowed to warm to room temperature overnight. The reaction was quenched with 2M HCl solution (10vol) and extracted with ethyl acetate (3 x 10vol). The aqueous was basified to pH 14 with NaOH (~30vol) and extracted with ethyl acetate (3 x 10vol). The organics were dried over MgSO₄, filtered and concentrated *in vacuo* at 35°C. Purification of the crude material by chromatography on silica, eluant (10%MeOH in DCM) provided the title compounds.

### Example 105: 4-[4-(2,2-Dimethyl-3-pyrrolidin-1-ylpropoxy)phenyl]tetrahydropyran-4-carbonitrile

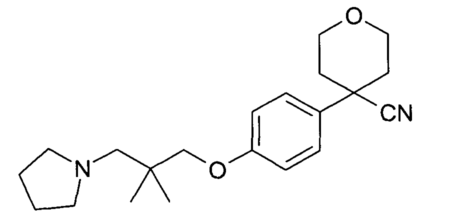

4-(4-Hydroxy-phenyl)-tetrahydro-pyran-4-carbonitrile (1.29g, 6.4mmol), 2,2-dimethyl-3-pyrrolidin-1-yl-propan-1-ol (0.8g, 5.1mmol), PPh₃ (1.67g, 6.4mmol), THF (16ml) and DIAD (1.25ml, 6.4mmol) were reacted together according to general procedure H. The crude material was subjected to chromatography on silica eluting with DCM :MeOH (98 :2). The resulting solid was slurried in TBME:heptanes (1:2, 1ml) to provide the title compound (120mg, 5%th) as a white solid.
¹H NMR (400MHz, CDCl₃), δ7.37 (d, 2H), 6.94 (d, 2H), 4.12-4.03 (m, 2H), 3.89 (td, 2H), 3.71 (s, 2H), 2.57 (br s, 4H), 2.47 (s, 2H), 2.15-1.99 (m, 4H), 1.70 (br s, 4H), 1.00 (s, 6H).

### Example 106: 4-[4-(3-Pyrrolidin-1-ylpropoxy)phenyl]tetrahydro-2H-pyran-4-carboxylic acid amide

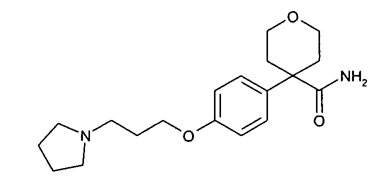

4-[4-(3-Pyrrolidin-1-yl-propoxy)-phenyl]-tetrahydro-pyran-4-carbonitrile (400 mg, 1.27 mmol) was treated with polyphosphoric acid at 80°C overnight. After allowing the mixture to cool to room temperature, ethyl acetate and water were added. The organic layer was separated. The aqueous layer was basified to pH 10 with concentrated NaOH and extracted with DCM. The combined organic extracts were dried over Na₂SO₄, filtered, concentrated *in vacuo* and purified by column chromatography on silica gel eluting to give the title compound (88 mg, 26%).
¹H NMR (400MHz, CDCl₃) δ7.29 (d, 2H), 6.91 (d, 2H), 5.64 -5.30 (d, 2H), 4.02 (t, 2H), 3.76 (t, 4H), 2.62 (t, 2H), 2.52 (m, 4H), 2.37-2.30 (m, 2H), 2.08-1.96 (m, 4H), 1.81-1.75 (m, 4H).

### Example 107: 4-[4-(3-Pyrrolidin-1-ylpropoxy)phenyl]tetrahydro-2H-pyran-4-carboxylic acid

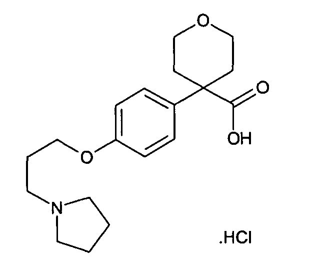

A solution of 4-[4-(3-pyrrolidin-1-ylpropoxy)phenyl]tetrahydro-2*H*-pyran-4-carbonitrile (20g, 0.0636mol) in concentrated hydrochloric acid (200ml, 10vol) was heated and stirred at reflux for 16h after which time LC/LCMS analysis showed the reaction had stalled at 85-90% conversion. The solution was evaporated to dryness *in vacuo,* azeo-dried with methanol (200ml, 20vol) and toluene (2x 200ml, 2x 20vol) to yield title compound as a beige solid which was used directly.
¹H NMR (400 MHz, CDCl₃) δ7.18 (d, 2H), 6.96 (d, 2H), 4.12 (t, 2H), 3.94-3.84 (m, 2H), 3.74-3.66 (m, 2H), 3.60 (dt, 2H), 3.42 (t, 2H), 2.50-2.44 (m, 2H), 2.26-2.00 (m, 8H), 2.96-2.84 (m, 2H);HRMS (ESI⁺) 334.2013 (M+H)⁺.

### Intermediate 36: Methyl 4-[4-(3-pyrrolidin-1-ylpropoxy)phenyl]tetrahydro-2H-pyran-4-carboxylate

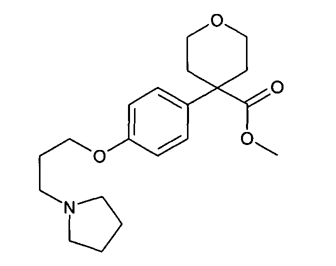

A suspension of 4-[4-(3-pyrrolidin-1-ylpropoxy)phenyl]tetrahydro-2*H*-pyran-4-carboxylic acid (23.5g, 0.0636mol) in methanol (235ml, 10vol) was stirred and cooled to 0 to -5°C under nitrogen. Thionyl chloride (9.3ml, 0.1272mol, 0.40vol) was charged dropwise over 20minutes, maintaining the temperature -5 to +5°C. The resulting slurry was then heated and stirred at reflux under nitrogen for 16h after which time LC analysis showed 1.4% by area of acid remaining. The solution was evaporated to dryness *in vacuo* at 40°C to give a brown sludge.

The sludge was dissolved in ethyl acetate (118ml, 5vol) and water (235ml, 10vol) and the layers separated. The aqueous layer was washed with ethyl acetate (118ml, 5vol) and basified to pH 11 with saturated aqueous potassium carbonate (118ml, 5vol). The product was extracted into dichloromethane (3x 118ml, 3x 5vol) and the combined extracts dried over magnesium sulfate (23g, 1wt) and concentrated *in vacuo* at 40°C to yield title compound (21.7g, 98%th from the nitrile) as a cream solid.
¹H NMR (400MHz, DMSO-*d*₆) δ7.15 (d, 2H), 6.76 (d, 2H), 3.90 (t, 2H), 3.70 (dt, 2H), 3.50 (s, 3H), 3.30 (td, 2H), 2.60 (t, 2H), 2.40-2.20 (m, 6H), 1.80-1.70 (m, 4H), 1.6-1.5 (m, 4H).

### Example 108: {4-[4-(3-Pyrrolidin-1-ylpropoxy)phenyl]tetrahydro-2H-pyran-4-yl}methanol

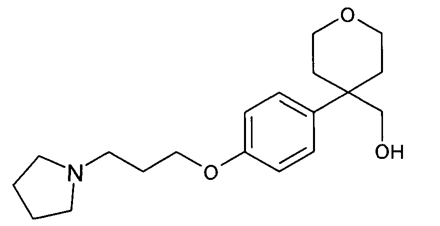

Anhydrous tetrahydrofuran (141ml, 10vol) was charged rapidly onto stirred and cooled (0-5°C) lithium aluminium hydride (6.2g, 0.1634mol, 0.44wt) under nitrogen (Note: very exothermic). The resulting slurry was cooled to 0-5°C and then a solution of methyl 4-[4-(3-pyrrolidin-1-ylpropoxy)phenyl]tetrahydro-2*H*-pyran-4-carboxylate (14.1 g, 0.04058mol) in tetrahydrofuran (99ml, 7vol) was added dropwise over 30 minutes maintaining 0-5°C. On complete addition the reaction was allowed to warm to room temperature over 30 minutes and stirred at 18-25°C for 30 minutes, LC analysis showed the reaction to be complete. The mixture was cooled to 0 to 5°C, a 1:1 mixture of tetrahydrofuran and water (18.6ml, 1.32vol) was added very slowly (Note: very exothermic, gas evolution, temperature maintained below 16°C, toward the end of the quench the reaction mixture sets and then becomes freer with continued stirring.). The mixture was further quenched with 20% w/v sodium hydroxide solution (6.2ml, 0.44vol) and water (18.6ml, 1.32vol) and the resulting white suspension stirred vigorously for 30 minutes at 18-25°C. The slats were removed by filtration and rinsed with tetrahydrofuran (3x 150ml). The combined filtrates were evaporated to dryness at 40°C to yield title compound (12.1 g, 94%th) as a white solid.
¹H NMR (400MHz, CDCl₃) δ7.25 (d, 2H), 6.90 (d, 2H), 4.05 (t, 2H), 3.80 (dt, 2H), 3.55 (td, 2H), 2.60 (t, 2H), 2.62-2.41 (m, 4H), 2.10 (dt, 2H), 2.00 (p, 2H), 2.00-1.85 (m, 2H), 1.95-1.72 (m, 4H).

### Example 109: 1-[4-(3-pyrrolidin-1-ylpropoxy)phenyl]cyclo-pentane-carbonitrile

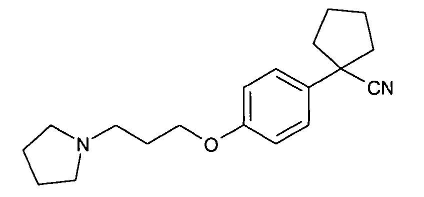

To a suspension of NaH (60%, 0.574 g, 14.35 mmol) in DMF (8 mL) at 0°C was added dropwise [4-(3-pyrrolidin-1-ylpropoxy)phenyl]acetonitrile (1 g, 4.10 mmol) in DMF (3 mL). The reaction mixture was stirred at 0°C for 5 min than at room temperature for 30 min. After cooling to 0°C, 1,4-dibromobutane (0.980 mL, 8.2 mmol) in DMF (2 mL) was added dropwise. The reaction mixture was allowed to warm up to room temperature then heated to 50°C overnight. It was poured into ice-cold water and extracted with DCM. The combined organic extracts were washed with brine, dried over Na₂SO₄, filtered and concentrated *in vacuo.* The crude mixture was purified by column chromatography, eluting with a gradient of DCM:MeOH:NH₄OH (from 99:0:1 to 90:10:1) to give the title compound (0.443 g, 36%).
¹H NMR (400MHz, DMSO-*d*₆) δ7.29 (d, 2H), 6.79 (d, 2H), 4.02 (m, 2H), 3.18 (m, 2H), 2.47-2.28 (m, 4H), 2.19-1.75 (m, 11 H), 1.19 (m, 3H).

### Example 110: 4-oxo-1-[4-(3-pyrrolidin-1-ylpropoxy)-phenyl]-cyclohexane-carbonitrile

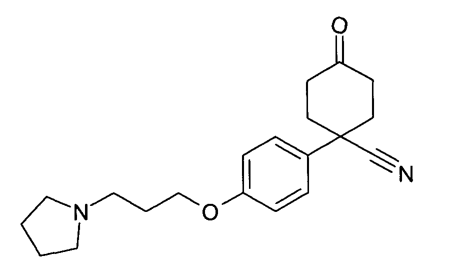

### Step 1:

A three neck reaction vessel was charged with [4-(3-pyrrolidin-1-ylpropoxy)phenyl]acetonitrile (7g, 28.6 mmol), benzyltrimethylammonium hydroxide (40% in methanol, 1.3 mL) in acetonitrile (185 mL). The solution was heated to reflux and methyl acrylate (25 mL, 286 mmol) was added dropwise. After refluxing for 5 hr, the mixture was concentrated to half, diethyl ether was added and the organics were washed with HCl 1N and brine, dried over Na₂SO₄, filtered and concentrated. The residue was purified by flash-chromatography to provide 4-cyano-4-[4-(3-pyrrolidin-1-ylpropoxy)-phenyl]-heptanedioic acid dimethyl ester (8.8 g, 74%).
¹H NMR (400MHz, CDCl₃) δ7.28 ( d,2H); 6.95 (d,2H); 4.08-4.0 ( m,2H ); 3.6 (s,6H); 2.68-2.60 (m,2H); 2.58-2.45 (m,6H); 2.40-2.20 (m,4H); 2.20-2.12 (m,2H); 2.08-2.0 (m,2H); 1.80-1.75 (m,4H)

### Step 2:

To a solution of 4-cyano-4-[4-(3-pyrrolidin-1-ylpropoxy)-phenyl]-heptanedioic acid dimethyl ester (8.8 g, 21.1 mmol) in 1,2-dimethoxyethane (176 mL) was charged portions of NaH ( 60% dispersion, 2.55 g, 63.4 mmol). The reaction mixture was heated to reflux. After 4.5 hr, the ¾ of solvent were evaporated and the mixture cooled to 20°C with an ice bath and quenched with water (100 mL), HCl 1N (100 mL). The aqueous layer was extracted with ether (100 mL). After basification with NaOH the aqueous phases were extracted with dichloromethane. The combined organics were washed with water, dried over Na₂SO₄, filtrered and concentrated to provide 5-cyano-2-oxo-5-[4-(3-pyrrolidin-1-ylpropoxy)-phenyl]-cyclohexanecarboxylic acid methyl ester (6.6 g , 81%) as an oil.
¹H NMR (400MHz, CDCl₃) δ12.2 (bs, 1H); 7.33 (d, 2H); 6.95 (d, 2H); 4.08-4.00 (m, 2H); 3.75 (s, 3H); 2.95 (d, 1 H); 2.80-2.70 (m, 1 H); 265-2.60 (m, 2H); 2.50-2.42 (m, 6H); 2.30-2.15 (m, 2H); 2.05-1.95 (m, 2H); 1.90-1.85 (m, 4H).

### Step 3:

To a solution of 5-cyano-2-oxo-5-[4-(3-pyrrolidin-1-ylpropoxy)-phenyl]-cyclohexanecarboxylic acid methyl ester (6.6 g, 17 mmol) and dimethylsulfoxide (128 mL) was added water (8.0 mL) and sodium chloride (6.4 g, 109 mmol). The reaction mixture was heated to 142-146°C. After 5 hr, the mixture was concentrated and the residue was dissolved in CH₂Cl₂ (120 mL), washed with water (100 mL), and brine, dried over Na₂SO₄, filtered and concentrated. The residue was purified by flash chromatography (70 g silica gel, eluant : gradient of CH₂Cl₂/MeOH 95/5 to 90/10) providing 4-oxo-1-[4-(3-pyrrolidin-1-ylpropoxy)-phenyl]-cyclohexanecarbonitrile as a crystallizing oil (2.2 g 45.5 %). After crystallization in ether, 0.2g of analytical sample were obtained.
¹H NMR (400MHz, CDCl₃) δ7.32 (d, 2H), 6.88(d,2H), 4. (t, 2H), 2.95 -2.8 (m, 2H), 2.65-2.38 (m, 10H), 2.3-2.15 (m, 2H), 2 - 1.9 (m, 2H), 18 - 1.7 (m, 4H),.

### Example 111: 4-hydroxy-1-[4-(3-pyrrolidin-1-ylpropoxy)-phenyl]cyclohexanecarbonitrile

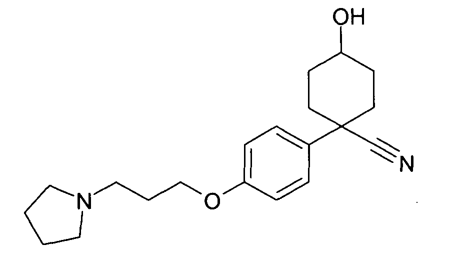

To a stirred suspension of LiAlH₄ (0.070 g, 1.85 mmol, 5 eq) in diethyl ether (1.9 mL) was added dropwise at 0°C a solution of 4-oxo-1-[4-(3-pyrrolidin-1-ylpropoxy)-phenyl]-cyclohexanecarbonitrile (0.12 g, 0.37 mmol) in diethyl ether (5.5 mL). The reaction mixture was stirred at ambient temperature for 4 h .The reaction mixture was cooled to 0°C and, successively, a solution of water, sodium hydroxide (15% w/v, 0.08 mL) and water again were carefully dropped. After being stirred for 15 min at room temperature, the mixture was filtered over diatomaceous earth and concentrated. The residue was purified by flash chromatography (10 g silica gel, CH₂Cl₂/ MeOH ( 10% NH₄OH) to provide 0.050 g of title compound.
¹H NMR (400MHz,DMSO-D6) δ10.6 (bs, 1 H), 7.42 (d, 2H), 7(d, 2H), 4.1-3.95 (m, 2H), 3.6 - 3.2 (m, 5H), 3.03-2.87 (m, 2H), 2.15- 1.8 (m, 10H), 1.65 - 1.5 (m, 2H).

### Intermediate 37: (R)-2-Methylpyrrolidine

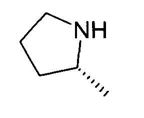

Pure enantiomers of 2-methyl pyrrolidine can be obtained by resolution with +/tartaric acid as described in *Acta. Pharm. Suecica* 15, 255-263; 1978.

### Intermediate 38: 3-[(2R)-2-Methylpyrrolidin-1-yl]propan-1-ol

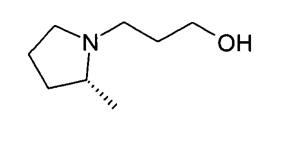

3-Bromopropan-1-ol (5.28 mL, 58.4 mmol) was added to a solution of (R)-2-methylpyrrolidine (7.1 g, 58.4 mmol) in water (200 mL). KOH (7.53 g, 134 mmol) was then added and the mixture stirred at ambient temperature for 48 hours. The reaction mixture was then extracted with dichloromethane (5 x 80 mL) and ethyl acetate (5 x 80 mL). The combined organic extracts were dried using MgSO₄, filtered and concentrated *in vacuo*. The compound was purified by column chromatography on Biotage® silica gel, eluting with dichloromethane:methanol:ammonia, (90:10:1) to give the title compound as a pale brown oil (5.44, 65 %).
¹H NMR (400MHz, CDCl₃) δ3.80 (m, 2H), 3.38 (m, 2H), 3.02-2.95 (m, 1H), 2.40 (m, 1H), 2.30 (m, 1 H), 2.12 (m, 1H), 1.99-1.82 (m, 2H), 1.80-1.60 (m, 2H), 1.55 (m, 1 H), 1.42-1.38 (m, 1 H), 1.18 (d, 3H). LCMS (APCl⁺) 144 (M+H)⁺.

### Intermediate 39: tert-Butyl (3S)-3-methoxypyrrolidine-1-carboxylate

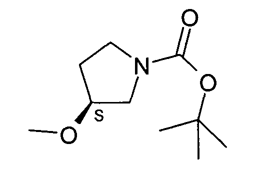

Sodium hydride (80 % dispersion in mineral oil, 940 mg, 0.024 mmol) was added in two portions at 0 °C to a solution of *tert*-butyl (3*S*)-3-hydroxypyrrolidine-1-carboxylate (4.00 g, 0.02 mmol). The reaction mixture was allowed to warm to room temperature and stirred for 1 h before being cooled once more to 0 °C. Methyl iodide (2.00 mL, 0.032 mmol) was added slowly and the reaction mixture allowed to warm to room temperature overnight. The mixture was poured slowly onto 200 mL of ice and stirred until the ice was fully thawed. The product was extracted using dichloromethane (2 x 250 mL), dried (Na₂SO4) and concentrated in vacuo. The compound was purified by column chromatography on Biotage® silica gel, eluting with dichloromethane to give the product as a colourless oil (2.88 g, 67 %).
¹H NMR (400MHz, CDCl₃) δ3.92-3.88 (m, 1 H), 3.42-3.36 (m, 4H), 3.30 (s, 3H), 2.02-1.82 (m, 2H), 1.42 (s, 9H).

### Intermediate 40: (3S)-3-Methoxypyrrolidine

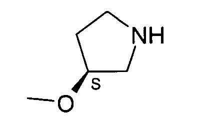

A solution of trifluoroacetic acid (2.5 ml) in dichloromethane (5 mL) was added slowly at 0 °C to a solution of *tert*-Butyl (3*S*)-3-methoxypyrrolidine-1-carboxylate (2.88 g, mmol) and the reaction allowed to warm to room temperature and stirred for 2.5 h. The reaction mixture was quenched with saturated sodium carbonate solution (100mL) and extracted with dichloromethane (2 x 200mL). The organics were combined, dried over magnesium sulphate and concentrated *in vacuo.* The residue was taken up in dichloromethane (30 mL) and cooled to 0 °C in an ice bath. Hydrogen chloride gas was bubbled through the suspension for 1 hour and the reaction mixture allowed to stir at room temperature for 48 hours. The reaction mixture was basified with saturated sodium hydrogencarbonate solution (100 mL) and extracted with dichloromethane (2 x 200mL) and ethyl acetate (3 x 150mL). The aqueous was concentrated *in vacuo* and then extracted with warm methanol to yield the title product, 2.00g.
¹HNMR(CD₃OD, 400MHz) δ: 1.96 (m, 1 H), 2.09 (m, 1 H), 3.08-3.37 (m, 4H), 4.06 (m, 1H), 4.80 (s, 3H).

### Intermediate 41: (3R)-1-Benzyl-3-methoxypyrrolidine

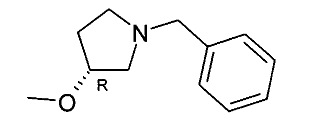

(3*R*)-1-Benzyl-3-methoxypyrrolidine was prepared as described in the following patent; WO 91/08206 and taken on to the next step as the crude mixture.

### Intermediate 42: (3R)-3-Methoxypyrrolidine

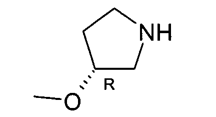

A solution of (3*R*)-1-Benzyl-3-methoxypyrrolidine (2.35 g, 12.3 mmol) in methanol (50 mL) containing concentrated HCl (1 mL) were hydrogenated at ambient temperature at 50 psi in the presence of a catalytic amount of 10 % Pd(OH)₂ on carbon (250 mg, 10% w/w). The reaction mixture was filtered over celite and rinsed with dichloromethane (60 mL) and methanol (60 mL) to remove the catalyst. The organic layer was basified with NaOH (2.0M, 10 mL) and extracted with diethylether (3 x 30 mL). The aqeous layer was further acidified to pH3 using concentrated HCl, concentrated in vacuo and azeotroped with toluene to give the product as a yellow solid which was further used without purification .

### Intermediate 43: 2,2-Dimethylpyrrolidine

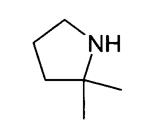

Step 1: 1-Benzyl-2,2-dimethylpyrrolidine was prepared according to the procedure described in the following reference. S. M. Denton and A. Wood, *Synlett*, 1999, 55.
Step 2: A solution of 1-Benzyl-2,2-dimethylpyrrolidine (1.02g, 5.40 mmol) in ethanol (80 mL) and concentrated HCl (0.5 mL) was hydrogenated over 20 % Pd(OH)₂ (100 mg, 10 % w/w) at 60 psi at ambient temperature for 4 hours. The reaction mixture was filtered over arbocel to remove the catalyst and a further 2 mL of concentrated HCl was added to the crude product. The reaction mixture was then concentrated in vacuo and azeotropically dried using toluene to give the product as a brown solid, which appeared to be slightly hygroscopic (732 mg, 100 %).
   ¹H NMR (400MHz, CDCl₃) δ3.40- 3.34 (m, 2H), 2.12 (pentet, 2H), 1.94 (t, 2H), 1.44 (s, 6H).

### Intermediate 44: (2R,5S)-2,5-Dimethylpyrrolidine

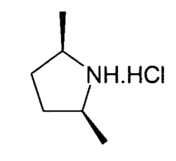

The free base of cis-2,5-dimethylpyrrolidine was prepared as described by C. G. Overberger, L. C. Palmer, B. S. Marks and N. R. Byrd, *J. Am. Chem. Soc*., 1955, 77, 4100. This was then acidified using HCl (2.0 M solution in diethylether) which, upon filtration and subsequent recrystallisation from acetonitrile and *i*-propylalcohol gave the title compound as a white crystalline solid (5.29 g, 22 %) in a ratio of approximately 10:1 ratio of cis:trans.
¹H NMR (400MHz, CDCl₃) δ3.86-3.80 (m, 0.2H), 3.70-3.58 (m, 1.8H), 2.24-2.10 (m, 2H), 1.88-1.76 (m, 2H), 1.58 (d, 5.4H), 1.52 (d, 0.6H).

### Intermediates 45 and 46: 1-[4-(3-bromo-propoxy)phenyl]cyclohexane-carbonitrile and 1-[4-(3-chloro-propoxy)-phenyl]-cyclohexane-carbonitrile

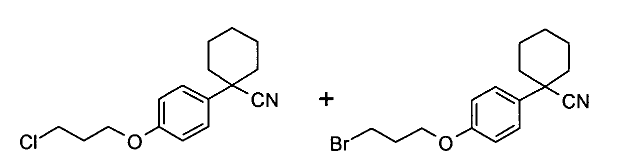

K₂CO₃ (3.02 g, 21.9 mmol) was added to a solution of 1-(4-hydroxyphenyl)cyclohexanecarbonitrile (4.0 g, 19.9 mmol) in DMF (50 mL). 1-bromo-3-chloroproprane (1.97 ml, 19.9 mmol) was then added dropwise and the reaction heated at 60 °C overnight. The reaction mixture was concentrated, quenched by adding water (50 mL), extracted with dichloromethane (2 x 50 mL) and the organic extracts were dried over Na₂SO₄, filtered and concentrated *in vacuo*. The compound was purified by column chromatography on Biotage® silica gel, eluting with diethylether:dichloromethane, 50:50 to 0:100. Yield: 5.32 g (96 % of a 3:1 mixture of chloro and bromo compound).
¹H NMR (400MHz, CDCl₃) δ 7.38 (d, 2H), 4.20-4.08 (m, 2H), 3.76 (t, 1.5H), 3.62 (t, 0.5H), 2.32 (pentet, 0.5H), 2.26 (pentet, 1.5H), 2.16-2.08 (m, 2H), 1.92-1.68 (m, 9H), 1.36-1.20 (m, 1H).

### Example 112: 1-(4-{3-[(2R)-2-(methoxymethyl)pyrrolidin-1-yl]propoxy}-phenyl)cyclohexanecarbonitrile

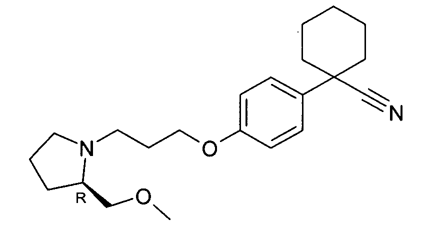

A (1:3) mixture of 1-[4-(3-bromo-propoxy)-phenyl]-cyclohexanecarbonitrile and 1-[4-(3-chloro-propoxy)-phenyl]-cyclohexanecarbonitrile (2.00g, 7.20 mmol), Cs₂CO₃ (2.58 g, 7.20 mmol), potassium iodide (0.20 g, 1.20 mmole) and (R)-(-)-2-(methoxymethyl)pyrrolidine (1.07 mL, 8.60 mmol) in NMP (20 mL) was heated to 75 °C overnight. The reaction mixture was cooled to ambient temperature, concentrated in *vacuo*, and quenched by adding water (30 mL). The crude product was extracted with ethyl acetate (30 x mL) and the organic extracts were dried over Na₂SO₄, filtered and concentrated *in vacuo*. The compound was purified by column chromatography on Biotage® silica gel, eluting with dichloromethane:methanol:ammonia, 100:0:0 to 90:10:1 to give the title compound as a brown oil (1.39 g, 54%).
¹H NMR (400MHz, CDCl₃) δ7.38 (d, 2H), 6.70 (d, 2H), 4.02 (t, 2H), 3.46-3.38 (m, 1H), 3.36-3.28 (m, 3H), 3.24-3.16 (m, 1H), 3.12-3.04 (m, 1H), 2.72-2.58 (m, 1H), 2.52-2.44 (m, 1H), 2.30-2.20 (m, 1H), 2.18-2.06 (m, 2H), 1.98 (pentet, 2H), 1.92-1.60 (m, 12H), 1.36-1.18 (m, 1H); HRMS (ESI⁺) 357.2537 (M+H)⁺.

### Example 113: 1-(4-{3-[(3S)-3-methoxypyrrolidin-1-yl]propoxy}phenyl)cyclohexanecarbonitrile

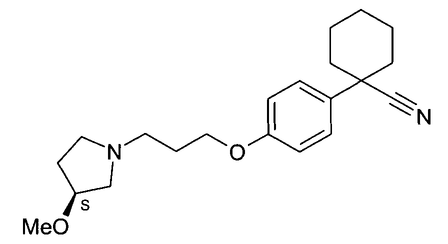

A (1:3) mixture of 1-[4-(3-bromo-propoxy)-phenyl]-cyclohexanecarbonitrile and 1-[4-(3-chloro-propoxy)-phenyl]-cyclohexanecarbonitrile (200 mg, 0.72 mmol), *N,N*-diisopropylethylamine (251 µL, 1.44 mmol) and (S)-3-methoxy-pyrrolidine (198 mg, 1.96 mmol) in NMP (0.5 mL) was heated at 160 °C in the microwave for 400 seconds. The reaction mixture was quenched with water (10 mL) and extracted with dichloromethane (2 x 10 mL), dried over Na₂SO₄, filtered and concentrated *in vacuo*. The compound was purified by column chromatography on Biotage® silica gel, eluting with dichloromethane:methanol:ammonia, 97:3:0.3 to 95:5:0.5. to give the product as a clear oil (102 mg, 41 %).
¹H NMR (400MHz, CDCl₃) δ7.36 (d, 2H), 6.90 (d, 2H), 4.02 (t, 2H), 3.96-3.88 (m, 1H), 3.28 (s, 3H), 2.86-2.52 (m, 6H), 2.18-1.96 (m, 5H), 1.88-1.66 (m, 8H), 1.38-1.16 (m, 1H); LCMS (APCI⁺) 343 (M+H)⁺.

### Example 114: 1-(4-{3-[(3R)-3-methoxypyrrolidin-1-yl]propoxy}phenyl)-cyclohexanecarbonitrile

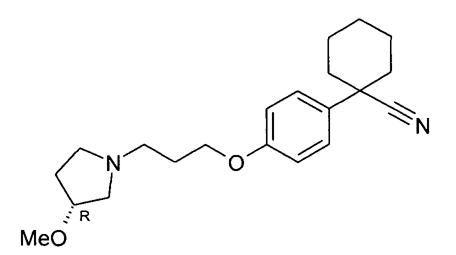

A (1:3) mixture of 1-[4-(3-bromo-propoxy)-phenyl]-cyclohexanecarbonitrile and 1-[4-(3-chloro-propoxy)-phenyl]-cyclohexanecarbonitrile (200 mg, 0.72 mmol), *N,N*-diisopropylethylamine (251 µL, 1.44 mmol) and (R)-3-methoxy-pyrrolidine (198 mg, 1.44 mmol) in NMP (0.5 mL) was heated at 160 °C in the microwave for 600 seconds. The reaction mixture was quenched with water (30 mL) and extracted with ethylacetate (2 x 30 mL), dried over Na₂SO₄, filtered and concentrated *in vacuo*. The compound was purified by column chromatography on Biotage® silica gel, eluting with dichloromethane:methanol:ammonia, 98:2:0.2 to 95:5:0.5 to give a brown oil (37 mg, 11 %).
¹H NMR (400MHz, CDCl₃) δ7.38 (d, 2H), 6.90 (d, 2H), 4.02 (t, 2H), 3.94-3.88 (m, 1H), 3.28 (s, 3H), 2.74-2.66 (m, 2H), 2.64-2.56 (m, 2H), 2.46 (quintet, 2H), 2.17-1.94 (m, 5H) 1.88-1.66 (m, 8H) 1.32-1.18 (m, 1H); HRMS (ESI⁺) 343.2380 (M+H)⁺.

### Example 115: 4-(4-{3-[(2R,5S)-2,5-dimethylpyrrolidin-1-yl]propoxy}-phenyl)-tetrahydro-2H-pyran-4-carbonitrile

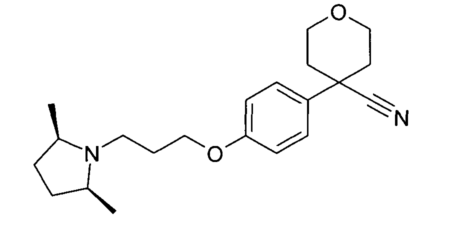

A (1:3) mixture of 1-[4-(3-bromo-propoxy)-phenyl]-cyclohexanecarbonitrile and 1-[4-(3-chloro-propoxy)-phenyl]-cyclohexanecarbonitrile (150 mg, 0.54 mmol), *N,N*-diisopropylethylamine ( 187 µL, 1.07 mmol) and (2*R*,5*S*)-2,5-dimethylpyrrolidine (145 mg, 1.07 mmol) in NMP (0.5 mL) was heated at 160°C in the microwave for 400 seconds then 170 °C for 600 seconds. The reaction mixture was quenched with NaHCO₃ (15 mL) and extracted with ethyl acetate (2 x 15 mL), dried over Na₂SO₄, filtered and concentrated *in vacuo*. The compound was purified by column chromatography on Biotage® silica gel, eluting with dichloromethane:methanol:ammonia, 97:3:0.3 to 90:10:1. Yield = 28 %
¹H NMR (400MHz, CDCl₃) δ7.36 (d, 2H), 6.92 (d, 2H), 4.10-4.04 (m, 2H), 4.00 (t, 2H), 3.88 (dt, 2H), 2.72 (t, 2H), 2.64-2.52 (m, 2H), 2.14-1.98 (m, 4H), 1.94 (pentet, 2H), 1.86-1.78 (m, 2H), 1.42-1.28 (m, 2H), 1.10 (d, 6H); HRMS (ESI⁺) 343.2380 (M+H)⁺.

### Example 116: 1-{4-[3-(2,2-dimethylpyrrolidin-1-yl)propoxy]phenyl}cyclohexanecarbonitrile

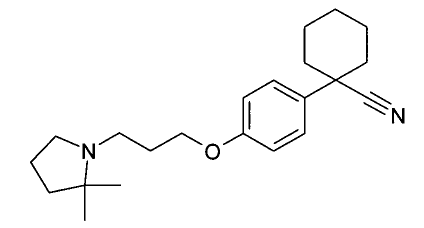

A (1:3) mixture of 1-[4-(3-bromo-propoxy)-phenyl]-cyclohexanecarbonitrile and 1-[4-(3-chloro-propoxy)-phenyl]-cyclohexanecarbonitrile (150 mg, 0.54 mmol), *N,N*-diisopropylethylamine (188 µL, 1.08 mmol) and 2,2-dimethyl-pyrrolidine (131 mg, 1.08 mmol) in NMP (0.5 mL) was heated at 180°C in the microwave for 600 seconds. The reaction mixture was quenched with water (15 mL) and extracted with ethyl acetate (2 x 15 mL), dried over Na₂SO₄, filtered and concentrated in *vacuo.* The compound was purified by column chromatography on Biotage® silica gel, eluting with dichloromethane:methanol:ammonia, 98:2:0.2 to 95:5:0.5 to give the title compound as a brown oil (31 mg, 17 %).
¹H NMR (400MHz, CDCl₃) δ7.38 (d, 2H), 6.90 (d, 2H), 4.04 (t, 2H), 2.78 (t, 2H), 2.54 (t, 2H), 2.16-2.12 (m, 2H), 1.94 (pentet, 2H), 1.90-1.64 (m, 11H), 1.20-1.34 (m, 1H), 0.98 (s, 6H); HRMS (ESI⁺) 341.2588 (M+H)⁺.

### Example 117: 1-[1-(4-{3-[(2R)-2-(methoxymethyl)pyrrolidin-1-yl]propoxy}-phenyl)cyclohexyl]methanamine

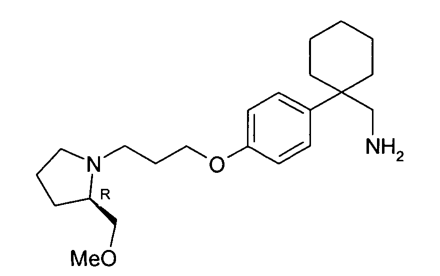

To a stirred suspension of LiAlH₄ (1.0M in diethyl ether, 13.7 ml, 13.7 mmol) at 0°C under an atmosphere of nitrogen was added 1-(4-{3-[(2R)-2-(methoxymethyl)pyrrolidin-1-yl]propoxy}phenyl)cyclohexanecarbonitrile (0.980 g, 2.7 mmol) in Et₂O (15 mL) over 15 minutes maintaining the temperature at 5 to 10°C. The reaction mixture was allowed to warm up to ambient temperature for 20 minutes then refluxed for 30 minutes until complete. The reaction mixture was cooled to 0°C, water (0.5 mL) was added followed by NaOH (2.0M, 1.5 mL) and water (0.5 mL). Ethyl acetate (5 mL) was added and the mixture filtered through a short pad of celite, eluting with ethyl acetate (2 x 15 mL). The organic washings were dried over Na₂SO₄ and concentrated *in vacuo*. The compound was purified by column chromatography on Biotage® silica gel, eluting with dichloromethane:methanol:ammonia, 98:2:0.2 to 95:5:0.5 to give the title compound as a clear oil (309 mg, 32 %).
¹H NMR (400MHz, CDCl₃) δ7.22 (d, 2H), 6.88 (d, 2H), 4.04-3.96 (m, 2H), 3.32 (m, 3H), 3.26-3.20 (m, 1H), 3.14-3.04 (m, 1H), 2.74-2.62 (m, 1H), 2.60 (s, 2H), 2.56-2.46 (m, 1H), 2.28 (quartet, 1 H), 2.12-1.98 (m, 4H), 1.96-1.90 (m, 1H), 1.82-1.70 (m, 2H), 1.68-1.46 (m, 8H), 1.36-1.30 (m, 3H); HRMS (ESI⁺) 361.2850 (M+H)⁺.

### Example 118: {[1-(4-{3-[(3S)-3-methoxypyrrolidin-1-yl]propoxy}-phenyl)-cyclohexyl]methyl}amine

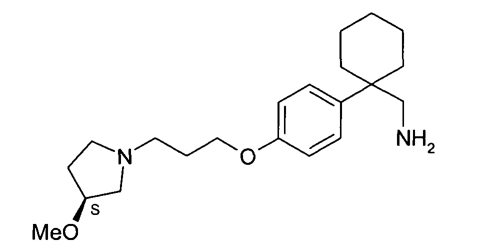

To a stirred suspension of LiAlH₄ (1.0M in diethyl ether, 1.46 ml, 1.46 mmol) at 0°C under an atmosphere of nitrogen was added a solution of 1-(4-{3-[(3S)-3-methoxypyrrolidin-1-yl]propoxylphenyl)cyclohexanecarbonitrile (0.10 g, 0.29 mmol) in Et₂O (2 mL) over 15 minutes maintaining the temperature at 5 to 10°C. The reaction mixture was allowed to warm up to ambient temperature for 20 minutes then refluxed for 30 minutes until complete. The reaction mixture was cooled to 0°C, water (0.5 ml) was added drop-wise followed by sodium hydroxide (2.0M, 1.5 ml) and water (0.5 ml). Ethyl acetate (5 mL) was added and the mixture filtered through a short pad of celite, eluting with ethyl acetate (2 x 15 mL). The organic washings were dried over Na₂SO₄ and concentrated *in vacuo*. The compound was purified by column chromatography on Biotage® silica gel, eluting with dichloromethane:methanol:ammonia, 97:3:0.3 to 90:10:1 to give the title compound as a clear oil (75 mg, 75 %).
¹H NMR (400MHz, CDCl₃) δ7.20 (d, 2H), 6.86 (d, 2H), 4.02 (t, 2H), 3.94-3.88 (m, 1 H), 3.28 (s, 3H), 2.76-2.68 (m, 2H), 2.58-2.66 (m, 3H), 2.45-2.52 (m, 1H), 2.12-2.04 (m, 3H), 1.98 (pentet, 2H), 1.84-1.74 (m, 1H), 1.56-1.44 (m, 5H), 1.38-1.30 (m, 3H), 1.16-1.25 (m, 2H); HRMS (ESI⁺) 347.2693 (M+H)⁺.

### Example 119: N-methyl-1-{1-[4-(3-pyrrolidin-1-ylpropoxy)phenyl]cyclohexyl}methanamine

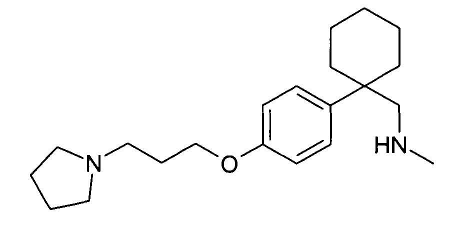

To a stirred solution of ({1-[4-(3-pyrrolidin-1-ylpropoxy)phenyl]cyclohexyl}methyl)amine (0.60 g, 1.90 mmol) and *N*,*N*-diisopropylethylamine (330µL, 1.90 mmol) in dry dichloromethane (6 mL) at 0 ° C was added dropwise a solution of di-*ter*t-butyldicarbonate (455 mg, 2.09 mmol) in dry dichloromethane (4 mL). The reaction was allowed to warm to ambient temperature and stirred for a further 2 hours. The reaction mixture was concentrated *in vacuo*, partitioned between NaHCO₃ (20 mL) and dichloromethane (20 mL) and extracted with a further 20 mL of dichloromethane. The organic layers were combined, dried over Na₂SO₄ and concentrated under reduced pressure to provide the crude product. The compound was purified by column chromatography on silica gel, eluting with DCM/MeOH/NH₃ (95:5:0.5) to give *tert*-butyl ({1-[4-(3-pyrrolidin-1-ylpropoxy)phenyl]cyclohexyl}methyl)-carbamate as a clear oil (642 mg, 81 %).
¹H NMR complicated by the presence of rotamers.
¹H NMR (400 MHz, CDCl₃) δ7.24 (d, 2H), 6.90 (d, 2H), 4.12 (broad singlet, 1H), 4.02 (t, 2H), 3.24-3.06 (m, 2H), 2.66 (t, 2H) 2.56-2.46 (m, 4H), 2.08-1.94 (m, 4H), 1.84-1.72 (m, 4H), 1.62-1.54 (m, 4H), 1.48-1.28 (m, 13H) LCMS (ESI⁺) 417 (M+H)⁺ 439 (M+Na)⁺.

### Step 2:

A solution of *tert*-butyl ({1-[4-(3-pyrrolidin-1-ylpropoxy)phenyl]cyclohexyl}methyl)-carbamate (600 mg, 1.44 mmol) in Et₂O (5 mL) was added dropwise to a stirred solution of LiAlH₄ (1.0M solution in Et₂O, 4.32 mL, 4.32 mmol) at 0 °C. THF (5 mL) was added and the reaction mixture heated at reflux overnight. Reaction mixture was cooled to 0 °C quenched with water (0.5 mL) aqueous NaOH (2.0M, 0.5 mL) and water (0.5 mL). Dichloromethane and ethyl acetate were added and the mixture filtered over a short pad of celite, and concentrated *in vacuo*. The compound was purified by column chromatography on Biotage® silica gel, eluting with dichloromethane:methanol:ammonia, 96:4:0.4 to 90:10:1 to give the title compound as a clear oil (230 mg, 48 %).
¹H NMR (400 MHz, CDCl₃) δ7.16 (d, 2H), 6.88 (d, 2H), 4.02 (t, 2H), 2.64 (t, 2H) 2.54 (s, 2H), 2.50-2.58 (m, 4H), 2.26 (s, 3H), 2.14-2.08 (m, 2H), 1.98 (pentet, 2H), 1.84-1.76 (m, 4H), 1.68-1.56 (m, 2H), 1.52-1.32 (m, 6H); HRMS (ESI⁺) 331.2744 (M+H)⁺.

### Example 120: [(1-{4-[3-(2,2-dimethylpyrrolidin-1-yl)propoxy]phenyl}-cyclohexyl)methyl]amine

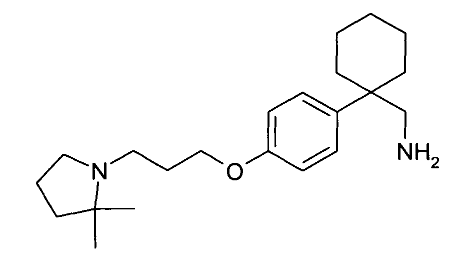

To a stirred suspension of LiAlH₄ (1.0M in diethyl ether, 0.4 ml, 0.40 mmol) at 0°C under an atmosphere of nitrogen was added 1-{4-[3-(2,2-dimethylpyrrolidin-1-yl)propoxy]phenyl}cyclohexanecarbonitrile (27 mg, 0.079 mmol) in Et₂O (15 mL) over 15 minutes maintaining the temperature at 5 to 10°C. The reaction mixture was allowed to warm up to ambient temperature for 20 minutes then refluxed for 30 minutes until complete. The reaction mixture was cooled to 0°C, water (0.5 mL) was added followed by NaOH (2.0M, 1.5 mL) and water (0.5 mL). Ethyl acetate (5 mL) was added and the mixture filtered through a short pad of celite, eluting with ethyl acetate (2 x 15 mL) and concentrated *in vacuo*. The compound was purified by column chromatography on Biotage® silica gel, eluting with dichloromethane:methanol:ammonia, 97:3:0.3 to give the title compound as a clear oil (10 mg, 37 %).
¹H NMR (400 MHz, CDCl₃) δ7.22 (d, 2H), 6.84 (d, 2H), 4.02 (t, 2H), 2.86-2.76 (m, 2H), 2.66 (s, 2H), 2.58-2.50 (m, 2H), 2.16-2.06 (m, 2H), 2.00-2.90 (m, 2H), 1.84-1.72 (m, 2H), 1.70-1.64 (m, 2H), 1.56-1.46 (m, 4H), 1.40-1.32 (m, 4H), 1.02 (s, 6H). HRMS (ESI⁺) 345.2901 (M+H)⁺.

### Example 121: N-ethyl-N-({4-[4-(3-pyrrolidin-1-ylpropoxy)phenyl]tetrahydro-2H-pyran-4-yl}methyl)ethanamine

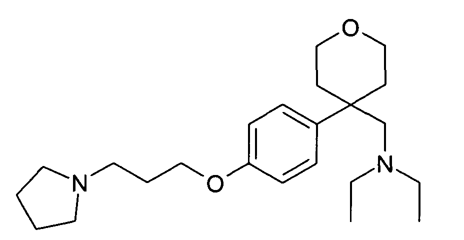

A solution of *N*-ethyl-*N*-({4-[4-(3-pyrrolidin-1-ylpropoxy)phenyl]tetrahydro-2*H*-pyran-4-yl}methyl)acetamide (119mg, 0.307 mmol) in tetrahydrofuran (1 mL) was added dropwise at 0°C to a solution of LiAlH₄ (1.0M in diethylether, 0.614 mL, 0.614 mmol) under an atmosphere of nitrogen. The reaction mixture was allowed to warm to ambient temperature overnight. The reaction mixture was cooled to 0°C, water (0.5 mL) was added drop-wise followed by sodium hydroxide (2.0M, 0.5 mL) and water (0.5 mL). The resulting solid was filtered over a small pad of celite, washed with DCM (100 mL) and concentrated *in vacuo*. The compound was purified by column chromatography on Biotage® silica gel, eluting with dichloromethane:methanol:ammonia, 94.5:5.5:0.55 to 91:9:0.9 to give the title compound as a pale yellow oil (61 mg, 53 %).
¹H NMR (400 MHz, CDCl₃) δ7.18 (d, 2H), 6.86 (d, 2H), 4.02 (t, 2H), 3.70-3.78 (m, 2H), 3.48 (dt, 2H), 2.64 (t, 2H), 2.58-2.46 (m, 4H), 2.38 (s, 2H), 2.20 (quartet, 4H), 2.13-1.96 (m, 4H), 1.92-1.86 (m, 2H), 1.82-1.78 (m, 4H), 0.82 (t, 6H); HRMS (ESI⁺) 375.3006 (M+H)⁺.

### Example 122: N-ethyl-N-({4-[4-(3-pyrrolidin-1-ylpropoxy)phenyl]tetrahydro-2H-pyran-4-yl}methyl)acetamide

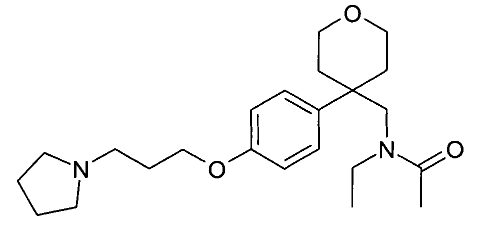

Acetyl chloride (14 µL, 0.20 mmol) was added dropwise to a solution of *N*-({4-[4-(3-pyrrolidin-1-ylpropoxy)phenyl]tetrahydro-2*H*-pyran-4-yl}methyl)ethanamine (70 mg, 0.20 mmol) and triethylamine (28 µL, 0.20 mmol) in THF (0.50 mL) at 0°C and the reaction stirred for 2 hours. The reaction was quenched using NaHCO₃ (10 mL), extracted with dichloromethane (3 x 10 mL) and the organic extracts dried over Na₂SO₄, filtered and concentrated *in vacuo*. The compound was purified by column chromatography on silica, eluting with DCM/MeOH/NH₃ (95:5:0.5) to provide the title compound as a clear oil (33 mg, 42 %).
¹H NMR complicated by the presence of rotamers.
¹H NMR (400 MHz, CDCl₃) δ7.20-7.12 (m, 2H), 6.92-6.86 (m, 2H), 4.06-4.00 (m, 2H), 3.88-3.76 (m, 2H), 2.56-2.48 (m, 2H), 3.42 (s, 2H), 2.66 (t, 2H), 2.58-2.52 (m, 4H), 2.48 (quartet, 2H), 2.08 (s, 3H), 2.06-1.92 (m, 6H), 1.84-1.76 (m, 4H), 0.98 (t, 0.6 H) 0.86 (t, 2.4H). HRMS (ESI⁺) 389.2799 (M+H)⁺.

### Example 123: N-methyl-N-({4-[4-(3-pyrrolidin-1-ylpropoxy)phenyl]-tetrahydro-2H-pyran-4-yl}methyl)ethanesulfonamide

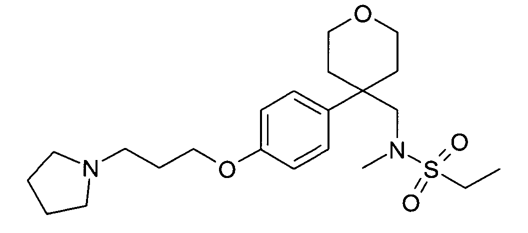

Ethanesulfonyl chloride (43 µL, 0.456 mmol) was added drop-wise to a solution of triethylamine (58 µL, 0.418 mmol) and *N*-methyl-1-{1-[4-(3-pyrrolidin-1-ylpropoxy)phenyl]cyclohexyl}methanamine (125 mg, 0.38 mmol) in dichloromethane (1 mL) at 0 °C and the reaction stirred for 30 minutes. The reaction was quenched with NaHCO₃ (20 mL), extracted using dichloromethane (3 x 10 mL), dried using Na₂SO₄ and concentrated *in vacuo*. The compound was purified by column chromatography on Biotage® silica gel, eluting with dichloromethane:methanol:ammonia, 98:2:0.2 to 96:4:0.4 to give the title compound as a pale brown oil (152 mg, 94 %).
¹H NMR (400 MHz, CDCl₃) δ7.24 (d, 2H), 6.90 (d, 2H), 4.04 (t, 2H), 3.84-3.76 (m, 2H), 2.56-2.50 (m, 2H), 3.26 (s, 2H), 2.88 (quartet, 2H), 2.68 (t, 2H), 2.60-2.52 (m, 4H), 2.22 (s, 3H), 2.30-2.12 (m, 2H), 2.02 (pentet, 2H), 1.98-1.90 (m, 2H), 1.86-1.78 (m, 4H) 1.34 (t, 3H); HRMS (ESI⁺) 425.2469 (M+H)⁺.

### Example 124: N-methyl-N-({4-[4-(3-pyrrolidin-1-ylpropoxy)phenyl]-tetrahydro-2H-pyran-4-yl}methyl)propane-1-sulfonamide

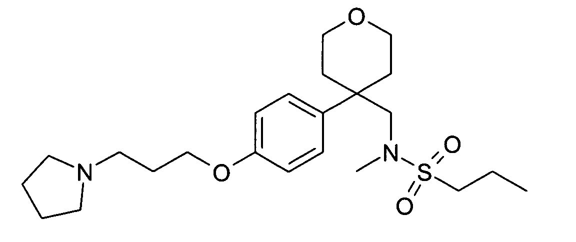

*n*-Propylsulfonyl chloride (33 µL, 0.289 mmol) was added drop-wise to a solution of triethylamine (37 µL, 0.265 mmol) and *N*-methyl-1-{1-[4-(3-pyrrolidin-1-ylpropoxy)phenyl]cyclohexyl}methanamine (80 mg, 0.24 mmol) in dichloromethane (1 mL) at 0 °C and the reaction stirred for 1 hour. The reaction was quenched with NaHCO₃ (20 mL), extracted using dichloromethane (3 x 15 mL), dried using Na₂SO₄ and concentrated *in vacuo*. The compound was purified by column chromatography on Biotage® silica gel, eluting with dichloromethane:methanol:ammonia, 98:2:0.2 to 92:8:0.8 to give the title compound as a pale brown oil (62 mg, 59 %).
¹H NMR (400 MHz, CDCl₃) δ7.22 (d, 2H), 6.90 (d, 2H), 4.02 (t, 2H), 3.82-3.74 (m, 2H), 3.50 (dt, 2H), 3.20 (s, 2H), 2.74 (dt, 2H), 2.64 (t, 2H), 2.56-2.48 (m, 4H), 2.20 (s, 3H), 2.18-2.12 (m, 2H), 2.02 (pentet, 2H), 1.98-1.84 (m, 2H), 1.82-1.68 (m, 6H) 1.00 (t, 3H); HRMS (ESI⁺) 439.2625 (M+H)⁺.

### Example 125: N-ethyl-N-({4-[4-(3-pyrrolidin-1-ylpropoxy)phenyl]tetrahydro-2H-pyran-4-yl}methyl)methanesulfonamide

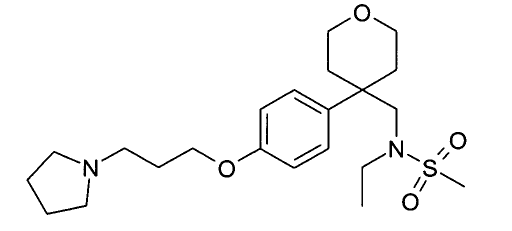

Methanesulfonyl chloride (19 µL, 0.24 mmol) was added drop-wise to a solution of triethylamine (31 µL, 0.22 mmol) and *N*-({4-[4-(3-pyrrolidin-1-ylpropoxy)phenyl]tetrahydro-2*H*-pyran-4-yl}methyl)ethanamine (70 mg, 0.20 mmol) in dichloromethane (0.5 mL) at 0 °C then was allowed to warm to ambient temperature and stirred overnight. The reaction was quenched with NaHCO₃ (10 mL), extracted using dichloromethane (3 x 15 mL), dried using Na₂SO₄ and concentrated *in vacuo*. The compound was purified by column chromatography on Biotage® silica gel, eluting with dichloromethane:methanol:ammonia, 96.5:3.5:0.35 to 95:5:0.5 to give the title compound as a pale brown oil (60 mg, 71 %).
¹H NMR (400 MHz, CDCl₃) δ7.24 (d, 2H), 6.90 (d, 2H), 4.02 (t, 2H), 3.84-3.72 (m, 2H), 3.50 (dt, 2H), 3.28 (s, 2H), 2.72-2.60 (m, 7H), 2.58-2.52 (m, 4H), 2.16 (m, 2H), 2.04 (pentet, 2H), 1.96-1.90 (m, 2H), 1.84-1.76 (m, 4H), 0.90 (t, 3H); HRMS (ESI⁺) 425.2469 (M+H)⁺.

### Example 126: N-ethyl-N-({4-[4-(3-pyrrolidin-1-ylpropoxy)phenyl]tetrahydro-2H-pyran-4-yl}methyl)ethanesulfonamide

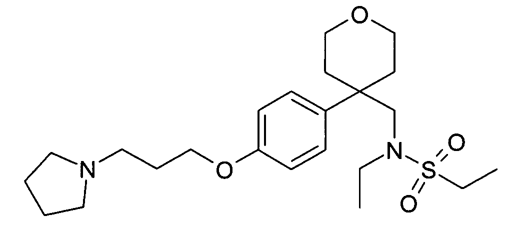

Ethanesulfonyl chloride (26 µL, 0.28 mmol) was added drop-wise to a solution of triethylamine (35 µL, 0.25 mmol) and *N*-({4-[4-(3-pyrrolidin-1-ylpropoxy)phenyl]tetrahydro-2*H*-pyran-4-yl}methyl)ethanamine (78 mg, 0.23 mmol) in dichloromethane (0.5 mL) at 0 °C allowed to warm to ambient temperature and stirred overnight. The reaction was quenched with NaHCO₃ (10 mL), extracted using dichloromethane (3 x 10 mL), dried using Na₂SO₄ and concentrated *in vacuo*. The compound was purified by column chromatography on Biotage® silica gel, eluting with dichloromethane:methanol:ammonia, 96.5:3.5:0.35 to 95:5:0.5 to give the title compound as a pale brown oil (55 mg, 54 %).
¹H NMR (400 MHz, CDCl₃) δ7.24 (d, 2H), 6.90 (d, 2H), 4.02 (t, 2H), 3.84-3.72 (m, 2H), 3.48 (t, 2H), 3.32 (s, 2H), 2.76 (quartet, 2H), 2.72-2.60 (m, 4H), 2.60-2.48 (m, 4H), 2.20-2.08 (m, 2H), 2.04 (pentet, 2H), 1.98-1.90 (m, 2H), 1.86-1.76 (m, 4H), 1.30 (t, 3H), 0.88 (t, 3H). HRMS (ESI⁺) 439.2625 (M+H)⁺.

### Example 127: N-({4-[4-(3-pyrrolidin-1-ylpropoxy)phenyl]tetrahydro-2H-pyran-4-yl}methyl)ethanamine

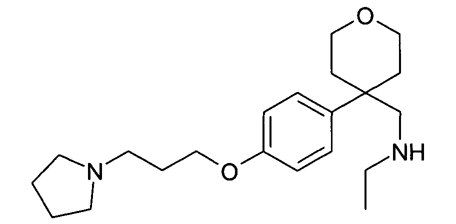

LiAlH₄ (1.0M in diethylether, 4.72 mL, 4.72 mmol) was added dropwise at 0°C to a solution of (*N*-{4-[4-(3-Pyrrolidin-1-yl-propoxy)-phenyl]-tetrahydro-pyran-4-ylmethyl}-acetamide (848 mg, 2.36 mmol) in tetrahydrofuran (2 mL) under an atmosphere of nitrogen. The reaction mixture was allowed to warm to ambient temperature for 2 hours then heated to 50 °C for 10 hours. The reaction mixture was cooled to 0 °C, water (3 mL) was added drop-wise followed by sodium hydroxide (2.0M, 6 mL) and water (3 mL). The resulting solid was filtered over a small pad of celite, washed with Et₂O/DCM (1:1 6 x 50 mL) and concentrated *in vacuo* to give the title compound as a pale yellow solid (635 mg, 78 %).
¹H NMR (400 MHz, CDCl₃) δ7.20 (d, 2H), 6.88 (d, 2H), 4.02 (t, 2H), 3.80-3.68 (m, 2H), 3.55 (dt, 2H), 2.68 (s, 2H), 2.60 (t, 2H), 2.58-2.42 (m, 6H), 2.18-2.08 (m, 2H), 2.00 (pentet, 2H) 1.94-1.86 (m, 2H), 1.84-1.76 (m, 4H), 1.92 (t, 3H). HRMS (ESI⁺) 347.2693 (M+H)⁺.

### Example 128: N-methyl-N-({4-[4-(3-pyrrolidin-1-ylpropoxy)phenyl]tetrahydro-2H-pyran-4-yl}methyl)ethanamine

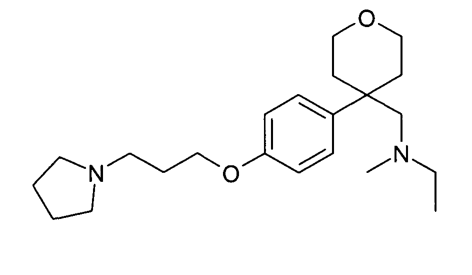

A solution of LiAlH₄ (1.0M in diethylether, 1.20 mL, 0.60 mmol) was added dropwise at 0°C to a solution of *N*-methyl-*N*-{4-[4-(3-Pyrrolidin-1-yl-propoxy)-phenyl]-tetrahydro-pyran-4-ylmethyl}-acetamide (220 mg, 0.60 mmol) in tetrahydrofuran (1 mL) under an atmosphere of nitrogen. The reaction mixture was allowed to warm to ambient temperature overnight. The reaction mixture was cooled to 0 °C, water (1 mL) was added drop-wise followed by sodium hydroxide (2.0M, 3 mL) and water (1 mL). The resulting solid was filtered over a small pad of celite, washed with DCM (150 mL) and concentrated *in vacuo*. The compound was purified by column chromatography on Biotage® silica gel, eluting with dichloromethane:methanol:ammonia, 97.5:2.5:0.25 to 95.5:4.5:0.45 to give the title compound as a pale yellow oil (114 mg, 88 %).
¹H NMR (400 MHz, CDCl₃) δ7.18 (d, 2H), 6.86 (d, 2H), 4.04 (t, 2H), 3.78-3.70 (m, 2H), 3.52 (dt, 2H), 2.66 (t, 2H), 2.52-2.66 (m, 4H), 2.38 (s, 2H), 2.18 (quartet, 2H), 2.00-2.12 (m, 4H), 1.92-1.86 (m, 5H), 1.82-1.76 (m, 4H), 0.86 (t, 3H); HRMS (ESI⁺) 361.2850 (M+H)⁺.

### Intermediate 47: 4-(4-{3-[(2S)-2-methylpyrrolidinyl]propoxy}phenyl)-tetrahydro-2H-pyran-4-carbonitrile

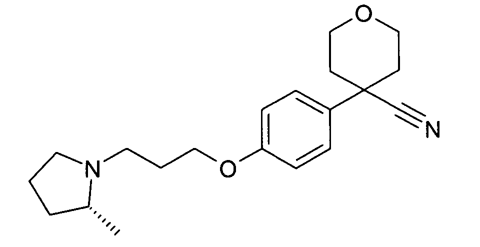

A solution of 4-(4-Hydroxy-phenyl)-tetrahydro-pyran-4-carbonitrile (203 mg, 1.0 mmol) and tri-*n*-butylphosphine (299 µL, 1.20 mmol) in toluene (6 mL) were added to a solution of 3-[(2*R*)-2-methylpyrrolidin-1-yl]propan-1-ol (172 mg, 1.20 mmol) in toluene (6 mL) and tetrahydrofuran (2 mL) at ambient temperature. 1'1-azobis(N,N-dimethylformamide) (206 mg, 1.20 mmol) was added and the reaction heated to 85°C overnight. The reaction mixture was concentrated *in vacuo* followed by addition on dichloromethane (30 mL). The organic solution was then washed with NaOH (2.0M, 2 x 20 mL), dried with Na₂SO₄ and concentrated *in vacuo.* The compound was purified by column chromatography on Biotage® silica gel, eluting with dichloromethane:methanol:ammonia, 98:2:0.2 to 96:4:0.4 to give the title compound as a pale yellow oil (70mg, 21 %).
¹H NMR (400 MHz, CDCl₃) δ7.18 (d, 2H), 6.92 (d, 2H), 4.10-4.00 (m, 4H), 3.88 (dt, 2H), 3.26-3.14 (m, 1 H), 3.02-2.92 (m, 1 H), 2.26-1.38 (m, 13H), 1.08 (d, 3H); HRMS (ESI⁺) 329.2224 (M+H)⁺.

### Example 129: 4-(4-{3-[(2R)-2-methylpyrrolidin-1-yl]propoxy}phenyl)-tetrahydro-2H-pyran-4-carboxylic acid

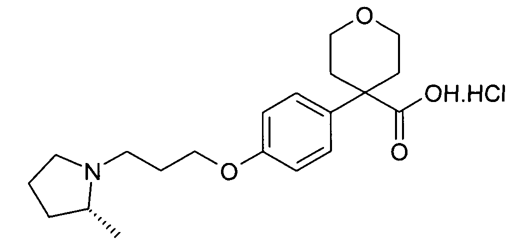

A solution of 4-(4-{3-[(2*S*)-2-methylpyrrolidinyl]propoxy}phenyl)tetrahydro-2*H*-pyran-4-carbonitrile (70 mg, 0.21 mmol) in concentrated hydrochloric acid (1 mL) was heated at reflux for 3 days. A further 2mL of concentrated hydrochloric acid was added and the reaction heated at reflux for a further 2 days. The solution was evaporated to dryness *in vacuo*, triturated using acetone and azeotropically dried with toluene to give a pale brown solid (51 mg, 73 %).
¹H NMR (400 MHz, CDCl₃) δ7.36 (d, 2H), 6.94 (d, 2H), 4.12 (t, 2H), 3.92-3.84 (m, 2H), 3.76-3.46 (m, 6H), 2.54-2.44 (m, 2H), 2.20-2.02 (m, 1 H), (m, 8H), (m, 3H); HRMS (ESI⁺) 348.2170 (M+H)⁺.

### Intermediate 48: tert-butyl 4-[4-(4-cyanotetrahydro-2H-pyran-4-yl)phenoxy]-1-piperidinecarboxylate

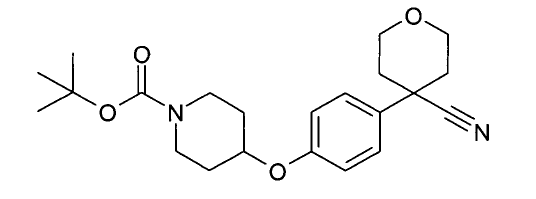

*tert*-butyl 4-[(methylsulfonyl)oxy]-1-piperidinecarboxylate (1.35g, 4.93mmol), 4-(4-hydroxyphenyl)tetrahydro-2*H*-pyran-4-carbonitrile (1.00g, 4.93mmol) and potassium carbonate (1.36g, 9.85mmol) were stirred in 10ml DMF at 56°C for 28 hours. The reaction mixture was partitioned between ethyl acetate (50ml) and water (50ml). The ethyl acetate was dried over Na₂SO₄ and evaporated to give the crude product which was then chromatographed on silica gel eluting with 2% diethylamine in ethylacetate. This only gave a poor separation, so the impure material was triturated with diisopropylether (20ml) to give a white powder (1.5g). This solid was dissolved in diethylether (100ml) and washed with 2M NaOH (50ml) then with water (50ml). The diethylether was dried over Na₂SO₄ and evaporated to give *tert*-butyl 4-[4-(4-cyanotetrahydro-2*H*-pyran-4-yl)phenoxy]-1-piperidinecarboxylate as white crystals (740mg, 39%)
¹H NMR (400MHz, CDCl₃) δ7.39 (d, 2H), 6.93 (d, 2H), 4.48 (m, 1H), 4.08 (m, 2H), 3.89 (m, 2H), 3.67 (m, 2H), 3.37 (m, 2H), 2.08 (m, 4H), 1.92 (m, 2H), 1.77 (m, 2H), 1.45 (s, 9H)

### Intermediate 49: 4-[4-(4-piperidinyloxy)phenyl]tetrahydro-2H-pyran-4-carbonitrile

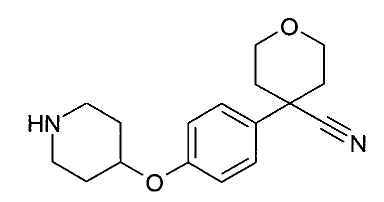

*tert*-butyl4-[4-(4-cyanotetrahydro-2*H*-pyran-4-yl)phenoxy]-1-piperidinecarboxylate (720mg) was dissolved in dichloromethane (50ml) at 0°C. TFA (10ml) was added and the mixture stirred whilst warming to room temperature over 2 hours. The reaction mixture was then concentrated *in vacuo,* and partitioned between dichloromethane (80ml) and 0.5M NaOH (50ml). The dichlorormethane solution was dried over Na₂SO₄ and evaporated to give 4-[4-(4-piperidinyloxy)phenyl]tetrahydro-2*H*-pyran-4-carbonitrile as a colurless oil which crystallized on standing (500mg, 94%).
¹H NMR (400MHz, CDCl₃) δ7.37 (d, 2H), 6.94 (d, 2H), 4.39 (m, 1 H), 4.07 (m, 2H), 2.83 (m, 2H), 3.16 (m, 2H), 2.72 (m, 2H), 2.17-1.98 (m, 6H), 1.68 (m, 2H), 1.53 (bs, 1H)

### Example 130: 4-{4-[(1-cyclobutyl-4-piperidinyl)oxy]phenyl}tetrahydro-2H-pyran-4-carbonitrile

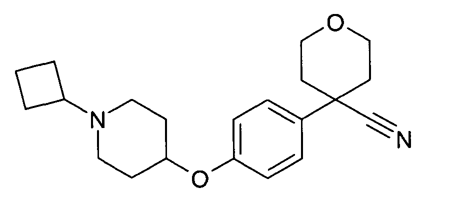

4-[4-(4-piperidinyloxy)phenyl]tetrahydro-2*H*-pyran-4-carbonitrile (130mg, 0.45mmol) and cyclobutanone (140µl, 1.9mmol) were stirred in THF (2ml) and acetic acid (26µl, 0.45mmol) at ambient temperature for 15 mins. Sodium triacetoxyborohydride (340mg, 1.6mmol) was added and the mixture stirred for 18 hrs. The reaction was quenched by adding 10% aqueous Na₂CO₃ (5ml) and then partitioned between ethyl acetate (50ml) and water (30ml). The organics were dried over Na₂SO₄ and evaporated to give a solid which was purified by column chromatography on silica gel eluting with dichloromethane:methanol:ammonia, 98:2:0.2 to 94:6:0.6 to give the product which was then crystallized from diisopropylether (5ml) to give 4-{4-[(1-cyclobutyl-4-piperidinyl)oxy]phenyl}tetrahydro-2*H*-pyran-4-carbonitrile as a white powder (50mg, 33%).
¹H NMR (400MHz, CDCl₃) δ7.38 (d, 2H), 6.93 (d, 2H), 4.33 (m, 1H), 4.08 (m, 2H), 3.89 (m, 2H), 2.75 (pentet, 1H), 2.62 (m, 2H), 2.20-1.58 (m, 16H)

### Example 131: 4-{4-[(1-isopropylpiperidin-4-yl)oxylphenyl}tetrahydro-2H-pyran-4-carboxamide

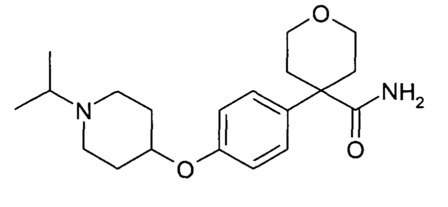

4-[4-(1-Isopropylpiperidin-4-yloxy)phenyl]tetrahydropyran-4-carbonitrile (50 mg, 0.152 mmol) was combined with boron trifluoride-acetic acid complex (500 µL, 3.6 mmol) and stirred at ambient temperature for 18 hours, followed by 65°C for 3 hours. The reaction mixture was allowed to cool, and was then basified with saturated NaHCO₃ solution (15 mL). The product was extracted with dichloromethane (2 x 35 mL). The combined organic extracts were dried using Na₂SO₄ filtered and concentrated *in vacuo*. The compound was purified by column chromatography on silica gel (15 g), eluting with dichloromethane:methanol:ammonia, (96:4:0.4) to dichloromethane:methanol:ammonia, (90:10:1) to give the title compound as a white solid (22 mg, 42%)
¹H NMR (400 MHz, CD₃OD) δ7.3 (d, 2H), 6.9 (d, 2H), 4.4 (m, 1H), 3.8 (m, 2H), 3.65 (m, 2H), 2.78-2.85 (m, 3H), 2.5 (m, 2H), 2.4 (d, 2H), 1.95-2.08 (m, 4H), 1.8 (m, 2H), 1.1(d, 6H)

### Example 132: 4-{4-[(1-isopropylpiperidin-4-yl)oxy]phenyl}tetrahydro-2H-pyran-4-carboxylic acid

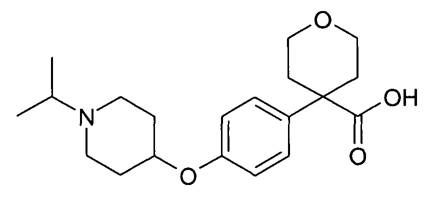

4-[4-(1-Isopropylpiperidin-4-yloxy)phenyl]tetrahydropyran-4-carbonitrile (1g, 3 mmol) and concentrated hydrochloric acid (10 mL) were combined and heated at reflux for 44 hours. Reaction mixture was concentrated *in vacuo* the resulting grey solid was triturated with acetone (20 mL) to give a cream coloured solid. The compound was purified using an SCX isolelute column which was flushed with methanol (60 mL) and then flushed with 2M NH₃ in methanol (150 mL). The title compound was isolated as a cream coloured solid (550mg, 52%).
¹H NMR (400 MHz, CD₃OD) δ7.4 (d, 2H), 6.9 (d, 2H), 4.4 (m, 1H), 3.85 (m, 2H), 3.65 (m, 2H), 3.4 (m, 1H), 3.2 (m, 2h), 3.03 (m, 2H), 2.5 (m, 2H), 2.0- 1.8 (m, 6H), 1.3 (d, 6H)

### Example 133: 4-{4-[(1-isopropylpiperidin-4-yl)oxylphenyl]-N,N-dimethyltetrahydro-2H-pyran-4-carboxamide

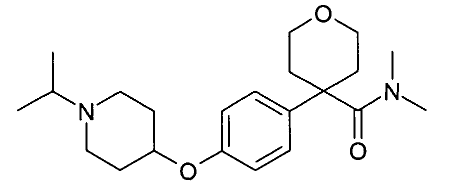

4-{4-[(1-isopropylpiperidin-4-yl)oxy]phenyl}tetrahydro-2*H*-pyran-4-carboxylic acid (150 mg, 3.9 mmol), O-(1H-Benzotriazol-1-yl)-N,N,N',N'-tetramethyluronium hexafluorophosphate (222 mg, 5.8 mmol), dimethylamine hydrochloride (96mg, 1.1 mmol), pyridine (126uL, 1.56 mmol) were combined and dissolved in dimethylformamide (2 mL). The reaction mixture was stirred at ambient temperature for 18 hours. Further O-(1H-Benzotriazol-1-yl)-N,N,N',N'-tetramethyluronium hexafluorophosphate (222 mg, 5.8 mmol) and dimethylamine in ethanol (33%) (500 µL) was added and the reaction mixture was stirred at ambient temperature for 18 hours. The reaction mixture was diluted with water (20 mL) and was then extracted with ethyl acetate (2 x 40 mL). The combined organic extracts were dried using Na₂SO₄, filtered and concentrated *in vacuo.* The compound was purified by column chromatography on silica gel, eluting with dichloromethane:methanol:ammonia (97:3:0.3) to dichloromethane:methanol:ammonia (90:10:1) to give a compound as a clear colourless oil. This was dissolved in ethyl acetate (70 mL) and washed with saturated NaHCO₃ solution (2 x 10 mL). The organic extract was dried using Na₂SO₄, filtered and concentrated *in vacuo* to give the title compound as a clear crystaline solid (72mg, 50%).
¹H NMR (400 MHz, CD₃OD) δ7.2 (d, 2H), 6.95 (d, 2H), 4.4 (m, 1 H), 3.85 (m, 2H), 3.7 (m, 2H), 2.9-2.5 (m, 11 H), 2.28 (m, 2H), 2.0 (m, 4H), 1.8 (m, 2H), 1.1 (d, 6H)

### Example 134: 4-{4-[(1-isopropylpiperidin-4-yl)oxy]phenyl]-N,N-diethyltetrahydro-2H-pyran-4-carboxamide

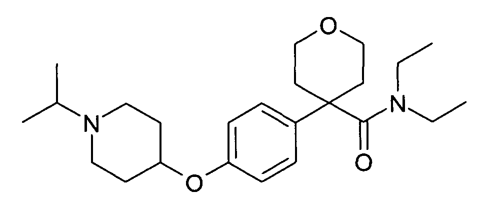

4-{4-[(1-isopropylpiperidin-4-yl)oxy]phenyl}tetrahydro-2*H*-pyran-4-carboxylic acid (86mg, 0.2 mol), diethylamine (27 µL, 0.26 mmol), O-(1H-Benzotriazol-1-yl)-N,N,N',N'-tetramethyluronium hexafluorophosphate (108mg, 0.29 mmol), pyridine (53 uL, 0.66 mmol) were combined and dissolved in dimethylformamide (1.5 mL). The reaction mixture was stirred at 60°C for 5 hours. Further diethylamine was added (500 µL). The reaction mixture was stirred at ambient temperature for 60 hours. The reaction mixture was diluted with water (25 mL), and extracted with ethyl acetate (2 x 35 mL). The combined organics were dried using Na₂SO₄, filtered and concentrated *in vacuo*. The compound was purified by column chromatography using 15g of silica gel, eluting with dichloromethane:methanol:ammonia (96:4:0.4) to dichloromethane:methanol:ammonia (90:10:1) to give the title compound as a clear yellow oil (10mg, 11 %).
¹H NMR (400 MHz, CD₃OD) δ7.2 (d, 2H), 6.95 (d, 2H), 4.4 (m, 1 H), 3.85-3.7 (m, 4H), 3.3 (m, 2H), 3.05-2.9 (m, 5H), 2.6 (m, 2H), 2.25 (d, 2H), 2.0 (m, 4H), 1.8 (m, 2H), 1.1 (m, 9H), 0.63(m,3H)

### Example 135: 4-{4-[(1-isopropylpiperidin-4-yl)oxylphenyl)tetrahydro-2H-pyran-4-yl]carbonyl}pyrrolidine

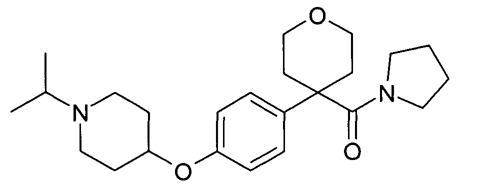

4-{4-[(1-isopropylpiperidin-4-yl)oxy]phenyl}tetrahydro-2*H*-pyran-4-carboxylic acid (93mg, 0.27 mmol) was supended in dimethylformamide (3mL), O-(1H-Benzotriazol-1-yl)-N,N,N',N'-tetramethyluronium hexafluorophosphate (203mg, 0.53 mmol), and triethylamine (112µL, 0.8 mmol) were added. The reaction mixture was stirred at ambient temperature for 1 hour. Pyrrolidine (300µL) was added, the reaction mixture was stirred at ambient temperature for 2.5 hours. The reaction mixture was concentrated *in vacuo,* then partitioned between saturated NaHCO₃ solution (50 mL) and dichloromethane (2 x 75 mL) The combined organics were dried using Na₂SO₄, filtered and concentrated *in vacuo*. The compound was purified by column chromatography using 20g of silica gel, eluting with dichloromethane:methanol:ammonia (96:4:0.4) to dichloromethane:methanol:ammonia (94:6:0.6) to give the title compound as a clear oil (34mg, 32%).
¹H NMR (400 MHz, CD₃OD) δ7.2 (d, 2H), 6.9 (d, 2H), 4.4 (m, 1H), 3.85-3.7 (m, 4H), 3.45 (m, 2H), 2.95 (m, 2H), 2.8-2.7 (m, 3H), 2.5 (m, 2H), 2.3 (m, 2H), 2.0 (m, 4H), 1.8-1.7 (m, 4H), 1.6 (m, 2H), 1.1 (d, 6H)

### Intermediate 50: 4-[4-(3-pyrrolidin-1-ylpropoxy)phenyl]tetrahydro-2H-pyran-4-carbothioamide

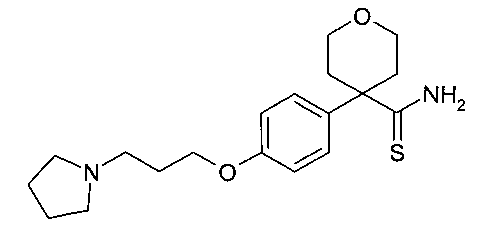

4-[4-(3-Pyrrolidin-1-yl-propoxy)-phenyl]-tetrahydro-pyran-4-carbonitrile (2g, 6.4 mmol), diethyl dithiophosphate (15 mL, 8.9 mmol), and water (1.5 mL) were combined and stirred at ambient temperature for 5 hours, 55 °C for 2 hours then 70°C for 2 hours. The reaction mixture was diluted with dichloromethane (100mL) and basified to pH 8 with a saturated solution of NaHCO₃ in water, and solid NaHCO₃. Layers were separated, and further dichloromethane (100 mL) was used to extract the product. The combined organics were dried using Na₂SO₄, filtered and concentrated *in vacuo* to leave a yellow oil. The compound was purified with column chromatography on 50g of silica, eluting with dichloromethane:methanol:ammonia (96:4:0.4) to dichloromethane:methanol:ammonia (90:10:1) to give a clear oil. The clear oil was dissolved in ethyl acetate (150 mL) and washed with 10% Na₂CO₃ solution. The organic layer was dried using Na₂SO₄ , filtered and concentrated *in vacuo* to give the title compound as a white solid (0.61g, 28%).
¹H NMR (400 MHz, CDCl₃) δ7.4 (s, 1 H), 7.3 (d, 2H), 6.93 (d, 2H), 6.5 (s, 1 H), 4.03 (t, 2H), 3.39 (m, 2H), 3.58 (m, 2H), 2.7-2.5 (m, 8H), 2.23 (m, 2H), 2.02 (m, 2H), 1.8 (m, 4H).

### Example 136: 4-methyl-2-[4-(4-(3-pyrrolidin-1-ylpropoxy)phenyl)tetrahydro-2H-pyran-4-yl]-1,3-thiazole

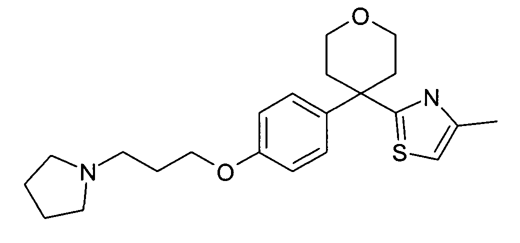

4-[4-(3-Pyrrolidin-1-ylpropoxy)phenyl]tetrahydro-2*H*-pyran-4-carbothioamide (100mg, 0.29 mmol), chloroacetone (46 µL, 0.57 mmol) and ethanol (5 mL) were combined and heated at reflux for 18 hours. The reaction mixture was concentrated *in vacuo* to give a cream coloured solid which was dissolved in dichloromethane (100 mL) and washed with saturated NaHCO₃ solution (50 mL). The organic extract was dried using Na₂SO₄ , filtered and concentrated *in vacuo*. The compound was purified by column chromatography using 15g of silica gel, eluting with dichloromethane:methanoal:ammonia (97:3:0.3) to dichloromethane:methanol:ammonia (94:6:0.6) to give the title compound as a white solid (68mg, 61 %).
¹H NMR (400 MHz, CDCl₃) δ7.3 (d, 2H), 6.9 (d, 2H), 6.75 (s, 1 H), 4.0 (t, 2H), 3.83 (m, 2H), 3.7 (m, 2H), 2.75-2.58 (m, 8H), 2.4 (s, 3H), 2.35 (m, 2H), 2.03 (m, 2H), 1.8 (m, 4H)

### Example 137: 2-[4-(4-(3-pyrrolidin-1-ylpropoxy)phenyl)tetrahydro-2H-pyran-4-yl]-1,3-thiazole

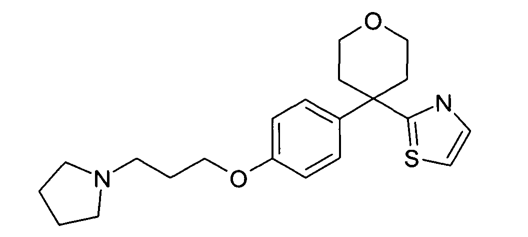

4-[4-(3-Pyrrolidin-1-ylpropoxy)phenyl]tetrahydro-2*H*-pyran-4-carbothioamide (150 mg, 0.43 mmol), bromoacetaldehyde diethylacetal (67 µL, 0.43 mmol), concentrated hydrochloric acid (5 drops) and ethanol were combined and heated at reflux for 5 hours. The reaction mixture was concentrated *in vacuo* and partitioned between 10% Na₂CO₃ (20mL) and dichloromethane (2 x 35 mL). The combined organics were dried using Na₂SO₄, filtered and concentrated *in vacuo.* The compound was purified using column chromatography using 15g of silica gel, eluting with dichloromethan:methanol:ammonia (95:5:0.5) to give a cream coloured solid, which was triturated with 3mL of diethyl ether to give the title compound as a cream coloured solid (25mg, 16%).
¹H NMR (400 MHz, CD₃OD) δ7.7 (s, 1H), 7.45 (s, 1 H), 7.3 (d, 2H), 6.9 (d, 2H), 4.0 (t, 2H), 3.8 (m, 2H), 3.7 (m, 2H), 2.85-2.75 (m, 6H), 2.6 (m, 2H), 2.4 (m, 2H), 2.03 (m , 2H), 1.9 (m, 4H)

### Intermediate 51: (4-{4-[(1-isopropylpiperidin-4-yl)oxylphenyl}tetrahydro-2H-pyran-4-yl)methylamine

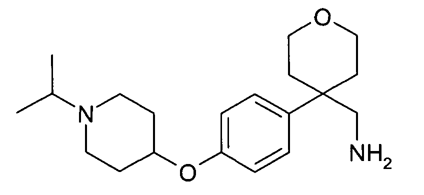

To a stirred solution 4-[4-(1-Isopropylpiperidin-4-yloxy)phenyl]tetrahydropyran-4-carbonitrile (2.5 g, 7.62 mmol) in dry diethyl ether (15 ml) at 0°C was added drop-wise a solution of LiAlH₄ (1.0M solution in Et₂O, 22.87 ml, 22.9 mmol). Reaction stirred at 0°C for 30 mins, then warmed up to ambient temperature overnight under a nitrogen atmosphere until complete. The reaction was cooled to 0°C, water (0.9 ml) was added drop-wise followed by sodium hydroxide (2.0M, 0.9 ml) and water (2.7 ml). Dichloromethane (25 ml) and methanol (1 ml) were added and the mixture filtered through a short pad of arbocel, eluting with 2% methanol in dichloromethane (200 ml). The organic washings were dried over Na₂SO₄ and concentrated *in vacuo* to give the title compound as an orange oil (2.59 g, 100%).
¹H NMR (400MHz, CDCl₃) δ7.25 (d, 2H), 6.90 (d, 2H), 4.22-4.30 (m, 1 H), 3.78-3.82 (m, 2h), 3.50-3.59 (m, 2H), 2.69-2.86 (m, 5H), 2.32-2.46 (m, 2H), 2.08-2.16 (m, 2H), 1.98-2.06 (m, 2H), 1.76-1.88 (m, 4H), 1.15 (d, 6H).

### Intermediate 52: tert-butyl [4-(4-[(1-isopropylpiperidin-4-yl)oxy]phenyl)tetrahydro-2H-pyran-4-yl]methylcarbamate

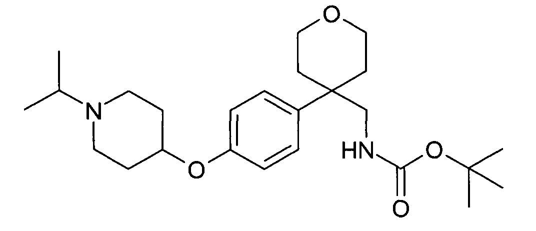

To a stirred solution of (4-{4-[(1-isopropylpiperidin-4-yl)oxy]phenyl}tetrahydro-2*H*-pyran-4-yl)methylamine (600 mg, 1.81 mmol) and triethylamine (578 µl. 5.42 mmol) in dichloromethane (2.5 ml) at 0°C, was added drop-wise a solution of di-tert-butyldicarbonate (540 µl, 2.35 mmol) in dichloromethane (2.5 ml). Solution stirred at 0°C for 2 hours then allowed to warm up to ambient temperature and stirred until reaction complete. Reaction mixture diluted with dichloromethane (10 ml), washed with water (3 x 10 ml) then brine (2 x 10 ml). Organic washings dried over Na₂SO₄ and concentrated *in vacuo.* The compound was purified by column chromatography on silica gel, eluting with dichloromethane:methanol:ammonia, 100:0:0 to 96:4:0:0.4 to give the title compound as a foam (532 mg, 68%).
¹H NMR (400MHz, CDCl₃) δ7.18 (d, 2H), 6.90 (d, 2H), 4.20-4.38 (m, 1H), 4.24-5.04 (m, 1 H), 3.74-3.88 (m, 2H), 3.52-3.64 (m, 2H), 3.20-3.38 (m, 2H), 2.70-2.88 (m, 3H), 2.30-2.50 (m, 2H), 1.98-2.10 (m, 4H), 1.79-1.97 (m, 4H), 1.32-1.50 (m, 9H), 1.10 (d, 6H).

### Example 138: N-[(4-{4-[(1-isopropylpiperidin-4-yl)oxy]phenyl}tetrahydro-2H-pyran-4-yl)methyl]-N-methylamine

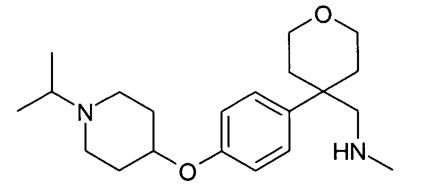

To a stirred solution of *tert*-butyl [4-(4-[(1-isopropylpiperidin-4-yl)oxy]phenyl)tetrahydro-2*H*-pyran-4-yl]methylcarbamate (532 mg, 1.23 mmol) in THF (3.5 ml) at 0°C was added drop-wise a solution of LiAlH₄ (1.0M solution in Et₂O, 3.69 ml, 3.69 mmol). Reaction stirred for 60 hours at ambient temperature before a further drop-wise addition of LiAlH₄ (1.0M solution in Et₂O, 0.6 ml, 0.6 mmol). Heated at 40°C until reaction complete. The reaction was then cooled to 0°C, water (0.2 ml) was added drop-wise followed by sodium hydroxide (2.0M, 0.2 ml) and water (0.6 ml). 1% methanol in dichloromethane (10ml) was added and the mixture filtered through a short pad of arbocel, eluting with 1% methanol in dichloromethane (25 ml). The organic washings were dried over Na₂SO₄ and concentrated *in vacuo* to give the title compound as a solid (399 mg, 94%).
¹H NMR (400MHz, CDCl₃) δ7.20 (d, 2H), 7.88 (d, 2H), 4.22-4.34 (m, 1H), 3.70-3.80 (m, 2H), 3.60-4.01 (d, 2H), 2.70-2.88 (m, 3H), 2.64 (s, 2H), 2.32-2.44 (m, 2H) 2.24 (s, 3H), 2.10-2.18 (M, 2H), 1.99-2.08 (m, 2H), 1.79-1.96 (m, 4H), 1.03 (d, 6H).

### Example 139: N-{[4-(4-[(1-isopropylpiperidin-4-yl)oxy]phenyl)tetrahydro-2H-pyran-4-yl]methyl}-N-methylacetamide

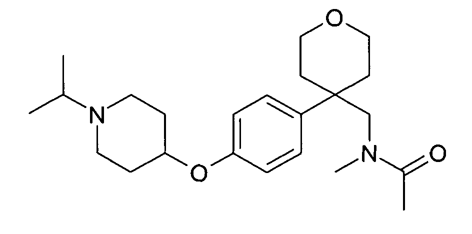

To a stirred solution of *N*-[(4-{4-[(1-isopropylpiperidin-4-yl)oxy]phenyl}tetrahydro-2*H*-pyran-4-yl)methyl]-*N*-methylamine (120 mg, 0.347 mmol) and triethylamine (121 µl, 0.867 mmol) in dichloromethane (1ml) at 0°C was added drop-wise acetic anhydride (36 µl, 0.382 mmol). Reaction allowed to warm up to ambient temperature until reaction complete. Water (1 ml) and dichloromethane (15 ml) added to the reaction, the extracted with saturated NaHCO₃ (2 x 10 ml). Organic washings were dried over Na₂SO₄ and concentrated *in vacuo* to give the title compound as an oil (113 mg, 83%).
¹H NMR (400MHz, CDCl₃) δ7.10-7.22 (2 doublets due to rotamers, 2H), 6.80 (d, 2H), 4.24-4.38 (m, 1H), 3.79-3.80 (m, 2H), 3.42-3.60 (m, 2H), 2.70-2.90 (m, 3H), 2.39-2.48 (m, 2H), 3.20-3.35 (singlet and doublet due to rotamers, 3H), 1.75-2.10 (m, 11 H), 1.10 (d, 6H).

### Example 140: N-{[4-(4-[(1-isopropylpiperidin-4-yl)oxy]phenyl)tetrahydro-2H-pyran-4-yl]methyl}-N-methylpropanamide

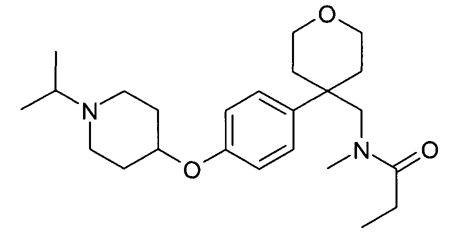

To a stirred solution of *N*-[(4-{4-[(1-isopropylpiperidin-4-yl)oxy]phenyl}tetrahydro-2*H*-pyran-4-yl)methyl]-*N*-methylamine (120 mg, 0.347 mmol) and triethylamine (121 µl, 0.867 mmol) in dichloromethane (1 ml) at 0°C was added drop-wise propionic anhydride (44 µl, 0.382 mmol). Reaction allowed to warm up to ambient temperature until reaction complete. Water (0.5 ml) and dichloromethane (15 ml) added to the reaction, the extracted with saturated NaHCO₃ (3 x 10 ml). Organic washings were dried over Na₂SO₄ and concentrated *in vacuo* to give the title compound as an oil (153 mg, 82%).
¹H NMR (400MHz, CD₃OD) δ7.20-7.32 (2 doublets due to rotamers, 2H), 7.95 (d, 2H), 3.95-4.05 (m, 1H), 3.76-3.85 (m, 2H), 3.40-3.60 (multiple peaks due to rotamers, 4H), 2.70-2.88 (multiple peaks due to rotamers, 4H), 2.42-2.58 (m, 2H), 2.40 (s, 2H), 2.20-2.38 (m, 2H), 1.52-2.58 (several multiplets, 8H), 1.10 (d, 6H), 0.70-1.08 (two triplets due to rotamers, 3H).

### Example 141: N-{[4-(4-[(1-isopropylpiperidin-4-yl)oxy]phenyl)tetrahydro-2H-pyran-4-yl]methyl}-acetamide

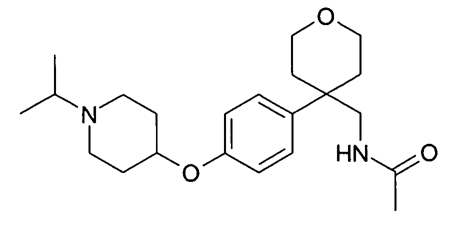

To a stirred solution of (4-{4-[(1-isopropylpiperidin-4-yl)oxy]phenyl}tetrahydro-2*H*-pyran-4-yl)methylamine (250 mg, 0.753 mmol) and triethylamine (200 µl. 1.88mmol) in dichloromethane (2ml) was added drop-wise acetic anhydride (78 µl, 0.828 mmol). Reaction left at ambient temperature until reaction complete. Dichloromethane (10 ml) added to the reaction, the extracted with saturated NaHCO₃ (3 x 10 ml). Organic washings were dried over Na₂SO₄ and concentrated *in vacuo* to give the title compound as an oil (203 mg, 72%).
¹H NMR (400MHz, CDCl₃) δ7.19 (d, 2H), 6.94 (d, 2H), 4.95-5.02 (broad, 1 H), 4.24-4.38 (m, 1H), 3.79-3.88 (m, 2H), 3.52-3.64 (m, 2H), 3.45 (d, 2H), 2.70-2.88 (m, 3H), 2.36-2.45 (m, 2H), 1.98-2.10 (m, 4H), 1.78-1.90 (m, 7H), 1.05 (d, 6H).

### Example 142: N-[(4-{4-[(1-isopropylpiperidin-4-yl)oxy]phenyl}tetrahydro-2H-pyran-4-yl)methyl]-N-ethylamine

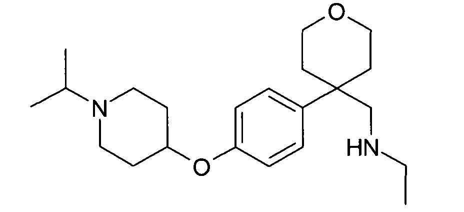

To a stirred solution of *N*-{[4-(4-[(1-isopropylpiperidin-4-yl)oxy]phenyl)tetrahydro-2*H*-pyran-4-yl]methyl}-acetamide (192 mg, 0.513 mmol) in THF (1.5 ml) at 0°C was added drop-wise a solution of LiAlH₄ (1.0M solution in Et₂O, 1.54 ml, 1.54 mmol). Reaction heated at 40°C for 24 hours, then increased to 50°C. A further drop-wise addition of LiAlH₄ (1.0M solution in Et₂O, 2 ml, 2 mmol) and further heating at 50°C for 24 hours was required for reaction to reach completion. The reaction was then cooled to 0°C, water (0.15 ml) was added drop-wise followed by sodium hydroxide (2.0M, 0.15 ml) and water (0.45 ml). 1% methanol in dichloromethane (15 ml) was added and the mixture filtered through a short pad of arbocel, eluting with 1% methanol in dichloromethane (25 ml). The organic washings were dried over Na₂SO₄ and concentrated *in vacuo* (185 mg crude, 82%). The compound was purified by column chromatography on silica gel, eluting with dichloromethane:methanol:ammonia, 98:2:0.2, to give the title compound as a white solid (9.9 mg, 4.4%).
¹H NMR (400MHz, CDCl₃) δ7.20 (d, 2H), 7.88 (d, 2H), 4.24-4.56 (m, 1H), 3.71-3.82 (m, 2H), 3.52-3.63 (m, 2H), 2.72-2.88 (m, 3H), 2.70 (s, 2H), 2.46-2.52 (quartet, 2H), 2.34-2.42 (m, 2H), 1.78-2.18 (several multiplets, 8H), 1.08 (d, 6H), 0.96 (t, 3H).

### Example 143: N-{[4-(4-[(1-isopropylpiperidin-4-yl)oxy]phenyl)tetrahydro-2H-pyran-4-yl]methyl}-N-ethylacetamide

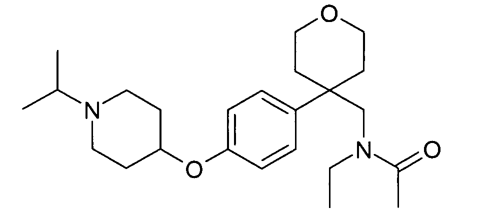

To a stirred solution of *N*-[(4-{4-[(1-isopropylpiperidin-4-yl)oxy]phenyl}tetrahydro-2H-pyran-4-yl)methyl]-*N*-ethylamine (102 mg, 0.283 mmol) and triethylamine (99 µl. 0.708 mmol) in dichloromethane (1 ml) was added drop-wise acetic anhydride (29 µl, 0.312 mmol). Reaction left at ambient temperature for 4 hours until reaction complete. Water (1 ml) and dichloromethane (10 ml) added to the reaction, the extracted with saturated NaHCO₃ (2 x 10 ml). Organic washings were dried over Na₂SO₄ and concentrated *in vacuo* to give the title compound as an oil (107mg, 94%).
¹H NMR (400MHz, CD₃OD) δ7.20-7.31 (multiplet due to rotamers, 2H), 6.92-7.00 (multiplet due to rotamers, 2H), 4.36-4.46 (m, 1H), 3.70-3.88 (m, 2H), 3.20-3.55 (several peaks due to rotamers, 4H), 2.78-2.90 (m, 3H), 2.60, (q, 2H), 2.25-2.52 (multiple peaks due to rotamers, 2H), 1.70-2.10 (multiple peaks, 11H), 1.10 (d, 6H), 0.80-1.00 (two triplets due to rotamers, 3H).

### Example 144: 1-{[4-(4-[(1-isopropylpiperidin-4-yl)oxylphenyl)tetrahydro-2H-pyran-4-yl]methyl}pyrrolidin-2-one

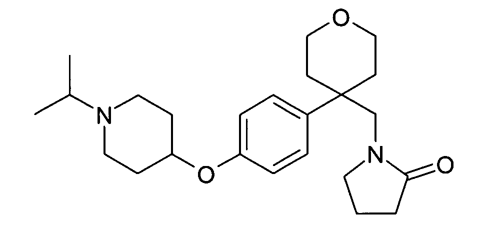

To a solution of (4-{4-[(1-isopropylpiperidin-4-yl)oxy]phenyl}tetrahydro-2*H*-pyran-4-yl)methylamine (100 mg, 0.301 mmol) in toluene (2 ml) was added ethyl-4-bromobutyrate (43 µl, 0.301 mmol), diisopropylethylamine (157 µl, 0.904 mmol) and potassium iodide (5 mg, 0.030 mmol). Reaction mixture heated at 80°C for 48 hours, then increased to reflux. Potassium carbonate (83 mg, 0.602 mmol) was added and reaction left at reflux for a further 24 hours until reaction complete. Reaction mixture was partitioned between ethyl acetate (20 ml) and saturated NaHCO₃ (10 ml). Organic washings were dried over Na₂SO₄ and concentrated *in vacuo* to give a brown oil (101 mg crude, 84%). The compound was purified by column chromatography on silica gel, eluting with dichloromethane:methanol:ammonia, 100:0:0 to 98:2:0.2, to give the title compound as an oil (7 mg, 6%).
¹H NMR (400MHz, CDCl₃) δ7.20 (d, 2H), 7.90 (d, 2H), 4.22-4.36 (m, 1H), 4.80-4.90 (m, 2H), 3.52-3.67 (m, 2H), 3.40 (s, 2H), 2.72-3.88 (several multiplets, 3H), 2.50 (t, 2H), 2.34-2.44 (m, 2H), 2.21-2.30 (t, 2H), 1.60-2.10 (several multiplets, 10H), 1.08 (d, 6H).

### Example 145: N-{[4-(4-[(1-isopropylpiperidin-4-yl)oxylphenyl)tetrahydro-2H-pyran-4-yl]methyl}pyrimidin-2-amine

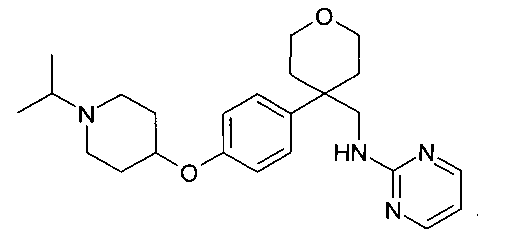

A mixture of (4-{4-[(1-isopropylpiperidin-4-yl)oxy]phenyl}tetrahydro-2*H*-pyran-4-yl)methylamine (150 mg, 0.422 mmol), 2-chloropyrimidine (52 mg, 0.422 mmol) and diisopropylethylamine (161 µl, 0.904 mmol) in N-methyl pyrrolidinone (0.5 ml) was heated at 150°C in the microwave for 900 seconds. The reaction mixture was partitioned between ethyl acetate (10 ml) and saturated NaHCO₃ (10 ml). The organic washings were dried over Na₂SO₄ and concentrated *in vacuo* to give an orange oil. The compound was purified by column chromatography on silica gel, eluting with dichloromethane:methanol:ammonia, 100:0:0 to 96:4:0.4, to give the title compound as a solid (87 mg, 47%).
¹H NMR (400MHz, CDCl₃) δ8.20 (d, 2H), 7.21 (d, 2H), 6.90 (d, 2H), 6.48 (t, 2H), 4.71 (m, 1 H), 4.20-4.35 (broad, 1 H), 3.80-3.90 (m, 2H), 3.55-3.70 (multiple peaks, 4H), 2.70-2.90 (m, 3H), 2.32-2.49 (m, 2H), 1.78-2.19 (several multiplets, 8H), 1.10 (d, 6H).

### Example 146: N-{[4-(4-(3-Pyrrolidin-1-ylpropoxy)phenyl)tetrahydro-2H-pyran-4-yl]methyl}pyrimidin-2-amine

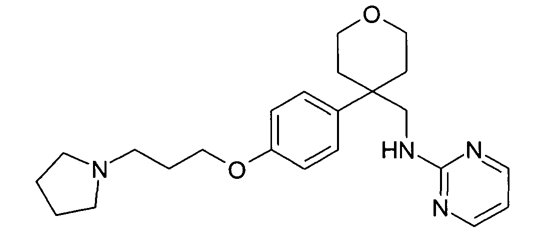

A mixture of {4-[4-(3-Pyrrolidin-1-ylpropoxy)phenyl]tetrahydropyran-4-yl} methylamine (100 mg, 0.314 mmol), 2-chloropyrimidine (50 mg, 0.346 mmol) and diisopropylethylamine (112 µl, 0.629 mmol) in N-methyl pyrrolidinone (0.5 ml) was heated at 180°C in the microwave for 600 seconds. The reaction mixture was partitioned between ethyl acetate (10 ml) and saturated NaHCO₃ (10 ml). The organic washings were dried over Na₂SO₄ and concentrated *in vacuo* to give an oil. The compound was purified by column chromatography on silica gel, eluting with dichloromethane:methanol:ammonia, 100:0:0 to 88:12:1.2. Compound still impure so purified further on the Fraction Lynx automated LC system to give the title compound as a solid (22 mg, 17%)
¹H NMR (400MHz, CDCl₃) δ8.30 (broad, 2H), 7.34 (d, 2H), 6.92 (d, 2H), 6.70 (t, 1H), 4.10 (t, 2H), 3.78-3.84 (m, 2H), 3.62-3.72 (multiple peaks, 4H), 3.44-3.55 (m, 2H), 3.40 (m, 2H), 3.04-3.18 (m, 2H), 1.85-2.25 (multiple peaks, 10H).

### Example 147: 2-{[4-(4-[(1-isopropylpiperidin-4-yl)oxylphenyl)tetrahydro-2H-pyran-4-yl]methyl}isothiazolidine 1,1-dioxide

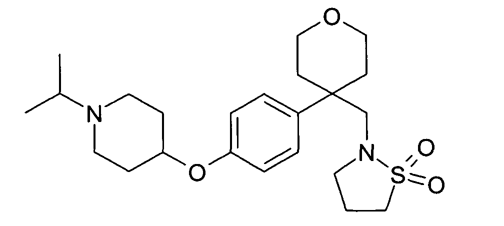

A solution of (4-{4-[(1-isopropylpiperidin-4-yl)oxy]phenyl}tetrahydro-2*H*-pyran-4-yl)methylamine (100mg, 0.30 mmol), 3-chloropropanesulphonyl chloride (51µl, 0.40 mmol) and diisopropylethylamine (52µl, 0.30 mmol) in dichloromethane (2ml) was stirred at room temperature for 18hrs. The reaction mixture was concentrated *in vacuo* and then partitioned between dichloromethane (10ml) and sodium bicarbonate solution (10ml). The aqueous layer was extracted with a further 10ml dichloromethane and the combined organics dried over sodium sulphate and concentrated *in vacuo* to give an oil. This was dissolved in tetrahydrofuran (2ml). Potassium tert-butoxide (65mg, 0.60 mmol) was added to the solution and the reaction mixture stirred at 40°C for 18hrs. This was then concentrated *in vacuo* and partitioned between diethyl ether (10ml) and water (10ml). The aqueous layer was extracted with a further 10ml diethyl ether and the combined organics dried over sodium sulphate and concentrated *in vacuo* to give a pale yellow oil. The compound was purified by column chromatography on Biotage® silica gel, eluting with dichloromethane:methanol:ammonia, 96:4:0.4 to give the title compound as a pale yellow oil (19mg, 15%).
¹H NMR (400 MHz, CDCl₃) δ7.20 (d, 2H), 6.90 (d, 2H), 4.26 (m, 2H), 3.80 (m, 2H), 3.58 (m, 2H), 3.15 (s, 2H), 2.99 (t, 2H), 2.80 (m, 2H), 2.75(m, 2H), 2.40 (t, 2H), 2.38 (m, 2H), 2.16 (m, 2H), 2.02 (m, 4H), 1.90 (m, 2H) 1.81 (m, 2H), 1.03 (d, 6H); HRMS (ESI⁺) 437.2457 (M+H)⁺.

### H₃ Cell Based Functional Assay

Compounds were evaluated using a cell based functional assay measuring cAMP through β-lactamase reporter gene activity. A stable cell line was generated from HEK-293 cells expressing a CRE β-lactamase reporter gene and transfected with human histamine H₃ receptor cDNA. Cells were seeded at a density of 500,000 cells/ml, and grown overnight in MEM (Invitrogen) supplemented with 1% dialysed FBS (Sigma), 2mM glutamine (Sigma), 1mM sodium pyruvate (Sigma), 0.1mM non essential amino acids (Invitrogen) and 25mM HEPES (Sigma) in poly D lysine coated 384 well plates (BD Biosciences). H₃ receptor agonist imetit (Tocris) dose dependently inhibited 10µM forskolin (Calbiochem) stimulated synthesis of cAMP measured after 4.5hours by β-lactamase cleavage of CCF4-AM dye (Invitrogen). For IC₅₀ determination, test compounds were prepared in PBS (Sigma) and DMSO (Sigma) at a dose response of 5x10⁻¹⁰ to 5x10⁻⁵M with a final DMSO concentration in the assay of 0.5%. Cells were incubated for 15 minutes plus/minus compound and their ability to permit 10µM forskolin-stimulated cAMP synthesis in the presence of 1nM imetit was measured as described above. Functional Kᵢ values were calculated from the IC₅₀ of compounds tested as antagonists based on an experimentally determined imetit EC₅₀ (represented in the equation as K_{d}) of 350pM, and an imetit concentration [L] of 1nM, according to the Cheng-Prussoff equation where Kᵢ = (IC₅₀)/(1+([L]/K_{d})).

All the examples were tested in the assay described above and found to have a Kᵢ value of less than 5 µM. Most of the examples have a Kᵢ value of less than 100 nM.

## Claims

1. A compound of the formula (1): 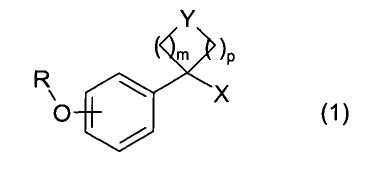 and pharmaceutically acceptable salts thereof wherein :
the substituent of formula -O-R is in the meta or para position of the phenyl group;
X is selected from -CN, -CH₂OH, -CH₂-O-(C₁-C₄)alkyl, -C(O)OH, -C(O)O(C₁-C₄)alkyl, -CH₂-NR¹R², -C(O)NR³R⁴ and het¹, het¹ being optionally substituted by one or two substituents independently selected from halo, cyano, (C₁-C₄)alkyl and (C₁-C₄)alkoxy;
wherein
R¹ ishydrogen or (C₁-C₄)alkyl;
R² is selected from the group consisting of :
hydrogen,
(C₁-C₄)alkyl optionally substituted by one or two substituents independently selected from (C₃-C₆)cycloalkyl, hydroxy, -S-(C₁-C₄)alkyl, -O-(C₁-C₄)alkyl, -SO₂-(C₁-C₄)alkyl, halo and phenyl, said phenyl being optionally substituted by one or two substituents independently selected from halo, cyano, (C₁-C₄)alkyl and (C₁-C₄)alkoxy,
(C₃-C₆)cycloalkyl,
het², optionally substituted by one or two substituents independently selected from halo, cyano, (C₁-C₄)alkyl and (C₁-C₄)alkoxy,
-SO₂-R⁵ wherein R⁵ is selected from the group consisting of (C₁-C₄)alkyl, amino, (C₁-C₄)alkylamino, [(C₁-C₄)alkyl]₂amino, phenyl and -(C₁-C₄)alkyl-phenyl, said phenyl being optionally substituted by one or two substituents independently selected from halo, cyano, (C₁-C₄)alkyl and (C₁-C₄)alkoxy, and
-C(O)-R⁶ wherein R⁶ is selected from the group consisting of (C₁-C₄)alkyl, amino, (C₁-C₄)alkylamino, [(C₁-C₄)alkyl]₂amino, phenyl, -(C₁-C₄)alkyl-phenyl, said phenyl being optionaly substituted by one or two substituents independently selected from halo, cyano, (C₁-C₄)alkyl and (C₁-C₄)alkoxy;
or R¹ and R² form together with the N atom to which they are attached a 4-, 5- or 6-membered saturated heterocycle wherein one C atom may be replaced by N, O, S, SO or SO₂ and wherein said saturated heterocycle is optionally substituted by one or two groups independently selected from hydroxy, halo, =O, (C₁-C₄)alkyl, (C₁-C₄)alkoxy, hydroxy(C₁-C₄)alkyl, (C₁-C₄)alkoxy(C₁-C₄)alkyl, -C(O)NH₂, C(O)O(C₁-C₄)alkyl and pyrrolidinone;
R³ and R⁴ are each independently selected from hydrogen and (C₁-C₄)alkyl or R³ and R⁴ form together with the N atom to which they are attached a 4-, 5- or 6- membered saturated heterocycle wherein one C atom may be replaced by N or O;
Y is selected from CH₂, CH(OH), O and C=O;
m and p are both integers which are independently 1, 2 or 3, with the condition that m+p is equal to or less than 4 so that the ring formed by : 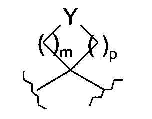 is a 4-, 5- or 6-membered ring;
and R is either a group of formula : 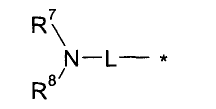 wherein * represents the attachment point to the O atom, L is a (C₂-C₆)alkylene and R⁷ and R⁸ are each independently selected from hydrogen, (C₁-C₆)alkyl, hydroxy(C₁-C₆)alkyl or R⁷ and R⁸ form together with the N atom to which they are attached a 4-, 5-, 6- or 7-membered saturated heterocycle wherein one C atom is optionally replaced by N, O, S, SO or SO₂ and wherein said saturated heterocycle is optionally substituted by one or two groups independently selected from (C₁-C₄)alkyl, (C₁-C₄)alkoxy, (C₁-C₄)alkoxy(C₁-C₄)alkyl, hydroxy(C₁-C₄)alkyl, hydroxy, C(O)O(C₁-C₄)alkyl, -C(O)-(C₁-C₄)alkyl-NH₂, -C(O)NH₂, halo, amino, (C₁-C₄)alkylamino and [(C₁-C₄)alkyl]₂amino,
or a group of formula: 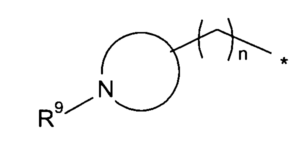 wherein * represents the attachment point to the O atom, the N-containing ring is a 4- to 7-membered saturated heterocycle, n is an integer equal to 0, 1 or 2, and R⁹ represents a substituent selected from hydrogen, (C₁-C₄)alkyl, hydroxy(C₁-C₆ alkyl) and (C₃-C₆)cycloalkyl;
het¹ is selected from monocyclic or bicyclic heteroaromatic groups having 5 to 10 ring members, which contain 1, 2, 3 or 4 heteroatom(s) selected from nitrogen, oxygen and sulphur and het² is selected from monocyclic or bicyclic heteroaromatic groups having 5 to 10 ring members, which contain 1, 2, 3 or 4 heteroatom(s) selected from nitrogen, oxygen and sulphur.

2. A compound of formula (1) as defined in claim 1, wherein X is selected from -CN, -CH₂OH, -C(O)OH, -CH₂-NR¹R², -C(O)NR³R⁴ and het¹, said het¹ being optionally substituted by one or two substituents independently selected from halo, cyano, (C₁-C₄)alkyl and (C₁-C₄)alkoxy, wherein R¹, R², R³, R⁴ and het¹ are as previously defined in claim 1.

3. A compound of formula (1) as defined in claim 2, wherein X is selected from -CN, -CH₂-NR¹R², -CONR³R⁴ and het¹, het¹ being optionally substituted by one or two substituents independently selected from halo, cyano, (C₁-C₄)alkyl and (C₁-C₄)alkoxy, wherein R¹, R², R³, R⁴ and het¹ are as previously defined in claim 1.

4. A compound of formula (1) as defined in any one of the preceding claims, wherein het¹ is selected from 5 or 6 membered monocyclic heteroaromatic groups containing 1 to 2 nitrogen atoms or 1 nitrogen atom and 1 oxygen atom or 1 nitrogen atom and 1 sulphur atom, optionally substituted by one or two substituents independently selected from halo, cyano, (C₁-C₄)alkyl and (C₁-C₄)alkoxy.

5. A compound of formula (1) as defined in claim 4, wherein het¹ is substituted by (C₁-C₄)alkyl.

6. A compound of formula (1) as defined in any one of claims 1 to 3, wherein R¹ is methyl or ethyl.

7. A compound of formula (1) as defined in any one of claims 1 to 3 and 6, wherein R² is selected from the group consisting of
hydrogen,
(C₁-C₄)alkyl optionally substituted by one or two substituents independently selected from -S-(C₁-C₄)alkyl, -O-(C₁-C₄)alkyl, -SO₂-(C₁-C₄)alkyl, and phenyl, said phenyl being optionally substituted by one or two substituents independently selected from halo, cyano, (C₁-C₄)alkyl and (C₁-C₄)alkoxy,
(C₃-C₆)cycloalkyl,
het² wherein het² is defined as in claim 1,
-SO₂-R⁵ wherein R⁵ is selected from the group consisting of (C₁-C₄)alkyl, [(C₁-C₄)alkyl]₂amino, phenyl, and -(C₁-C₄)alkyl-phenyl, wherein said phenyl is optionally substituted by 1 substituent independently selected from halo and cyano and
-C(O)-R⁶ wherein R⁶ is selected from the group consisting of (C₁-C₄)alkyl, ((C₁-C₄)alkyl]₂amino, amino, and -(C₁-C₄)alkyl-phenyl, said phenyl being optionally substituted by one or two substituents independently selected from halo, cyano, (C₁-C₄)alkyl and (C₁-C₄)alkoxy.

8. A compound of formula (1) as defined in claim 7, wherein R² is selected from the group consisting of
(C₁-C₃)alkyl optionally substituted by -O-(C₁-C₃)alkyl, preferably methoxy, (C₃-C₅)cycloalkyl,
het², wherein het² is selected from the group consisting of 5 or 6 membered monocyclic heteroaromatic groups containing 1 to 2 nitrogen atoms or 1 nitrogen atom and 1 oxygen atom or 1 nitrogen atom and 1 sulphur atom, optionally substituted by one or two substituents independently selected from halo, cyano, (C₁-C₄)alkyl and (C₁-C₄)alkoxy, preferably (C₁-C₄)alkyl,
SO₂-R⁵ wherein R⁵ is preferably (C₁-C₄)alkyl and
C(O)-R⁶ wherein R⁶ is preferably (C₁-C₄)alkyl.

9. A compound of formula (1) as defined in claim 8, wherein het² is selected from the group consisting of 5 or 6 membered monocyclic heteroaromatic ring systems containing 1 or 2 nitrogen atoms.

10. A compound of formula (1) as defined in any one of claims 1 to 3, wherein R¹ and R² form together with the N atom to which they are attached a 5- or 6- membered saturated heterocycle wherein a C atom may be replaced by N, O, S, SO or SO₂ and wherein said saturated heterocycle is optionally substituted by one or two groups independently selected from hydroxy, halo, =O, (C₁-C₄)alkyl, (C₁-C₄)alkoxy, hydroxy(C₁-C₄)alkyl, (C₁-C₄)alkoxy(C₁-C₄)alkyl, -C(O)NH₂, C(O)O(C₁-C₄)alkyl and pyrrolidinone.

11. A compound of formula (1) as defined in any one of claims 1 to 3, wherein R³ and R⁴ are each independently selected from hydrogen and (C₁-C₄)alkyl or R³ and R⁴ form together with the N atom to which they are attached a 5-membered saturated heterocycle.

12. A compound of formula (1) as defined in any one of the preceding claims, wherein Y is selected from CH₂, CH(OH), O and C=O.

13. A compound of formula (1) as defined in claim 12, wherein Y is O.

14. A compound of formula (1) as defined in any one of the preceding claims, wherein m is equal to 1 or 2 and p is equal to 2.

15. A compound of formula (1) as defined in claim 14, wherein m and p are both 2.

16. A compound of formula (1) as defined in any one of the preceding claims, wherein
R is a group of formula : 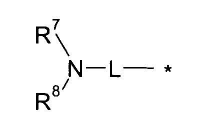 wherein * represents the attachment point to the O atom, L is a (C₂-C₅)alkylene and R⁷ and R⁸ are each independently selected from hydrogen, (C₁-C₆)alkyl, hydroxy(C₁-C₆)alkyl or R⁷ and R⁸ form together with the N atom to which they are attached a 5-, 6- or 7-membered saturated heterocycle wherein one C atom is optionally replaced by N, O, S, SO or SO₂ and wherein said saturated heterocycle is optionally substituted by one or two groups independently selected from (C₁-C₄)alkyl, (C₁-C₄)alkoxy, (C₁-C₄)alkoxy(C₁-C₄)alkyl, hydroxy(C₁-C₄)alkyl, hydroxy, C(O)O(C₁-C₄)alkyl, -C(O)-(C₁-C₄)alkyl-NH₂, -C(O)NH₂ and halo.

17. A compound of formula (1) as defined in claim 16, wherein R⁷ and R⁸ form together with the N atom to which they are attached a 5 or 6 membered saturated heterocycle, optionally substituted by one or two (C₁-C₄)alkyl.

18. A compound of formula (1) as defined in claim 17, wherein the 5 or 6 membered saturated heterocycle is substituted by one or two methyl.

19. A compound of formula (1) as defined in any one of claims 1 to 15, wherein
R is a group of formula : 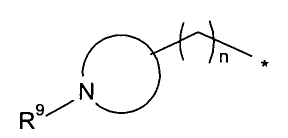 wherein * represents the attachment point to the O atom, the N-containing ring is a 6-membered saturated heterocycle, n is an integer equal to 0, and R⁹ represents a substituent selected from hydrogen, (C₁-C₄)alkyl and (C₃-C₆)cycloalkyl.

20. Compound selected from :
{4-[4-(1-Isopropylpiperidin-4-yloxy)phenyl]tetrahydropyran-4-ylmethyl}dimethylamine
Dimethyl-{4-[4-(4-pyrrolidin-1-ylbutoxy)phenyl]tetrahydro-pyran-4-ylmethyl}amine
{4-[4-(3-Pyrrolidin-1-ylpropoxy)phenyl]tetrahydropyran-4-yl} methylamine
Dimethyl-{4-[4-(3-pyrrolidin-1-ylpropoxy)phenyl]-tetrahydropyran-4-ylmethyl}amine
{4-[4-(3-Piperidin-1-ylpropoxy)phenyl]tetrahydropyran-4-yl} methylamine
Dimethyl-{4-[4-(3-piperidin-1-ylpropoxy)phenyl]tetra-hydropyran-4-ylmethyl}amine
1-Methyl-4-{4-[4-(3-pyrrolidin-1-yl-propoxy)-phenyl]-tetrahydro-pyran-4-ylmethyl}piperazine
(R)-2-Methoxymethyl-1-{4-[4-(3-pyrrolidin-1-yl-propoxy)-phenyl]-tetrahydro-pyran-4-ylmethyl}-pyrrolidine
(S)-2-Methoxymethyl-1-{4-[4-(3-pyrrolidin-1-ylpropoxy)-phenyl]tetrahydropyran-4-ylmethyl}pyrrolidine
1-{4-[4-(3-Pyrrolidin-1-ylpropoxy)phenyl]tetrahydropyran-4-ylmethyl}piperidine
methyl-{4-[4-(3-pyrrolidin-1-ylpropoxy)phenyl]-tetrahydropyran-4-ylmethyl}amine
isopropyl-methyl-{4-[4-(3-pyrrolidin-1-ylpropoxy)phenyl]-tetrahydropyran-4-ylmethyl}amine
1-{4-[4-(3-Pyrrolidin-1-ylpropoxy)phenyl]tetrahydropyran-4-ylmethyl}pyrrolidine
4-{4-[4-(3-Pyrrolidin-1-ylpropoxy)phenyl]tetrahydropyran-4-ylmethyl}-morpholine
(2-methoxyethyl)-methyl-{4-[4-(3-pyrrolidin-1-ylpropoxy)-phenyl]tetrahydropyran-4-ylmethyl}amine
4-[4-(1-Isopropylpiperidin-4-yloxy)phenyl]tetrahydropyran-4-carbonitrile
4-[4-(3-Pyrrolidin-1-ylpropoxy)phenyl]tetrahydro-2H-pyran-4-carboxylic acid amide
*N*-methyl-1-{1-[4-(3-pyrrolidin-1-ylpropoxy)phenyl]-cyclohexyl}methanamine
*N*-ethyl-*N*-({4-[4-(3-pyrrolidin-1-ylpropoxy)phenyl]tetrahydro-2*H*-pyran-4-yl}methyl)ethanamine
*N*-({4-[4-(3-pyrrolidin-1-ylpropoxy)phenyl]tetrahydro-2*H*-pyran-4-yl}methyl)ethanamine
*N*-methyl-*N*-({4-[4-(3-pyrrolidin-1-ylpropoxy)phenyl]-tetrahydro-2*H*-pyran-4-yl}methyl)ethanamine
4-{4-[(1-cyclobutyl-4-piperidinyl)oxy]phenyl}tetrahydro-2*H*-pyran-4-carbonitrile
4-{4-[(1-isopropylpiperidin-4-yl)oxy]phenyl}tetrahydro-2*H*-pyran-4-carboxamide
4-{4-[(1-isopropylpiperidin-4-yl)oxy]phenyl]-*N*,*N*-dimethyltetrahydro-2*H*-pyran-4-carboxamide
4-{4-[(1-isopropylpiperidin-4-yl)oxy]phenyl]-*N*,*N*-diethyltetra-hydro-2*H*-pyran-4-carboxamide
4-{4-[(1-isopropylpiperidin-4-yl)oxy]phenyl)tetrahydro-2*H*-pyran-4-yl]carbonyl}pyrrolidine
4-methyl-2-[4-(4-(3-pyrrolidin-1-ylpropoxy)phenyl)tetrahydro-2*H*-pyran-4-yl]-1,3-thiazole
2-[4-(4-(3-pyrrolidin-1-ylpropoxy)phenyl)tetrahydro-2*H*-pyran-4-yl]-1,3-thiazole
*N*-[(4-{4-[(1-isopropylpiperidin-4-yl)oxy]phenyl}tetrahydro-2*H*-pyran-4-yl)methyl]-N-methylamine
*N*-[(4-{4-[(1-isopropylpiperidin-4-yl)oxy]phenyl}tetrahydro-2*H*-pyran-4-yl)methyl]-N-ethylamine
*N*-{[4-(4-[(1-isopropylpiperidin-4-yl)oxy]phenyl)tetrahydro-2*H*-pyran-4-yl]methyl}pyrimidin-2-amine and
*N*-{[4-(4-(3-Pyrrolidin-1-ylpropoxy)phenyl)tetrahydro-2*H*-pyran-4-yl]methyl}pyrimidin-2-amine.

21. A pharmaceutical composition including a compound of the formula (1) or a pharmaceutically acceptable salt or solvate thereof, as defined in any one of the preceding claims, together with a pharmaceutically acceptable excipient.

22. A compound of the formula (1) as defined in any one of claims 1 to 20 or a pharmaceutically acceptable salt or solvate thereof, for use as a medicament.

23. The use of a compound of the formula (1) as defined in any one of claims 1 to 20 or of a pharmaceutically acceptable salt or solvate thereof, for the manufacture of a medicament to treat a disease for which a H₃ ligand is indicated.

24. The use of a compound of the formula (1) according to claim 23, for the manufacture of a medicament for the treatment of sleep disorders, migraine, dyskinesia, stress-induced anxiety, psychotic disorders, epilepsy, Cognition deficiency diseases such as Alzheimer's disease or mild cognitive impairment, depression, mood disorders, schizophrenia, anxiety disorders, attention-deficit hyperactivity disorder (ADHD), psychotic disorders, obesity, dizziness, vertigo, epilepsy, motion sickness, inflammatory diseases, adult respiratory distress syndrome, acute respiratory distress syndrome, bronchitis, chronic bronchitis, chronic obstructive pulmonary disease, cystic fibrosis, asthma, emphysema, rhinitis, chronic sinusitis, allergy, allergy-induced airway responses, allergic rhinitis, viral rhinitis, non-allergic rhinitis, perennial and seasonal rhinitis, nasal congestion and allergic congestion.

25. The use according to claim 24, for the manufacture of a medicament for the treatment of allergy, allergy-induced airway responses, allergic rhinitis, viral rhinitis, non-allergic rhinitis, perennial and seasonal rhinitis, nasal congestion and allergic congestion.

26. A method of treatment of a mammal, including a human being, suffering from a disease for which a H₃ ligand is indicated, comprising administering to said mammal an effective amount of a compound of the formula (1) as defined in any one of claims 1 to 20 or a pharmaceutically acceptable salt, solvate or composition thereof.

27. A combination of a compound of formula (1) as defined in any one of claims 1 to 20 and another pharmacologically active agent.

28. A combination as claimed in claim 27, wherein the other pharmalogically active agent is an histamine H₁ receptor antagonist.
